# EUROPEAN PATENT APPLICATION

(11) **EP 4 635 939 A1**
(43) Date of publication of application: **22.10.2025**
(21) Application number: 23903729.4
(22) Date of filing: 25.10.2023
(51) Int. Cl.: C07C 235/12, C07D 207/08, C07D 211/14, C07D 295/108, C07D 231/12, C07D 213/46, C07D 339/02, C07D 413/12, A61K 9/51, A61K 47/18

(54) **NOVEL LIPID COMPOUND AND LIPID NANOPARTICLE COMPOSITION COMPRISING SAME**

(30) Priority: 12.12.2022 KR 20220172496; 13.10.2023 KR 20230136909
(71) Applicant: Korea Institute of Science and Technology, Seoul 02792 (KR); The Catholic University Of Korea Industry-Academic Cooperation Foundation, Seocho-gu Seoul 06591 (KR)
(72) Inventor: BANG, Eun-Kyoung, Seoul 02792 (KR); KEUM, Gyo Chang, Seoul 02792 (KR); NAM, Jae-Hwan, Bucheon-si Gyeonggi-do 14779 (KR); BAE, Seohyeon, Gimhae-si Gyeongsangnam-do 50898 (KR); FAISAL, Muhammad, Seoul 02792 (KR); KWON, Sung Pil, Seoul 02792 (KR); YOO, Soyeon, Seoul 02792 (KR)
(74) Representative: Mewburn Ellis LLP
(86) International application number: PCT/KR2023/016630
(87) International publication number: WO 2024/128525

(57) **Abstract**

The present invention relates to a novel lipid compound and a lipid nanoparticle composition comprising same. More particularly, the lipid nanoparticle composition comprises ionized lipids, helper lipids, PEG-lipids, and additives, and comprises: a novel lipid compound based on a bio-friendly vitamin; and helper lipids containing neutral lipids, and thus can alleviate changes and side effects from a delivery mechanism, and improve protein expression efficiency.

## Description

### Technical Field

The present disclosure relates to a novel lipid compound and a lipid nanoparticle composition including the same.

### Background Art

Originated more than 40 years ago to support production of deficient proteins through injection of plasmid DNA into the human body, various types of nucleic acid-based medicines such as antigen, decoy, antisense, siRNA, and miRNA that inhibit transcription and translation of genes have been reported afterwards. Nucleic acid-based medicines have attracted attention as personalized drugs through complementary binding with specific sequences of DNA or RNA by targeting DNA or RNA rather than proteins. Nucleic acid-based medicines are applied not only as therapeutic agents but also as prophylactic agents to defend against diseases by injecting genes capable of expressing antigens against specific diseases. Gene-based vaccines are divided into DNA vaccines, RNA vaccines, and viral vector vaccines, wherein the RNA vaccines, in a form in which mRNA encoding an antigen is injected into the human body to express antigens in the human body and cause antibody formation thereagainst, became popular as an effective countermeasure against COVID-19, which broke out in 2019, since there is no potential risk of infection that viral vector-based vaccines have or genetic mutations shown in DNA vaccines while ensuring rapid development.

However, nucleic acid-based medicines are easily degraded by nucleases in the human body and are negatively charged macromolecules that they cannot be easily delivered intracellularly, such that a method of stably, efficiently delivering the nucleic acid-based medicines to a desired site is required. Delivery techniques based on various materials such as lipids, polymers, dendrimers, and inorganic metal materials have been reported as delivery systems for nucleic acids, wherein lipid nanoparticles are applied to patisiran ONPATTRO^{®}, the first FDA-approved new siRNA drug in 2018 as well as mRNA vaccines against COVID-19 approved in 2020 for emergency use. Currently, lipid nanoparticles which are in general use are in a form in which four components including ionized lipid, phospholipid (helper lipid), cholesterol (structure-retaining lipid), and PEG-lipid are mixed in a constant ratio.

As such, with commercialization of new siRNA drugs and mRNA vaccines, various issues are rising on lipid nanoparticle-based delivery, concerning the targeting, the securing of easiness in storage and distribution, mitigation of side effects, cost reduction, and response to breakthrough infection, aside from enhancement of stability and cell permeability, which are the challenges in the field of delivery for traditional nucleic acid drugs. Therefore, development of lipid nanoparticles capable of solving new challenges is required in the nucleic acid drug market.

### Disclosure of the Invention

### Technical Goals

An object of the present disclosure is to provide novel vitamin-based ionized lipid and helper lipid compounds, and a lipid nanoparticle composition which includes the same and is biocompatibility and capable of improving the protein expression efficiency and persistence. Technical Solutions

In order to achieve the above object, the present disclosure provides a compound selected from a compound represented by the following Chemical Formula 1, a stereoisomer thereof, a racemate thereof, or a pharmaceutically acceptable salt thereof: 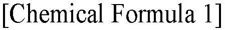 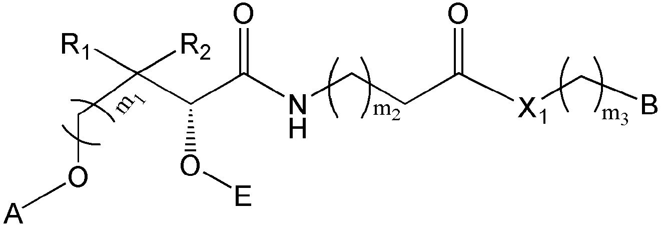
wherein, in the Chemical Formula 1, R₁ and R₂ are the same or different and each is hydrogen or a saturated hydrocarbon with 1 to 2 carbon atoms, X₁ is O, NH, or S, m₁, m₂, and m₃ are the same or different and each is an integer of 1 to 3, and A is hydrogen or a compound represented by the following Chemical Formula 1-1, 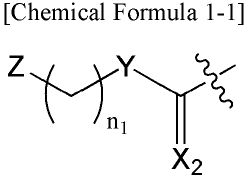
wherein, in the Chemical Formula 1-1, Z is NR¹R²; a heterocyclic or aromatic compound with 3 to 8 carbon atoms; or linear or branched, saturated or unsaturated hydrocarbon with 6 to 22 carbon atoms, the R¹ and R² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and the hydrocarbon includes or does not include ester, ether, amide, carbamate, carbonate, or disulfide bonds, X₂ is O or S, Y is CH₂, NH, or O, n₁ is an integer of 0 to 3, and
B is a compound represented by the following Chemical Formula 1-2 or NR¹'R²', wherein the R¹' and R²' are the same or different, and each is linear or branched, saturated or unsaturated hydrocarbon with 1 to 6 carbon atoms, wherein the hydrocarbon includes or does not include ester, ether, amide, carbamate, carbonate, or disulfide bonds, and R¹' and R²' are connected to each other to form a heterocyclic or aromatic ring with 3 to 8 carbon atoms, 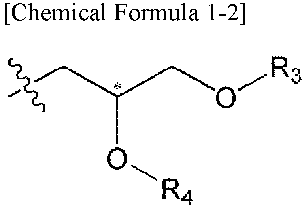
wherein, in the Chemical Formula 1-2, R₃ and R₄ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and E is hydrogen or a compound represented by the following Chemical Formula 1-3, 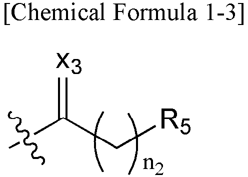
wherein, in the Chemical Formula 1-3, X₃ is O or S, n₂ is an integer of 0 to 3, and R₅ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with thiolane or dithiolane.

The present disclosure provides a lipid nanoparticle composition comprising the compounds.

The present disclosure provides a lipid nanoparticle composition comprising one or more compounds selected from a compound represented by the Chemical Formula 1, a stereoisomer thereof, a racemate thereof, or a pharmaceutically acceptable salt thereof.

The present disclosure provides a composition for drug delivery, comprising the lipid nanoparticle composition described above; and a therapeutic or prophylactic agent.

In addition, the present disclosure provides an immune boosting composition comprising the lipid nanoparticle composition.

### Advantageous Effects

According to the present disclosure, it is possible to form lipid nanoparticles that stably express proteins by including biofriendly vitamin-based novel lipid compounds, and it is expected to solve a problem concerning toxicity of lipid nanoparticles resulting from ionized lipids in most cases.

### Brief Description of Drawings

FIG. 1 shows results of analyzing an amount of R/L expressed 6 hours and 24 hours after injecting lipid nanoparticles (LNP 127, 130) of a novel helper lipid composition including Renilla Luciferase (R/L) mRNA and control lipid nanoparticles (LNP 98, 126) into the ears of mice (i.d.).
FIG. 2 shows results of analyzing an amount of R/L expressed 6 hours and 24 hours after injecting lipid nanoparticles (LNP 131, 132, 133) of a novel helper lipid composition including Renilla Luciferase (R/L) mRNA and control lipid nanoparticles (LNP 128) into the ears of mice (i.d.).
FIG. 3 shows results of analyzing an amount of R/L expressed 6 hours and 24 hours after injecting lipid nanoparticles (LNP 136, 138, 139) of a novel helper lipid composition including Renilla Luciferase (R/L) mRNA and control lipid nanoparticles (LNP 98, 128) into the ears of mice (i.d.).
FIG. 4 shows results of analyzing an amount of R/L expressed 6 hours and 24 hours after injecting lipid nanoparticles (LNP 109, 111, 114) of a novel cholesterol replaced composition including Renilla Luciferase (R/L) mRNA and control lipid nanoparticles (LNP 98) into the ears of mice (i.d.).
FIG. 5 shows results of analyzing an amount of R/L expressed 6 hours and 24 hours after injecting lipid nanoparticles (LNP 110, 115, 117, 118) of a novel cholesterol replaced composition including Renilla Luciferase (R/L) mRNA and control lipid nanoparticles (LNP 98) into the ears of mice (i.d.).
FIG. 6 shows results of analyzing an amount of R/L expressed 6 hours and 24 hours after injecting lipid nanoparticles (LNP 119, 123) of a novel cholesterol replaced composition including Renilla Luciferase (R/L) mRNA and control lipid nanoparticles (LNP 98) into the ears of mice (i.d.).
FIG. 7 shows results of analyzing an amount of R/L expressed 6 hours and 24 hours after injecting lipid nanoparticles (LNP 168) of a novel cholesterol replaced composition including Renilla Luciferase (R/L) mRNA and control lipid nanoparticles (LNP 98) into the ears of mice (i.d.).
FIG. 8 shows results of analyzing an amount of R/L expressed 6 hours and 24 hours after injecting lipid nanoparticles (LNP 200) of a novel cholesterol replaced composition including Renilla Luciferase (R/L) mRNA and control lipid nanoparticles (LNP 126) into the ears of mice (i.d.).
FIG. 9 shows results of analyzing an amount of R/L expressed 6 hours and 24 hours after injecting lipid nanoparticles (LNP 146, 147) of a novel ionized lipid composition including Renilla Luciferase (R/L) mRNA and control lipid nanoparticles (LNP 98) into the ears of mice (i.d.).
FIG. 10 shows results of analyzing an amount of R/L expressed 6 hours and 24 hours after injecting lipid nanoparticles (LNP 152, 153) of a novel ionized lipid composition including Renilla Luciferase (R/L) mRNA and control lipid nanoparticles (LNP 98, 128) into the ears of mice (i.d.).
FIG. 11 shows results of analyzing an amount of R/L expressed 6 hours and 24 hours after injecting lipid nanoparticles (LNP 159, 160) of a novel ionized lipid composition including Renilla Luciferase (R/L) mRNA and control lipid nanoparticles (LNP 98, 128) into the ears of mice (i.d.).
FIG. 12 shows results of analyzing an amount of R/L expressed 6 hours and 24 hours after injecting lipid nanoparticles (LNP 166, 167) of a novel ionized lipid composition including Renilla Luciferase (R/L) mRNA and control lipid nanoparticles (LNP 98, 128) into the ears of mice (i.d.).
FIG. 13 shows results of analyzing an amount of R/L expressed 6 hours and 24 hours after injecting lipid nanoparticles (LNP 178, 179, 181, 182) of a novel ionized lipid composition including Renilla Luciferase (R/L) mRNA and control lipid nanoparticles (LNP 138, 139) into the ears of mice (i.d.).
FIG. 14 shows results of analyzing an amount of R/L expressed 6 hours and 24 hours after injecting lipid nanoparticles (LNP 185, 187) of a novel ionized lipid composition including Renilla Luciferase (R/L) mRNA and control lipid nanoparticles (LNP 126) into the ears of mice (i.d.).
FIG. 15 shows results of analyzing an amount of R/L expressed 6 hours and 24 hours after injecting lipid nanoparticles (LNP 192, 193) of a novel ionized lipid composition including Renilla Luciferase (R/L) mRNA and control lipid nanoparticles (LNP 126, 128) into the ears of mice (i.d.).
FIG. 16 shows results of analyzing an amount of R/L expressed 6 hours and 24 hours after injecting lipid nanoparticles (LNP 205, 208, 209, 210) of a novel ionized lipid composition including Renilla Luciferase (R/L) mRNA and control lipid nanoparticles (LNP 187) into the ears of mice (i.d.).
FIG. 17 shows results of analyzing an amount of R/L expressed 6 hours and 24 hours after injecting lipid nanoparticles (LNP 211, 212, 213, 214, 216, 217) of a novel ionized lipid composition including Renilla Luciferase (R/L) mRNA and control lipid nanoparticles (LNP 126, 166) into the ears of mice (i.d.).
FIG. 18 shows results of analyzing an amount of R/L expressed 6 hours and 24 hours after injecting lipid nanoparticles (LNP 224, 225, 226) of a novel ionized lipid composition including Renilla Luciferase (R/L) mRNA and control lipid nanoparticles (LNP 126) into the ears of mice (i.d.).
FIG. 19 shows results of analyzing an amount of R/L expressed 6 hours and 24 hours after injecting lipid nanoparticles (LNP 227, 228, 229, 230, 231, 232) of a novel ionized lipid composition including Renilla Luciferase (R/L) mRNA and control lipid nanoparticles (LNP 126) into the ears of mice (i.d.).
FIG. 20 shows results of analyzing an amount of R/L expressed 6 hours and 24 hours after injecting lipid nanoparticles (LNP 256, 257) of a novel ionized lipid composition including Renilla Luciferase (R/L) mRNA and control lipid nanoparticles (LNP 126, 128) into the ears of mice (i.d.).
FIG. 21 shows results of analyzing an amount of R/L expressed 6 hours and 24 hours after injecting lipid nanoparticles (LNP 278, 279, 280, 281) of a novel ionized lipid composition including Renilla Luciferase (R/L) mRNA and control lipid nanoparticles (LNP 126, 128) into the ears of mice (i.d.).
FIG. 22 shows results of analyzing an amount of R/L expressed 6 hours and 24 hours after injecting lipid nanoparticles (LNP 291, 292, 293, 294, 295, 296) of a novel ionized lipid-included composition including Renilla Luciferase (R/L) mRNA and control lipid nanoparticles (LNP 126, 128) into the ears of mice (i.d.).
FIG. 23 shows results of analyzing an amount of R/L expressed 6 hours and 24 hours after injecting lipid nanoparticles (LNP 310, 311, 312, 313, 314, 315) of a novel cholesterol replaced composition including Renilla Luciferase (R/L) mRNA and control lipid nanoparticles (LNP 126, 128) into the ears of mice (i.d.).
FIG. 24 shows results of analyzing an amount of R/L expressed 6 hours and 24 hours after injecting lipid nanoparticles (LNP 323, 324, 325, 326, 327, 328) of a composition including novel ionized lipid including Renilla Luciferase (R/L) mRNA and control lipid nanoparticles (LNP 126, 128) into the ears of mice (i.d.).
FIG. 25 shows results of analyzing an amount of R/L expressed 6 hours and 24 hours after injecting lipid nanoparticles (LNP 329, 330, 331, 332, 333, 334) of a composition including novel ionized lipid including Renilla Luciferase (R/L) mRNA and control lipid nanoparticles (LNP 126, 128) into the ears of mice (i.d.).
FIG. 26 shows results of analyzing an amount of R/L expressed 6 hours and 24 hours after injecting lipid nanoparticles (LNP 335, 336, 337, 338) of a composition including novel ionized lipid including Renilla Luciferase (R/L) mRNA and control lipid nanoparticles (LNP 126, 128) into the ears of mice (i.d.).
FIG. 27 shows a result of analyzing an amount of R/L expressed 6 hours after injecting lipid nanoparticles (LNP 412, 417, 422, 425) of a composition including novel ionized lipid including Renilla Luciferase (R/L) mRNA and control lipid nanoparticles (LNP 126, 128, 295, 334) into the ears of mice (i.d.).
FIG. 28 shows results obtained by formulating (LNP 126, 181, 187, 200) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d).
FIG. 29 shows results obtained by formulating (LNP 126, 128, 230, 231, 232) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d).
FIG. 30 shows results obtained by formulating (LNP 128, 295) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d).
FIG. 31 shows results obtained by formulating (LNP 126, 159, 211, 224, 227, 230, 291, 294, 310, 313, 323, 325, 327, 329, 331, 333, 335, 337) mRNA encoding Renilla Luciferase (R/L) to administer the mRNA into the ear of ICR mice by I.D. and measuring the concentration of MCP-1 from the blood collected after 6 hours.
FIG. 32 shows results obtained by formulating (LNP 128, 160, 212, 225, 228, 231, 292, 295, 311, 314, 324, 326, 328, 330, 332, 334, 336, 338) mRNA encoding Renilla Luciferase (R/L) to administer the mRNA into the ear of ICR mice by I.D. and measuring the concentration of MCP-1 from the blood collected after 6 hours.

### Best Mode for Carrying Out the Invention

Hereinafter, the present disclosure will be described in more detail.

The present disclosure provides a compound selected from a compound represented by the following Chemical Formula 1, a stereoisomer thereof, a racemate thereof, or a pharmaceutically acceptable salt thereof: 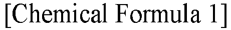 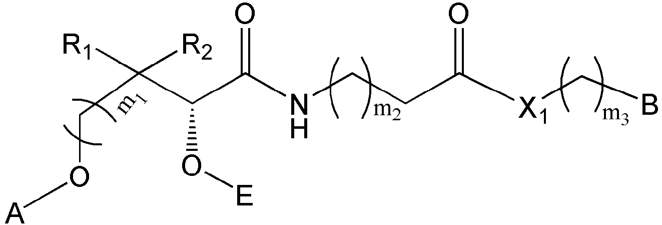
wherein, in the Chemical Formula 1, R₁ and R₂ are the same or different and each is hydrogen or a saturated hydrocarbon with 1 to 2 carbon atoms, X₁ is O, NH, or S, m₁, m₂, and m₃ are the same or different and each is an integer of 1 to 3, and A is hydrogen or a compound represented by the following Chemical Formula 1-1, wherein, 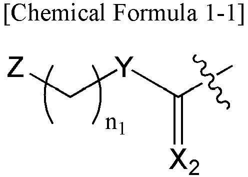
wherein, in the Chemical Formula 1-1, Z is NR¹R²; a heterocyclic or aromatic compound with 3 to 8 carbon atoms; or linear or branched, saturated or unsaturated hydrocarbon with 6 to 22 carbon atoms, wherein the R¹ and R² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and the hydrocarbon includes or does not include ester, ether, amide, carbamate, carbonate, or disulfide bonds, X₂ is O or S, Y is CH₂, NH, or O, n₁ is an integer of 0 to 3, and
B is a compound represented by the following Chemical Formula 1-2 or NR¹'R²', wherein the R¹' and R²' are the same or different, and each is linear or branched, saturated or unsaturated hydrocarbon with 1 to 6 carbon atoms, wherein the hydrocarbon includes or does not include ester, ether, amide, carbamate, carbonate, or disulfide bonds, and R¹' and R²' are connected to each other to form a heterocyclic or aromatic ring with 3 to 8 carbon atoms, wherein, 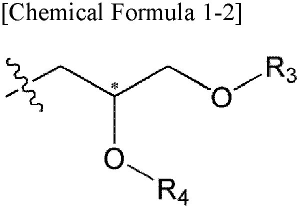
wherein, in the Chemical Formula 1-2, R₃ and R₄ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and E is hydrogen or a compound represented by the following Chemical Formula 1-3, wherein, 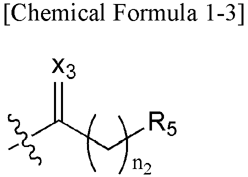
wherein, in the Chemical Formula 1-3, X₃ is O or S, n₂ is an integer of 0 to 3, R₅ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with thiolane or dithiolane.

The heterocyclic compound may be selected from the group consisting of thiophene, furan, pyrosol, pyridine, pyran, oxazine, thiazine, morpholine, pyrrolidine, piperidine, piperazine, pyrazole, pyridine, and dithiolane that are substituted or unsubstituted with alkyl groups with 1 to 4 carbon atoms.

The aromatic compound may be benzene that is substituted or unsubstituted with a di(C1~C4) alkylamino(C1~2) alkyl group.

Specifically, the compound represented by the Chemical Formula 1 may be a compound represented by the following Chemical Formula 2.

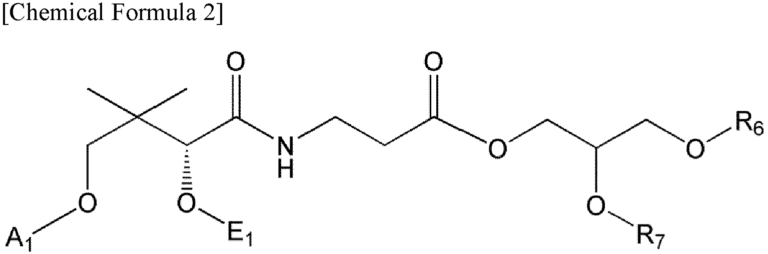

In the Chemical Formula 2, A₁ is hydrogen or a compound represented by the following Chemical Formula 2-1, wherein,

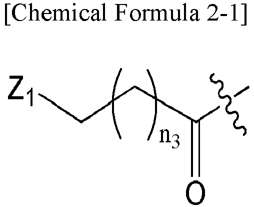

in the Chemical Formula 2-1, Z₁ is NR³R⁴; or a heterocyclic or aromatic compound with 3 to 8 carbon atoms, wherein the R³ and R⁴ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4) alkylamino(C1~2) alkyl, n₃ is an integer of 0 to 3, and
E₁ is hydrogen or a compound represented by the following Chemical Formula 2-2, wherein, 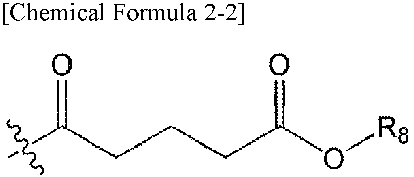
in the Chemical Formula 2-2, R₈ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with dithiolane, and
R₆ and R₇ are the same or different, and each is linear or branched, saturated or unsaturated hydrocarbon with 6 to 22 carbon atoms.

In addition, the compound represented by the Chemical Formula 1 may be a compound represented by the following Chemical Formula 3.

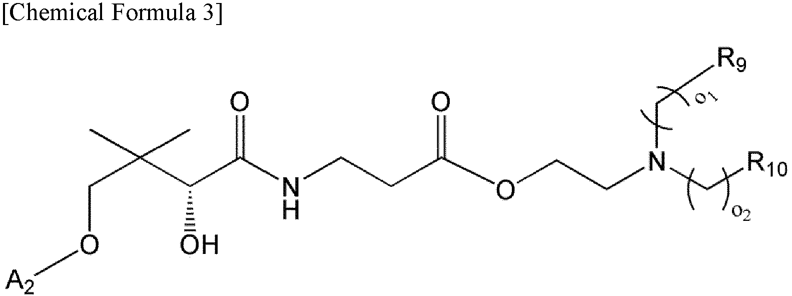

In the Chemical Formula 3, A₂ is hydrogen or a compound represented by the following Chemical Formula 3-1, wherein,

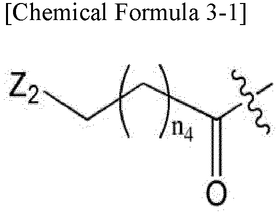

in the Chemical Formula 3-1, Z₂ is NR⁵R⁶; or a heterocyclic or aromatic compound with 3 to 8 carbon atoms, wherein R⁵ and R⁶ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and n₄ is an integer of 0 to 3, and
R₉ and R₁₀ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and o₁ and o₂ are the same or different, and each is an integer of 2 to 10.

In addition, the compound represented by the Chemical Formula 1 may be a compound represented by the following Chemical Formula 4.

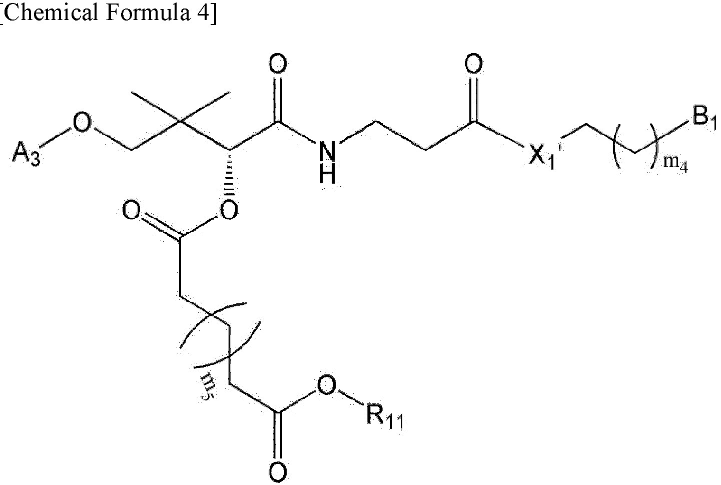

In the Chemical Formula 4, X₁' is O, NH, or S, m₄ and m₅ are the same or different respectively, and each is an integer of 0 to 3, and A₃ is a compound represented by the following Chemical Formula 4-1, wherein,

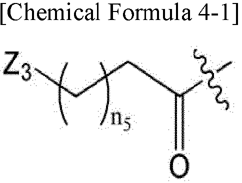

in the Chemical Formula 4-1, Z₃ is NR⁷R⁸; a heterocyclic or aromatic compound with 3 to 8 carbon atoms; or linear or branched, saturated or unsaturated hydrocarbon with 6 to 22 carbon atoms, the R⁷ and R⁸ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, wherein the hydrocarbon includes or does not include ester, ether, amide, carbamate, carbonate, or disulfide bonds, n₅ is an integer of 0 to 3,
B₁ is a compound represented by the following Chemical Formula 4-2; NR⁹R¹⁰; or linear or branched, saturated or unsaturated hydrocarbon with 6 to 22 carbon atoms, wherein R⁹ and R¹⁰ are the same or different, and each is linear or branched, saturated or unsaturated hydrocarbon with 1 to 6 carbon atoms, wherein the hydrocarbon includes or does not include ester, ether, amide, carbamate, carbonate, or disulfide bonds, and R⁹ and R¹⁰ are connected to each other to form a heterocyclic or aromatic ring with 3 to 8 carbon atoms, wherein, 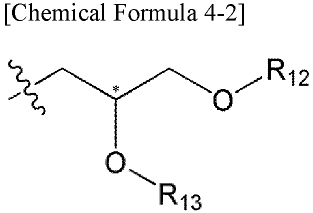
in the Chemical Formula 4-2, R₁₂ and R₁₃ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
R₁₁ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with dithiolane.

More specifically, the compound represented by the Chemical Formula 2 may be a compound represented by Chemical Formula 5 or Chemical Formula 6.

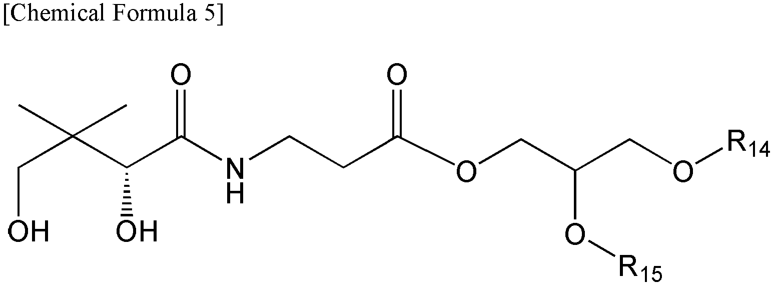

In the Chemical Formula 5, R₁₄ and R₁₅ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds.

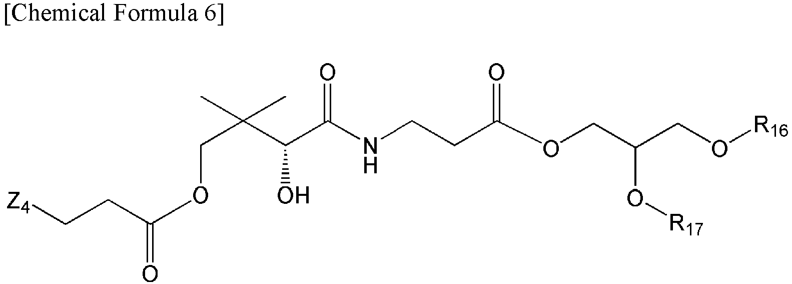

In the Chemical Formula 6, Z₄ is NR¹¹R¹², or a heterocyclic or aromatic compound with 3 to 8 carbon atoms, wherein R¹¹ and R¹² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and
R₁₆ and R₁₇ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds.

In addition, the compound represented by Chemical Formula 3 may be a compound represented by Chemical Formula 7.

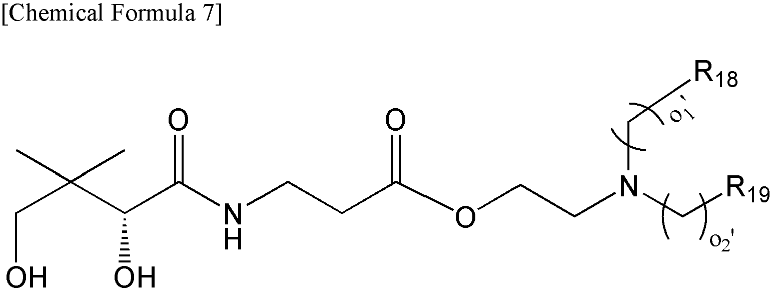

In the Chemical Formula 7, R₁₈ and R₁₉ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and o₁' and o₂' are the same or different, and each is an integer of 3 to 8.

In addition, the compound represented by Chemical Formula 4 may be a compound represented by Chemical Formula 8 or Chemical Formula 9.

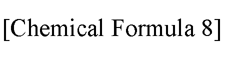

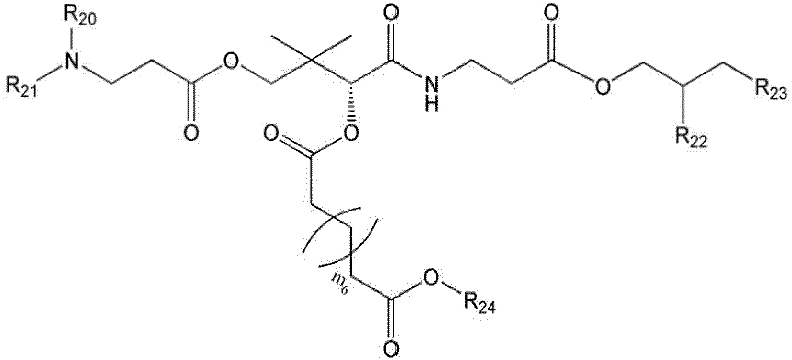

**In** the Chemical Formula 8, m₆ is an integer of 0 to 3, R₂₀ and R₂₁ are the same or different, and each is linear or branched, saturated or unsaturated hydrocarbon with 1 to 6 carbon atoms; the R₂₀ and R₂₁ are connected to each other to form a heterocyclic ring with 3 to 8 carbon atoms,
R₂₂ and R₂₃ are the same or different, and each is hydrogen or linear or branched, saturated or unsaturated hydrocarbon with 6 to 22 carbon atoms, wherein the hydrocarbon includes or does not include ester, ether, amide, carbamate, carbonate, or disulfide bonds, and R₂₄ is linear or branched, saturated or unsaturated hydrocarbon with 6 to 22 carbon atoms, wherein the hydrocarbon includes or does not include ester bonds or disulfide bonds and is substituted or unsubstituted with dithiolane.

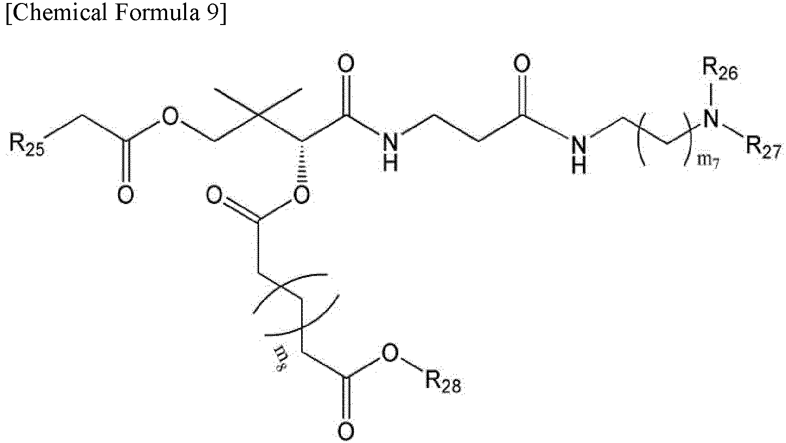

In the Chemical Formula 9, m₇ and m₈ are the same or different and each is an integer of 0 to 3, R₂₅ is linear or branched, saturated or unsaturated hydrocarbon with 1 to 6 carbon atoms, wherein the hydrocarbon includes or does not include ester bonds or disulfide bonds, R₂₆ and R₂₇ are the same or different, and each is linear or branched, saturated or unsaturated hydrocarbon with 6 to 22 carbon atoms; the R₂₆ and R₂₇ are connected to each other to form a heterocyclic or aromatic ring with 3 to 8 carbon atoms, and R₂₈ is linear or branched, saturated or unsaturated hydrocarbon with 6 to 22 carbon atoms, wherein the hydrocarbon includes or does not include ester bonds or disulfide bonds and is substituted or unsubstituted with dithiolane.

For example, a compound represented by Chemical Formula 5 may be helper lipid represented by the following Chemical Formula 10 or Chemical Formula 11.

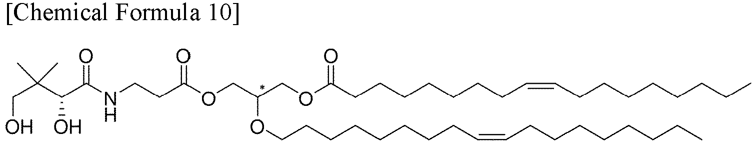

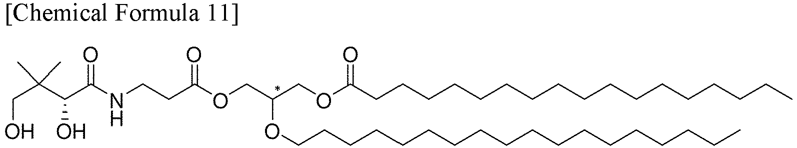

In addition, the compound represented by Chemical Formula 6 may be ionized lipid represented by any one selected from the group consisting of the following Chemical Formula 12 to Chemical Formula 23.

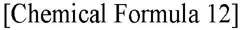

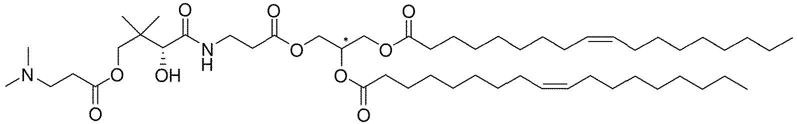

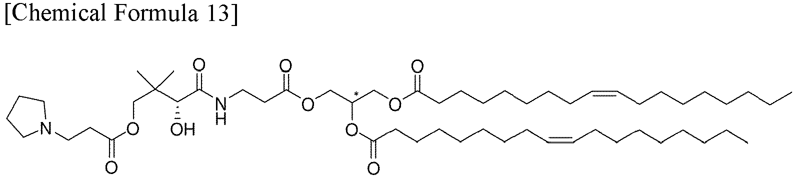

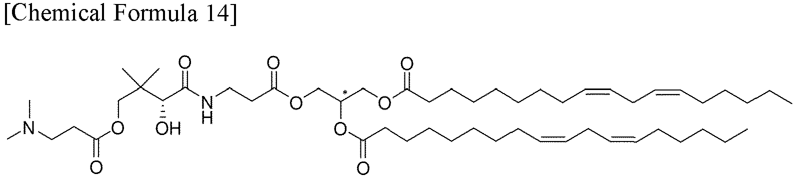

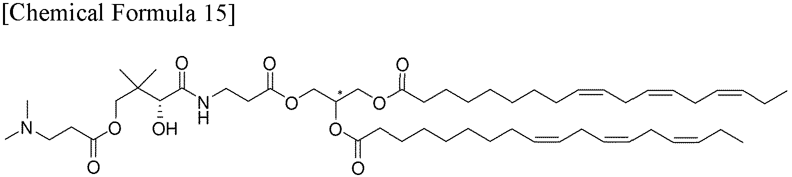

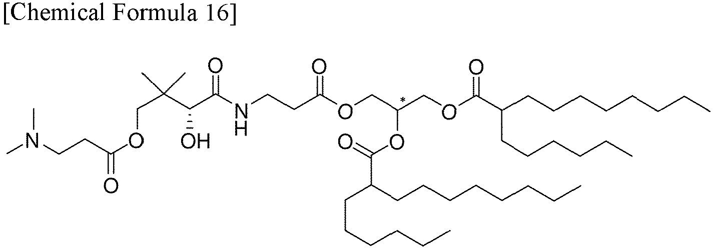

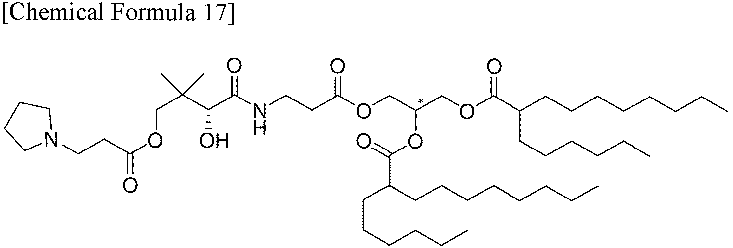

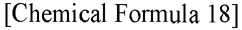

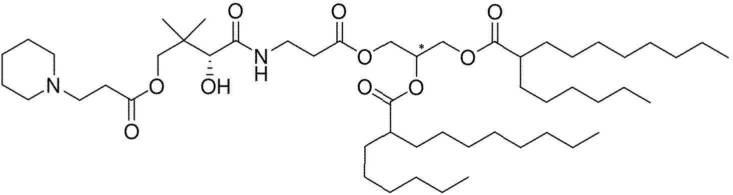

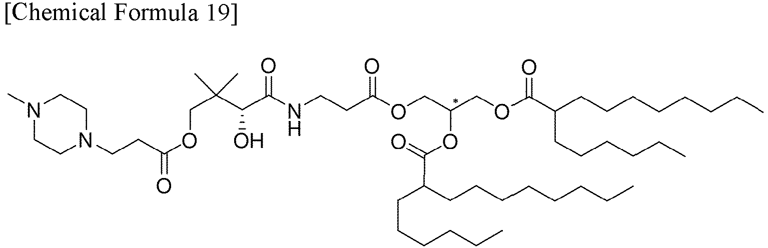

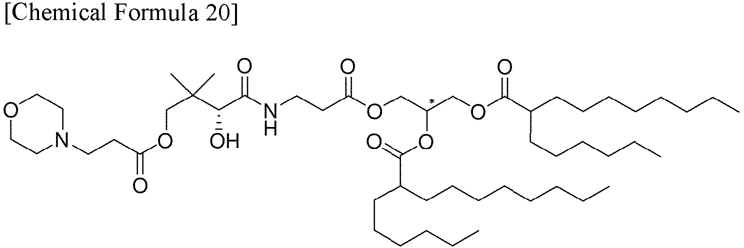

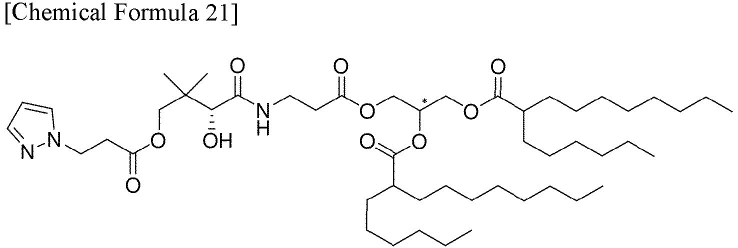

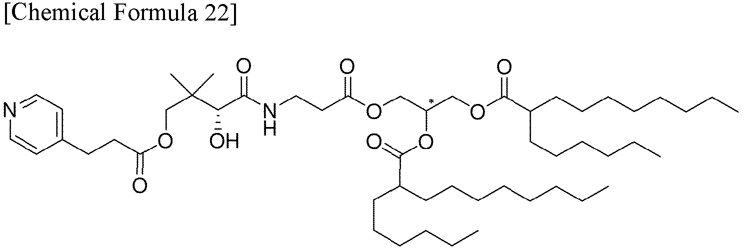

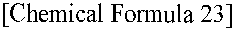

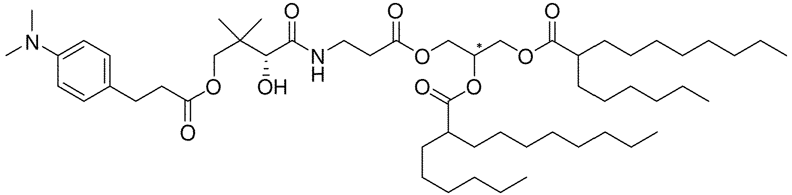

In addition, the compound represented by Chemical Formula 7 may be ionized lipid represented by the following Chemical Formula 24.

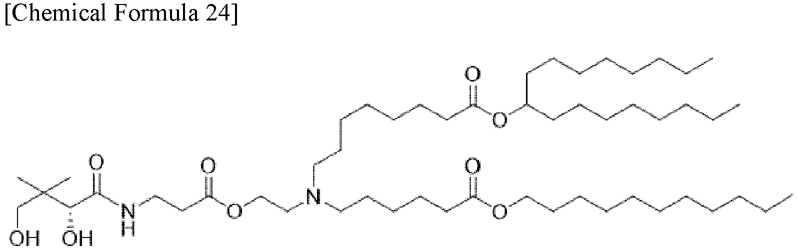

In addition, the compound represented by the Chemical Formula 8 may be ionized lipid represented by any one selected from the group consisting of the following Chemical Formula 25 to Chemical Formula 28 and Chemical Formula 37 to Chemical Formula 38.

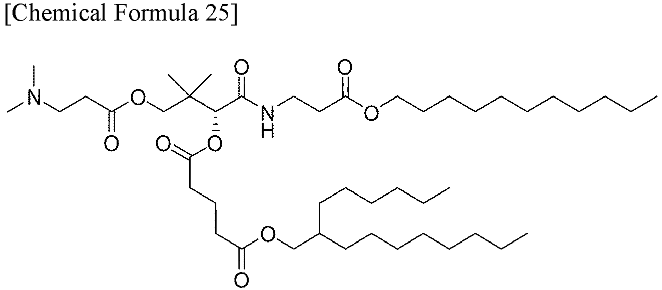

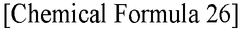

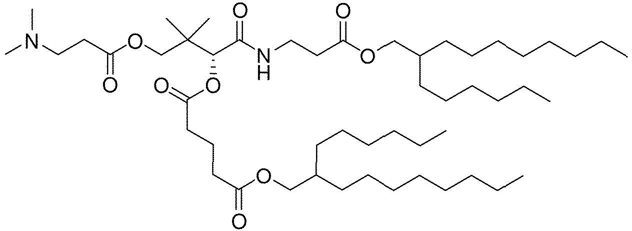

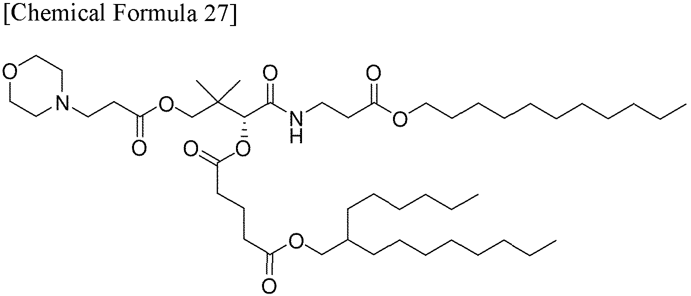

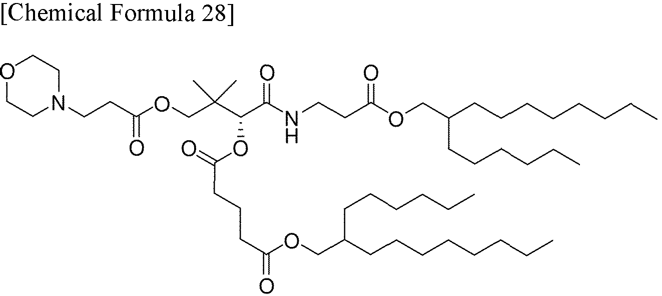

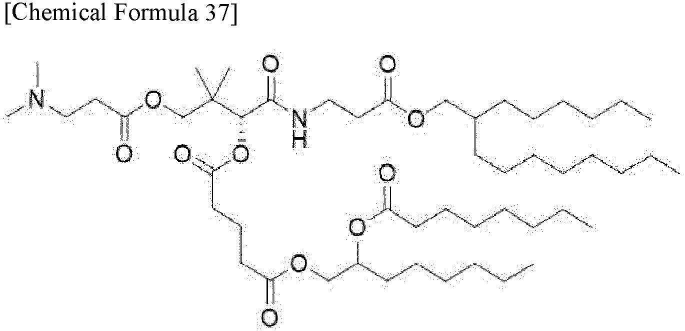

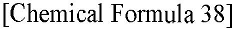

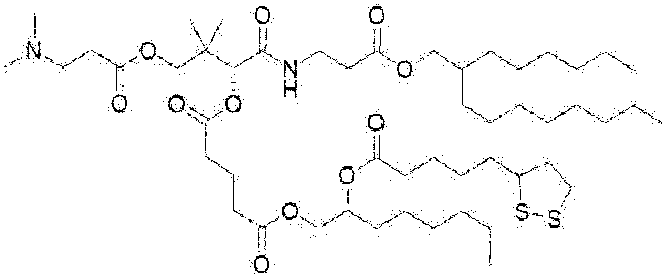

In addition, the compound represented by Chemical Formula 9 may be ionized lipid represented by any one selected from the group consisting of the following Chemical Formula 29 to Chemical Formula 36 and Chemical Formula 39 to Chemical Formula 40.

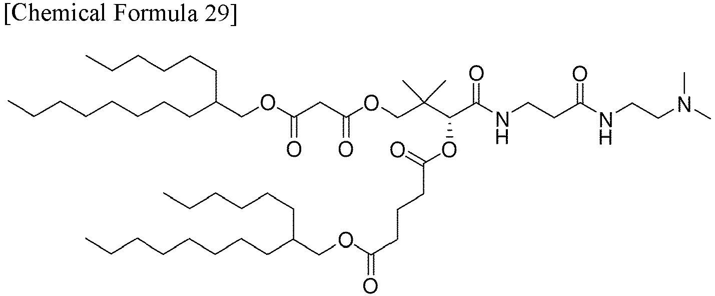

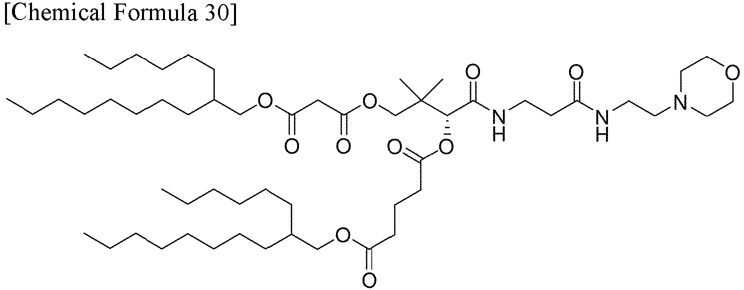

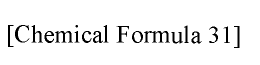

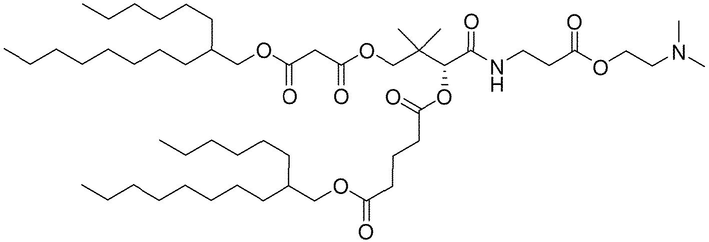

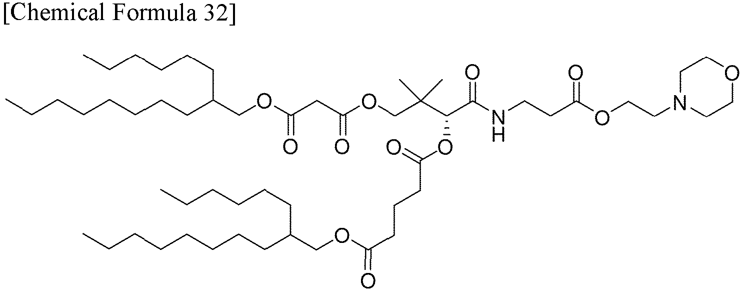

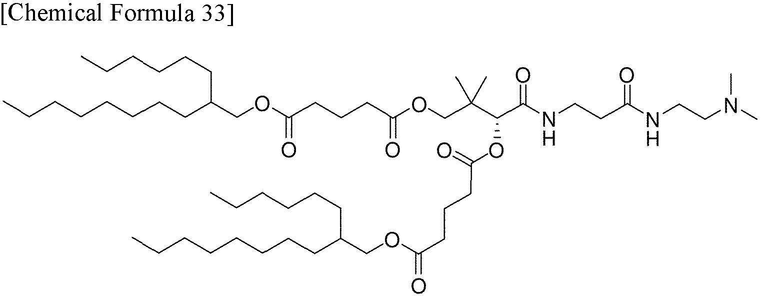

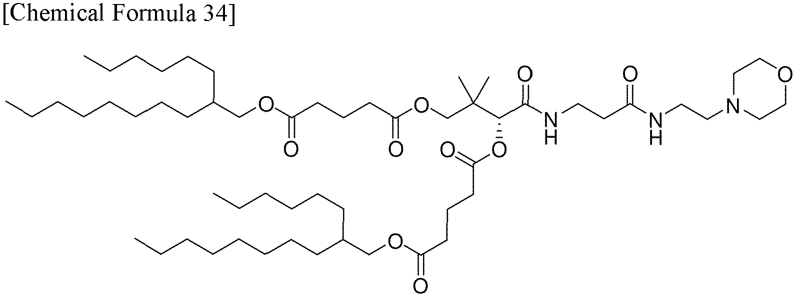

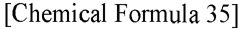

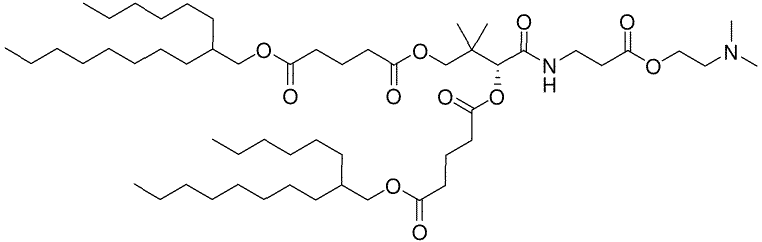

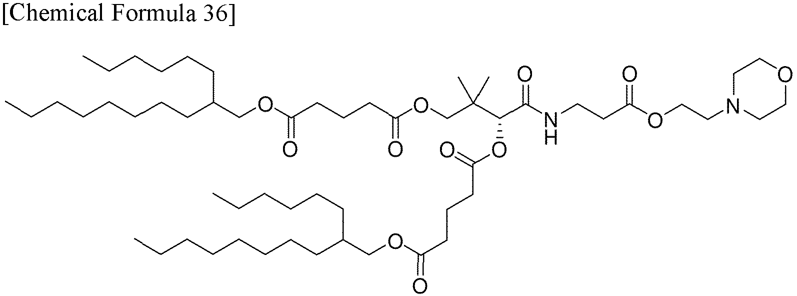

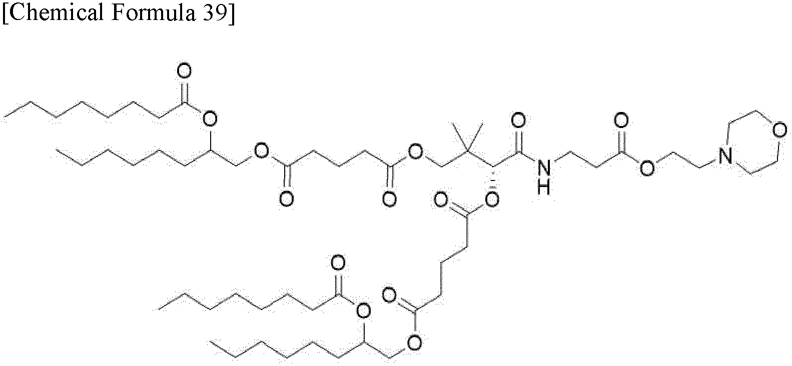

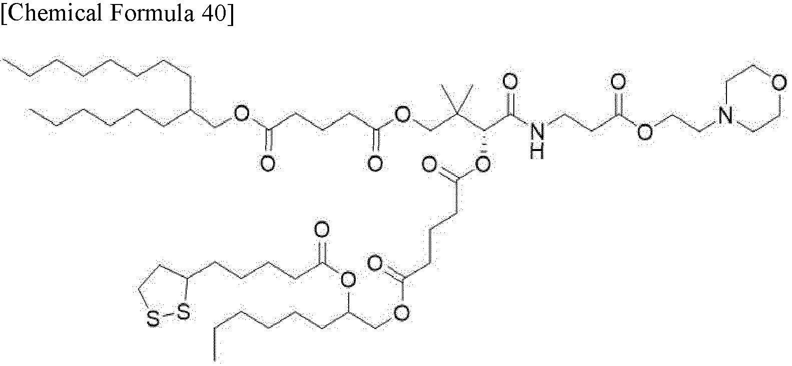

In addition, the present disclosure provides a lipid nanoparticle composition comprising a compound selected from the above-described compound represented by Chemical Formula 1, a stereoisomer thereof, a racemate thereof, or a pharmaceutically acceptable salt thereof.

The compound may be ionized lipid or helper lipid.

The corresponding features may be substituted in the above-described part.

The lipid nanoparticle composition includes one or more selected from the group consisting of helper lipid, structure-retaining lipid, PEG-lipid, and additives.

The helper lipid may be one or more selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinoleoyl-sn-glycero-3-phosphocholine, 1,2-diaracydonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diaracydonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidylethanolamine (DSPE), dipalmitoyl-phosphatidylethanolamine (DPPE), 1,2-dimyristoyl-sn-glycero-3-phosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, and lysophosphatidylethanolamine (LPE).

The structure-retaining lipid may be one or more selected from the group consisting of cholesterol, bile acid derivatives including butyl lithocholate, cholanic acid derivatives, lithocholic acid derivatives, flavonoid, vitamin A and derivatives thereof, vitamin E, vitamin K, coenzyme Q10, and beta-carotene.

The lithocholic acid derivative may be a compound represented by the following Chemical Formula 41:

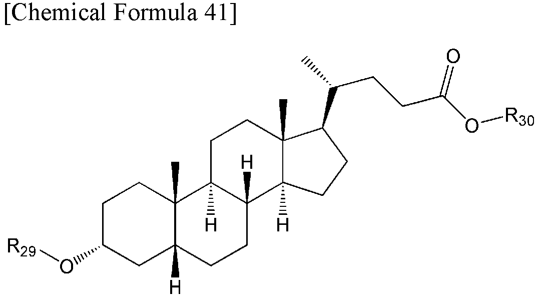

In the Chemical Formula 41, R₂₉ and R₃₀ are the same or different and each is linear or branched, saturated or unsaturated hydrocarbon with 1 to 22 carbon atoms. Provided is a lipid nanoparticle composition in which at least one of the R₂₉ and R₃₀ is saturated or unsaturated hydrocarbon with 2 to 20 carbon atoms.

Specifically, the compound represented by the Chemical Formula 41 may be a compound represented by Chemical Formula 42 in which R₂₉ is hydrogen and R₃₀ has an alkyl group with 4 carbon atoms:

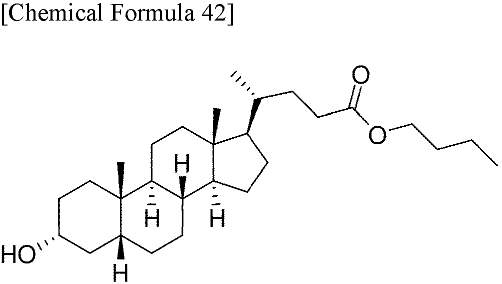

In addition, the PEG-lipid may be one or more selected from the group consisting of PEG-modified phosphatidylethanolamine, PEG-modified phosphatidic acid, PEG-modified ceramide, PEG-modified dialkylamine, PEG-modified diacylglycerol, PEG-modified dialkyl glycerol, and a mixture thereof.

In addition, the additive may be a trehalose derivative represented by the following Chemical Formula 43:

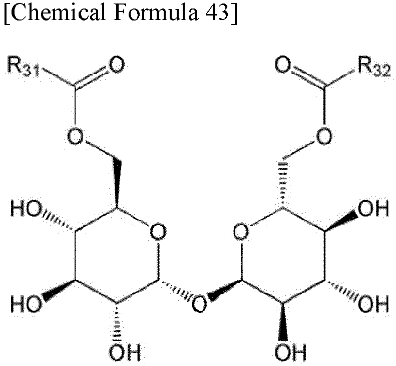

in the Chemical Formula 43, R₃₁ and R₃₂ are the same or different, and each is linear or branched, saturated or unsaturated hydrocarbon with 6 to 22 carbon atoms.

Specifically, the compound represented by the Chemical Formula 43 may be a compound represented by the following Chemical Formula 44:

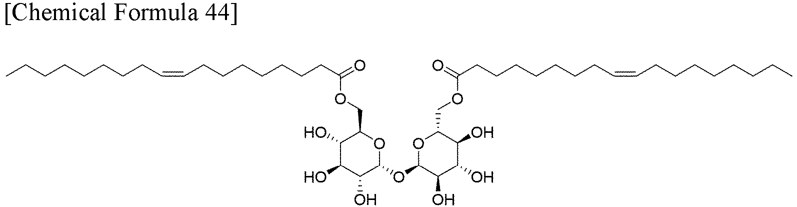

Specifically, the lipid nanoparticle composition may include ionized lipid, helper lipid, structure-retaining lipid, PEG-lipid, and additives, in a ratio of 20 to 60 mol% of ionized lipid, 5 to 40 mol% of helper lipid, 25 to 45 mol% of structure-retaining lipid, 1 to 3 mol% of PEG-lipid, and 0 to 25 mol% of additives.

More specifically, the composition may include 20 to 50 mol% of ionized lipid, 5 to 15 mol% of helper lipid, 25 to 45 mol% of structure-retaining lipid, 1 to 3 mol% of PEG-lipid, and 0 to 25 mol% of additives.

More specifically, the composition may include any one compound of Chemical Formula 12 to Chemical Formula 40 as the ionized lipid, DSPC, DOPE, and a compound of Chemical Formula 10 or Chemical Formula 11 as the helper lipid, cholesterol, lithocholic acid derivative or cholanic acid derivative as the structure-retaining lipid, myristoyl diglyceride (DMG)-PEG as the PEG-lipid, and 6,6'-trehalose dioleate as the additive.

The lipid nanoparticle composition of the present disclosure may further include a therapeutic and/or prophylactic agent.

In addition, the therapeutic and/or prophylactic agent may be a vaccine or compound capable of inducing an immune response.

The therapeutic and/or prophylactic agent may be selected from the group consisting of DNA, small interfering RNA (siRNA), asymmetric interfering RNA (aiRNA), microRNA (miRNA), dicer-substrate RNA (dsRNA), small hairpin RNA (shRNA), messenger RNA (mRNA), and a mixture thereof.

The encapsulation efficiency of the therapeutic and/or prophylactic agent may be at least 50% or higher independently.

The wt/wt ratio of the lipid component to the therapeutic and/or prophylactic agent may be about 10:1 to about 60:1.

The N:P ratio of the therapeutic and/or prophylactic agent may be about 2:1 to about 30:1. The N/P ratio is a value obtained by dividing the number of ionizable nitrogen in ionized lipids by the number of phosphate groups in nucleic acid molecules.

In addition, the present disclosure provides a composition for drug delivery, including the above-described lipid nanoparticle composition and the therapeutic and/or prophylactic agent.

The corresponding features may be substituted in the above-described part.

In addition, the present disclosure provides a method of delivering therapeutic and/or prophylactic agents to mammalian cells by the composition for drug delivery.

The corresponding features may be substituted in the above-described part.

A method of delivering the therapeutic and/or prophylactic agent to mammalian cells includes administering the lipid nanoparticle composition to a subject, wherein the administration brings the cells into contact with the lipid nanoparticle composition to have the therapeutic and/or prophylactic agent delivered to cells.

The mammalian cells are derived from mammals.

The mammal is human.

In addition, the composition for drug delivery may be administered intravenously, intramuscularly, intradermally, subcutaneously, intranasally, or by inhalation. The therapeutic and/or prophylactic agents may be administered to mammals in a dose from about 0.01 mg/kg to about 10 mg/kg.

In addition, the present disclosure provides a method of producing desired polypeptides in mammalian cells.

The method of producing desired polypeptides in mammalian cells includes bringing the cells into contact with the composition for drug delivery which includes the lipid nanoparticle composition and the therapeutic and/or prophylactic agent, wherein the therapeutic and/or prophylactic agent is mRNA which encodes the desired polypeptides and translated in cells to produce the desired polypeptide.

The corresponding features may be substituted in the above-described part.

In addition, the present disclosure provides an immune boosting composition including the above-described lipid nanoparticle composition.

The corresponding features may be substituted in the above-described part. Modes for Carrying Out the Invention

Hereinafter, to help the understanding of the present disclosure, example embodiments will be described in detail. However, the following example embodiments are merely illustrative of the contents of the present disclosure, and the scope of the present disclosure is not limited to the following example embodiments. Example embodiments of the present disclosure are provided to more fully describe the present disclosure to those skilled in the art.

### Example 1: Synthesis of pantothenic acid-based helper lipid

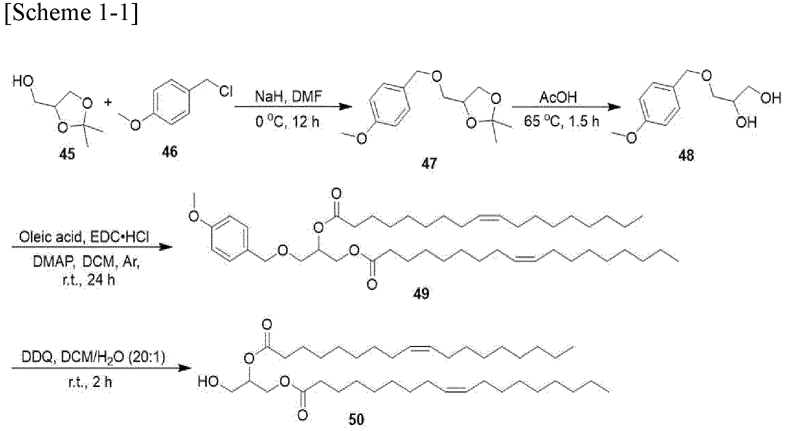

### 1-1-1. 4-(((4-Methoxybenzyl)oxy)methyl)-2,2-dimethyl-1,3-dioxolane (47)

Sodium hydride (0.50 g, 12 mmol, 60% dispersion in mineral oil) was put into anhydrous DMF (10 mL), stirred, and cooled to 0°C. Thereafter, solketal (compound 45) (1 g, 7.55 mmol) was added dropwise to the solution, then raised to room temperature, and stirred in the presence of argon gas for 10 minutes. The reaction mixture was cooled again to 0°C, p-methoxybenzyl chloride (compound 46) (1.75, 11 mmol) was added dropwise, and the mixture was vigorously stirred at room temperature for 12 hours. When the reaction was terminated, the reaction mixture was cooled to 0°C, slowly added with anhydrous methanol (1 mL), and diluted with EtOAc (30 mL), and then water (2 x 20 mL) and saturated aqueous NaCl solution (20 mL) were used to wash off organic layers sequentially. The organic layer was dried with anhydrous MgSO₄, and then the filtered filtrate was concentrated with a rotary vacuum distiller and purified by flash column chromatography (SiO₂, EtOAc/hexane 1:9 → 2:8) to obtain compound 47 (6.34 g, 93%) in a clear oil state.

¹H NMR (CDCl₃, 400 MHz): δ 1.36 (s, 3H), 1.43 (s, 3H), 3.50-3.61 (m, 1H), 3.60-3.72 (m, 1H), 3.75-3.82 (m, 1H), 3.83 (s, 3H), 3.91-4.05 (m, 1H), 4.16-4.26 (m, 1H), 4.56 (s, 3H), 6.83 (d, *J =* 7 Hz, 2H), 7.24 (d, *J =* 7 Hz, 2H); ¹³C NMR (CDCl₃, 100 MHz): δ 25.4, 26.76, 55.2, 66.8, 70.7, 73.2, 74.7, 109.4, 113.8, 114.2, 130.2, 130.2, 129.4.

### 1-1-2. 3-((4-Methoxybenzyl)oxy)propane-1,2-diol (48)

The synthesized acetal compound 47 (2 g, 8 mmol) was dissolved in 80% aqueous AcOH solution (40 mL), and then the mixture was heated to 65°C and stirred vigorously within 1.5 hours. After checking that the reaction was terminated with TLC (SiO₂, hexane/EtOAc 1:9, CAM stain), the reaction mixture was removed with the rotary vacuum distiller using toluene (30 mL x 2) and purified by column chromatography (SiO₂, EtOAc/hexane 8:2) to obtain compound 48 (6.34 g, 84%) in a clear oil state.

¹H NMR (CDCl₃, 400 MHz): δ 7.24 (d, *J =* 8.8 Hz), 6.88 (d, *J =* 8.8 Hz), 4.47 (s, 2H), 3.84-3.89 (m, 1H), 3.78 (s, 3H), 3.67 (dd, *J=* 11.5, 3.9 Hz), 3.59 (dd, *J =* 11.6, 5.7 Hz, 1H), 3.47-3.53 (m, 2H), 2.69 (s, 2H); ¹³C NMR (CDCl₃, 100 MHz): δ 159.4, 129.8, 129.5, 113.9, 73.1, 71.3, 70.5, 64.1, 55.3.

### 1-1-3. 3-((4-Methoxybenzyl)oxy)propane-1,2-diyl dioleate (49)

Oleic acid (665 mg, 2.5 equiv.) was put into a reaction vessel, dissolved in DCM (40 mL), and added with EDCI•HCl (580 mg, 2.5 equiv.) and 4-dimethylaminopyridine (DMAP) (55 mg, 0.3 equiv.), followed by stirring vigorously at 5°C in the presence of argon gas for 10 minutes. Thereafter, (3-((4-methoxybenzyl)oxy)propane-1,2-diol (200 mg, 1 equiv.) solution dissolved in DCM (10 mL) was added dropwise while being maintained at 5°C, followed by stirring at room temperature for 24 hours. After checking that the reaction was terminated with TLC (SiO₂, EtOAc/hexane 4:6), DCM (50 mL) was added to the reaction mixture, washing was followed using saturated aqueous NaHCO₃ solution (50 mL x 2) and saturated aqueous NaCl solution (20 mL), drying was performed with anhydrous MgSO₄, and filtered filtrate was concentrated with the rotary vacuum distiller. Thereafter, purification was followed using column chromatography (SiO₂, EtOAc/hexane 1:9) to obtain compound 49 (594 mg, 85%) in a clear solution state.

¹H NMR (CDCl₃, 400 MHz): δ 0.86-0.89 (t, *J =* 5.6 Hz, 6H), 1.26-1.29 (m, 41H), 1.59-1.60 (m, 5H), 1.98-2.00 (m, 8H), 2.01-2.33 (m, 4H), 3.54-3.55 (m, 2H), 3.56-3.80 (s, 3H), 4.14-4.34 (m, 2H), 4.47 (dd, *J =* 3.6, 4.8 Hz, 2H), 5.21-5.22 (m, 2H), 5.32-5.35 (m, 4H), 7.21 (d, *J =* 8.8 Hz, 2H), 7.26 (d, *J* = 9 Hz, 2H); ¹³CNMR (CDCl₃, 100 MHz): δ 14.11, 22.69, 24.88, 24.96, 27.19, 27.24, 29.08, 29.11, 29.14, 29.21, 29.33, 29.54, 29.73, 29.78, 31.92, 34.11, 34.33, 55.26, 62.71, 67.93, 70.06, 72.98, 113.83, 129.30, 129.72, 129.80, 130.02, 159.34, 173.08, 173.38.

### 1-1-4. 3-Hydroxypropane-1,2-diyl dioleate(1,2-diolein; 1,2-dioleoyl-rac-glycerol) (50)

The compound 49 (210 mg, 0.28 mmol, 1 equiv.) was put into in a mixed solvent (20: 1, 20 mL) of DCM and distilled water, stirred for 5 minutes, and then added with DDQ (96.48 mg, 0.368 mmol, 1.5 equiv.), followed by stirring at room temperature for 2 hours. After the reaction was completed, NaHCO₃ (200 mg) was added to the solid-filtered filtrate to separate layers, the aqueous solution layer was extracted with DCM (25 mL x 2), and then the organic layers were collected together to be washed with saturated aqueous NaHCO₃ solution (25 mL x 2) and saturated aqueous NaCl solution (25 mL), followed by drying with anhydrous MgSO₄. Thereafter, the filtered organic layer was purified by flash column chromatography (SiO₂, EtOAc/hexane 1:9) by removing the solvent to obtain compound 50 (125 mg, 71%) in the form of a clear oil.

¹H NMR (CDCl₃, 400 MHz): δ 0.83-0.89 (m, 6H), 1.25-1.37 (m, 40H), 1.41-1.44 (m, 4H), 1.99-2.07 (m, 8H), 2.30-2.36 (m, 4H), 3.72-3.73 (m, 2H), 4.21-4.25 (m, 1H), 4.30-4.33 (m, 2H), 5.07-5.09 (m, 1H), 5.32-5.39 (m, 4H) ; ¹³C NMR (CDCl₃, 100 MHz): δ 27.31, 27.42, 29.21, 29.32, 29.43, 29.52, 29.61, 29.62, 29.84, 29.85, 29.87, 30.31, 31.56, 31.71, 32.04, 32.05, 33.85, 33.95, 34.21, 34.42, 34.64, 61.71, 62.27, 72.35, 129.87, 130.14, 130.34, 173.51, 173.81.

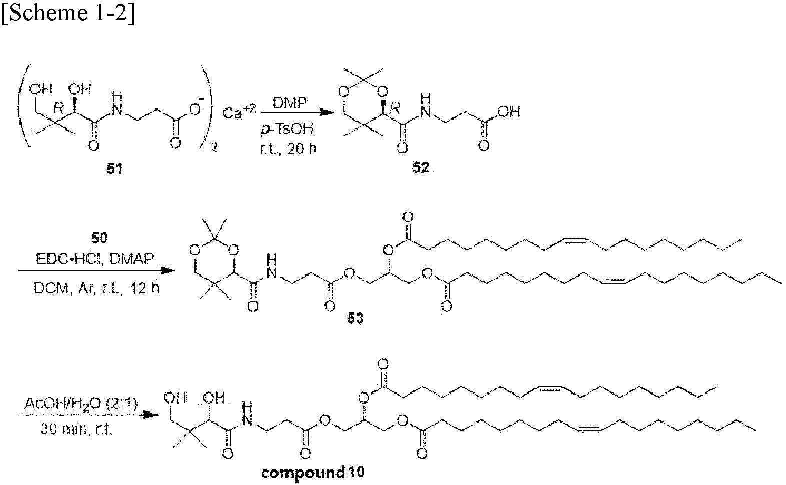

### 1-2-1. (R)-3-(2,2,5,5-Tetramethyl-1,3-dioxane-4-carboxamido)propanoic acid (52)

p-Toluenesulfonic acid (PTSA, 3.97 g, 1.1 equiv.) was added at once while stirring D-pantothenic acid hemicalcium salt, (compound 51, 5 g, 1 equiv.) and 2,2-dimethoxypropane (DMP, 60 mL) in a reaction vessel. The reaction mixture solution was stirred at room temperature for 20 hours, and then the produced white solid was washed with acetone for filtration. The yellow solid obtained by vacuum distillation of the filtrate was washed with warm n-hexane to obtain compound 52 (5.45 g, 100%) as a white solid.

¹H NMR (CDCl₃, 400 MHz): δ 0.98 (s, 3H), 1.04 (s, 3H), 1.43 (s, 3H), 1.46 (s, 3H), 2.62 (dt, *J =* 6.0, 2.0, 2H), 3.29 (d, *J =* 1.5, 1H), 3.45-3.53 (m, 1H), 3.56-3.64 (m, 1H), 3.70 (d, *J =* 11.5, 1H), 4.11 (s, 1H), 7.05 (app bs, 1H); ¹³C NMR (CDCl₃, 100 MHz): δ 18.7, 18.8, 22.0, 29.4, 30.9, 33.0, 33.9, 34.1, 71.4, 77.1, 99.1, 170.2.

### 1-2-2. 3-((3-(2,2,5,5-Tetramethyl-1,3-dioxane-4-carboxamido)propanoyl)oxy)propane-1,2-diyl dioleate (53)

Carboxylic acid compound 52 (62 mg, 1.5 equiv.) was put into a reaction vessel to dissolve in DCM (30 mL), EDCI•HCl (46 mg, 1.5 equiv.), and 4-dimethylaminopyridine (DMAP) (4 mg, 0.2 equiv.) were added, and the reaction mixture was stirred vigorously in the presence of argon gas at 5°C for 10 minutes. Thereafter, DCM (10 mL) solution obtained by dissolving compound 50 (100 mg, 1 equiv.) was added dropwise at 5°C, and then the mixture was stirred at room temperature for 12 hours. After checking that the reaction was terminated using TLC (SiO₂, EtOAc/hexane 2:8), DCM (50 mL) was further added, and then organic layers were washed off with saturated aqueous NaHCO₃ solution (2 x 50 mL) and saturated aqueous NaCl solution (20 mL). The filtrate filtered after removing moisture from the organic layer using anhydrous MgSO₄ was distilled under reduced pressure to remove the solvent, and the remaining mixture was separated by flash column chromatography (SiO₂, EtOAc/hexane 3:7) to obtain compound 53 (100 mg, 72%) in a clear liquid state.

¹H NMR (CDCl₃, 400 MHz): δ 0.87 (t, *J =* 7.1 Hz, 6H), 0.95 (s, 3H), 1.03 (s, 3H), 1.25-1.30 (m, 43H), 1.41 (s, 3H), 1.45 (s, 3H), 1.61 (brs, 4H), 1.98-2.01 (m, 8H), 2.28-2.32 (m, 4H), 2.56-2.58 (t, *J =* 6.5 Hz, 2H), 3.28 (d, *J =* 11.5 Hz, 1H), 3.48-3.58 (m, 2H), 3.66 (d, *J* = 11.5 Hz, 1H), 4.07 (s, 1H), 4.11-4.17 (m, 2H), 4.26-4.34 (m, 2H), 5.24-5.31 (m, 1H), 5.32-5.35 (m, 4H), 7.25 (s, 1H); ¹³C NMR (CDCl₃, 100 MHz): δ 14.02, 18.58, 18.59, 18.73, 21.99, 22.58, 24.73, 24.76, 27.73, 24.76, 27.07, 27.12, 28.94, 48.99, 29.04, 29.08, 29.22, 29.36, 29.42, 29.61, 29.66, 31.79, 31.80, 32.86, 33.78, 33.80, 33.91, 34.03, 34.05, 34.07, 61.88, 68.64, 71.37, 76.71, 76.93, 77.14, 98.92, 129.58, 126.60, 129.92, 129.93, 136.71, 171.55, 171.57, 172.76, 172.78, 173.13.

### 1-2-3. 3-((3-(2,4-dihydroxy-3,3-dimethylbutanamido)propanoyl)oxy)propane-1,2-diyl dioleate (compound 10)

The compound 53 (100 mg) was put into a reaction vessel to dissolve in the mixed solvent of AcOH/H₂O (2:1, 20 mL), followed by stirring at room temperature for 40 minutes. Saturated aqueous NaHCO₃ solution (50 mL) was added to the reaction mixture solution to extract with EtOAc (50 mL x 1, 20 mL x 2). After collecting all the EtOAc layers and then washing with saturated aqueous NaCl solution (20 mL), moisture was removed with anhydrous MgSO₄, the filtered filtrate was distilled under reduced pressure to remove the solvent, and then the remaining mixture was purified by flash column chromatography (SiO₂, EtOAc/hexane 1:1) to obtain compound 10 (36.22 mg, 38%) in a clear oil state.

¹H NMR (CDCl₃, 400 MHz): δ 0.87 (t, *J =* 7.1 Hz, 6H), 0.90 (d, *J =* 12 Hz, 3H), 1.03 (d, *J =* 12 Hz, 3H), 1.23-1.34 (m, 42H), 1.60 (s, 4H), 1.98-2.01 (m, 8H), 2.30-2.33 (m, 4H), 2.55-2.56 (m, 2H), 3.15-3.21 (m, 1H), 3.47-3.65 (m,3H), 3.75-3.78 (m,1H), 4.00-4.06 (m, 1H), 4.16-4.17 (m, 1H), 4.20-4.39 (m, 4H), 5.30-5.36 (m, 5H), 7.22 (s, 1H); ¹³C NMR (CDCl₃, 100 MHz): δ 14.01, 19.86, 20.11, 21.61, 21.73, 22.58, 24.71, 24.72, 24.77, 27.07, 27.12, 28.92, 28.94, 28.98, 29.01, 29.02, 29.07, 23.13, 29.21, 29.23, 29.42, 29.60, 29.66, 31.80, 33.89, 33.93, 33.94, 34.09, 34.41, 39.27, 61.90, 61.95, 62.51, 62.60, 68.71, 68.75, 71.05, 71.09, 129.57, 129.58, 129.94, 129.95, 171.76, 173.09, 173.38, 173.56.

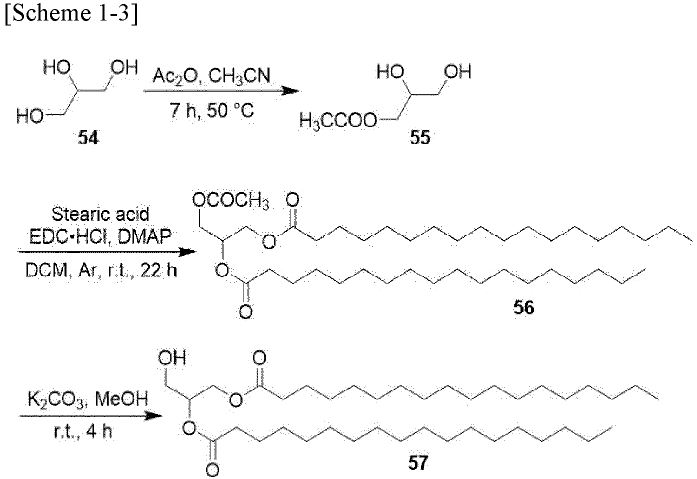

### 1-3-1. 2,3-Dihydroxypropyl acetate (55)

After dissolving propane-1,2,3-triol, (compound 54) (300 mg, 1 equiv.) and ethanoic anhydride (432 mg, 1.3 equiv.) in anhydrous acetonitrile solvent (20 mL), tetra-n-butylammonium acetate (TBAAc; 687 mg, 0.7 equiv.) was added, and the reaction mixture was stirred at 50 °C for 7 hours. After the termination of the reaction, the reaction mixture was loaded by the solid deposition method and purified by flash column chromatography (SiO₂, n-hexane/EtOAc 5:5) to obtain compound 55 (297 mg, 68%) in a colorless oil state.

¹HNMR (CDCl₃, 400 MHz): δ 2.11 (s, 3H), 2.7-2.8 (bs, 1H), 3.09-3.19 (bs, 1H), 3.59 (dd, 1H, *J =* 6, 11.6 Hz), 3.69 (dd, 1H, *J =* 3.6, 11.6 Hz), 3.93 (pentet, 1H, *J =* 4.8 Hz), 4.22-4.12 (m, 2H); ¹³C NMR (CDCl₃, 100 MHz): δ 20.83, 63.31, 65.29, 70.12, 171.56.

### 1-3-2. 3-Acetoxypropane-1,2-diyl distearate (56)

Stearic acid (1060 mg, 2.5 equiv.) was put into a reaction vessel to dissolve in DCM (40 mL), and then EDCI•HCl (580 mg, 2.5 equiv.) and 4-dimethylaminopyridine (DMAP) (55 mg, 0.3 equiv.) were added. The reaction mixture was vigorously stirred in the presence of argon gas at 5°C for 10 minutes, and then a solution obtained by dissolving compound 55 (200 mg, 1 equiv.) in DCM (20mL) was added dropwise at 5°C, followed by stirring at room temperature for 22 hours. After checking that the reaction was terminated with TLC (SiO₂, EtOAc/hexane 4:6), DCM (50mL) was added to the reaction mixture, the mixture was then placed in a fractionation funnel and washed with saturated aqueous NaHCO₃ solution (2 x 50 mL) and saturated aqueous NaCl solution (30 mL), and then organic layers were collected to remove moisture using anhydrous MgSO₄, followed by distillation under reduced pressure using the filtered filtrate. The obtained mixture was purified by flash column chromatography (SiO₂, EtOAc/hexane 2:8) to obtain compound 56 (846 mg, 85%) in a clear liquid state.

¹H NMR (CDCl₃, 400 MHz): δ 0.87 (t, *J =* 6 Hz, 6H), 1.01 (m, 9H), 1.21 (m, 56H), 1.62 (m, 4H), 2.08 (s, 1H), 2.25 (m, 4H), 3.74 (m, 2H), 4.28 (dd, *J =* 5, 12 Hz, 1H), 4.41 (dd, *J* = 4, 12 Hz, 1H), 5.14 (m, 1H), 7.31-7.41 (m, 6H), 7.52-7.65 (m, 4 H); ¹³C NMR (CDCl₃, 100 MHz): δ 173.12, 172.22, 170.11, 135.89, 135.63, 133.09, 129.82, 127.35, 72.04, 62.85, 34.97, 34.76, 32.55, 30.37, 30.14, 30.04, 29.93, 29.76, 29.53, 27.46, 25.49, 25.36, 19.35, 14.41.

### 1-3-3. 3-Hydroxypropane-1,2-diyl distearate (57)

The compound 56 (protected glycerol; 200 mg, 1 equiv.) was put into a reaction vessel which was then filled with nitrogen gas and closed with a rubber stopper. Thereafter, methanol (30 mL) was added using cannula for dissolution, and K₂CO₃ (83 mg, 2 equiv.) was then added in order, followed by stirring in the presence of nitrogen gas at room temperature for 4 hours. When the reaction was terminated, the reaction mixture was distilled under reduced pressure to remove the solvent, and the obtained mixture was dissolved by adding DCM (50 mL) and distilled water (50 mL), which was then placed in a fractionation funnel to separate the organic layer and the aqueous solution layer. The aqueous solution layer was extracted with DCM (2 x 20 mL), and all the organic layers were collected and washed with saturated aqueous NaCl solution (20 mL). The filtrate filtered by removing moisture from the separated organic layer using anhydrous MgSO₄ was distilled under reduced pressure to remove DCM, and then the remaining mixture was purified by flash column chromatography (SiO₂, EtOAc/hexane 2:8 → 5:5) to obtain compound 57 (131 mg, 70%) in a clear liquid state.

¹H NMR (CDCl₃, 400 MHz): δ 0.88 (t, *J =* 6 Hz, 6H), 1.05 (m, 9H), 1.26 (m, 56H), 1.63 (m, 4H), 2.25 (m, 4H), 3.77 (m, 2H), 4.22 (dd, *J =* 5, 12 Hz, 1H), 4.42 (dd, *J =* 4, 12 Hz, 1H), 5.18 (m, 1H), 7.31-7.42 (m, 6H), 7.65-7.68 (m, 4 H); ¹³C NMR (CDCl₃, 100 MHz): δ 173.25, 172.77, 135.83, 135.63, 133.09, 129.82, 127.35, 72.04, 62.85, 34.97, 34.76, 32.55, 30.37, 30.14, 30.04, 29.93, 29.76, 29.53, 27.46, 25.49, 25.36, 19.35, 14.41.

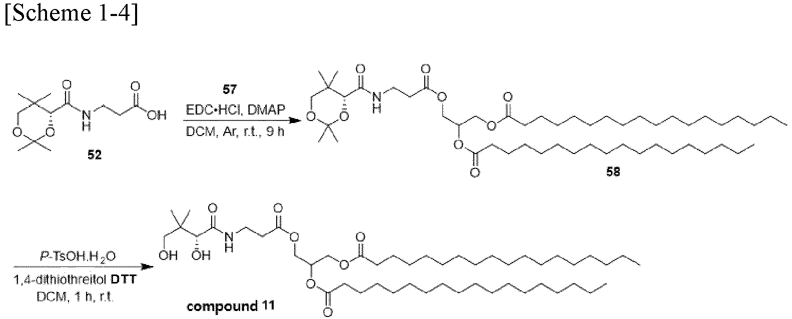

### 1-4-1. 3-((3-((R)-2,2,5,5-Tetramethyl-1,3-dioxane-4-carboxamido)propanoyl)oxy)propane-1,2-diyl distearate (58)

The compound 52 (186 mg, 1.5 equiv.) was put into a reaction vessel to dissolve in DCM (30 mL), EDCI•HCl (111 mg, 1.5 equiv.) and DMAP (12 mg, 0.2 equiv.) were added, and the reaction mixture was stirred vigorously in the presence of argon gas at 5°C for 10 minutes. Thereafter, a solution obtained by dissolving compound 57 (300 mg, 1 equiv.) in DCM (10 mL) was added dropwise at 5°C, followed by stirring at room temperature for 9 hours. After checking the reaction termination with TLC (SiO₂, EtOAc/hexane 3: 7), DCM (50 mL) was further poured into the reaction mixture solution for dissolution, which was then transferred to a fractionation funnel to wash with saturated aqueous NaHCO₃ solution (2 x 50 mL) and saturated aqueous NaCl solution (30 mL). The filtrate obtained by removing moisture from the organic layer with anhydrous MgSO₄ was distilled under reduced pressure and purified by flash column chromatography (SiO₂, EtOAc/hexane 4:6) to obtain compound 58 (306 mg, 72%) in a clear oil state.

¹H NMR (CDCl₃, 400 MHz): δ 0.86 (t, *J =* 11.1 Hz, 6H), 0.95 (s, 3H), 1.02 (s, 3H), 1.24-1.31 (m, 58H), 1.45 (s, 3H), 1.56 (s, 3H), 1.57-1.69 (m, 4H), 2.28-2.31 (q, *J =* 6.2 Hz, 4H), 2.57 (t, *J =* 6.5 Hz, 2H), 3.27 (d, *J =* 11.5 Hz, 1H), 3.44-3.57 (m, 2H), 3.68 (d, *J =* 11.5 Hz, 1H), 4.11 (s, 1H), 4.12-4.33 (m, 4H), 5.23-5.26 (m, 1H), 7.27 (s, 1H); ¹³C NMR (CDCl₃, 100 MHz): δ 14.01, 18.57, 18.58, 18.73, 21.99, 22.58, 24.75, 24.78, 28.97, 29.02, 29.17, 29.26, 29.35, 29.38, 29.39, 29.52, 29.56, 29.60, 31.81, 39.82, 32.86, 33.79, 33.81, 33.93, 34.03, 34.07, 61.87, 62.51, 68.63, 68.64, 71.37, 77.03, 98.92, 169.71, 171.54, 172.79, 173.14.

### 1-4-2. 3-((3-((R)-2,4-Dihydroxy-3,3-dimethylbutanamido)propanoyl)oxy)propane-1,2-diyl distearate (compound 11)

The compound 58 (200 mg, 1 equiv.) and DL-1,4-dithiothreitol (DTT, 71 mg, 2 equiv.) were dissolved in DCM by stirring in a reaction vessel, and *p*-toluenesulfonic acid (20 mg, 0.5 equiv.) was added at room temperature, followed by stirring for 1 hour. After checking that the reaction was terminated with TLC, the mixture was transferred to a fractionation funnel, extracted with EtOAc (30 mL x 2), and washed with saturated aqueous NaCl solution. The filtrate filtered by removing moisture from the organic layer with anhydrous Na₂SO₄ was distilled under reduced pressure and purified by flash column chromatography (SiO₂, EtOAc/hexane 2:8 → 6:4) to obtain compound 11 (105 mg, 55%) in a yellow oil state.

¹H NMR (CDCl₃, 400 MHz): δ 0.87 (t, *J =* 10.2 Hz, 6H), 0.90 (s, 3H), 1.03 (s, 3H), 1.25-1.31 (m, 59H), 1.60-1.62 (m, 4H), 2.30-2.35 (q, *J =* 6.3 Hz, 4H), 2.54-2.62 (m, 2H), 3.48-3.49 (m, 1H), 3.52-3.55 (m, 3H), 3.59-3.62 (m, 1H), 3.91-4.01 (m, 1H), 4.04-4.06 (m, 1H), 4.14-4.41 (m, 4H), 5.27-5.33 (m, 1H), 7.27 (s, 1H); ¹³C NMR (CDCl₃, 100 MHz): δ 14.11, 20.00, 20.23, 21.64, 21.77, 22.69, 24.85, 24.90, 29.06, 29.12, 29.27, 29.36, 29.49, 29.64, 29.67, 29.71, 31.93, 33.99, 34.02, 34.06, 34.22, 34.50, 39.36, 62.01, 62.07, 62.64, 62.71, 68.82, 68.86, 71.15, 71.19, 77.72, 171.79, 171.85, 173.22, 173.33, 173.48, 173.56, 173.68.

### Example 2: Synthesis of pantothenic acid-based ionized lipid

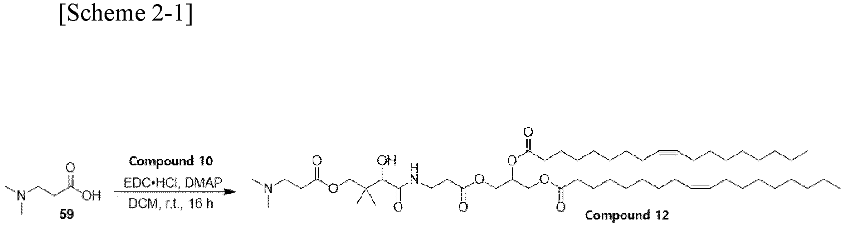

### 2-1. 9-Hydroxy-2.8.8-trimethyl-5,10,14-trioxo-6,15-dioxa-2,11-diazaoctadecane-17,18-diyl dioleate (compound 12)

3-(Dimethylamino)propanoicacid (compound 59); 26 mg, 1.2 equiv.) was dissolved in DCM (20 mL), and then EDCI•HCl (35 mg, 1.5 equiv.) and DMAP (3 mg, 0.2 equiv.) were added, followed by stirring vigorously in the presence of argon gas at 5°C for 10 minutes. Thereafter, a solution obtained by dissolving the compound 10 (100 mg, 1 equiv.) in DCM (5 mL) was added dropwise at 5°C and stirred at room temperature for 16 hours. After checking that the reaction was terminated using TLC (SiO₂, EtOAc/MeOH 9: 1), DCM (50 mL) was further added to the reaction mixture solution, and then organic layers were washed off with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure and purified by column chromatography (SiO₂, EtOAc/MeOH 1:0 →→9:1) to obtain compound 12 (37 mg, 37%) in a clear oil state.

¹H NMR (CDCl₃, 400 MHz): δ 0.87-0.89 (m, 9H), 1.18 (s, 3H), 1.30-1.41 (m, 40H), 1.60-1.61 (m, 4H), 1.99-2.03 (m, 8H), 2.31 (s, 6H), 2.32-2.34 (m, 4H), 2.52-2.72 (m, 7H), 3.52-3.61 (m, 3H), 3.90 (s, 1H), 4.14-4.33 (m, 5H), 5.26-5.39 (m, 5H), 7.28 (s, 1H); ¹³C NMR (CDCl₃, 100 MHz): δ 14.06, 14.10, 19.49, 22.29, 22.67, 24.82, 24.85, 27.16, 27.21, 29.97, 29.08, 29.11, 29.17, 29.30, 29.48, 29.51, 29.58, 29.64, 29.70, 29.75, 31.89, 32.55, 32.59, 33.23, 34.01, 34.15, 34.47, 34.49, 38.44, 38.47, 45.03, 55.66, 62.01, 62.55, 62.59, 68.76, 71.37, 74.49, 74.55, 129.68, 130.01, 130.15, 171.57, 172.09, 172.69.

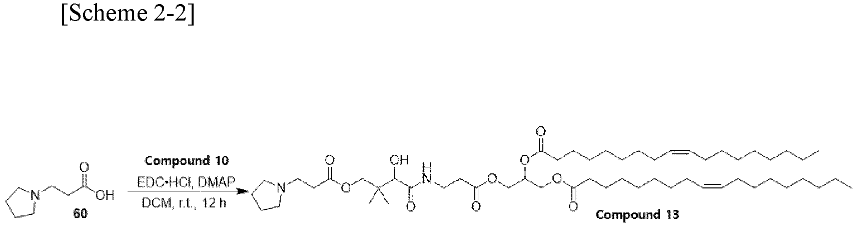

### 2-2. 3-((3-((R)-2-Hydroxy-3,3-dimethyl-4-((3-(pyrrolidin-1-yl)propanoyl)oxy)butanamido)propanoyl)oxy)propane-1,2-diyl dioleate (compound 13)

3-(Pyrrolidin-1-yl)propanoic acid (compound 60, 21 mg, 1.2 equiv.) was dissolved in DCM (20 mL) in a reaction vessel, and then EDCI•HCl (28 mg, 1.5 equiv.) and DMAP (3 mg, 0.2 equiv.) were added and stirred vigorously in the presence of argon gas at 5°C for 10 minutes. Thereafter, a solution obtained by dissolving the compound 10 (100 mg, 1 equiv.) in DCM (5 mL) was added dropwise to the reaction mixture at 5°C, followed by stirring at room temperature for 12 hours. After checking that the reaction was terminated with TLC (SiO₂, EtOAc/MeOH 9:1), DCM (50 mL) was further added, followed by washing with saturated aqueous NaHCO₃ solution (2 x 50 mL) and saturated aqueous NaCl solution (20 mL) sequentially. The filtrate filtered after drying the separated organic layer with anhydrous MgSO₄ was distilled under reduced pressure and purified by column chromatography (SiO₂, EtOAc/MeOH 1:0 → 9:1) to obtain compound 13 (47 mg, 41%) in a clear oil state.

¹H NMR (CDCl₃, 400 MHz): δ 0.77-0.82 (m, 9H), 1.15 (s, 3H), 1.23-1.41 (m, 40H), 1.52-1.55 (m, 4H), 1.88 (m, 4H), 1.89-1.94 (m, 8H), 2.22-2.26 (m, 4H), 2.24-2.53 (m, 10H), 2.64-2.66 (m, 1H), 3.42-3.46 (m, 3H), 3.83 (s,1H), 4.05-4.09 (m,2H), 4.20-4.24 (m, 2H), 4.31-4.33 (m, 1H), 5.25-5.31 (m, 5H), 7.53 (s, 1H); ¹³C NMR (CDCl₃, 100 MHz): δ 14.10, 14.10, 19.04, 22.67, 22.83, 23.12, 24.83, 24.83, 24.85, 27.16, 27.21, 28.97, 29.03, 29.08, 29.11, 29.17, 29.31, 29.48, 29.51, 29.58, 29.65, 29.70, 29.75, 31.89, 32.55, 32.60, 34.01, 34.15, 34.32, 34.40, 38.47, 38.49, 52.77, 52.81, 54.11, 62.01, 62.54, 62.60, 68.75, 68.75, 71.48, 74.47, 74.51, 129.68, 130.01, 130.15, 130.47, 171.56, 172.25, 172.82, 172.87, 173.24.

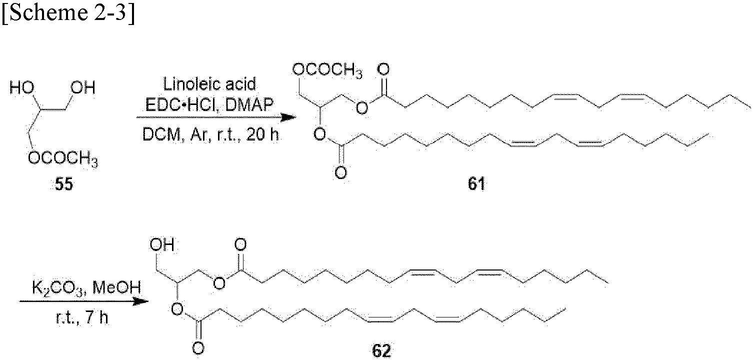

### 2-3-1. 3-Acetoxypropane-1,2-diyl (9Z,9'Z,12Z,12'Z)-bis(octadeca-9,12-dienoate) (61)

After dissolving (9Z,12Z)-octadeca-9,12-dienoic acid (710 mg, 2.5 equiv.) in DCM (40 mL) in a reaction vessel, EDCI•HCl (578 mg, 2.5 equiv.) and 4-dimethylaminopyridine (DMAP) (36 mg, 0.2 equiv.) were added and stirred vigorously in the presence of argon gas at 5°C for 10 minutes. Thereafter, compound 55 (200 mg, 1 equiv.) was added dropwise to DCM (20 mL) at 5°C and stirred at room temperature for 20 hours. After checking that the reaction was terminated with TLC (SiO₂, EtOAc/hexane 1:1), DCM (50 mL) was further added, followed by washing with saturated aqueous NaHCO₃ solution (2 x 50 mL) and saturated aqueous NaCl solution (30 mL) sequentially. The filtrate filtered after drying the separated organic layer with anhydrous MgSO₄ was distilled under reduced pressure and purified by column chromatography (SiO₂, EtOAc/hexane 2:8) to obtain glycerol compound 61 (845 mg, 86%) in a clear oil state.

¹H NMR (CDCl₃, 400 MHz): δ 0.89 (t, *J =* 6.9 Hz, 6H), 1.40-1.26 (m, 28H), 1.63 (m, 4H), 2.05 (dt, *J =* 6.6, 6.9 Hz, 8H), 2.11 (s, 3H), 2.33 (t, *J =* 7.8 Hz, 2H), 2.36 (t, *J =* 7.8 Hz, 2H), 2.79 (t, *J =* 6.5 Hz, 4H), 3.74 (s, 3H), 4.23 (dd, *J =* 5.8, 11.7 Hz, 1H), 4.33 (dd, *J* = 4.7, 11.8 Hz, 1H), 4.44 (d, *J =* 11.8 Hz, 1H), 4.48 (d, *J =* 11.8 Hz, 1H), 5.06 (tt, *J =* 5.6, 5.6 Hz, 1H), 5.39-5.31 (m, 8H); ¹³C NMR (CDCl₃, 100 MHz): δ 14.08 (2C), 20.83, 22.58 (2C), 24.87 (2C), 24.92 (2C), 25.62 (2C), 27.18 (4C), 29.06 (2C), 29.13 (2C), 29.18 (2C), 29.36 (2C), 29.61 (2C), 31.53 (2C), 34.06 (2C), 34.25 (2C), 61.54, 61.99, 72.11, 127.89, 128.08, 130.01, 130.21, 173.39, 173.76, 175.87.

### 2-3-2. 3-Hydroxypropane-1,2-diyl (9Z,9'Z,12Z,12'Z)-bis(octadeca-9,12-dienoate) (62)

The glycerol compound 61 (845 mg, 1 equiv.) was put into a reaction vessel which was then closed with a rubber stopper and filled with nitrogen gas, wherein the state was maintained until the reaction was terminated. Thereafter, CH₃OH (70 mL) was added using cannula, and K₂CO₃ (354 mg, 2 equiv.) was added subsequently, followed by stirring at room temperature for 7 hours. The reaction mixture was distilled under reduced pressure to remove the solvent, then DCM (50 mL) and distilled water (50 mL) were added, and the obtained solution was transferred to a fractionation funnel. The aqueous layer was extracted with DCM (2 x 20 mL), then all the organic layers were collected and washed with saturated aqueous NaCl solution (20 mL). The filtrate filtered by drying the separated organic layer with anhydrous MgSO₄ was distilled under reduced pressure and purified by column chromatography (SiO₂, EtOAc/hexane 1:9 → 5:5) to obtain compound 62 (561 mg, 71%) in a clear oil state.

¹H NMR (CDCl₃, 400 MHz): δ 0.89 (t, *J* = 6.9 Hz, 6H), 1.26-1.40 (m, 28H), 1.63 (m, 4H), 2.05 (dt, *J* = 6.6, 6.9 Hz, 8H), 2.33 (t, *J =* 7.8 Hz, 2H), 2.36 (t, *J* = 7.8 Hz, 2H), 2.79 (t, *J* = 6.5 Hz, 4H), 3.74 (s, 3H), 4.23 (dd, *J* = 5.8, 11.7 Hz, 1H), 4.33 (dd, *J* = 4.7, 11.8 Hz, 1H), 4.44 (d, *J* = 11.8 Hz, 1H), 4.48 (d, *J* = 11.8 Hz, 1H), 5.06 (tt, *J* = 5.6, 5.6 Hz, 1H), 5.31-5.39 (m, 8H); ¹³C NMR (CDCl₃, 100 MHz): δ 14.08 (2C), 22.58 (2C), 24.87 (2C), 24.92 (2C), 25.62 (2C), 27.18 (4C), 29.06 (2C), 29.13 (2C), 29.18 (2C), 29.36 (2C), 29.61 (2C), 31.53 (2C), 34.06 (2C), 34.25 (2C), 61.54, 61.99, 72.11, 127.89, 128.08, 130.01, 130.21, 173.39, 173.76.

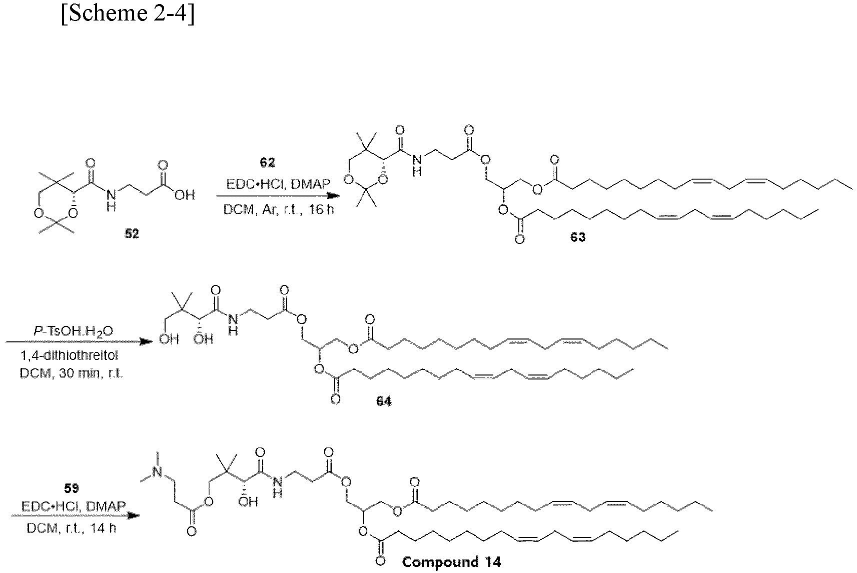

### 2-4-1. 3-((3-((R)-2,2,5,5-Tetramethyl-1,3-dioxane-4-carboxamido)propanoyl)oxy)propane-1,2-diyl (9Z,9'Z,12Z,12'Z)-bis(octadeca-9,12-dienoate) (63)

After dissolving the compound 52 (354 mg, 1.5 equiv.) in DCM (30 mL) in a reaction vessel, EDCI•HCl (211 mg, 1.5 equiv.) and DMAP (22 mg, 0.2 equiv.) were added and stirred vigorously in the presence of argon gas at 5°C for 10 minutes. Thereafter, the solution obtained by dissolving the compound 62 (560 mg, 1 equiv.) in DCM (10 mL) was added dropwise in the reaction mixture at 5°C, followed by stirring at room temperature for 16 hours. After checking that the reaction was terminated with TLC (SiO₂, EtOAc/hexane 3:7), DCM (50 mL) was further added, followed by washing with saturated aqueous NaHCO₃ solution (2 x 50 mL) and saturated aqueous NaCl solution (30 mL) sequentially. The filtrate filtered by drying the separated organic layer with anhydrous MgSO₄ was distilled under reduced pressure and purified by column chromatography (SiO₂, EtOAc/hexane 4:6) to obtain compound 63 (583 mg, 75%) in a clear oil state.

¹H NMR (CDCl₃, 400 MHz): δ 0.90 (t, *J* = 6.8 Hz, 6H), 0.98 (s, 3H), 1.05 (s, 3H), 1.28-1.35 (m, 29H), 1.43 (s, 3H), 1.47 (s, 3H), 1.61-1.64 (m, 4H), 2.07 (dd, *J =* 6.4, 10.1 Hz, 8H), 2.41 (m, 4H), 2.61 (t, *J =* 6.7, 3H), 2.78 (t, *J =* 5.8 Hz, 3H), 3.27-3.71 (m, 4H), 4.09 (s, 1H), 4.17-4.34 (m, 4H), 5.27-5.41 (m, 9H), 7.01 (s, 1H); ¹³C NMR (CDCl₃, 100 MHz): δ 14.06, 18.69, 18.83, 22.10, 22.56, 24.82, 24.85, 25.63, 27.19, 29.03, 29.08, 29.11, 29.17, 29.34, 29.45, 29.60, 31.52, 32.52, 33.90, 34.00, 34.14, 37.18, 61.98, 62.55, 62.60, 68.76. 71.47, 99.02, 127.89, 128.08, 129.97, 130.21, 169.81, 171.63, 172.81, 173.81.

### 2-4-2. 3-((3-((R)-2,4-Dihydroxy-3,3-dimethylbutanamido)propanoyl)oxy)propane-1,2-diyl (9Z,9'Z,12Z,12'Z)-bis(octadeca-9,12-dienoate) (64)

After mixing the compound 63 (580 mg, 1 equiv.) and DL-1,4-dithiothreitol (DTT, 208 mg, 2 equiv.) in DCM solvent in a reaction vessel, *p*-toluenesulfonic acid (78 mg, 0.5 equiv.) was added at room temperature. After stirring the reaction mixture at room temperature for 30 minutes and then checking that the reaction was terminated with TLC, the reaction mixture was extracted with EtOAc (2 X 30 mL), and the organic layer was washed with saturated aqueous NaCl solution. A mixture obtained by distillation under reduced pressure using the filtrate that was filtered by drying the separated organic layer with anhydrous Na₂SO₄ was purified by flash column chromatography (SiO₂, EtOAc/hexane 3:7 → 7:3) to obtain diol compound 64 (408 mg, 55%) in a light-yellow oil form.

¹H NMR (CDCl₃, 400 MHz): δ 0.91 (t, *J* = 6.8 Hz, 6H), 0.94 (s, 3H), 1.05 (s, 3H), 1.27-1.41 (m, 29H), 1.61-1.63 (m, 4H), 2.07 (dd, *J* = 6.5, 10.0 Hz, 8H), 2.37-2.43 (m, 4H), 2.58-2.77 (m, 2H), 2.79-2.80 (t, *J* = 5.7 Hz, 4H), 3.25 (m, 2H), 3.50-3.55 (m, 4H), 4.03 (s, 1H), 4.04-4.31 (m, 4H), 5.31-5.42 (m, 9H), 7.92 (s, 1H); ¹³C NMR (CDCl₃, 100 MHz): δ 14.07, 20.00, 20.23, 21.63, 21.75, 22.57, 24.82, 24.87, 25.64, 27.16, 27.20, 29.06, 29.08, 29.12, 29.17, 29.34, 29.61, 31.35, 33.99, 34.99, 34.19, 34.52, 39.35, 39.36, 62.01, 62.06, 62.62, 62.70, 68.83, 68.68, 71.16, 71.19, 127.89, 128.10, 128.11, 129.97, 129.98, 130.24, 171.77, 171.83, 173.83, 173.16, 173.32, 173.43, 173.46, 173.50, 173.62.

### 2-4-3. (9R)-9-Hydroxy-2,8,8-trimethyl-5,10,14-trioxo-6,15-dioxa-2,11-diazaoctadecane-17,18-diyl (9Z,9'Z,12Z,12'Z)-bis(octadeca-9,12-dienoate) (compound 14)

After dissolving compound 59 (34 mg, 1.2 equiv.) in DCM (20 mL) in a reaction vessel, EDCI•HCl (210 mg, 1.5 equiv.) and 4-dimethylaminopyridine (DMAP) (22 mg, 0.2 equiv.) were added and stirred vigorously in the presence of argon gas at 5°C for 10 minutes. Thereafter, compound 64 (200 mg, 1 equiv.) was dissolved in DCM (5 mL), the mixture was then added dropwise to the reaction mixture at 5°C and stirred at room temperature for 14 hours. After checking that the reaction was terminated with TLC (SiO₂, EtOAc/MeOH 9:1), DCM (50 mL) was further added, followed by washing with saturated aqueous NaHCO₃ solution (2 x 50 mL) and saturated aqueous NaCl solution (20 mL) sequentially. After distilling under reduced pressure using the filtrate filtered by drying the separated organic layer with anhydrous MgSO₄, the obtained mixture was purified by flash column chromatography (SiO₂, EtOAc/MeOH 1:0 → 9:1) to obtain compound 14 (241 mg, 29%) in a clear oil state.

¹H NMR (CDCl₃, 400 MHz): δ 0.87-0.90 (m, 9H), 0.71 (s, 3H), 1.25-1.37 (m, 30H), 1.59-1.62 (m, 4H), 2.05 (dd, *J* = 6.5, 10.0 Hz, 8H), 2.26 (s, 6H), 2.30-2.34 (m, 4H), 2.52-2.65 (m, 6H), 2.76 (t, *J* = 5.7 Hz, 4H), 3.52-3.61 (m, 3H), 3.90 (s, 1H), 4.12-4.31 (m, 6H), 5.30-5.39 (m, 9H), 7.41 (s, 1H); ¹³C NMR (CDCl₃, 100 MHz): δ 14.05, 19.47, 22.22, 22.55, 24.81, 24.83, 25.61, 27.17, 29.01, 29.06, 20.10, 19.16, 29.32, 29.59, 31.50, 33.09, 33.99, 34.13, 34.47, 38.42, 38.44, 44.95, 55.50, 62.01, 62.54, 62.85, 68.76, 71.33, 74.43, 74.48, 127.87, 124.06, 129.97, 130.19, 171,55, 171.55, 172.04, 172.78, 172.89, 172.94, 173.24, 173.26.

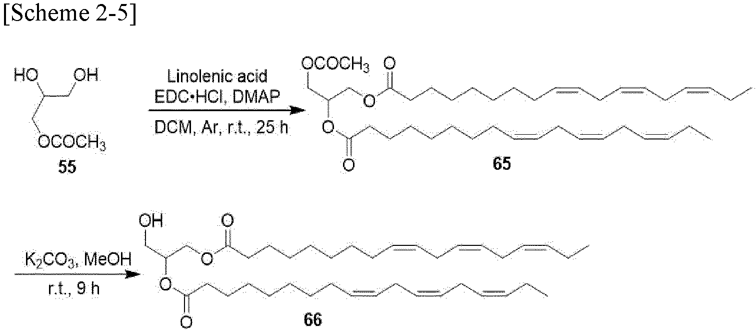

### 2-5-1. 3-Acetoxypropane-1,2-diyl (9Z,9'Z,12Z,12'Z,15Z,15'Z)-bis(octadeca-9,12,15-trienoate) (65)

After dissolving (9Z,12Z,15Z)-octadeca-9,12,15-trienoic acid (Linolenic acid) (520 mg, 2.5 equiv.) in DCM (40 mL) in a reaction vessel, EDCI•HCl (290 mg, 2.5 equiv.) and 4-dimethylaminopyridine (DMAP) (18 mg, 0.2 equiv.) were added and stirred vigorously in the presence of argon gas at 5°C for 10 minutes. Thereafter, the solution obtained by dissolving compound 55 (100 mg, 1 equiv.) in DCM (20 mL) was added dropwise to the reaction mixture at 5°C and stirred at room temperature for 25 hours. After checking that the reaction was terminated with TLC (SiO₂, EtOAc/hexane 1:1), DCM (50 mL) was further added, followed by washing with saturated aqueous NaHCO₃ solution (2 x 50 mL) and saturated aqueous NaCl solution (30 mL). A mixture obtained by distillation under reduced pressure using the filtrate that was filtered by drying the separated organic layer with anhydrous MgSO₄ was purified by flash column chromatography (SiO₂, EtOAc/hexane 3:7) to obtain compound 65 (391 mg, 80%) in a clear oil state.

¹H NMR (CDCl₃, 400 MHz): δ 0.91 (t, *J* = 6.3 Hz, 6H), 1.25-1.41 (m, 20H), 1.60-1.62 (m, 5H), 2.04-2.14 (m, 8H), 2.11 (s, 3H), 2.31-2.37 (m, 4H), 2.84-2.88 (m, 8H), 4.12 (s, 1H), 4.32-4.38 (m, 4H), 5.27-5.33 (m, 1H), 5.34-5.43 (m, 12H); ¹³C NMR (CDCl₃, 100 MHz): δ 14.06 (2C), 21.57 (2C), 24.88 (2C), 24.97 (2C), 26.62 (2C), 27.16 (4C), 29.06 (2C), 30.13 (2C), 29.11 (2C), 29.35 (2C), 29.61 (2C), 31.55 (2C), 34.06 (2C), 34.27 (2C), 61.53, 61.98, 72.12, 127.90, 128.08, 130.02, 130.22, 173.40, 173.77, 175.88.

### 2-5-2. 3-Hydroxypropane-1,2-diyl (9Z,9'Z,12Z,12'Z,15Z,15'Z)-bis(octadeca-9,12,15- trienoate) (66)

The compound 65 (390, 1 equiv.) was put into a reaction vessel which was then closed with a rubber stopper and filled with nitrogen gas, wherein the state was maintained until the reaction was terminated. Thereafter, CH₃OH (70 mL) was added using cannula, and then K₂CO₃ (164 mg, 2 equiv.) was added subsequently, followed by stirring at room temperature for 9 hours. The solution obtained by adding DCM (50 mL) and distilled water (50 mL) to the reaction mixture was transferred to a fractionation funnel to extract the aqueous layer with DCM (2 x 20 mL), and then all the organic layers were collected and washed with saturated aqueous NaCl solution (20 mL). The filtrate filtered by drying the obtained organic layer with anhydrous MgSO₄ was distilled under reduced pressure and purified by flash column chromatography (SiO₂, EtOAc/hexane 2:8 → 5:5) to obtain compound 66 (222 mg, 61%) in a clear oil state.

¹H NMR (CDCl₃, 400 MHz): δ 0.90 (t, *J* = 6.9 Hz, 6H), 1.26-1.41 (m, 20H), 1.61-1.63 (m, 5H), 2.04-2.13 (m, 8H), 2.31-2.35 (m, 4H), 2.84-2.89 (m, 8H), 4.10 (s, 1H), 4.32-4.39 (m, 4H), 5.27-5.32 (m, 1H), 5.34-5.42 (m, 12H); ¹³C NMR (CDCl₃, 100 MHz): δ 14.06 (2C), 21.57 (2C), 24.88 (2C), 24.97 (2C), 26.62 (2C), 27.16 (4C), 29.06 (2C), 30.13 (2C), 29.11 (2C), 29.35 (2C), 29.61 (2C), 31.55 (2C), 34.06 (2C), 34.27 (2C), 61.53, 61.98, 72.12, 127.90, 128.08, 130.02, 130.22, 173.40, 173.77.

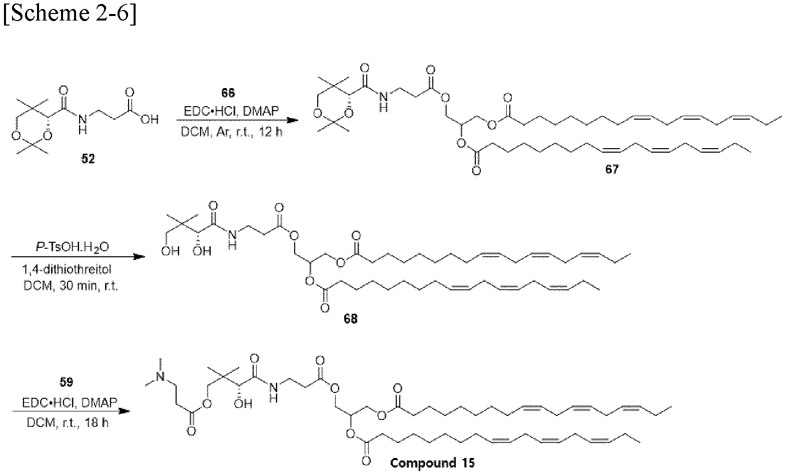

### 2-6-1. 3-((3-((R)-2,2,5,5-Tetramethyl-1,3-dioxane-4-carboxamido)propanoyl)oxy)propane-1,2-diyl (9Z,9'Z,12Z,12'Z,15Z,15'Z)-bis(octadeca-9,12,15-trienoate) (67)

After putting the compound 55 (141 mg, 1.5 equiv.) into a reaction vessel and dissolving in DCM (30 mL), EDCI•HCl (82 mg, 1.5 equiv.) and DMAP (9 mg, 0.2 equiv.) were added and stirred vigorously in the presence of argon gas at 5°C for 10 minutes. Thereafter, the solution obtained by dissolving the compound 66 (222 mg, 1 equiv.) in DCM (10 mL) was added dropwise to the reaction mixture at 5°C, followed by stirring at room temperature for 12 hours. After checking that the reaction was terminated with TLC (SiO₂, EtOAc/hexane 3:7), DCM (50 mL) was further added, and the mixture was transferred to a fractionation funnel, followed by washing with saturated aqueous NaHCO₃ solution (2 x 50 mL) and saturated aqueous NaCl solution (30 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/hexane 2:8) to obtain compound 67 (232 mg, 75%) in a clear oil state.

¹H NMR (CDCl₃, 400 MHz): δ 0.97-1.01 (m, 9H), 1.06 (m, 3H), 1.32-1.33 (m, 20H), 1.44 (m, 3H), 1.48 (m, 3H), 1.61-1.63 (m, 5H), 1.73 (s, 1H), 2.04-2.13 (m, 8H), 2.31-2.35 (m, 4H), 2.58-2.61 (t, *J* = 6.9 Hz, 2H), 2.81-2.84 (m, 8H), 3.32 (d, *J* = 11.6 Hz, 1H), 3.41-3.68 (m, 2H), 3.75 (d, *J* = 11.8 Hz, 1H), 4.10 (s, 1H), 4.15-4.34 (m, 4H), 5.27-5.32 (m, 1H), 5.34-5.42 (m, 12H), 7.29 (s, 1H); ¹³C NMR (CDCl₃, 100 MHz): δ 14.12, 18.84, 20.55, 22.10, 24.82, 24.84, 25.52, 25.61, 27.20, 29.03, 29.08, 29.11, 29.17, 29.46, 29.59, 32.97, 33.91, 34.00, 34.14, 34.18, 61.98, 61.55, 62.60, 68.75, 71.47, 76.72, 77.04, 77.15, 77.36, 99.03, 127.11, 127.76, 127.77, 128.23, 129.30, 130.20, 130.21, 131.95, 168.82, 171.64, 172.82, 173.20.

### 2-6-2. 3-((3-((R)-2,4-Dihydroxy-3,3-dimethylbutanamido)propanoyl)oxy)propane-1,2-diyl (9Z,9'Z,12Z,12'Z,15Z,15'Z)-bis(octadeca-9,12,15-trienoate) (68)

After dissolving the compound 67 (230 mg, 1 equiv.) and DL-1,4-dithiothreitol (DTT, 83 mg, 2 equiv.) in DCM, p-toluenesulfonic acid (23 mg, 0.5 equiv.) was added at room temperature and stirred for 30 minutes. After the reaction was terminated, the reaction mixture solution was extracted with EtOAc (30 mL x 2), and all the organic layers were collected and washed with saturated aqueous NaCl solution. The filtrate filtered by drying the separated organic layer with anhydrous Na₂SO₄ was distilled under reduced pressure to remove the solvent, and the obtained mixture was purified by flash column chromatography (SiO₂, EtOAc/hexane 3:7 → 5:5) to obtain compound 68 (121 mg, 55%) in a light-yellow oil state.

¹H NMR (CDCl₃, 400 MHz): δ 0.98-1.02 (m, 9H), 1.03 (m, 3H), 1.32-1.34 (m, 20H), 1.61-1.65 (m, 5H), 1.72 (s, 1H), 2.04-2.15 (m, 8H), 2.34-2.37 (m, 4H), 2.59-2.65 (t, *J* = 6.9 Hz, 2H), 2.81-2.84 (m, 8H), 3.32-3.33 (m, 1H), 3.41-3.69 (m, 2H), 3.75-3.78 (m, 1H), 4.10 (s, 1H), 4.16-4.34 (m, 4H), 5.28-5.32 (m, 1H), 5.34-5.42 (m, 12H), 7.30 (s, 1H); ¹³C NMR (CDCl₃, 100 MHz): δ 14.13, 18.85, 24.83, 24.85, 25.53, 25.63, 27.23, 29.04, 29.07, 29.12, 29.18, 29.45, 29.60, 32.98, 33.91, 34.11, 34.12, 34.12, 61.98, 61.56, 62.61, 68.76, 71.48, 76.71, 77.03, 77.16, 77.36, 99.04, 127.12, 127.74, 127.77, 128.24, 129.30, 130.21, 130.25, 131.94, 168.82, 171.64, 172.82, 173.20.

### 2-6-3. (9R)-9-hydroxy-2,8,8-trimethyl-5,10,14-trioxo-6,15-dioxa-2,11-diazaoctadecane-17,18-diyl (9Z,9'Z,12Z,12'Z,15Z,15'Z)-bis(octadeca-9,12,15-trienoate) (Compound 15)

After putting compound 59 (31 mg, 1.2 equiv.) in a reaction vessel and dissolving in DCM (20 mL), EDCI•HCl (34 mg, 1.5 equiv.) and 4-dimethylaminopyridine (DMAP) (3.6 mg, 0.2 equiv.) were stirred vigorously in the presence of argon gas at 5°C for 10 minutes. Thereafter, the solution obtained by dissolving the compound 68 (120 mg, 1 equiv.) in DCM (5 mL) was added dropwise to the reaction mixture at 5°C, followed by stirring at room temperature for 18 hours. After checking that the reaction was terminated with TLC (SiO₂, EtOAc/MeOH 9:1), DCM (50 mL) was further added, and the mixture was transferred to a fractionation funnel, followed by washing with saturated aqueous NaHCO₃ solution (2 x 50 mL) and saturated aqueous NaCl solution (20 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/MeOH 1:0 → 9:1) to obtain compound 15 (48 mg, 36%) in a clear oil state.

¹H NMR (CDCl₃, 400 MHz): δ 0.89 (s, 3H), 0.97 (t, *J* = 7.6 Hz, 6H), 1.09 (s, 3H), 1.25-1.30 (m, 20 H), 1.60 (s, 5H), 2.02-2.11 (m, 8H), 2.29-2.34 (m, 4H), 2.57 (s, 2H), 2.79-3.01 (m, 16H), 3.35-3.36 (m, 4H), 3.73-3.76 (m, 1H), 4.05 (s, 1H), 4.11-4.17 (m, 3H), 4.25-4.31 (m, 3H), 5.27 (m, 1H), 5.30-5.36 (m, 12H), 7.41 (s, 1H); ¹³C NMR (CDCl₃, 100 MHz): δ 14.26, 19.48, 20.53, 21.71, 24.80, 24.84, 24.71, 24.80, 24.84, 25.51, 25.60, 27.18, 29.02, 29.06, 29.10, 29.16, 29.54, 30.27, 34.00, 34.15, 34.49, 38.17, 43.79, 53.76, 62.05, 62.56, 68.78, 71.62, 73.65, 73.73, 127.09, 127.73, 128.21, 128.27, 130.20, 131.93, 169.91, 171.57, 173.01, 173.32.

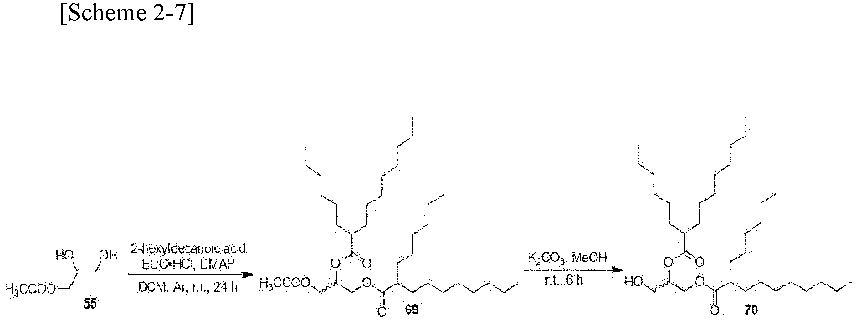

### 2-7-1. 3-Acetoxypropane-1,2-diyl bis(2-hexyldecanoate) (69)

After putting 2-hexyldecanoic acid (956 mg, 2.5 equiv.) into a reaction vessel and dissolving in DCM (40 mL), EDCI•HCl (580 mg, 2.5 equiv.) and 4-dimethylaminopyridine (DMAP) (55 mg, 0.3 equiv.) were added and stirred vigorously in the presence of argon gas at 5°C for 10 minutes. Thereafter, the solution obtained by dissolving compound 55 (200 mg, 1 equiv.) in DCM (20 mL) was added dropwise to the reaction mixture at 5°C, followed by stirring at room temperature for 24 hours. After checking that the reaction was terminated with TLC (SiO₂, EtOAc/hexane 4:6), DCM (50 mL) was further added, and the mixture was transferred to a fractionation funnel, followed by washing with saturated aqueous NaHCO₃ solution (2 x 50 mL) and saturated aqueous NaCl solution (30 mL). The filtrate filtered by drying the separated organic layer with anhydrous MgSO₄ was distilled under reduced pressure to remove the solvent, and the obtained mixture was purified by flash column chromatography (SiO₂, EtOAc/hexane 1:9) to obtain compound 69 (801 mg, 88%) in a clear liquid state.

¹H NMR (CDCl₃, 400 MHz): δ 0.81-0.82 (t, *J* = 5.2 Hz, 12H), 1.18-1.25 (m, 42H), 1.36-1.41 (m, 5H), 1.49-1.55 (m, 5H), 2.11 (s, 3H), 2.25-2.31 (m, 2H), 3.54-3.57 (dd, *J* = 3.6, 3.9 Hz, 1H), 3.60-3.64 (dd, *J* = 3.6, 3.9 Hz, 1H), 4.10-4.15 (dd, *J* = 3.4, 4.3 Hz, 1H), 4.28-4.32 (dd, *J* = 3.4, 4.3 Hz, 1H), 5.12-5.16 (m, 1H); ¹³C NMR (CDCl₃, 100 MHz): δ 14.04, 14.08, 20.83, 22.61, 22.66, 27.31, 27.34, 27.31, 27.42, 27.46, 29.20, 29.23, 29.28, 29.45, 29.53, 29.56, 31.68, 32.31, 32.34, 32.19, 42.32, 45.65, 45.74, 62.09, 70.17, 171.56, 175.46, 175.87.

### 2-7-2. 3-Hydroxypropane-1,2-diyl bis(2-hexyldecanoate) (70)

Glycerol compound 69 (700 mg, 1 equiv.) was put into a reaction vessel which was then closed with a rubber stopper and filled with nitrogen gas, wherein the state was maintained until the reaction was terminated. Thereafter, CH₃OH (70 mL) was added using cannula, and then K₂CO₃ (316 mg, 2 equiv.) was added subsequently, followed by stirring at room temperature for 6 hours. The reaction mixture was distilled under reduced pressure to remove the solvent, then DCM (50 mL) and distilled water (50 mL) were added, and the mixture was transferred to a fractionation funnel. The moisture layer was extracted with DCM (2 x 20 mL), and all organic layers were collected and washed with saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, EtOAc/hexane 2:8 → 3:6) to obtain compound 70 (502 mg, 77%) in a clear oil state.

¹H NMR (CDCl₃, 400 MHz): δ 0.80-0.82 (t, *J* = 5.1 Hz, 12H), 1.18-1.24 (m, 42H), 1.36-1.41 (m, 5H), 1.49-1.57 (m, 5H), 2.25-2.31 (m, 2H), 3.54-3.58 (dd, *J* = 3.6, 3.9 Hz, 1H), 3.60-3.64 (dd, *J* = 3.6, 3.9 Hz, 1H), 4.10-4.14 (dd, *J* = 3.4, 4.3 Hz, 1H), 4.28-4.32 (dd, *J* = 3.4, 4.3 Hz, 1H), 5.12-5.16 (m, 1H); ¹³C NMR (CDCl₃, 100 MHz): δ 14.04, 14.08, 22.60, 22.66, 27.30, 27.34, 27.37, 27.41, 27.45, 29.19, 29.23, 29.28, 29.44, 29.53, 29.58, 31.68, 32.30, 32.33, 32.18, 42.32, 45.64, 45.73, 62.08, 70.16, 175.46, 175.86.

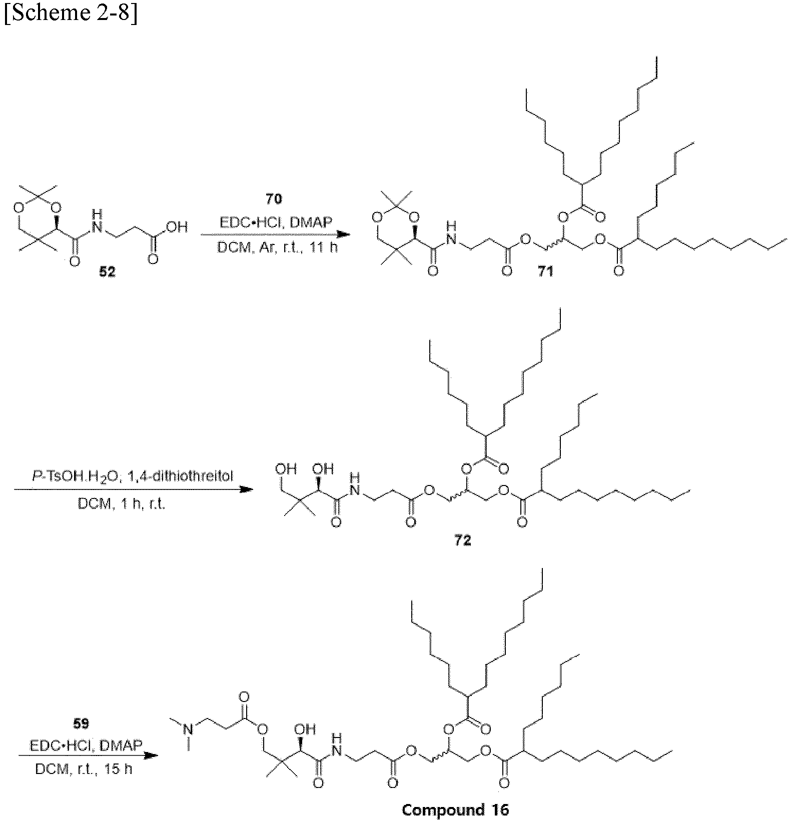

### 2-8-1. 3-((3-((R)-2,2,5,5-Tetramethyl-1,3-dioxane-4-carboxamido)propanoyl)oxy)propane-1,2-diyl bis(2-hexyldecanoate) (71)

After putting acetal-protected pantothenic acid compound 52 (376 mg, 1.5 equiv.) into a reaction vessel and dissolving in DCM (30 mL), EDCI•HCl (225 mg, 1.5 equiv.) and DMAP (24 mg, 0.2 equiv.) were added and stirred vigorously in the presence of argon gas at 5°C for 10 minutes. Thereafter, the solution obtained by dissolving compound 70 (550 mg, 1 equiv.) in DCM (10 mL) was added dropwise to the reaction mixture at 5°C, followed by stirring at room temperature for 11 hours. After checking that the reaction was terminated with TLC (SiO₂, EtOAc/hexane 2:8), DCM (50 mL) was further added, and the mixture was transferred to a fractionation funnel, followed by washing with saturated aqueous NaHCO₃ solution (2 x 50 mL) and saturated aqueous NaCl solution (30 mL). The filtrate filtered by drying the separated organic layer with anhydrous MgSO₄ was distilled under reduced pressure to remove the solvent, and the obtained mixture was removed by column chromatography (SiO₂, EtOAc/hexane 3:7) to obtain compound 71 (440 mg, 77%) in a clear oil state.

¹H NMR (CDCl₃, 400 MHz): δ 0.61-0.63 (t, *J* = 5.1 Hz, 12H), 0.73 (s, 3H), 0.80 (s, 3H), 1.01-1.07 (m, 41H), 1.18-1.22 (m, 11H), 1.32-1.37 (m, 5H), 2.08-2.11 (m, 2H), 2.30-2.33 (t, *J* = 4.1 Hz, 2H), 3.02-3.05 *(d,J =* 6.1 Hz, 1H), 3.20-3.42 (m, 2H), 3.43-3.45 (d, *J* = 6.1 Hz, 1H), 3.84-3.94 (m, 3H), 4.06-4.31 (m, 2H), 5.04-5.06 (m, 1H), 7.04 (s, 1H); ¹³C NMR (CDCl₃, 100 MHz): δ 14.03, 14.07, 18.66, 22.08, 22.58, 22.64, 27.26, 27.32, 27.37, 27.42, 29.18, 29.21, 29.27, 29.42, 29.54, 29.57, 31.65, 31.84, 32.21, 32.27, 32.95, 33.83, 33.85, 34.09, 32.12, 45.57, 45.63, 61.95, 62.72, 62.79, 68.64, 71.46, 99.00, 169.78, 171.55, 175.49, 175.87.

### 2-8-2. 3-((3-((R)-2,4-Dihydroxy-3,3-dimethylbutanamido)propanoyl)oxy)propane-1,2-diyl bis(2-hexyldecanoate) (72)

The compound 71 (420 mg, 1 equiv.) and DL-1,4-dithiothreitol (DTT, 160 mg, 2 equiv.) were put into a reaction vessel to dissolve in DCM, and p-toluenesulfonic acid (45 mg, 0.5 equiv.) was added at room temperature, followed by stirring for 1 hour. When the reaction was terminated, the mixture was extracted with EtOAc (30 mL x 2), and all the organic layers were collected and washed with saturated aqueous NaCl solution. The filtrate filtered by drying the separated organic layer with anhydrous Na₂SO₄ was distilled under reduced pressure to remove the solvent, and the obtained mixture was purified by flash column chromatography (SiO₂, EtOAc/hexane 3:7 → 6:4) to obtain compound 72 (243 mg, 61%) in a light-yellow oil state.

¹H NMR (CDCl₃, 400 MHz): δ 0.79-0.82 (t, *J* = 5.1 Hz, 12H), 0.95 (s, 3H), 1.00 (s, 3H), 1.18-1.24 (m, 45H), 1.38 (m, 5H), 1.48-1.52 (m, 5H), 2.25-2.29 (m, 2H), 2.43-2.47 (m, 2H), 3.31-3.43 (m, 1H), 3.59-3.62 (m, 1H), 4.02-4.06 (m, 3H), 4.15-4.28 (m, 4H), 4.38-4.42 (m, 1H), 5.32-.5.34 (m, 1H), 7.02-7.20 (m, 1H); ¹³C NMR (CDCl₃, 100 MHz): δ 14.03, 14.07, 19.91, 19.98, 20.84, 22.59, 22.65, 27.23, 27.28, 27.33, 27.38, 27.44, 29.11, 29.20, 29.27, 29.43, 29.56, 31.62, 31.65, 31.85, 32.21, 32.26, 32.28, 32.32, 32.43, 33.88, 34.41, 34.61, 38.29, 38.52, 45.62, 45.74, 61.95, 62.08, 62.80, 63.03, 68.70, 68.76, 73.27, 73.32, 74.70, 74.96, 169.66, 172.05, 173.54, 173.71.

### 2-8-3. (9R)-9-Hydroxy-2,8,8-trimethyl-5,10,14-trioxo-6,15-dioxa-2,11-diazaoctadecane-17,18-diyl bis(2-hexyldecanoate) (Compound 16)

After compound 59 (37 mg, 1.2 equiv.) into a reaction vessel and dissolving in DCM (20 mL), EDCI•HCl (60 mg, 1.5 equiv.) and 4-dimethylaminopyridine (DMAP) (6.3 mg, 0.2 equiv.) were stirred vigorously in the presence of argon gas at 5°C for 10 minutes. Thereafter, the solution obtained by dissolving the compound 72 (200 mg, 1 equiv.) in DCM (5 mL) was added dropwise to the reaction mixture at 5°C, followed by stirring at room temperature for 15 hours. After checking that the reaction was terminated with TLC (SiO₂, EtOAc/MeOH 9:1), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, EtOAc/MeOH 1:0 → 9:1, 28% aqueous NH₃ solution added) to obtain compound 16 (104 mg, 46%) in a clear oil state.

¹H NMR (CDCl₃, 400 MHz): δ 0.79-0.80 (m, 12H), 0.81 (s, 3H), 1.11 (s, 3H), 1.18-1.22 (m, 42H), 1.36-1.39 (m, 4H), 1.48-1.51 (m, 4H), 2.21-2.26 (m, 8H), 2.43-2.55 (m, 4H), 2.56-2.67 (m, 2H), 3.43-3.54 (m, 3H), 3.83 (s, 1H), 3.99-4.05 (m, 2H), 4.22-4.30 (m, 3H), 5.21-5.23 (m, 1H), 7.39 (s, 1H); ¹³C NMR (CDCl₃, 100 MHz): δ 14.03, 14.07, 19.91, 19.98, 20.84, 22.59, 22.65, 27.23, 27.28, 27.33, 27.38, 27.44, 29.11, 29.20, 29.27, 29.43, 29.56, 31.62, 31.65, 31.85, 32.21, 32.26, 32.28, 32.32, 32.43, 33.88, 34.41, 34.61, 38.29, 38.52, 45.62, 45.74, 61.95, 62.08, 62.80, 63.03, 68.70, 68.76, 73.27, 73.32, 74.70, 74.96, 169.66, 172.05, 173.54, 173.71.

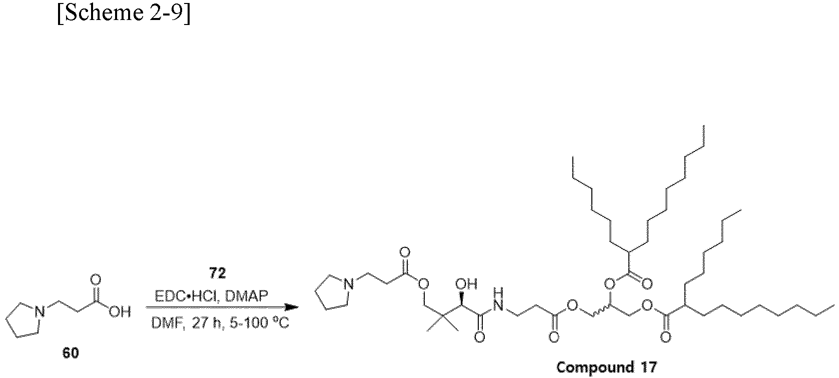

### 2-9. 3-((3-((R)-2-Hydroxy-3,3-dimethyl-4-((3-(pyrrolidin-1-yl)propanoyl)oxy)butanamido)propanoyl)oxy)propane-1,2-diyl bis(2-hexyldecanoate) (Compound 17)

After putting compound 60 (41 mg, 1.1 equiv.) into a reaction vessel and dissolving in DMF (20 mL), EDCI•HCl (75 mg, 1.5 equiv.) and 4-dimethylaminopyridine (DMAP) (6.3 mg, 0.2 equiv.) were added and stirred vigorously in the presence of argon gas at room temperature for 20 minutes. The compound 72 (200 mg, 1 equiv.) was put into another reaction vessel, dissolved in DMF (10 mL), and cooled to 5°C, and then the mixture solution of compound 60 was added dropwise at 5°C for 30 minutes, followed by stirring at 100°C for 27 minutes. After checking that the reaction was terminated with TLC (SiO₂, EtOAc/MeOH 1:1 and 9:1), the reaction mixture was distilled under reduced pressure to remove the solvent, DCM (50 mL) was added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, DCM/MeOH 10:0 → 9:1, 28% aqueous NH₃ solution added) to obtain compound 17 (151 mg, 65%) in a clear oil state.

¹H NMR (CDCl₃, 400 MHz): δ 0.77-0.82 (m, 16H), 1.15-1.22 (m, 46H), 1.34-1.39 (m, 4H), 1.48-1.51 (m, 4H), 1.72 (s, 4H), 2.25-2.27 (m, 2H), 2.44-2.57 (m, 8H), 2.67-2.68 (m, 1H), 2.81-2.91 (m, 1H), 3.43-3.48 (m, 3H), 3.83 (s, 1H), 4.01-4.06 (m, 2H), 4.22-4.34 (m, 3H), 5.20-5.23 (m, 1H), 7.3 (m, 1H); ¹³C NMR (CDCl₃, 100 MHz): δ 14.05, 14.09, 19.00, 22.60, 22.65, 22.85, 23.12, 27.26, 27.33, 27.35, 27.38, 27.43, 29.19, 29.23, 29.29, 29.44, 29.55, 29.58, 31.66, 31.85, 32.21, 32.27, 33.96, 34.28, 38.45, 38.48, 45.58, 45.63, 45.65, 52.72, 52.77, 54.12, 62.01, 62.69, 62.77, 68.64, 71.51, 71.55, 74.45, 74.51, 171.51, 171.55, 172.79, 175.53, 175.63, 175.65, 175.95, 175.98.

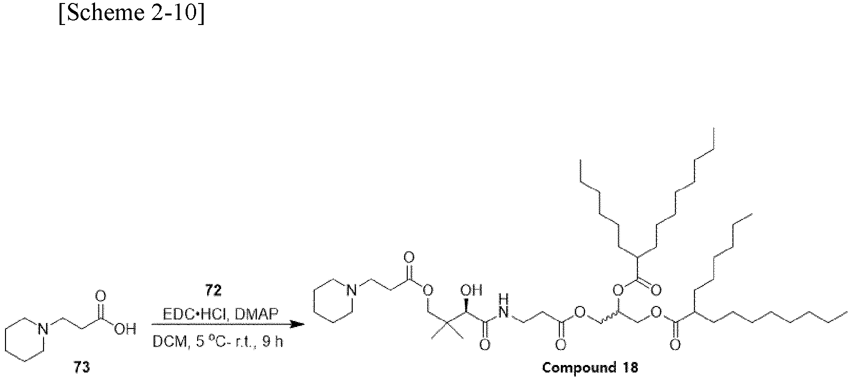

### 2-10. 3-((3-((R)-2-Hydroxy-3,3-dimethyl-4-((3-(piperidin-1-yl)propanoyl)oxy)butanamido)propanoyl)oxy)propane-1,2-diyl bis(2-hexyldecanoate) (Compound 18)

After putting 3-(piperidin-1-yl)propanoic acid (compound 73, 40.40 mg, 1.1 equiv.) into a reaction vessel and dissolving in DCM (20 mL), EDCI•HCl (67 mg, 1.5 equiv.) and 4-dimethylaminopyridine (DMAP) (5.7 mg, 0.2 equiv.) were added and stirred vigorously at room temperature for 20 minutes. The compound 72 (180 mg, 1 equiv.) was put into another reaction vessel, dissolved in DCM (10 mL), and cooled to 5°C. Thereafter, the mixture solution of compound 73 was added dropwise at 5°C for 30 minutes, followed by stirring at room temperature for 9 hours. After checking that the reaction was terminated with TLC (SiO₂, EtOAc/MeOH 1:1 and 9: 1), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the mixture was purified by flash column chromatography (SiO₂, DCM/MeOH 10:0 → 8:1, 28% aqueous NH₃ solution added) to obtain compound 18 (130 mg, 61%) in a clear liquid state.

¹H NMR (CDCl₃, 400 MHz): δ 0.77-0.82 (m, 15H), 0.14-1.24 (m, 43H), 1.34-1.39 (m, 6H), 1.50 (s, 8H), 2.24-2.52 (m, 10H), 2.71 (m, 1H), 3.42-3.49 (m, 3H), 3.83 (s, 1H), 4.02-4.06 (m, 2H), 4.24-4.35 (m, 2H), 4.37-4.38 (m, 1H), 5.20-5.22 (s, 1H), 7.30-7.34 (m, 1H); ¹³C NMR (CDCl₃, 100 MHz): δ 14.04, 14.08, 18.95, 19.98, 22.60, 22.65, 22.80, 22.84, 23.96, 25.00, 27.26, 27.30, 27.32, 27.35, 27.38, 27.43, 29.18, 29.22, 29.28, 29.43, 29.55, 29.58, 31.66, 31.85, 32.51, 32.27, 32.40, 32.97, 34.23, 38.48, 38.52, 45.58, 45.62, 45.64, 54.44, 55.23, 55.28, 62.00, 62.69, 62.44, 68.63, 71.27, 71.32, 74.20, 74.29, 171.52, 171.57, 172.24, 172.70, 175.56, 175.66, 175.94, 175.98.

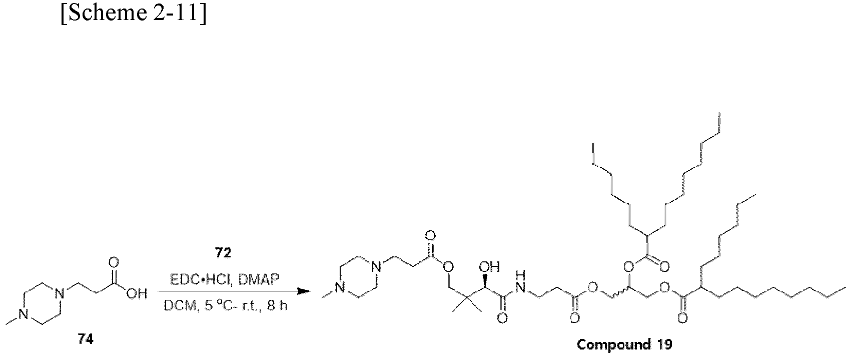

### 2-11. 3-((3-((R)-2-Hydroxy-3,3-dimethyl-4-((3-(4-methylpiperazin-1-yl)propanoyl)oxy)butanamido)propanoyl)oxy)propane-1,2-diyl bis(2-hexyldecanoate) (Compound 19)

After putting 3-(4-methylpiperazin-1-yl)propanoic acid (compound 74, 50 mg, 1.1 equiv.) into a reaction vessel and dissolving in DCM (20 mL), EDCI•HCl (75 mg, 1.5 equiv.) and 4-dimethylaminopyridine (DMAP) (6.3 mg, 0.2 equiv.) were added and stirred vigorously at room temperature for 20 minutes. The compound 72 (200 mg, 1 equiv.) was put into another reaction vessel, dissolved in DMF (10 mL), and cooled to 5°C, and the mixture solution of compound 74 was added dropwise at 5°C for 30 minutes, followed by stirring at room temperature for 8 hours. After checking that the reaction was terminated with TLC (SiO₂, EtOAc/MeOH 1:1 and 9:1, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, DCM/MeOH 10:0 → 9:1, 28% aqueous NH₃ solution added) to obtain compound 19 (60 mg, 67%) in a clear oil state.

¹H NMR (CDCl₃, 400 MHz): δ 0.86-0.90 (m, 15H), 1.75 (d, *J* = 1.4 Hz, 3H), 1.26-1.44 (m, 46H), 1.45-1.47 (m, 4H), 1.56-1.59 (m, 4H), 2.31-2.35 (m, 5H), 2.49-2.71 (m, 13H), 2.78-2.83 (m, 1H), 3.54-3.62 (m, 3H), 3.94 (s, 1H), 4.07-4.17 (m, 2H), 4.30-4.38 (m, 3H), 4.71 (s, 1H), 5.28-5.31 (m, 1H), 7.31 (s, 1H); ¹³C NMR (CDCl₃, 100 MHz): δ 14.05, 14.09, 19.09, 19.12, 22.34, 22.60, 22.65, 27.26, 27.32, 27.35, 27.38, 27.43, 29.18, 29.22, 29.28, 29.43, 29.54, 29.58, 31.66, 31.85, 32.21, 32.31, 32.42, 33.96, 34.30, 38.55, 38.61, 45.58, 45.65, 52.46, 54.20, 54.24, 54.36, 54.39, 62.00, 62.74, 62.80, 68.63, 71.03, 71.08, 74.12, 74.52, 171.58, 171.64, 172.41, 172.61, 175.59, 175.74, 175.98, 176.02.

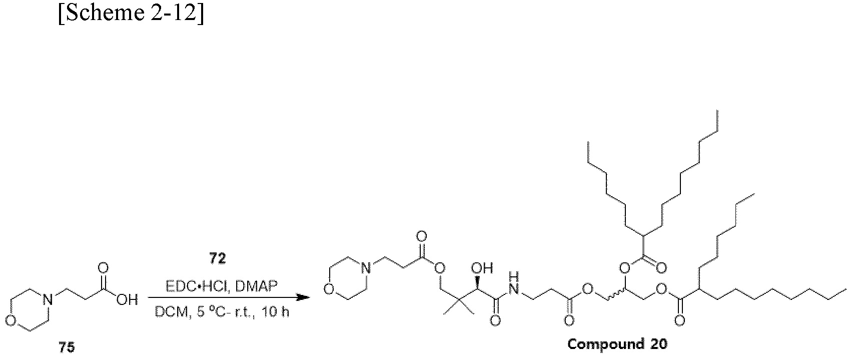

### 2-12. 3-((3-((R)-2-Hydroxy-3,3-dimethyl-4-((3-morpholinopropanoyl)oxy)butanamido)propanoyl)oxy)propane-1,2-diyl bis(2-hexyldecanoate) (Compound 20)

After putting 3-morpholinopropanoic acid (compound 75, 45.42 mg, 1.1 equiv.) into a reaction vessel and dissolving in DCM (20 mL), EDCI•HCl (75 mg, 1.5 equiv.) and 4-dimethylaminopyridine (DMAP) (6.3 mg, 0.2 equiv.) were added and stirred vigorously in the presence of argon gas at room temperature for 20 minutes. The compound 72 (200 mg, 1 equiv.) was put into another reaction vessel, dissolved in DMF (10 mL), and cooled to 5°C, and the mixture solution of compound 75 was added dropwise at 5°C for 30 minutes, followed by stirring at room temperature for 10 hours. After checking that the reaction was terminated with TLC (SiO₂, EtOAc/MeOH 1:1 and 9:1, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, DCM-MeOH 10:0 → 10:0.2, 28% aqueous NH₃ solution added) to obtain compound 20 (151 mg, 64%) in a clear liquid state.

¹H NMR (CDCl₃, 400 MHz): δ 0.79-0.82 (m, 15H), 1.09 (d, *J* = 2.48 Hz, 3H), 1.18-1.24 (m, 43H), 1.34-1.39 (m, 4H), 1.48-1.51 (m, 4H), 2.24-2.28 (m, 2H), 2.41-2.52 (m, 10H), 3.48-3.63 (m, 7H), 3.85 (d, J = 6 Hz, 1H), 4.00-4.04 (m, 2H), 4.23-4.37 (m, 4H), 5.23 (m, 1H), 7.19 (s, 1H); ¹³C NMR (CDCl₃, 100 MHz): δ 14.06, 14.10, 19.22, 19.27, 22.21, 22.61, 22.66, 27.34, 27.39, 24.44, 29.19, 29.24, 29.30, 29.45, 29.59, 31.68, 31.87, 32.22, 32.27, 33.93, 34.33, 34.37, 38.57, 38.65, 45.60, 45.66, 53.45, 54.62, 62.03, 62.80, 62.86, 66.22, 68.65, 71.04, 71.10, 74.42, 74.58, 171.64, 171.72, 172.17, 175.66, 175.84, 176.13, 176.08.

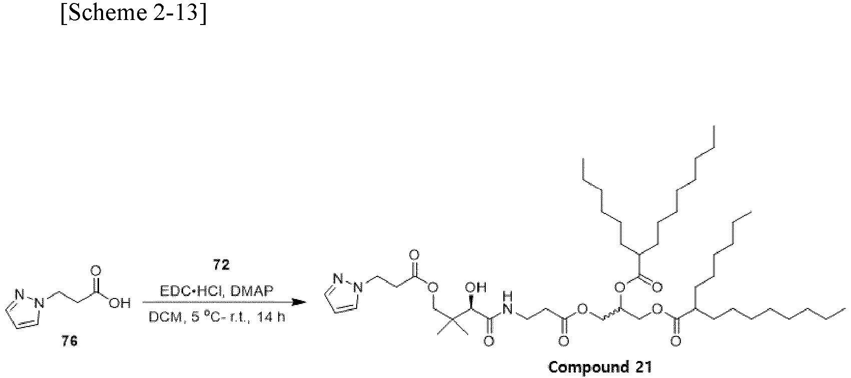

### 2-13. 3-((3-((R)-4-((3-(1H-Pyrazol-1-yl)propanoyl)oxy)-2-hydroxy-3,3-dimethylbutanamido)propanoyl)oxy)propane-1,2-diyl bis(2-hexyldecanoate) (Compound 21)

After putting 3-(1H-pyrazol-1-yl)propanoic acid (compound 76, 36.75 mg, 1.01 equiv.) into a reaction vessel and dissolving with DCM (20 mL), EDCI•HCl (79.65 mg, 1.6 equiv.) and 4-dimethylaminopyridine (DMAP) (6.34 mg, 0.2 equiv.) were added and stirred vigorously in the presence of argon gas at room temperature for 20 minutes. The compound 72 (200 mg, 1 equiv.) was put into another reaction vessel, dissolved in DCM (10 mL), and cooled to 5°C, and the mixture solution of compound 76 was added dropwise at 5°C for 30 minutes, followed by stirring at room temperature for 14 hours. After checking that the reaction was terminated with TLC (SiO₂, EtOAc/MeOH 1: 1 and 9: 1, PMA stain), the reaction mixture solution was distilled under reduced pressure to remove the solvent, DCM (50 mL) was added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture solution was purified by column chromatography (SiO₂, DCM/MeOH 10:0 → 9:1, 28% aqueous NH₃ solution added) to obtain compound 21 (127 mg, 55%) in a clear oil state.

¹H NMR (CDCl₃, 400 MHz): δ 0.78-0.80 (m, 15H), 0.82 (s, 3H), 1.05-1.18 (m, 42H), 1.22-1.34 (m, 4H), 1.36-1.39 (m, 4H), 2.25-2.28 (m, 4H), 2.41-2.56 (m, 3H), 3.44-3.48 (m, 3H), 3.84 (s, 1H), 4.01-4.06 (m, 2H), 4.22-4.35 (m, 3H), 5.20-5.23 (m, 1H), 7.40 (m, 1H), 7.42-7.75 (m, 3H); ¹³C NMR (CDCl₃, 100 MHz): δ 14.05, 14.09, 19.16, 19.23, 20.80, 21.26, 21.87, 22.60, 22.65, 27.26, 27.33, 27.38, 27.43, 29.12, 29.28, 29.44, 29.54, 29.58, 31.66, 31.86, 32.26, 33.62, 33.63, 34.35, 34.40, 34.87, 35.18, 35.88, 35.93, 37.35, 37.36, 38.38, 38.46, 47.15, 47.21, 47.77, 62.06, 62.75, 62.80, 68.65, 69.69, 71.24, 74.07, 74.25, 76.73, 77.04, 77.16, 77.36, 105.45, 105.81, 106.84, 129.64, 129.70, 129.73, 139.70, 129.73, 139.70, 167.69, 170.76, 171.61, 172.34, 175.61, 175.92.

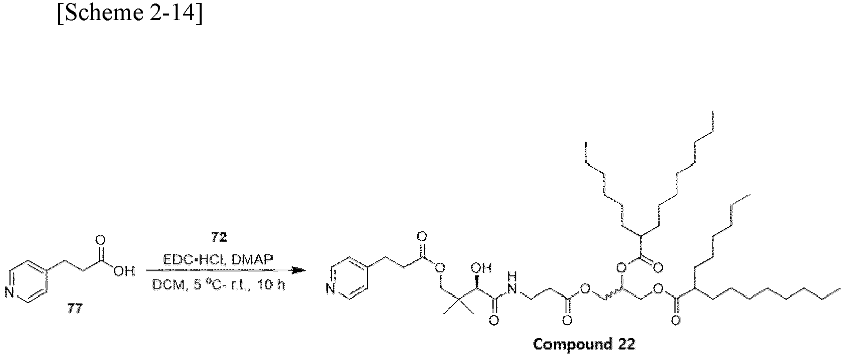

### 2-14. 3-((3-((R)-2-Hydroxy-3,3-dimethyl-4-((3-(pyridin-4-yl)propanoyl)oxy)butanamido)propanoyl)oxy)propane-1,2-diyl bis(2-hexyldecanoate) (Compound 22)

After putting 3-(pyridin-4-yl)propanoic acid (compound 77, 32.38 mg, 1.1 equiv.) into a reaction vessel and dissolving in DCM (20 mL), EDCI•HCl (60 mg, 1.6 equiv.) and 4-dimethylaminopyridine (DMAP) (4.75 mg, 0.2 equiv.) were added and stirred in the presence of argon gas at room temperature for 20 minutes. The compound 72 (150 mg, 1 equiv.) was put into another reaction vessel, dissolved in DCM (10 mL), and cooled to 5°C, and the mixture solution of compound 77 was added dropwise at 5°C for 30 minutes, followed by stirring at room temperature for 10 hours. After checking that the reaction was terminated with TLC (SiO₂, EtOAc/MeOH 1:1 and 9:1, PMA stain), the reaction mixture solution was distilled under reduced pressure to remove the solvent, DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture solution was purified by column chromatography (SiO₂, DCM/MeOH 10:0 → 9.5:0.1, 28% aqueous NH₃ solution added) to obtain compound 22 (121 mg, 69%) in a clear oil state.

¹H NMR (CDCl₃, 400 MHz): δ 0.78-0.84 (m, 15H),0.94 (s, 3H) 1.18-1.23 (m, 42H), 1.36-1.37 (m, 4H), 1.49-1.50 (m, 4H), 2.24-2.28 (m, 2H), 2.45-2.61 (m, 4H), 2.85-2.89 (t, *J* = 6 Hz, 2H), 3.40-3.52 (m, 2H), 3.77-3.82 (m, 2H), 3.95-4.12 (m, 4H), 4.21-4.32 (m, 2H), 5.20-5.23 (m, 1H), 7.00-7.20 (m, 3H), 8.42 (m, 2H); ¹³C NMR (CDCl₃, 100 MHz): δ 14.05, 14.09, 19.77, 19.83, 21.23, 22.60, 22.65, 27.25, 27.33, 27.38, 27.43, 29.18, 29.22, 29.28, 29.44, 29.55, 29.58, 30.06, 31.66, 32.21, 32.26, 32.37, 33.92, 34.31, 34.33, 34.39, 34.50, 38.40, 38.54, 45.60, 45.68, 61.97, 62.03, 62.80, 62.88, 68.66, 68.69, 70.59, 70.62, 74.68, 74.94, 123.69, 149.41, 149.80, 171.71, 171.84, 172.05, 172.09, 172.49, 175.75, 176.01, 176.22.

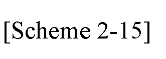

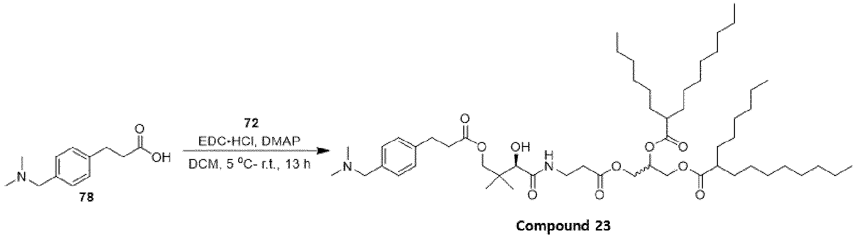

### 2-15. 3-((3-((R)-4-((3-(4-((Dimethylamino)methyl)phenyl)propanoyl)oxy)-2-hydroxy-3,3-dimethylbutanamido)propanoyl)oxy)propane-1,2-diyl bis(2-hexyldecanoate) (Compound 23)

After putting 3-(4-((dimethylamino)methyl)phenyl)propanoic acid (compound 78, 42.38 mg, 1.05 equiv.) into a reaction vessel and dissolving in DCM (20 mL), EDCI•HCl (59.73 mg, 1.6 equiv.) and 4-dimethylaminopyridine (DMAP) (4.75 mg, 0.2 equiv.) were added and stirred vigorously in the presence of argon gas at room temperature for 20 minutes. The compound 72 (150 mg, 1 equiv.) was put into another reaction vessel, dissolved in DCM (10 mL), and cooled to 5°C, and the mixture solution of compound 78 was added dropwise at 5°C for 30 minutes, followed by stirring at room temperature for 13 hours. After checking that the reaction was terminated with TLC (SiO₂, EtOAc/MeOH 1: 1 and 1:0, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, DCM 100%, 28% aqueous NH₃ solution added) to obtain compound 23 (127 mg, 68%) in a clear oil state.

¹H NMR (CDCl₃, 400 MHz): δ 0.78-0.82 (m, 15H), 0.91 (s, 3H), 1.18-1.22 (m, 41H), 1.36-1.37 (m, 4H), 1.38-1.49 (m, 4H), 2.16 (s, 6H), 2.21-2.27 (m, 2H), 2.44-2.57 (m, 3H), 2.91 (t, *J* = 6 Hz, 2H), 3.27-3.34 (m, 2H), 3.43-3.56 (m, 2H), 3.66 (d, *J* = 6 Hz, 1H), 3.71-3.81 (t, *J* = 8 Hz, 1H), 3.96-4.34 (m, 5H), 5.21-5.26 (m, 1H), 6.97-6.99 (m, 1H), 7.06-7.19 (m, 4H); ¹³C NMR (CDCl₃, 100 MHz): δ 14.07, 14.11, 19.75, 19.78, 21.61, 12.20, 22.61, 22.66, 27.32, 27.34, 27.39, 27.45, 29.20, 29.23, 29.29, 29.45, 29.59, 30.77, 31.67, 31.87, 32.23, 32.23, 32.37, 33.92, 34.37, 34.45, 35.95, 38.37, 38.50, 45.30, 45.61, 45.86, 61.98, 62.03, 62.81, 62.89, 63.98, 68.67, 70.40, 70.44, 74.44, 74.67, 128.17, 129.43, 136.60, 139.22, 171.71, 171.81, 172.12, 172.17, 173.32, 175.71, 175.99, 176.06, 176.16.

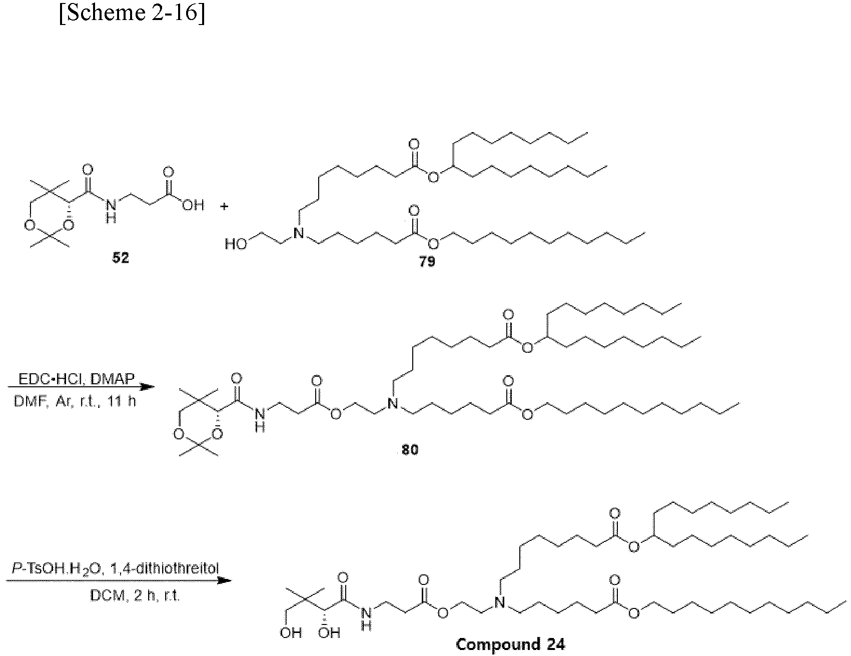

### 2-16-1. Heptadecan-9-yl (R)-8-((6-oxo-6-fundecyloxy)hexyl)(2-((3-(2,2,5,5-tetramethyl-1,3-dioxane-4-carboxamido)propanoyl)oxy)ethyl)amino)octanoate (80)

After dissolving carboxylic acid compound 52 (30 mg, 1.5 equiv.) in DCM (30 mL) in a reaction vessel, EDCI•HCl(18 mg, 1.5 equiv.) and DMAP (2 mg, 0.2 equiv.) were added and stirred vigorously in the presence of argon gas at 5°C for 10 minutes. Thereafter, the solution (10 mL) obtained by dissolving compound 79 (55 mg, 1 equiv.) in DCM was added dropwise at 5°C, followed by stirring at room temperature for 11 hours. After checking that the reaction was terminated with TLC (SiO₂, EtOAc/hexane 2:8), DCM (50 mL) was poured, and the mixture was transferred to a fractionation funnel, followed by washing with saturated aqueous NaHCO₃ solution (2 x 50 mL) and saturated aqueous NaCl solution (30 mL). The filtrate filtered by removing moisture from the separated organic layer with anhydrous MgSO₄ was distilled under reduced pressure and purified by column chromatography (SiO₂, EtOAc/hexane 8:2) to obtain compound 80 (50 mg, 68%) in a clear liquid state.

¹H NMR (CDCl₃, 400 MHz): δ 0.87 (t, *J* = 7.1 Hz, 9H), 0.96 (s, 3H), 1.03 (s, 3H), 1.20-1.35 (m, 48H), 1.35-1.55 (m, 15H), 1.55-3.73 (m, 7H), 2.26 (qui, *J* = 3.9, 4H), 2.30-2.44 (m, 4H), 2.54 (t, *J* = 4.7 Hz, 2H), 2.66 (t, *J* = 4.7 Hz, 2H), 3.25-3.28 (d, *J* = 5.7 Hz, 1H), 3.43-3.61 (m, 2H), 3.66-3.69 (d, *J* = 5.1 Hz, 1H), 4.02-4.11 (m, 3H), 4.12-4.14 (t, *J* = 4.9 Hz, 2H), 4.82-4.87 (qui, *J* = 4.7 Hz, 1H), 6.95 (s, 1H); ¹³C NMR (CDCl₃, 100 MHz): δ 14.07, 18.67, 18.82, 22.07, 22.63, 24.91, 25.09, 25.28, 25.90, 26.94, 27.16, 27.28, 28.63, 29.20, 29.22, 29.26, 29.30, 29.47, 29.50, 29.55, 31.83, 31.87, 32.94, 34.12, 34.19, 34.66, 52.14, 54.54, 54.74, 64.40, 71.46, 74.04, 77.13, 98.96, 169.66, 172.05, 173.54, 173.71.

### 2-16-2. Heptadecan-9-yl (R)-8-((2-((3-(2,4-dihydroxy-3,3-dimethylbutanamido)propanoyl)oxy)ethyl)(6-oxo-6-(undecyloxy)hexyl)amino)octanoate (Compound 24)

After dissolving the compound 80 (420 mg, 1 equiv.) and DL-1,4-dithiothreitol (DTT, 16 mg, 2 equiv.) in DCM by stirring in a reaction vessel, p-toluenesulfonic acid (4.5 mg, 0.5 equiv.) was added and stirred at room temperature for 2 hours. After checking that the reaction was terminated with TLC, the mixture was transferred to a fractionation funnel, extracted with EtOAc (30 mL x 2), and washed with saturated aqueous NaCl solution, and then the filtrate filtered by removing moisture from organic layer using anhydrous Na₂SO₄ was distilled under reduced pressure to remove the solvent and purified by column chromatography (SiO₂, EtOAc/hexane 1:1) to obtain a compound of Chemical Formula 24 (Compound 24, 21 mg, 44%) in a light-yellow oil form.

¹H NMR (CDCl₃, 400 MHz): δ 0.79-0.84 (m, 12H), 0.97 (s, 3H), 1.15-1.35 (m, 44H), 1.40-1.1.51 (m, 6H), 1.55-1.65 (m, 6H), 2.11 (qui, *J* = 3.3, 4H), 2.30 (m, 4H), 2.57 (m, 2H), 2.68 (t, *J* = 5.6 Hz, 2H), 3.27 (d, *J* = 4.2 Hz, 1H), 3.43 (m, 2H), 3.69 (d, *J* = 6.2 Hz, 1H), 4.11-4.12 (m, 3H), 4.12-4.14 (t, *J* = 5.0 Hz, 2H), 4.78 (qui, *J* = 4.7 Hz, 1H), 7.34 (s, 1H); ¹³C NMR (CDCl₃, 100 MHz): δ 14.08, 18.55, 18.84, 22.04, 22.65, 24.44, 25.45, 25.23, 25.44, 26.87, 27.22, 28.64, 28.91, 29.13, 29.17, 29.23, 29.26, 29.33, 29.50, 29.53, 29.53, 29.58, 26.60, 29.70, 31.86, 32.94, 34.33, 34.19, 34.67, 52.55, 54.56, 54.78, 64.42, 71.47, 74.05, 77.14, 98.95, 169.55, 171.99, 173.55, 173.61.

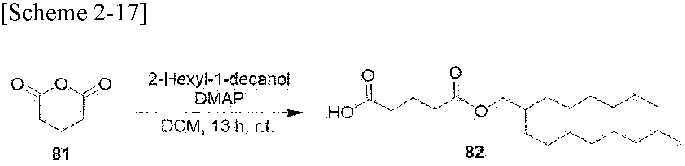

### 2-17. 5-((2-Hexyldecyl)oxy)-5-oxopentanoic acid (82)

After putting 2-hexyl-1-decanoic acid (500 mg, 1 equiv.) into a reaction vessel and dissolving in DCM (50 ml), glutaric anhydride (compound 81, 470 mg, 2 equiv.) and DMAP (630 mg, 2.5 equiv.) were added and stirred vigorously at room temperature for 13 hours. After checking that the reaction was terminated with TLC (SiO₂, EtOAc/Hexane 1:1, PMA stain), 2N aqueous HCl solution (10 ml) was added to the reaction mixture solution and then extracted with DCM (3 x 25 ml). All organic layers were collected, washed with the 2N aqueous HCl solution (2 x 20 ml) to remove residual bases, and then washed with saturated aqueous NaCl solution (30 ml). The filtrate filtered by drying the separated organic layer with anhydrous Na₂SO₄ was distilled under reduced pressure to remove the solvent, and the mixture was purified by flash column chromatography (SiO₂, EtOAc/hexane 1:9 → 2:8) to obtain compound 82 (603 mg, 82%) in a clear liquid state.

¹H NMR (CDCl₃, 400 MHz): δ 0.79-0.82 (t, *J* = 5.1 Hz, 6H), 1.19 (m, 25H), 1.54 (br. s, 1H), 1.87-1.92 (qui, *J* = 5.2 Hz, 2H), 2.31-2.38 (m, 4H), 3.92 (d, *J* = 7.2 Hz, 2H); ¹³C NMR (CDCl₃, 100 MHz): δ 14.07, 14.08, 19.86, 22.64, 22.67, 26.69, 29.30, 29.54, 29.60, 29.93, 31.25, 31.80, 31.89, 33.06, 33.25, 37.27, 67.39, 173.10, 179.25.

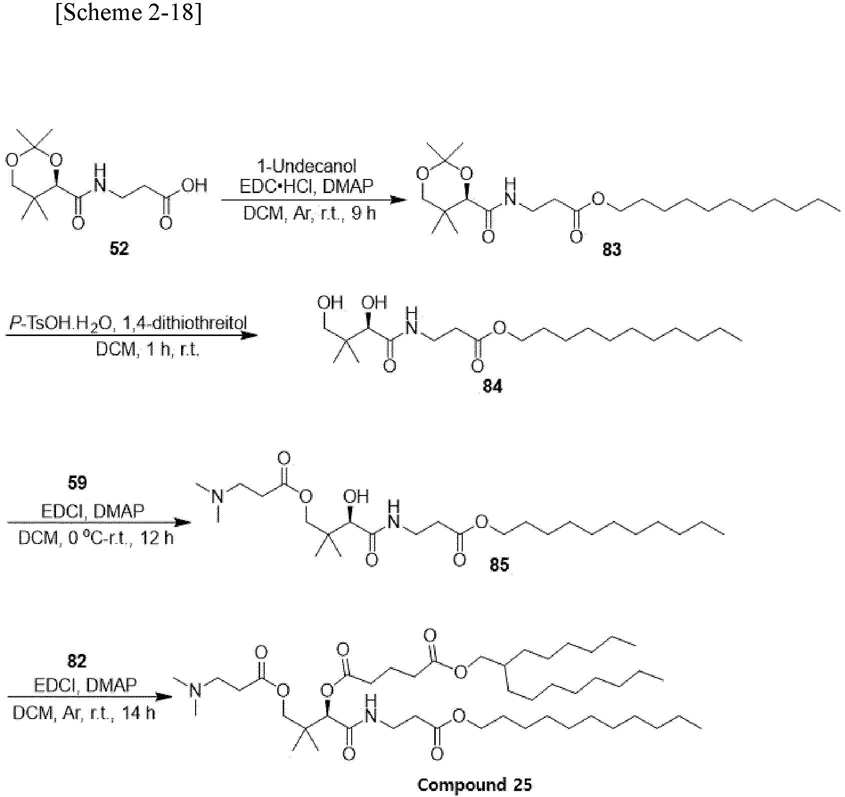

### 2-18-1. Undecyl(R)-33-(2,2,5,5-tetramethyl-1,3-dioxane-4-carboxamido)propanoate (83)

After putting acetal-protected pantothenic acid compound 52 (195 mg, 1.3 equiv.) into a reaction vessel and dissolving in DCM (30 mL), EDCI•HCl (167 mg, 1.5 equiv.) and 4-dimethylaminopyridine (DMAP) (14 mg, 0.2 equiv.) were added and stirred vigorously in the presence of argon gas at 5°C for 10 minutes. Thereafter, the solution obtained by dissolving undecan-1-ol (100 mg, 1 equiv.) in DCM (10 mL) was added dropwise to the reaction mixture at 5°C, followed by stirring at room temperature for 9 hours. After checking that the reaction was terminated with TLC (SiO₂, EtOAc/hexane 2:8, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the mixture was purified by flash column chromatography (SiO₂, EtOAc/hexane 1:9 → 2:8) to obtain compound 83 (206 mg, 86%) in a clear oil state.

¹H NMR (CDCl₃, 400 MHz): δ 0.79-0.82 *(t, J* = 5.6 Hz, 3H), 0.89 (s, 3H), 0.97 (s, 3H), 1.19-1.23 (m, 16H), 1.35 (s, 3H), 1.39 (s, 3H), 1.51-1.58 (qui, *J* = 6.6 Hz, 2H), 2.46-2.50 (m, 2H), 3.19-3.22 (d, *J* = 8.2 Hz, 1H), 3.37-3.56 (m, 2H), 3.60-3.62 (d, *J* = 8.7 Hz, 1H), 4.00-4.04 (m, 3H), 6.89 (s, 1H); ¹³C NMR (CDCl₃, 100 MHz): δ 14.09, 18.68, 18.81, 22.08, 22.66, 25.87, 28.59, 29.22, 29.30, 29.46, 29.48, 29.55, 29.57, 31.88, 32.96, 34.17, 34.24, 64.89, 71.49, 98.99, 169.68, 172.19.

### 2-18-2. Undegyl(R)-3-(2,4-dihydroxy-3,3-dimethylbutanamido)propanoate (84)

After dissolving the compound 83 (200 mg, 1 equiv.) and DL-1,4-dithiothreitol (DTT, 149 mg, 2 equiv.) in DCM, p-toluenesulfonic acid (84 mg, 0.5 equiv.) was added and stirred at room temperature for 1 hour. After the reaction was terminated, the mixture was extracted with EtOAc (30 mL x 2), and all the organic layers were collected and washed with saturated aqueous NaCl solution. The filtrate filtered by drying the separated organic layer with anhydrous Na₂SO₄ was distilled under reduced pressure to remove the solvent, and the mixture was purified by flash column chromatography (SiO₂, EtOAc/hexane 1:1 → 8:2) to obtain compound 84 (124 mg, 69%) in a clear oil state.

¹H NMR (CDCl₃, 400 MHz): δ 0.79-0.83 (m, 6H), 0.90 (s, 3H), 1.19-1.23 (m, 16H), 1.51-1.58 (qui, J = 6.7 Hz, 2H), 2.47-2.50 (t, *J* = 5.9 Hz, 2H), 3.40-3.53 (m, 4H), 3.39 (s, 1H), 3.99-4.03 (t, *J* = 8.7 Hz, 2H), 7.31 (m, 1H); ¹³C NMR (CDCl₃, 100 MHz): δ 14.08, 20.26, 21.04, 22.64, 25.87, 28.53, 29.22, 29.29, 29.48, 19.55, 29.56, 31.86, 34.03, 34.62, 39.22, 65.07, 71.09, 172.36, 173.59.

### 2-18-3. (R)-3-Hydroxy-2,2-dimethyl-4-oxo-4-((3-oxo-3-(undecyloxy)propyl)amino)butyl 3-(dimethylamino)propanoate (85)

After putting compound 59 (42.77 mg, 1.1 equiv.) into a reaction vessel and dissolving in DCM (20 mL), EDCI•HCl (102 mg, 1.6 equiv.) and 4-dimethylaminopyridine (DMAP) (8 mg, 0.2 equiv.) were added and stirred in the presence of argon gas at room temperature for 20 minutes. The compound 84 (124 mg, 1 equiv.) was put into another reaction vessel, dissolved in DCM (10 mL), and cooled to 5°C, and the mixture solution of compound 55 was added dropwise at 5°C for 30 minutes, followed by stirring at room temperature for 12 hours. After checking that the reaction was terminated with TLC (SiO₂, EtOAc/MeOH 1:1, PMA stain), the reaction mixture solution was distilled under reduced pressure to remove the solvent. Thereafter, DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the mixture was purified by flash column chromatography (SiO₂, DCM/MeOH 9:1, 28% aqueous NH₃ solution added) to obtain compound 85 (110 mg, 70%) in a clear oil state.

¹H NMR (CDCl₃, 400 MHz): δ 0.79-0.83 *(t, J* = 5.7 Hz, 3H), 0.89 (s, 3H), 0.92 (s, 3H), 1.19-1.23 (m, 16H), 1.35 (s, 3H), 1.39 (s, 3H), 1.51-1.58 (qui, *J* = 6.6 Hz, 2H), 2.16 (s, 6H), 2.46-2.50 (m, 6H), 3.19-3.22 (d, *J* = 8.2 Hz, 1H), 3.37-3.56 (m, 2H), 3.60-3.62 (d, *J* = 8.7 Hz, 1H), 4.00-4.04 (m, 3H), 6.89 (s, 1H); ¹³C NMR (CDCl₃, 100 MHz): δ 14.08, 18.69, 18.81, 22.08, 22.67, 25.87, 28.55, 29.23, 29.31, 29.46, 29.47, 29.54, 29.58, 31.88, 32.97, 34.18, 34.24, 64.88, 71.50, 98.98, 169.67, 172.18.

### 2-18-4. 2-Hexyldecyl ((R)-2,8,8-trimethyl-5,10,14-trioxo-6,15-dioxa-2,11-diazahexacosan-9-yl) glutarate (Compound 25)

After putting the compound 82 (98 mg, 1.3 equiv.) into a reaction vessel and dissolving in DCM (30 mL), EDCI•HCl (61 mg, 1.5 equiv.) and 4-dimethylaminopyridine (DMAP) (5 mg, 0.2 equiv.) were added and stirred vigorously in the presence of argon gas at 5°C for 10 minutes. Thereafter, the solution obtained by dissolving the compound 85 (100 mg, 1 equiv.) in DCM (10 mL) was added dropwise to the reaction mixture solution at 5°C, followed by stirring at room temperature for 14 hours. After checking that the reaction was terminated with TLC (SiO₂, EtOAc/hexane 7:3, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the mixture was purified by flash column chromatography (SiO₂, EtOAc/hexane 5:5 → 8:2) to obtain compound 25 (149 mg, 87%) in a clear oil state.

¹H NMR (CDCl₃, 400 MHz): δ 0.88-0.91 (t, *J* = 5.1 Hz, 9H), 1.07-1.08 (m, 6H), 1.25-1.40 (m, 42H), 1.59-1.64 (m, 3H), 1.97-2.02 (qui, *J* = 6.9 Hz, 2H), 2.39 (s, 6H), 2.41-2.48 (t, *J* = 6.1 Hz, 2H), 2.50-2.74 (m, 8H), 3.36-3.42 (m, 1H), 3.56-3.62 (m, 1H), 3.81-4.06 (m, 4H), 4.08-4.11 (t, *J* = 8.1 Hz, 2H), 4.88 (s, 1H), 7.44 (s, 1H); ¹³C NMR (CDCl₃, 100 MHz): δ 14.09, 14.11, 20.06, 20.27, 22.05, 22.64, 22.67, 25.91, 26.64, 26.69, 28.57, 29.31, 29.52, 29.58, 29.61, 29.95, 31.21, 31.81, 31.90, 33.07, 33.10, 33.26, 33.82, 35.04, 37.21, 37.28, 45.22, 55.13, 64.90, 67.34, 69.60, 76.63, 167.85, 171.72, 171.99, 172.34, 173.06.

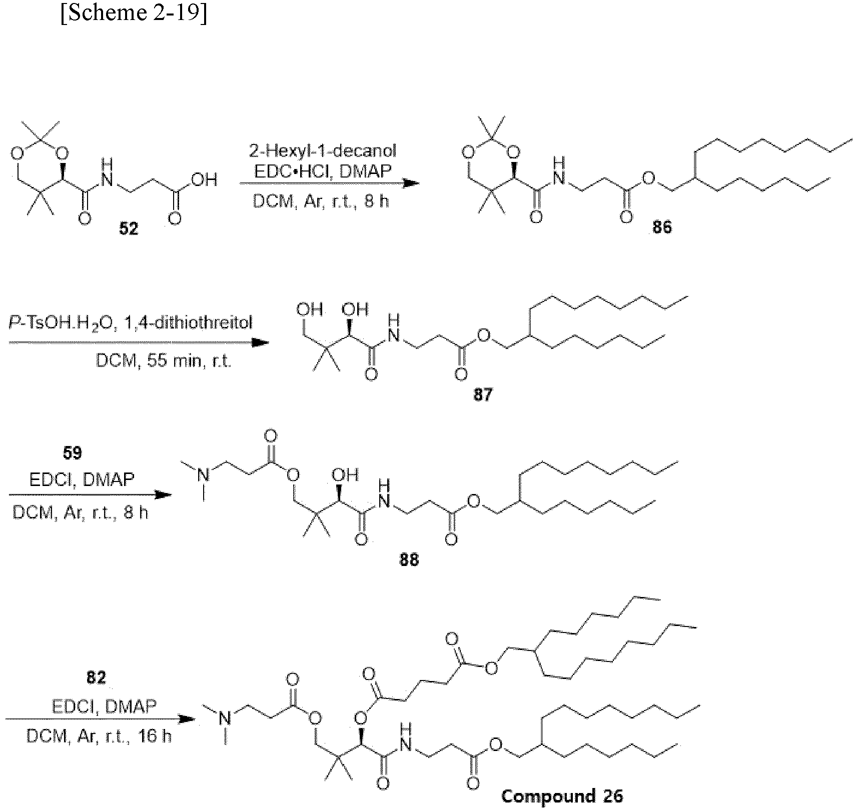

### 2-19-1. 2-Hexyldecyl 3-((R)-2,2,5,5-tetramethyl-1,3-dioxane-4-carboxamido)propanoate (86)

After putting acetal-protected pantothenic acid compound 52 (107 mg, 1.2 equiv.) into a reaction vessel and dissolving in DCM (30 mL), EDCI•HCl (119 mg, 1.5 equiv.) and 4-dimethylaminopyridine (DMAP) (10 mg, 0.2 equiv.) were added and stirred vigorously in the presence of argon gas at 5°C for 10 minutes. Thereafter, the solution obtained by dissolving 2-hexyldecan-1-ol (100 mg, 0.41mmol) in DCM (10 mL) was added dropwise at 5°C, followed by stirring at room temperature for 8 hours. After checking that the reaction was terminated with TLC (SiO₂, EtOAc/hexane 3:7, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, EtOAc/hexane 1:9 → 4:6) to obtain compound 86 (177 mg, 89%) in a clear oil state.

¹H NMR (CDCl₃, 400 MHz): δ 0.87-0.90 *(t, J* = 5.2 Hz, 3H), 0.97 (s, 3H), 1.05 (s, 3H), 1.27-1.32 (m, 25H), 1.43 (s, 3H), 1.47 (s, 3H), 1.62 (br. s, 1H), 2.55-2.58 (m, 2H), 3.27-3.30 (d, *J* = 9.2 Hz, 1H), 3.45-3.63 (m, 2H), 3.67-3.70 (d, *J* = 8.1 Hz, 1H), 4.00 (d, *J* = 9.8 Hz, 2H), 4.08 (s, 1H), 6.95-6.96 (m, 1H); ¹³C NMR (CDCl₃, 100 MHz): δ 14.07, 14.09, 18.68, 18.82, 22.09, 22.63, 22.66, 26.64, 26.67, 29.29, 29.46, 29.54, 29.58, 29.92, 31.20, 31.79, 31.87, 32.69, 34.14, 34.24, 37.27, 67.56, 71.49, 77.15, 98.99, 169.69, 172.30.

### 2-19-2. 2-Hexyldecyl 3-((R)-2,4-dihydroxy-3,3-dimethylbutanamido)propanoate (87)

After dissolving the compound 86 (177 mg, 1 equiv.) and DL-1,4-dithiothreitol (DTT, 122 mg, 2 equiv.) in DCM, p-toluenesulfonic acid (64 mg, 0.5 equiv.) was added and stirred at room temperature for 55 minutes. After the reaction was terminated, the mixture was extracted with EtOAc (2 x 30 mL), all the organic layers were collected and washed with saturated aqueous NaCl solution, the separated organic layers were dried with anhydrous Na₂SO₄, and the filtered filtrate was distilled under reduced pressure to remove the solvent. The remaining mixture was purified by flash column chromatography (SiO₂, EtOAc/hexane 1:1 → 7:3) to obtain compound 87 (105 mg, 65%) in a clear oil state.

¹H NMR (CDCl₃, 400 MHz): δ 0.79-0.83 (m, 9H), 0.92 (s, 3H), 1.19-1.23 (m, 25H), 2.47-2.51 (t, *J* = 6.2 Hz, 2H), 1.55 (br. s, 1H), 3.38-3.54 (m, 4H), 3.91-3.94 (m, 3H), 7.22-7.25 (s, 1H); ¹³C NMR (CDCl₃, 100 MHz): δ 14.05, 14.03, 22.05, 22.64, 22.63, 26.64, 26.63, 29.29, 29.44, 29.55, 29.58, 29.91, 31.20, 31.79, 31.86, 32.69, 34.14, 34.24, 37.27, 67.56, 71.49, 77.15, 98.99, 169.69, 172.30.

### 2-19-3. 2-Hexyldecyl 3-((R)-4-((3-(dimethylamino)propanoyl)oxy)-2-hydroxy-3,3-dimethylbutanamido)propanoate (88)

After putting compound 59 (29 mg, 1.1 equiv.) into a reaction vessel and dissolving in DCM (20 mL), EDCI•HCl (69 mg, 1.6 equiv.) and 4-dimethylaminopyridine (DMAP) (5 mg, 0.2 equiv.) were added and stirred vigorously in the presence of argon gas at 5°C for 20 minutes. The compound 87 (100 mg, 1 equiv.) was put into another reaction vessel, dissolved in DCM (10 mL), and cooled to 5°C, and the mixture solution of compound 59 was added dropwise at 5°C for 30 minutes, followed by stirring at room temperature for 8 hours. After checking that the reaction was terminated with TLC (SiO₂, EtOAc/MeOH 1:1, PMA stain), the reaction mixture was distilled under reduced pressure to remove the solvent, DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the obtained mixture was purified by flash column chromatography (SiO₂, DCM/MeOH 9:1/28% aqueous NH₃ solution) to obtain compound 88 (84 mg, 69%) in a clear oil state.

¹H NMR (CDCl₃, 400 MHz): δ 0.79-0.82 (m, 9H), 1.12 (s, 3H), 1.19-1.31 (m, 25H), 1.53 (br. s, 1H), 2.16 (s, 6H), 2.38-2.66 (m, 6H), 3.38-3.53 (m, 3H), 3.81 (s, 1H), 3.91 (d, *J* = 6.2 Hz, 2H), 4.23-4.26 (d, *J* = 8.2 Hz, 1H), 7.39-7.40 (s, 1H); ¹³C NMR (CDCl₃, 100 MHz): δ 14.08, 14.10, 19.31, 22.49, 22.63, 22.66, 26.64, 26.68, 29.29, 29.55, 29.58, 29.93, 31.18, 31.80, 31.88, 33.33, 34.22, 34.56, 37.25, 38.48, 37.25, 38.48, 45.05, 55.75, 67.56, 71.49, 74.47, 172.17, 172.24, 172.62.

### 2-19-4. (9R)-17-Hexyl-2,8,8-trimethyl-5,10,14-trioxo-6,15-dioxa-2,11-diazapentacosan-9-yl (2-hexyldecyl) glutarate (Compound 26)

After putting the compound 82 (71 mg, 1.3 equiv.) into a reaction vessel and dissolving in DCM (30 mL), EDCI•HCl (45 mg, 1.5 equiv.) and 4-dimethylaminopyridine (DMAP) (19 mg, 0.2 equiv.) were added and stirred vigorously in the presence of argon gas at 5°C for 10 minutes. Thereafter, the solution obtained by dissolving the compound 88 (84 mg, 1 equiv.) in DCM (10 mL) was added dropwise to the reaction mixture at 5°C, followed by stirring at room temperature for 16 hours. After checking that the reaction was terminated with TLC (SiO₂, EtOAc/hexane 5:5, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the mixture was purified by flash column chromatography (SiO₂, EtOAc/hexane 5:5 → 6:4) to obtain compound 26 (120 mg, 88%) in a clear oil state.

¹H NMR (CDCl₃, 400 MHz): δ 0.79-0.82 (m, 12H), 0.98-0.99 (m, 6H), 1.11-1.36 (m, 47H), 1.53 (br. s, 2H), 1.88-1.92 (qui, J = 7.3 Hz, 2H), 2.21 (s, 6H), 2.31 (t, *J* = 6.7 Hz, 2H), 2.41-2.65 (m, 8H), 3.27-3.32 (m, 1H), 3.46-3.53 (m, 1H), 3.72-3.75 (d, *J* = 9.3 Hz, 1H), 3.38-3.94 (m, 5H), 4.79 (s, 1H), 7.38 (s, 1H); ¹³C NMR (CDCl₃, 100 MHz): δ 14.09, 20.05, 20.27, 22.04, 22.64, 22.66, 26.64, 26.69, 23.30, 29.56, 29.60, 29.94, 31.16, 31.21, 31.81, 31.88, 33.09, 33.24, 33.78, 35.04, 37.20, 37.24, 37.27, 24.21, 55.13, 67.33, 67.57, 69.59, 76.61, 76.73, 77.05, 77.25, 167.84, 171.69, 191.97, 172.43, 173.04.

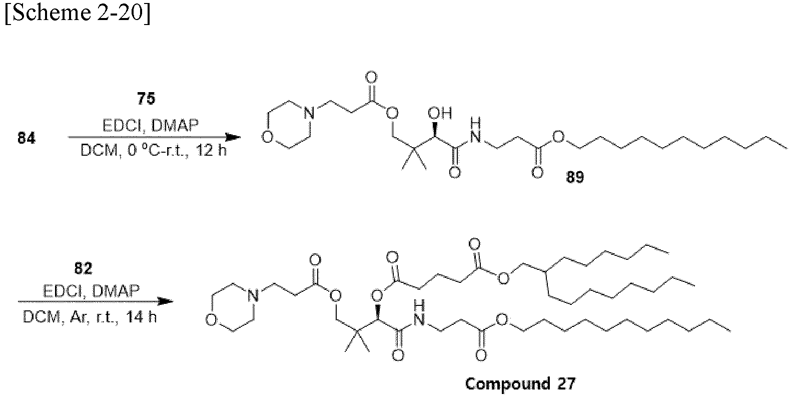

### 2-20-1. (R)-3-Hydroxy-2,2-dimethyl-4-oxo-4-((3-oxo-3-(undecyloxy)propyl)amino)butyl 3-morpholinopropanoate (89)

After putting compound 75 (140 mg, 1.1 equiv.) into a reaction vessel and dissolving in DCM (20 mL), EDCI•HCl (245 mg, 1.6 equiv.) and 4-dimethylaminopyridine (DMAP) (20 mg, 0.2 equiv.) were added and stirred vigorously in the presence of argon gas at room temperature for 20 minutes. The compound 84 (300 mg, 1 equiv.) was put into another reaction vessel, dissolved in DCM (10 mL), and cooled to 5°C, and the mixture solution of compound 75 was added dropwise at 5°C for 30 minutes, followed by stirring at room temperature for 10 hours. After checking that the reaction was terminated with TLC (SiO₂, EtOAc/MeOH 1:1, PMA stain), the reaction mixture solution was distilled under reduced pressure to remove the solvent, DCM (50 mL) was further added, and the mixture was transferred to a fractionation funnel, followed by washing with saturated aqueous NaHCO₃ solution (2 x 50 mL) and saturated aqueous NaCl solution (20 mL). The filtrate filtered by drying the separated organic layer with anhydrous MgSO₄ was distilled under reduced pressure to remove the solvent, and the remaining mixture was purified by flash column chromatography (SiO₂, DCM/MeOH 7:3, 28% aqueous NH₃ solution added) to obtain compound 89 (300 mg, 73%) in a clear oil state.

¹H NMR (CDCl₃, 400 MHz): δ 0.78-0.85 *(t, J* = 5.8 Hz, 3H), 0.90 (s, 3H), 0.91 (s, 3H), 1.20-1.25 (m, 16H), 1.36 (s, 3H), 1.40 (s, 3H), 1.52-1.59 (qui, J = 6.8 Hz, 2H), 2.18 (s, 4H), 2.47-2.55 (m, 6H), 3.20-3.44 (d, *J* = 8.4 Hz, 1H), 3.38-3.60 (m, 2H), 3.61-3.65 (d, *J* = 8.9 Hz, 5H), 4.11-4.14 (m, 3H), 6.92 (s, 1H); ¹³C NMR (CDCl₃, 100 MHz): δ 14.09, 18.70, 18.84, 22.09, 22.64, 25.89, 28.54, 29.22, 29.32, 29.47, 29.49, 29.57, 29.59, 31.87, 32.95, 34.17, 34.23, 64.84, 71.55, 98.94, 169.63, 172.13.

### 2-20-2. (R)-3,3-Dimethyl-4-((3-morpholinopropanoyl)oxy)-1-oxo-1-((3-oxo-3-(undecyloxy)propyl)amino)butan-2-yl (2-hexyldecyl) glutarate (Compound 27)

After putting the compound 82 (270 mg, 1.3 equiv.) into a reaction vessel and dissolving in DCM (30 mL), EDCI•HCl (167 mg, 1.5 equiv.) and 4-dimethylaminopyridine (DMAP) (14 mg, 0.2 equiv.) were added and stirred vigorously in the presence of argon gas at 5 °C for 10 minutes. Thereafter, the solution obtained by dissolving the compound 89 (300 mg, 1 equiv.) in DCM (10 mL) was added dropwise to the reaction mixture at 5°C, followed by stirring at room temperature for 11 hours. After checking that the reaction was terminated with TLC (SiO₂, EtOAc/hexane 4:6, PMA stain), DCM (100 mL) was further added, followed by washing with saturated aqueous NaHCO₃ solution (2 x 100 mL) and saturated aqueous NaCl solution (50 mL). The filtrate filtered by drying the separated organic layer with anhydrous MgSO₄ was distilled under reduced pressure to remove the solvent, and the remaining mixture was purified by flash column chromatography (SiO₂, EtOAc/hexane 4:6 → 6:4) to obtain compound 27 (433 mg, 87%) in a clear oil state.

¹H NMR (CDCl₃, 400 MHz): δ 0.88-0.92 (t, *J* = 5.5 Hz, 9H), 1.07-1.09 (m, 6H), 1.25-1.43 (m, 42H), 1.59-1.67 (m, 3H), 1.97-2.06 (qui, J = 6.5 Hz, 2H), 2.30-2.34 (t, *J* = 8.3 Hz, 2H), 2.42-2.64 (m, *J* = 6.5 Hz, 12H), 3.38-3.58 (m, 2H), 3.38-3.65 (t, *J* = 6.6 Hz, 4H), 3.85-3.97 (m, 6H), 4.89 (s, 1H), 7.45 (s, 1H); ¹³C NMR (CDCl₃, 100 MHz): δ 14.10, 14.12, 20.07, 20.28, 22.04, 22.68, 22.65, 25.95, 26.64, 26.66, 28.54, 29.34, 29.55, 29.55, 29.62, 29.97, 31.22, 31.84, 31.92, 33.08, 33.12, 33.27, 33.84, 35.07, 37.28, 37.29, 53.49, 54.07, 64.95, 67.37, 69.67, 76.68, 167.88, 171.77, 171.95, 172.35, 173.03.

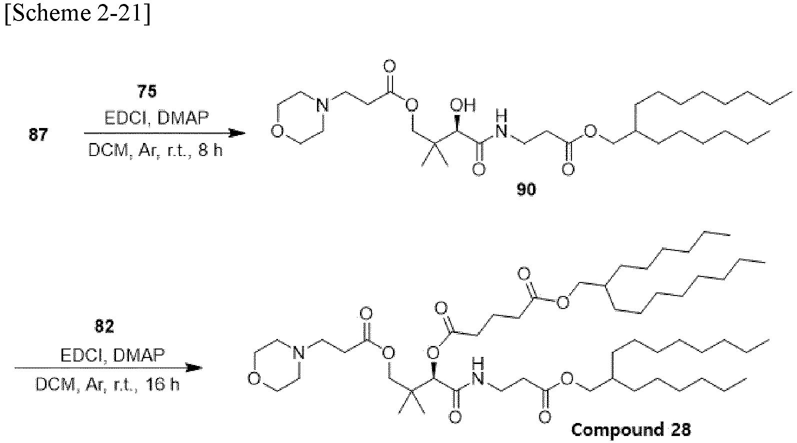

### 2-21-1. 2-Hexyldecyl 3-((R)-2-hydroxy-3,3-dimethyl-4-((3-morpholinopropanoyl)oxy)butanamido)propanoate (90)

After putting compound 75 (237 mg, 1.1 equiv.) into a reaction vessel and dissolving in DCM (20 mL), EDCI•HCl (414 mg, 1.6 equiv.) and 4-dimethylaminopyridine (DMAP) (33 mg, 0.2 equiv.) were added and stirred in the presence of argon gas at room temperature for 20 minutes. The compound 87 (600 mg, 1 equiv.) was put into another reaction vessel, dissolved in DCM (10 mL), and cooled to 5°C, and the mixture solution of compound 75 was added dropwise at 5°C, followed by stirring at room temperature for 7 hours. After checking that the reaction was terminated with TLC (SiO₂, EtOAc/MeOH 9.5:0.5, PMA stain), the reaction mixture solution was distilled under reduced pressure, DCM (50 mL) was further added, and the mixture was transferred to a fractionation funnel, followed by washing with saturated aqueous NaHCO₃ solution (2 x 100 mL) and saturated aqueous NaCl solution (100 mL). The filtrate filtered by drying the separated organic layer with anhydrous MgSO₄ was distilled under reduced pressure to remove the solvent, and the remaining mixture was purified by flash column chromatography (SiO₂, DCM/MeOH 9:1, 28% aqueous NH₃ solution added) to obtain compound 90 (609 mg, 77%) in a clear oil state.

¹H NMR (CDCl₃, 400 MHz): δ 0.80-0.83 (m, 6H), 0.95 (s, 3H), 0.97 (s, 3H), 1.20-1.24 (m, 25H), 1.55 (br. s, 1H), 2.30 (t, *J* = 8.2 Hz, 2H), 2.33 (t, *J* = 8.2 Hz, 2H), 2.41-2.55 (m, *J* = 6.9 Hz, 6H), 3.37-3.52 (m, 2H), 3.36-3.63 (t, *J* = 6.5 Hz, 4H), 3.44-3.96 (m, 4H), 4.93 (s, 1H), 7.21 (s, 1H); ¹³C NMR (CDCl₃, 100 MHz): δ 14.07, 14.10, 20.01, 21.02, 21.13, 22.74, 22.68, 26.66, 26.63, 29.32, 29.54, 29.60, 29.61, 29.94, 29.96, 31.22, 31.80, 31.88, 32.07, 33.18, 34.64, 37.25, 37.30, 37.44, 53.46, 54.03, 66.75, 67.75, 69.11, 69.29, 76.57, 167.88, 171.55, 171.90.

### 2-21-2. 2-Hexyldecyl ((2R)-1-((3-((2-hexyldecyl)oxy)-3-oxopropyl)amino)-3,3-dimethyl-4-((3-morpholinopropanoyl)oxy)-1-oxobutan-2-yl) glutarate (Compound 28)

After putting the compound 82 (474 mg, 1.3 equiv.) into a reaction vessel and dissolving in DCM (30 mL), EDCI•HCl (294 mg, 1.5 equiv.) and 4-dimethylaminopyridine (DMAP) (25 mg, 0.2 equiv.) were added and stirred vigorously in the presence of argon gas at 5 °C for 10 minutes. Thereafter, the solution obtained by dissolving the compound 90 (600 mg, 1 equiv.) in DCM (10 mL) was added dropwise into the reaction mixture at 5°C, followed by stirring at room temperature for 12 hours. After checking that the reaction was terminated with TLC (SiO₂, EtOAc/hexane 5:5, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL). The filtrate filtered by drying the separated organic layer with anhydrous MgSO₄ was distilled under reduced pressure to remove the solvent, and the remaining mixture was purified by flash column chromatography (SiO₂, EtOAc/hexane 3:7 → 5:5) to obtain compound 28 (862 mg, 91%) in a clear oil state.

¹H NMR (CDCl₃, 400 MHz): δ 0.79-0.82 (m, 12H), 0.94 (s, 3H), 0.99 (s, 3H), 1.19-1.23 (m, 49H), 1.54 (br. s, 2H), 1.88-1.92 (qui, J = 7.6 Hz, 2H), 2.29-2.33 (t, *J* = 8.2 Hz, 2H), 2.40-2.63 (m, *J* = 6.9 Hz, 12H), 3.37-3.51 (m, 2H), 3.36-3.64 (t, *J* = 6.5 Hz, 4H), 3.84-3.96 (m, 6H), 4.91 (s, 1H), 7.20 (s, 1H); ¹³C NMR (CDCl₃, 100 MHz): δ 14.08, 14.09, 20.02, 21.02, 21.10, 22.64, 22.64, 26.64, 26.69, 29.30, 29.55, 29.59, 29.60, 29.93, 29.95, 31.17, 31.21, 31.80, 31.88, 32.07, 33.18, 33.60, 34.64, 37.25, 37.29, 37.44, 53.45, 54.04, 66.79, 67.39, 67.71, 69.26, 69.26, 76.56, 167.89, 171.60, 171.88, 172.79, 172.97.

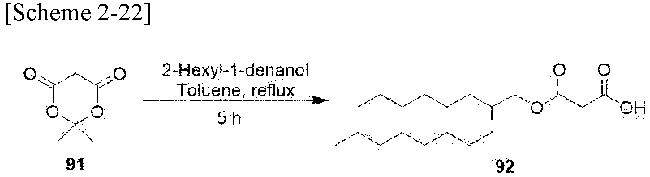

### 2-22. 3-((2-Hexyldecyl)oxy)-3-oxopropanoic acid (92)

Meldrum's acid (compound 91, 500 mg, 1 equiv.) and 2-hexyl-1-decanol (841 mg, 1 equiv.) were put into a reaction vessel in the same amount, dissolved in toluene (50 mL), and heated by reflux for 5 hours. After checking that the reaction was terminated with TLC (SiO₂, EtOAc/hexane 5:5), the reaction mixture was cooled to room temperature and then to 4°C again. Saturated aqueous NaHCO₃ solution was put into the reaction mixture, and 3M aqueous HCl solution was added to neutralize the mixture. Thereafter, the reaction mixture solution was placed in a fractionation funnel to extract with EtOAc (2 x 100 mL), and all organic layers were collected, followed by washing with saturated aqueous NaCl solution (1 x 100 mL). The filtrate filtered by drying the separated organic layer with anhydrous Na₂SO₄ was distilled under reduced pressure to remove the solvent, and the remaining mixture solution was purified by column chromatography (SiO₂, EtOAc/hexane 5:5, 1% AcOH added) to obtain compound 92 (1103 mg, 97%) in a clear oil state.

¹H NMR (CDCl₃, 400 MHz): δ 0.87-0.92 (m, 6H), 1.30 (m, 26H), 1.68 (s, 1H), 3.44-3.47 (m, 2H), 4.08-4.13 (m, 2H), 10.68 (br. s, 1H); ¹³C NMR (CDCl₃, 100 MHz): δ 14.09, 22.66, 22.69, 26.61, 26.66, 29.32, 29.56, 29.93, 31.10, 31.10, 31.89, 31.91, 37.22, 40.80, 68.79, 167.04, 171.69.

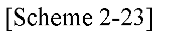

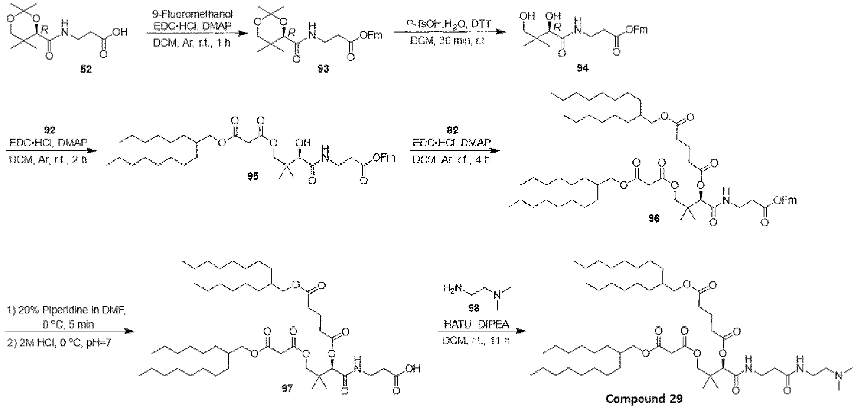

### 2-23-1. (9H-Fluoren-9-yl)methyl (R)-3-(2,2,5,5-tetramethyl-1,3-dioxane-4-carboxamido)propanoate (93)

After putting acetal-protected pantothenic acid compound 52 (476 mg, 1.2 equiv.) into a reaction vessel and dissolving in DCM (30 mL), EDCI•HCl (438 mg, 1.5 equiv.) and 4-dimethylaminopyridine (DMAP) (38 mg, 0.2 equiv.) were added and stirred vigorously in the presence of argon gas at 5°C for 10 minutes. Thereafter, the solution obtained by dissolving 9-fluorenemethanol (300 mg, 1 equiv.) in DCM (10 mL) was added dropwise into the reaction mixture at 5°C, followed by stirring at room temperature for 1 hour. After checking that the reaction was terminated with TLC (SiO₂, EtOAc/hexane 1:9, PMA stain), DCM (50 mL) was further added, and the mixture was transferred to a fractionation funnel, followed by washing with saturated aqueous NaHCO₃ solution (2 x 50 mL) and saturated aqueous NaCl solution (20 mL) sequentially. The filtrate filtered by drying the separated organic layer with anhydrous MgSO4 was distilled under reduced pressure to remove the solvent, and the obtained mixture was purified by flash column chromatography (SiO₂, EtOAc/hexane 1:9) to obtain compound 93 (541 mg, 81%) in a clear oil state.

¹H NMR (CDCl₃, 400 MHz): δ 0.86 (s, 3H), 0.94 (s, 3H), 1.27 (s, 3H), 1.31 (s, 3H), 2.54-2.57 (t, *J* = 8.2 Hz, 2H), 3.14-3.17 (d, *J* = 5.1 Hz, 1H), 3.38-3.52 (m, 2H), 3.54-3.57 (d, *J* = 5.5 Hz, 1H), 3.98 (s, 1H), 4.07-4.10 (t, *J* = 8.3 Hz, 1H), 4.27-4.29 (d, *J* = 7.1 Hz, 2H), 6.87-6.90 (s, 1H), 7.16-7.18 (t, *J* = 5.1 Hz, 2H), 7.20-7.27 (t, *J* = 5.5 Hz, 2H), 7.29-7.46 (d, *J* = 5.0 Hz, 2H), 7.47-7.66 (d, *J* = 4.8 Hz, 2H); ¹³C NMR (CDCl₃, 100 MHz): δ 14.23, 18.67, 18.89, 21.03, 22.14, 29.45, 32.79, 32.98, 34.21, 34.25, 46.72, 46.92, 60.36, 66.66, 71.45, 76.87, 77.19, 77.50, 99.02, 119.78, 120.09, 120.35, 124.69, 125.01, 127.16, 127.87, 141.29, 143.64, 169.82, 171.06.

### 2-23-2. (9H-Fluoren-9-yl)methyl (R)-3-(2,4-dihydroxy-3,3-dimethylbutanamido)propanoate (94)

After dissolving the compound 93 (500 mg, 1 equiv.) and DL-1,4-dithiothreitol (DTT, 352 mg, 2 equiv.) in DCM, and *p*-toluenesulfonic acid (98 mg, 0.5 equiv.) was added at room temperature, followed by stirring for 15 minutes. When the reaction was terminated, the reaction mixture solution was extracted with EtOAc (30 mL x 2), and all the organic layers were collected and washed with saturated aqueous NaCl solution. The filtrate filtered by drying the separated organic layer with anhydrous Na₂SO₄ was distilled under reduced pressure to remove the solvent, and the obtained mixture was purified by flash column chromatography (SiO₂, EtOAc/hexane 1:1 → 7:3) to obtain compound 94 (277 mg, 61%) in a clear oil state.

¹H NMR (CDCl₃, 400 MHz): δ 0.90 (s, 3H), 0.97 (s, 3H), 2.61-2.64 (t, *J* = 8.1 Hz, 2H), 3.47-3.58 (m, 4H), 4.00 (s, 1H), 4.18-4.21 (t, *J* = 5.1 Hz, 1H), 4.40-4.42 (d, 2H), 7.27-7.30 (m, 3H), 7.32-7.34 (t, *J* = 5.5 Hz, 2H), 7.39-7.42 (d, *J* = 5.0 Hz, 2H), 7.56-7.77 (d, *J* = 4.8 Hz, 2H); ¹³C NMR (CDCl₃, 100 MHz): δ 14.19, 20.34, 21.05, 21.31, 34.06, 34.63, 39.26, 46.71, 53.47, 60.47, 66.62, 71.15, 76.82, 77.14, 77.45, 77.48, 120.09, 124.95, 127.18, 127.89, 141.29, 143.59, 171.33, 172.17, 173.54.

### 2-23-3. 4-((3-((9H-Fluoren-9-yl)methoxy)-3-oxopropyl)amino)-3-hydroxy-2,2-dimethyl-4-oxobutyl (2-hexyldecyl) malonate (95)

After putting the compound 92 (181 mg, 1.1 equiv.) into a reaction vessel and dissolving in DCM (20 mL), EDCI•HCl (154 mg, 1.6 equiv.) and 4-dimethylaminopyridine (DMAP) (13 mg, 0.2 equiv.) were added and stirred in the presence of argon gas at room temperature for 20 minutes. The compound 94 (200 mg, 1 equiv.) was put into another reaction vessel, dissolved in DCM (10 mL), and cooled to 5°C, and the mixture of compound 92 was added dropwise in the presence of argon gas at 5°C for 30 minutes, followed by stirring at room temperature for 2 hours. After checking that the reaction was terminated with TLC (SiO₂, EtOAc/hexane 2:8, PMA stain), the mixture was distilled under reduced pressure to remove the solvent, dissolved in DCM (50 mL) again, and transferred to a fractionation funnel, followed by washing with saturated aqueous NaHCO₃ solution (2 x 100 mL) and saturated aqueous NaCl solution (100 mL) sequentially. The filtrate filtered by drying the separated organic layer with anhydrous MgSO₄ was distilled under reduced pressure to remove the solvent, and the obtained mixture was purified by flash column chromatography (SiO₂, DCM/hexane 1:9 → 1:1) to obtain compound 95 (256 mg, 72%) in a clear oil state.

¹H NMR (CDCl₃, 400 MHz): δ 0.78-0.81 (t, *J* = 8.5 Hz, 6H), 0.95-0.95 (d, *J =* 8.9 Hz, 6H), 1.15-1.28 (m, 41H), 1.54 (s, 1H), 2.52-2.53 (t, *J* = 5.7 Hz, 2H), 3.32 (s, 2H), 3.35-3.51 (m, 2H), 3.83-3.99 (m, 4H), 4.12-4.26 (t, *J* = 5.0 Hz, 1H), 4.22-4.34 (m, 2H), 4.81 (s, 1H); ¹³C NMR (CDCl₃, 100 MHz): δ 14.10, 20.03, 20.94, 21.32, 22.63, 22.65, 26.61, 26.63, 26.68, 29.32, 29.56, 29.60, 29.61, 29.93, 29.96, 31.08, 31.21, 31.82, 31.89, 33.01, 33.18, 33.66, 34.75, 37.20, 37.26, 37.32, 41.37, 46.70, 66.70, 67.39, 68.61, 70.34, 76.79, 77.11, 77.31, 77.42, 120.05, 125.01, 125.03, 127.15, 129.84, 141.28, 143.62, 143.72, 166.13, 166.95, 167.84, 171.73.

### 2-23-4. 1-(9H-Fluoren-9-yl)-17-hexyl-9,9-dimethyl-3,7,12,14-tetraoxo-2,11,15-trioxa-6-azapentacosan-8-yl (2-hexyldecyl) glutarate (96)

After putting the compound 82 (164 mg, 1.3 equiv.) into a reaction vessel and dissolving in DCM (30 mL), EDCI•HCl (101 mg, 1.5 equiv.) and 4-dimethylaminopyridine (DMAP) (7 mg, 0.2 equiv.) were added and stirred vigorously in the presence of argon gas at 5°C. Thereafter, the solution obtained by dissolving the compound 95 (250 mg, 1 equiv.) in DCM (10 mL) was added dropwise into the reaction mixture at 5°C, followed by stirring at room temperature for 4 hours. After checking that the reaction was terminated with TLC (SiO₂, EtOAc/hexane 1:9, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/hexane 1:9) to obtain compound 96 (300 mg, 81%) in a clear oil state.

¹H NMR (CDCl₃, 400 MHz): δ 0.78-0.81 (t, *J* = 8.5 Hz, 12H), 0.95-0.97 (d, *J* = 8.9 Hz, 6H), 1.15-1.27 (m, 53H), 1.53 (s, 2H), (qui, *J* = 5.1 Hz, 2H), 2.26-2.30 (t, *J* = 5.5 Hz, 2H), 2.37-2.40 (t, *J* = 5.1 Hz, 2H), 2.53-2.56 (t, *J* = 5.7 Hz, 2H), 3.32 (s, 2H), 3.35-3.52 (m, 2H), 3.83-3.99 (m, 6H), 4.10-4.26 (t, *J* = 5.0 Hz, 1H), 4.29-4.34 (m, 2H), 4.82 (s, 1H); ¹³C NMR (CDCl₃, 100 MHz): δ 14.09, 14.11, 20.01, 20.92, 21.30, 22.65, 22.67, 26.60, 26.64, 26.69, 29.31, 29.56, 29.59, 29.61, 29.93, 29.95, 31.07, 31.21, 31.81, 31.89, 33.02, 33.18, 33.67, 34.75, 37.20, 37.28, 37.32, 41.36, 46.70, 66.70, 67.35, 68.61, 70.34, 76.79, 77.11, 77.31, 77.42, 120.05, 125.01, 125.03, 127.15, 129.84, 141.28, 143.62, 143.70, 166.19, 166.98, 167.85, 171.72, 172.30, 173.01.

### 2-23-5. 3-(4-((3-((2-Hexyldecyl)oxy)-3-oxopropanoyl)oxy)-2-((5-((2-hexyldecyl)oxy)-5-oxopentanoyl)oxy)-3,3-dimethylbutanamido)propanoic acid (97)

Piperidine and DMF were mixed in a volume ratio of 2:8 in a reaction vessel and then cooled to 0°C to prepare a 20% piperidine solution. Compound 96 (300 mg) was put into another reaction vessel, dissolved in DMF (20 ml), and stirred at 0°C for 5 minutes. Thereafter, the prepared 20% piperidine solution (40 ml) was added in the presence of argon gas and stirred at 0°C for 5 minutes. After the reaction was terminated, 3M aqueous HCl solution was added, the pH was adjusted to 7, dilution was performed using EtOAc (200 mL), and the mixture was transferred to a fractionation funnel, followed by washing with distilled water (2 x 200 mL) and saturated aqueous NaCl solution (2 x 200 mL) sequentially. The filtrate filtered by drying the separated organic layer with anhydrous Na₂SO₄ was distilled under reduced pressure to remove the solvent, and the obtained mixture was purified by flash column chromatography (SiO₂, EtOAc/hexane 0:1 → 4:6, 1% AcOH added) to obtain compound 97 (226 mg, 91%) in a clear oil state.

¹H NMR (CDCl₃, 400 MHz): δ 0.82 (t, *J* = 8.1 Hz, 12H), 0.97-1.00 (d, *J* = 8.3 Hz, 6H), 1.15-1.29 (m, 50H), 1.56 (s, 2H), 1.85-1.93 (m, 2H), 2.30-2.55 (m, 6H), 3.28-3.38 (m, 3H), 3.55-3.63 (m, 1H), 3.85-4.01 (m, 6H), 4.87 (s, 1H), 6.76 (t, *J* = 5.7 Hz, 1H); ¹³C NMR (CDCl₃, 100 MHz): δ 14.08, 14.09, 19.86, 20.99, 21.24, 22.64, 22.66, 26.60, 26.64, 26.68, 29.30, 29.35, 29.55, 29.59, 29.94, 31.07, 31.20, 31.80, 31.89, 32.87, 33.18, 33.39, 34.49, 37.21, 37.22, 37.25, 41.32, 67.79, 68.67, 70.34, 77.23, 166.30, 167.09, 167.97, 171.70, 173.96, 175.31.

### 2-23-6. 20-Hexyl-2,12,12-trimethyl-6,10,15,17-tetraoxo-14,18-dioxa-2,5,9-triazaoctacosan-11-yl (2-hexyldecyl) glutarate (Compound 29)

After putting compound 97 (100 mg, 1 equiv.) into a reaction vessel and dissolving in DCM (50 mL), HATU (66 mg, 1.5 equiv.), Hünig's base (22.32 mg, 1.5 equiv.), and compound 98 (12 mg, 1.1 equiv.) were added and stirred vigorously at room temperature for 11 hours. After checking that the reaction was terminated with TLC (SiO₂, EtOAc/MeOH 9:1, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/MeOH 10:0 → 7:3) to obtain compound 29 (89 mg, 82%) in a clear oil state.

¹H NMR (CDCl₃, 400 MHz): δ 0.81 (t, *J* = 7.1 Hz, 12H), 0.97-0.99 (d, *J* = 8.1 Hz, 6H), 1.14-1.30 (m, 50H), 1.55 (s, 2H), 1.87-1.93 (qui, *J* = 8.2 Hz, 2H), 2.14 (s, 6H), 2.17-2.45 (m, 8H), 3.15-3.51 (m, 6H), 3.87-4.02 (m, 6H), 4.78 (s, 1H), 6.24 (m, 1H), 7.16-7.22 (m, 1H); ¹³C NMR (CDCl₃, 100 MHz): δ 14.07, 19.99, 21.03, 21.06, 22.62, 22.64, 26.58, 26.62, 26.67, 29.58, 26.62, 26.67, 29.28, 29.53, 29.57, 29.58, 29.91, 31.05, 31.20, 31.78, 31.86, 33.06, 33.21, 35.26, 35.56, 26.67, 37.19, 37.27, 41.37, 45.08, 57.67, 67.34, 68.50, 70.28, 166.27, 166.81, 167.80, 171.54, 171.99, 173.00.

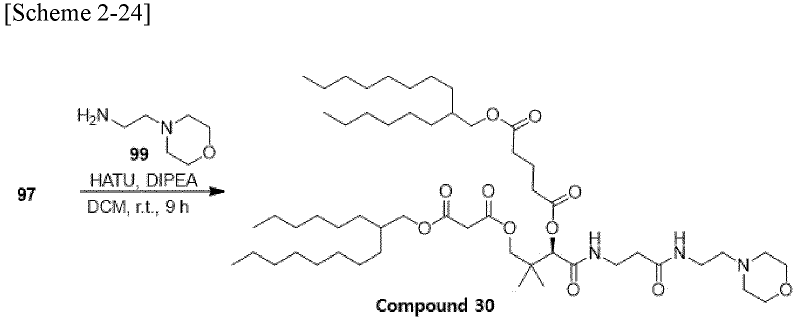

### 2-24. 18-Hexyl-10,10-dimethyl-1-morpholino-4,8,13,15-tetraoxo-12,16-dioxa-3,7-diazahexacosan-9-yl (2-hexyldecyl) glutarate (Compound 30)

After putting compound 97 (100 mg, 1 equiv.) into a reaction vessel and dissolving in DCM (50 mL), HATU (66 mg, 1.5 equiv.), Hünig's base (22.32 mg, 1.5 equiv.), and compound 99 (17 mg, 1.1 equiv.) were added and stirred vigorously at room temperature for 9 hours. After checking that the reaction was terminated with TLC (SiO₂, EtOAc/MeOH 9:1, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL). The filtrate filtered by drying the separated organic layer with anhydrous MgSO₄ was distilled under reduced pressure to remove the solvent, and the obtained mixture was purified by flash column chromatography (SiO₂, EtOAc/MeOH 10:0 → 9:1) to obtain compound 30 (99 mg, 88%) in a clear oil state.

¹H NMR (CDCl₃, 400 MHz): δ 0.81 (t, *J* = 7.9 Hz, 12H), 0.97-0.99 (d, *J* = 8.2 Hz, 6H), 1.15-1.31 (m, 51H), 1.55 (s, 2H), 1.85-1.93 (qui, *J* = 8.1 Hz, 2H), 2.29-2.44 (m, 10H), 3.22-3.30 (m, 2H), 3.32 (s, 2H), 3.35-3.51 (m, 2H), 3.63-3.65 (t, *J* = 4.1 Hz, 4H), 3.87-4.01 (m, 6H), 6.15 (s, 1H), 7.02-7.05 (m, 1H); ¹³C NMR (CDCl₃, 100 MHz): δ 14.08, 19.99, 20.99, 21.20, 22.63, 22.65, 26.58, 26.62, 26.67, 29.29, 29.54, 29.57, 29.59, 29.92, 29.93, 31.05, 31.19, 31.79, 31.79, 31.87, 33.05, 33.21, 35.17, 35.42, 35.51, 37.18, 37.23, 37.26, 41.36, 53.32, 57.02, 66.78, 67.35, 66.53, 70.28, 76.95, 166.26, 166.87, 167.86, 171.32, 171.87, 172.99.

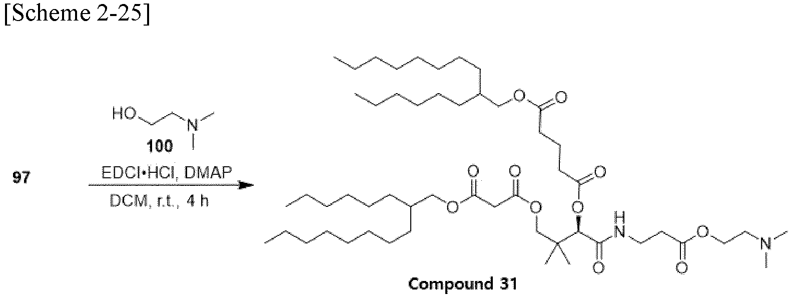

### 2-25. 20-Hexyl-2,12,12-trimethyl-6,10,15,17-tetraoxo-5,14,18-trioxa-2,9-diazaoctacosan-11-yl (2-hexyldecyl) glutarate (Compound 31)

After putting compound 97 (100 mg, 1 equiv.) into a reaction vessel and dissolving in DCM (30 mL), EDCI•HCl (33 mg, 1.5 equiv.) and 4-dimethylaminopyridine (DMAP) (3 mg, 0.2 equiv.) were added and stirred vigorously in the presence of argon gas at 5°C for 10 minutes. Thereafter, the solution obtained by dissolving compound 100 (11 mg, 1.1 equiv.) in DCM (10 mL) was added dropwise into the reaction mixture at 5°C, followed by stirring at room temperature for 4 hours. After checking that the reaction was terminated with TLC (SiO₂, EtOAc 100%, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/MeOH 20:0 → 19:1) to obtain compound 31 (97 mg, 90%) in a clear oil state.

¹H NMR (CDCl₃, 400 MHz): δ 0.81 (t, *J* = 7.5 Hz, 12H), 0.97-0.99 (d, *J* = 8.2 Hz, 6H), 1.13-1.31 (m, 50H), 1.56 (s, 2H), 1.88-1.93 (qui, *J* = 8.2 Hz, 2H), 2.22 (s, 6H), 2.31 (t, *J* = 7.6 Hz, 2H), 2.41(t, *J* = 7.1 Hz, 2H), 2.47-2.52 (m, 4H), 3.34 (s, 2H), 3.37-3.52 (m, 2H), 3.86-4.14 (m, 8H), 4.83 (s, 1H), 6.83 (t, *J* = 7.8 Hz, 1H); ¹³CNMR (CDCl₃, 100 MHz): δ 14.07, 14.08, 20.01, 20.98, 21.17, 22.63, 22.65, 26.59, 26.64, 26.68, 29.29, 29.55, 29.58, 29.60, 29.93, 29.94, 31.07, 31.21, 31.79, 31.88, 33.03, 33.20, 33.76, 34.75, 37.20, 37.29, 41.37, 45.58, 57.68, 62.14,67.37, 68.58, 70.32, 166.21, 166.91, 167.72, 171.72, 172.36, 173.02.

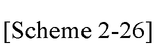

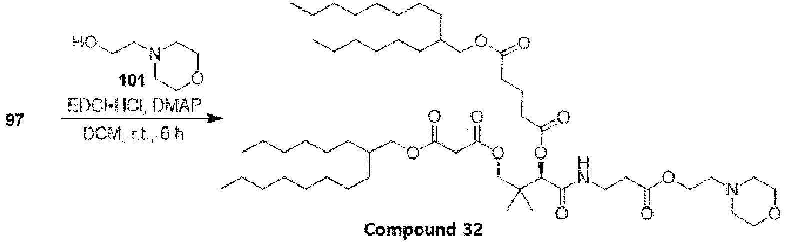

### 2-26. 18-Hexyl-10,10-dimethyl-1-morpholino-4,8,13,15-tetraoxo-3,12,16-trioxa-7-azahexacosan-9-yl (2-hexyldecyl) glutarate (Compound 32)

After putting compound 97 (100 mg, 1.3 equiv.) into a reaction vessel and dissolving in DCM (30 mL), EDCI•HCl (33 mg, 1.5 equiv.) and 4-dimethylaminopyridine (DMAP) (3 mg, 0.2 equiv.) were added and stirred vigorously in the presence of argon gas at 5°C for 10 minutes. Thereafter, the solution obtained by dissolving compound 101 (17 mg, 1.1 equiv.) in DCM (10 mL) was added dropwise into the reaction mixture at 5°C, followed by stirring at room temperature for 6 hours. After checking that the reaction was terminated with TLC (SiO₂, EtOAc 100%, PMA stain), DCM (100 mL) was further added, and the mixture was transferred to a fractionation funnel, followed by washing with saturated aqueous NaHCO₃ solution (2 x 200 mL) and saturated aqueous NaCl solution (200 mL). The filtrate filtered by drying the separated organic layer with anhydrous MgSO₄ was distilled under reduced pressure to remove the solvent, and the obtained mixture was purified by flash column chromatography (SiO₂, EtOAc/hexane 8:3 → 10:0) to obtain compound 32 (89 mg, 79%) in a clear oil state.

¹H NMR (CDCl₃, 400 MHz): δ 0.82 (t, *J* = 7.1 Hz, 12H), 0.97-0.99 (d, *J* = 8.9 Hz, 6H), 1.13-1.31 (m, 51H), 1.56 (s, 2H), 1.88-1.93 (qui, J = 8.1 Hz, 2H), 2.23-2.33 (t, *J =* 7.5 Hz, 2H), 2.39-2.49 (m, 8H), 2.53-2.56 (t, *J* = 7.0 Hz, 2H), 3.34 (s, 2H), 3.37-3.52 (m, 2H), 3.62-3.64 (t, *J* = 3.9 Hz, 4H), 3.86-4.00 (m, 6H), 4.11-4.17 (m, 2H), 4.83 (s, 1H), 6.75 (t, *J* = 7.9 Hz, 1H); ¹³C NMR (CDCl₃, 100 MHz): δ 14.06, 19.99, 20.89, 21.28, 22.61, 22.63, 26.57, 26.62, 26.66, 29.27, 26.62, 26.66, 29.27, 29.52, 29.55, 29.57, 29.90, 29.92, 31.05, 31.19, 31.77, 31.86, 33.00, 33.17, 33.65, 34.73, 37.18, 37.28, 41.34, 53.78, 56.98, 61.99, 66.80, 67.69, 66.80, 67.34, 68.58, 70.30, 166.16, 166.94, 167.75, 171.68, 172.24, 172.99.

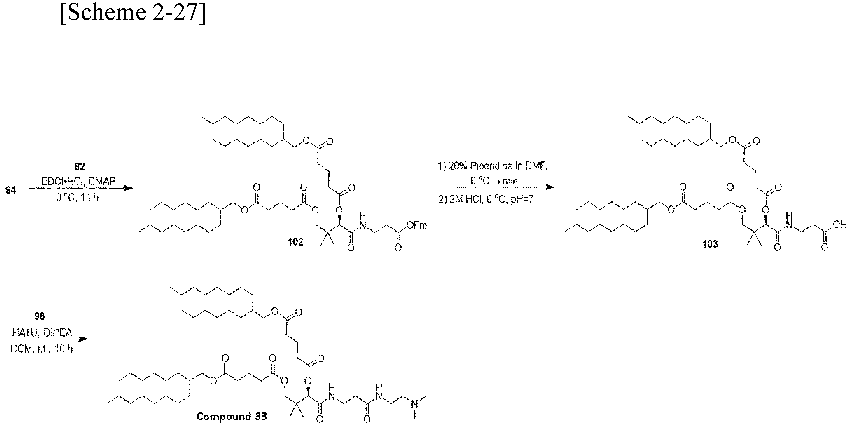

### 2-27-1. O,O'-(4-((3-((9H-Fluoren-9-yl)methoxy)-3-oxopropyl)amino)-2,2-dimethyl-4-oxobutane-1,3-diyl) bis(2-hexyldecyl) diglutarate (102)

After putting compound 82 (164 mg, 2.2 equiv.) into a reaction vessel and dissolving in DCM (30 mL), EDCI•HCl (101 mg, 1.5 equiv.) and 4-dimethylaminopyridine (DMAP) (7 mg, 0.2 equiv.) were added and stirred vigorously in the presence of argon gas at 5°C for 10 minutes. Thereafter, the solution obtained by dissolving compound 94 (250 mg, 1 equiv.) in DCM (10 mL) was added dropwise into the reaction mixture at 5°C, followed by stirring at room temperature for 4 hours. After checking that the reaction was terminated with TLC (SiO₂, EtOAc/hexane 1:9, PMA stain), DCM (100 mL) was further added, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure to remove the solvent, and the obtained mixture was purified by flash column chromatography (SiO₂, EtOAc/hexane 1:9) to obtain compound 102 (300 mg, 81%) in a clear oil state.

¹H NMR (CDCl₃, 400 MHz): δ 0.80 (t, *J* = 8.0 Hz, 12H), 0.92-0.97 (d, *J* = 9.2 Hz, 6H), 1.11-1.28 (m, 50H), 1.52 (s, 2H), 1.63 (s, 1H), (qui, J = 5.5 Hz, 4H), 2.26-2.33 (m, 6H), 2.38 (t, *J =* 5.7 Hz, 2H), 2.53-2.56 (t, *J =* 5.9 Hz, 2H), 3.38-3.51 (m, 2H), 3.76-3.97 (m, 6H), 4.12-4.31 (t, *J* = 5.0 Hz, 1H), 4.33 (d, *J* = 9.1 Hz, 2H), 4.88 (s, 1H), 6.61 (m, 1H), 7.24 (t, *J* = 7.1 Hz, 2H), 7.26 (t, *J* = 8.1 Hz, 2H), 7.49 (d, *J* = 8.3 Hz, 2H), 7.68 (d, *J* = 8.2 Hz, 2H); ¹³C NMR (CDCl₃, 100 MHz): δ 14.08, 14.10, 20.00, 20.13, 20.90, 21.32, 22.64, 22.67, 26.65, 26.69, 29.30, 29.56, 29.60, 29.95, 31.21, 31.24, 31.81, 31.89, 33.05, 33.17, 33.24, 33.37, 33.66, 34.65, 37.29, 37.38, 46.70, 66.71, 67.35, 67.41, 69.23, 120.08, 124.98, 125.00, 127.17, 127.88, 141.30, 143.57, 143.65, 167.95, 171.62, 172.48, 172.62, 173.01, 173.09.

### 2-27-2. 3-(2,4-bis((5-((2-Hexyldecyl)oxy)-5-oxopentanoyl)oxy)-3,3-dimethylbutanamido)propanoic acid (103)

Piperidine and DMF were mixed and stirred in a volume ratio of 2:8 in a reaction vessel and then cooled to 0°C to prepare a 20% piperidine solution. Compound 102 (300 mg) was put into another reaction vessel, dissolved in DMF (20 mL), stirred at for 5 minutes, and cooled to 0°C. The prepared 20% piperidine solution (40 mL) was added in the presence of argon gas and stirred at 0°C for 5 minutes. After the reaction was terminated, 3M aqueous HCl solution was added to the reaction mixture, the pH was adjusted to 7, EtOAc (200 mL) was added, and the mixture was transferred to a fractionation funnel, followed by washing with distilled water (2 x 200 mL) and saturated aqueous NaCl solution (2 x 200 mL) sequentially. The filtrate filtered by drying the separated organic layer with anhydrous Na₂SO₄ was distilled under reduced pressure to remove the solvent, and the obtained mixture was purified by flash column chromatography (SiO₂, EtOAc/hexane 0:1 → 4:6, 1% AcOH added) to obtain compound 103 (226 mg, 91%) in a clear oil state.

¹H NMR (CDCl₃, 400 MHz): δ 0.81 (t, *J* = 8.1 Hz, 12H), 0.91-0.92 (d, *J* = 9.0 Hz, 6H), 1.12-1.21 (m, 51H), 1.52 (s, 2H), 1.62 (s, 1H), (qui, J = 5.5 Hz, 4H), 2.27-2.33 (m, 6H), 2.39 (t, *J =* 5.8 Hz, 2H), 2.53-2.55 (t, *J =* 5.3 Hz, 2H), 3.38-3.52 (m, 2H), 3.77-3.97 (m, 6H), 4.89 (s, 1H), 6.62 (m, 1H); ¹³C NMR (CDCl₃, 100 MHz): δ 14.09, 14.11, 20.02, 20.14, 20.91, 21.31, 22.66, 22.68, 26.61, 26.65, 29.31, 29.56, 29.61, 29.95, 31.22, 31.24, 31.81, 31.89, 33.05, 33.17, 33.24, 33.37, 33.66, 34.65, 37.29, 37.38, 46.70, 66.71, 67.35, 67.41, 69.23, 167.95, 171.62, 172.48, 172.62, 173.01, 173.09.

### 2-27-3. O,O'-(4-((3-((2-(Dimethylamino)ethyl)amino)-3-oxopropyl)amino)-2,2-dimethyl-4-oxobutane-1,3-diyl) bis(2-hexyldecyl) diglutarate (Compound 33)

After putting compound 103 (100 mg, 1 equiv.) into a reaction vessel and dissolving in DCM (50 mL), HATU (66 mg, 1.5 equiv.), Hünig's base (22.32 mg, 1.5 equiv.), and compound 98 (12 mg, 1.1 equiv.) were added and stirred vigorously at room temperature for 11 hours. After checking that the reaction was terminated with TLC (SiO₂, EtOAc/MeOH 9:1, PMA stain), DCM (100 mL) was further added, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure, and the obtained mixture was purified by flash column chromatography (SiO₂, EtOAc/MeOH 10:0 → 7:3) to obtain compound 33 (89 mg, 82%) in a clear liquid state.

¹H NMR (CDCl₃, 400 MHz): δ 0.81 (t, *J* = 7.1 Hz, 12H), 0.97-0.99 (d, *J* = 8.1 Hz, 6H), 1.14-1.30 (m, 50H), 1.55 (s, 2H), 1.87-1.93 (qui, *J* = 8.2 Hz, 4H), 2.14 (s, 6H), 2.17-2.45 (m, 8H), 3.38-3.51 (m, 2H), 3.37 (d, *J* = 9.6 Hz, 2H), 3.91 (d, *J* = 9.7 Hz, 4H), 3.96 (d, *J* = 9.9 Hz, 2H), 4.10-4.14 (m, 2H), 4.87 (s, 1H), 6.24 (m, 1H), 7.16-7.22 (m, 1H); ¹³C NMR (CDCl₃, 100 MHz): δ 14.07, 19.99, 21.03, 21.06, 22.62, 22.64, 26.58, 26.62, 26.67, 29.58, 26.62, 26.67, 29.28, 29.53, 29.57, 29.58, 29.91, 31.05, 31.20, 31.78, 31.86, 33.06, 33.21, 35.26, 35.56, 26.67, 37.19, 37.27, 41.37, 45.08, 57.67, 67.34, 68.50, 70.28, 166.27, 166.81, 167.80, 171.54, 171.99, 173.00.

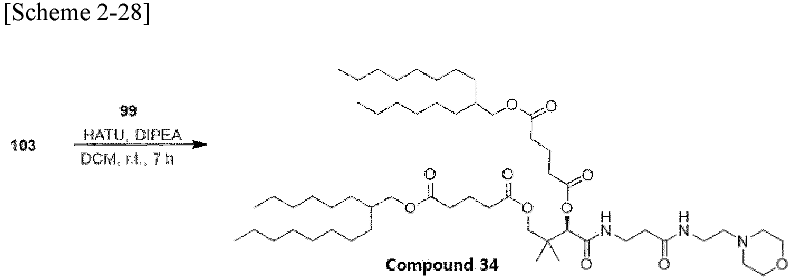

### 2-28. O,O'-(2,2-Dimethyl-4-((3-((2-morpholinoethyl)amino)-3-oxopropyl)amino)-4-oxobutane-1,3-diyl) bis(2-hexyldecyl) diglutarate (Compound 34)

After putting compound 103 (100 mg, 1 equiv.) into a reaction vessel and dissolving in DCM (50 mL), HATU (66 mg, 1.5 equiv.), Hünig's base (22.32 mg, 1.5 equiv.), and compound 99 (17 mg, 1.1 equiv.) were added and stirred vigorously at room temperature for 9 hours. After checking that the reaction was terminated with TLC (SiO₂, EtOAc/MeOH 9:1, PMA stain), DCM (100 mL) was further added, followed by washing with saturated aqueous NaHCO₃ solution (2 x 200 mL) and saturated aqueous NaCl solution (200 mL) sequentially. The solution filtered by drying the separated organic layer with anhydrous MgSO4 was distilled under reduced pressure, and the obtained mixture was purified by flash column chromatography (SiO₂, EtOAc/MeOH 10:0 → 9:1) to obtain compound 34 (99 mg, 88%) in a clear oil state.

¹H NMR (CDCl₃, 400 MHz): δ 0.81-0.82 (t, *J* = 7.0 Hz, 12H), 0.95-0.98 (d, *J* = 8.5 Hz, 6H), 1.11-1.35 (m, 50H), 1.54 (s, 2H), 1.83-1.92 (m, 4H), 2.28-2.47 (m, 16H), 3.21-2.32 (m, 4H), 3.40 (t, *J* = 3.2 Hz, 4H), 3.78-3.81 (d, *J* = 8.2 Hz, 1H), 3.89-3.91 (d, *J* = 8.0 Hz, 4H), 3.94-3.97 (d, *J =* 9.2 Hz, 1H), 4.84 (s, 1H), 6.18 (m, 1H), 7.00 (t, *J =* 6.9 Hz, 1H); ¹³C NMR (CDCl₃, 100 MHz): δ 14.04, 14.05, 19.97, 20.09, 20.90, 21.30, 22.59, 22.62, 26.61, 26.65, 29.25, 29.51, 29.56, 29.90, 31.19, 31.31.76, 31.84, 33.05, 33.18, 33.32, 35.07, 35.37, 35.54, 37.25, 53.33, 57.06, 66.76, 67.28, 67.34, 69.21, 76.77, 76.91, 77.09, 77.41, 167.99, 171.45, 191.77, 172.57, 172.95, 173.02.

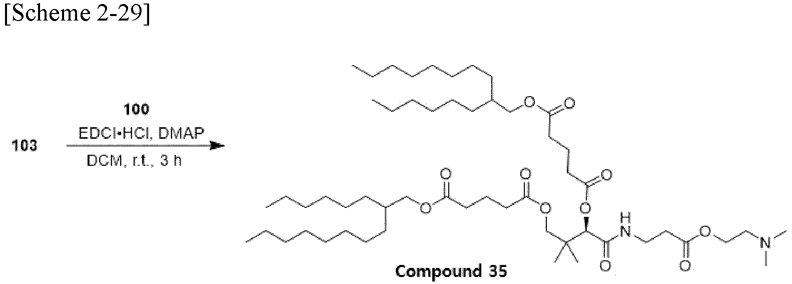

### 2-29. O,O'-(4-((3-((2-(Dimethylamino)ethyl)amino)-3-oxopropyl)amino)-2,2-dimethyl-4-oxobutane-1,3-diyl) bis(2-hexyldecyl) diglutarate (Compound 35)

After putting compound 103 (100 mg, 1 equiv.) into a reaction vessel and dissolving in DCM (30 mL), EDCI•HCl (33 mg, 1.5 equiv.) and 4-dimethylaminopyridine (DMAP) (3 mg, 0.2 equiv.) were added and stirred vigorously in the presence of argon gas at 5°C for 10 minutes. Thereafter, the solution obtained by dissolving the compound 100 (11 mg, 1.1 equiv.) in DCM (10 mL) was added dropwise at 5°C, followed by stirring at room temperature for 4 hours. After checking that the reaction was terminated with TLC (SiO₂, EtOAc 100%, PMA stain), DCM (100 mL) was further added, followed by washing with saturated aqueous NaHCO₃ solution (2 x 200 mL) and saturated aqueous NaCl solution (200 mL) sequentially. The filtrate filtered by drying the separated organic layer with anhydrous MgSO₄ was distilled under reduced pressure, and the mixture was purified by column chromatography (SiO₂, EtOAc/MeOH 20:0 → 19:1) to obtain compound 35 (97 mg, 90%) in a clear oil state.

¹H NMR (CDCl₃, 400 MHz): δ 0.81-0.82 (t, *J =* 7.1 Hz, 12H), 0.95-0.98 (d, *J =* 8.1 Hz, 6H), 1.11-1.32 (m, 50H), 1.54 (s, 2H), 1.85-1.91 (qui, *J* = 8.0 Hz, 4H), 2.22 (s, 6H), 2.30-2.32 (t, *J =* 7.0 Hz, 6H), 2.39 (t, *J =* 7.9 Hz, 2H), 2.43-2.51 (m, 4H), 3.37 (d, *J =* 9.6 Hz, 2H), 3.38-3.51 (m, 2H), 3.91 (d, *J =* 9.7 Hz, 4H), 3.96 (d, *J =* 9.9 Hz, 2H), 4.10-4.14 (m, 2H), 4.87 (s, 1H), 6.77 (t, *J* = 6.9 Hz, 1H); ¹³C NMR (CDCl₃, 100 MHz): δ 14.04, 19.99, 20.09, 20.79, 21.32, 22.60.22.62, 26.61, 26.66, 29.26, 29.52, 29.56, 29.91, 31.20, 31.77, 31.85, 33.02, 33.16, 33.19, 33.33, 33.75, 34.66, 37.26, 37.30, 45.57, 57.68, 62.16, 67.28, 67.34, 69.21, 167.80, 171.59, 172.44, 192.55, 172.95, 173.01.

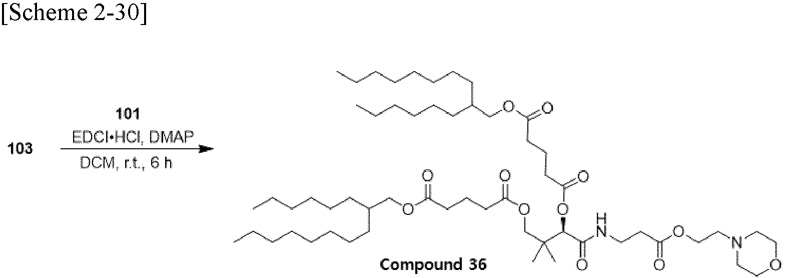

### 2-30. O,O ' -(2,2-Dimethyl-4-((3-((2-morpholinoethyl)amino)-3-oxopropyl)amino)-4-oxobutane-1,3-diyl) bis(2-hexyldecyl) diglutarate (Compound 36)

After putting the compound 103 (100 mg, 1.3 equiv.) into a reaction vessel and dissolving in DCM (30 mL), EDCI•HCl (33 mg, 1.5 equiv.) and 4-dimethylaminopyridine (DMAP) (3 mg, 0.2 equiv.) were added and stirred vigorously in the presence of argon gas at 5°C for 10 minutes. Thereafter, the solution obtained by dissolving the compound 101 (17 mg, 1.1 equiv.) in DCM (10 mL) was added dropwise into the reaction mixture at 5°C, followed by stirring at room temperature for 6 hours. After checking that the reaction was terminated with TLC (SiO₂, EtOAc 100%, PMA stain), DCM (100 mL) was further added, followed by washing with saturated aqueous NaHCO₃ solution (2 x 200 mL) and saturated aqueous NaCl solution (1 x 200 mL) sequentially. The mixture obtained by distillation under reduced pressure using the filtrate filtered by drying the separated organic layer with anhydrous MgSO₄ was purified by flash column chromatography (SiO₂, EtOAc/hexane 8:3 → 10:0) to obtain a compound 36 (89 mg, 79%) in a clear oil state.

¹H NMR (CDCl₃, 400 MHz): δ 0.81 (t, *J =* 7.6 Hz, 12H), 0.94-0.98 (d, *J =* 8.7 Hz, 6H), 1.10-1.31 (m, 47H), 1.54 (s, 2H), 1.85-1.93 (m, 4H), 2.28-2.57 (m, 16H), 2.33-2.50 (m, 2H), 3.65 (m, 4H), 3.76-3.79 (d, 1H), 3.89-3.98 (m, 5H), 4.13-1.19 (m, 2H), 4.88 (s, 1H); ¹³C NMR (CDCl₃, 100 MHz): δ 14.06, 20.01, 20.12, 20.89, 21.31, 22.62, 22.64, 26.64, 26.68, 29.28, 29.54, 29.58, 29.93, 31.22, 31.79, 31.87, 33.06, 33.18, 33.23, 33.35, 33.66, 34.68, 37.29, 37.35, 53.76, 56.97, 61.62, 66.72, 67.34, 37.41, 69.21, 167.88, 171.62, 172.38, 172.59, 173.00, 173.05.

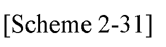

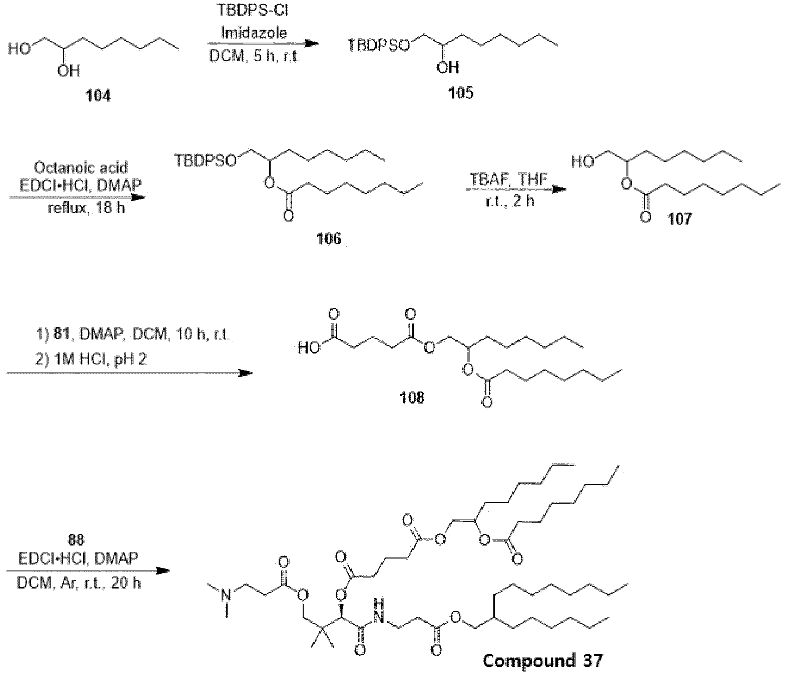

### 2-31-1. 1-((tert-Butyldiphenylsilyl)oxy)octan-2-ol (105)

After putting 1,2-octanediol (compound 104, 500 mg, 1 equiv.) and imidazole (279.32, 1.2 equiv.) into a reaction vessel and then dissolving in DCM (30 mL) to stir for 5 minutes, a solution, in which tert-butyldiphenylsilyl chloride (TBDPS-Cl; 936.38 mg, 1 equiv.) was dissolved in DCM (20 mL), was added dropwise for 20 minutes. The reaction mixture solution was stirred vigorously at room temperature for 5 hours. After checking that the reaction was terminated with TLC (SiO₂; hexane/ethyl acetate, 19:1), the reaction mixture solution was concentrated to a level of 35 mL. The filtrate remaining after filtering solids precipitated by adding 70 mL of n-hexane was concentrated and purified by column chromatography (SiO₂; hexane/ethyl acetate, 39:1 to 19:1, v/v) to obtain compound 105 (1183 mg, 90%) in a clear liquid state.

¹H NMR (CDCl₃, 400 MHz): δ 0.98-0.95 (t, *J* = 6.2 Hz, 3H), 1.14 (s, 9H), 1.32 (s, 7H), 1.44-1.46 (m, 3H), 2.57 (s, 1H), 3.54-3.58 (m, 1H), 3.72-3.79 (m, 2H), 7.43-7.49 (m, 6H), 7.73-7.75 (d, *J =* 6.7 Hz, 4H); ¹³C NMR (CDCl₃, 100 MHz): δ 14.15, 19.10, 22.65, 25.54, 26.92, 29.39, 31.82, 32.85, 68.12, 72.02, 127.83, 129.86, 129.86, 133.25, 133.29, 235.59, 135.61.

### 2-31-2. 1-((tert-Butyldiphenylsilyl)oxy)octan-2-yl octanoate (106)

After putting compound 105 (100 mg, 1 equiv.) into a reaction vessel and dissolving in DCM (50 ml), octanoic acid (62 mg, 1.3 equiv.), EDCI•HCl (46 mg, 1.5 equiv.), and DMAP (4 mg, 0.2 equiv.) were added to be subjected to heating and refluxing for 18 hours. After checking that the reaction was terminated with TLC (SiO₂; hexane/ethyl acetate, 39:1), DCM (50 mL) was further added, and the mixture was washed sequentially with distilled water (2 × 50 mL), saturated aqueous NaHCO₃ solution (2 × 50 mL), and brine (1 × 20 mL). The filtrate filtered after collecting an organic layer and then removing moisture with anhydrous MgSO₄ was distilled under reduced pressure and then purified by column chromatography (SiO₂; hexane/ethyl acetate, 39:1 to 19:1, v/v) to obtain compound 106 (118 mg, 89 %) in a clear liquid state.

¹H NMR (CDCl₃, 400 MHz): δ 0.97-0.98 (t, *J =* 6.1 Hz, 6H), 1.14 (s, 9H), 1.22-1.33 (m, 16H), 1.47-1.55 (m, 4H), 2.17-2.22 (m, 2H), 3.55-3.63 (m, 2H), 4.90-4.95 (m, 1H), 7.27-7.36 (m, 6H), 7.57-7.59 (d, *J =* 6.8 Hz, 4H); ¹³C NMR (CDCl₃, 100 MHz): δ 14.09, 19.26, 22.58, 22.63, 25.10, 25.16, 26.76, 29.00, 29.19, 30.56, 31.69, 31.72, 34.64, 65.09, 74.20, 127.67, 129.67, 129.66, 129.69, 133.53, 135.58, 135.65, 173.51.

### 2-31-3. 1-Hydroxyoctan-2-yl octanoate (107)

The reaction vessel was subjected to flame-drying to remove moisture, cooled to 20°C, and filled with nitrogen gas. Thereafter, compound 106 (200 mg, 1 equiv.) and dry THF (15 mL) were injected with a syringe and stirred for 1 minute. Then, n-Bu₄NF solution (1M, THF, 0.5 mL, 1.2 equiv.) was added dropwise with a syringe for 10 minutes, and then the reaction mixture solution was stirred at 20°C for 2 hours. After checking that the reaction was terminated with TLC (SiO₂; hexane/ethyl acetate, 9:1), dilution was performed by adding distilled water (2 × 50 mL), followed by extraction with DCM (3 × 40 mL). After collecting an organic layer and washing with saturated aqueous NaCl solution, a mixture obtained by distilling, under reduced pressure, the filtrate filtered after removing moisture with anhydrous Na₂SO₄ was purified by column chromatography (SiO₂; hexane/ethyl acetate, 1:19, v/v) to obtain compound 107 (96 mg, 90 %) in a clear oil state.

¹H NMR (CDCl₃, 400 MHz): δ 0.81 (t, *J* = 6.3 Hz, 6H), 1.20 (m, 16H), 1.47-1.55 (m, 4H), 2.18-2.22 (m, 2H), 3.56-3.64 (m, 2H), 4.91-4.94 (m, 1H); ¹³C NMR (CDCl₃, 100 MHz): δ 14.09, 19.27, 22.54, 22.65, 25.14, 25.17, 26.88, 29.54, 30.56, 31.74, 31.72, 34.77, 65.09, 74.21, 173.56.

### 2-31-4. 6-((2-(Octanoyloxy)octyl)oxy)-6-oxohexanoic acid (108)

After putting compound 107 to a reaction vessel and adding DCM (25 mL) to dissolve, compound 81 (84 mg, 2 equiv.) and DMAP (112 mg, 2.5 equiv.) were added and stirred vigorously at room temperature for 10 hours. After checking that the reaction was terminated with TLC (SiO₂; hexane/ethyl acetate, 9:1), acidification was performed by adding 1N aqueous HCl solution, followed by extraction with DCM (3 × 25 mL). After collecting an organic layer and washing with saturated aqueous NaCl solution (1 × 30 mL), a mixture obtained by distilling, under reduced pressure, the filtrate filtered after removing moisture with anhydrous Na₂SO₄ was purified by column chromatography (SiO₂; hexane/ethyl acetate, 9:1 to 8:2, v/v) to obtain compound 108 (99 mg, 70 %) in a clear liquid state.

¹H NMR (CDCl₃, 400 MHz): δ 0.87 (t, *J* = 6.9 Hz, 6H), 1.24-1.28 (m, 18H), 1.55-1.60 (m, 4H), 1.90-1.98 (m, 2H), 2.27-2.30 (t, *J =* 6.1 Hz, 2H), 2.37-2.44 (m, 4H), 3.98-4.02 (m, 1H), 4.21-4.25 (dd, *J =* 2.2, 9.1 Hz, 1H), 5.05-5.10 (m, 1H); ¹³C NMR (CDCl₃, 100 MHz): δ 14.12, 19.84, 22.48, 22.55, 24.84, 25.02, 28.87, 28.98, 29.01, 30.66, 31.56, 31.62, 32.90, 33.25, 32.90, 33.25, 34.05, 60.41, 64.81, 71.73, 172.37, 173.60, 178.67.

### 2-31-5. 17-Hexyl-2,8,8-trimethyl-5,10,14-trioxo-6,15-dioxa-2,11-diazapentacosan-9-yl (2-(octanoyloxy)octyl) glutarate (Compound 37)

After dissolving compound 108 (170.90 mg, 1.2 equiv.) in DCM (30 mL) in a reaction vessel, EDCI•HCl (113 mg, 1.6 equiv.) and DMAP (9 mg, 0.2 equiv.) were added and stirred in the presence of argon gas at 5°C for 10 minutes. A solution obtained by dissolving compound 88 (200 mg, 1 equiv.) in DCM (10 mL) was added dropwise at 5°C and stirred at room temperature for 20 hours. After checking that the reaction was terminated with TLC (SiO₂; hexane/ethyl acetate, 7:3), DCM (50 mL) was further added, and the mixture was washed sequentially with saturated aqueous NaHCO₃ solution (2 × 50 mL) and saturated aqueous NaCl solution (1 × 20 mL). The filtrate filtered after removing moisture from an organic layer with anhydrous MgSO₄ was distilled under reduced pressure and then purified by column chromatography (SiO₂; ethyl acetate/hexane, 2:8 to 5:5, v/v) to obtain compound 37 (292 mg, 87 %) in a clear liquid state.

¹H NMR (CDCl₃, 400 MHz): δ 0.87 (t, *J =* 6.5 Hz, 12H), 0.98-0.99 (d, *J =* 4.7 Hz, 6H), 1.19 (m, 47H), 1.53 (m, 1H), 1.88-1.92 (m, 2H), 2.21 (s, 6H), 2.30-2.65 (m, 12H), 3.27-3.32 (m, 1H), 3.46-3.53 (m, 1H), 3.72-3.75 (d, *J =* 11.2 Hz, 1H), 3.86-3.94 (m, 5H), 4.79 (s, 1H), 5.16 (bs, 1H), 7.35-7.38 (t, *J* = 5.8 Hz, 1H); ¹³C NMR (CDCl₃, 100 MHz): δ 14.09, 20.05, 20.27, 22.04, 22.64, 22.66, 26.64, 26.69, 29.30, 29.56, 29.60, 29.94, 31.16, 31.21, 31.81, 31.88, 33.09, 33.24, 33.78, 35.04, 37.20, 37.24, 37.27, 45.21, 55.13, 67.33, 67.57, 69.59, 74.47, 76.61, 167.84, 171.69, 171.97, 172.43, 173.04, 176.04.

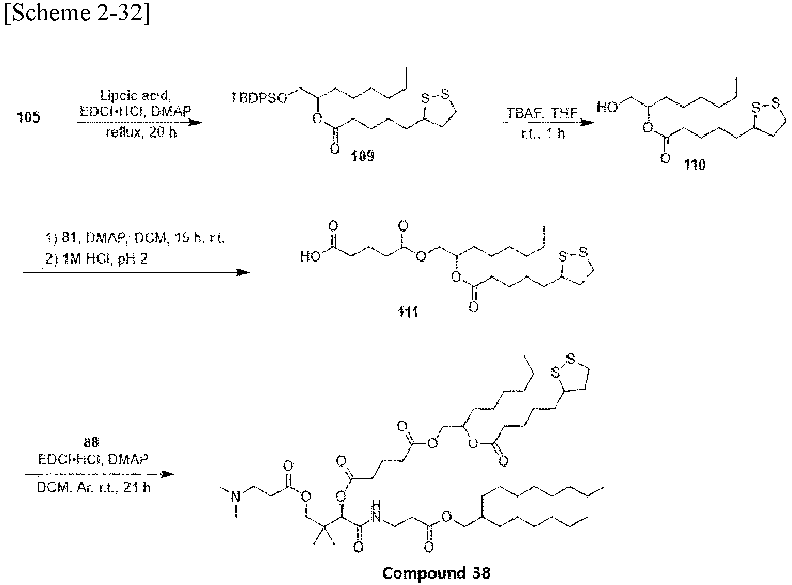

### 2-32-1. 1-((tert-Butyldiphenylsilyl)oxy)octan-2-yl 5-(1,2-dithiolan-3-yl)pentanoate (109)

After putting compound 105 (500 mg, 1 equiv.) to a reaction vessel and dissolving in DCM (50 ml), lipoic acid (349 mg, 1.3 equiv.), EDCI•HCl (374 mg, 1.5 equiv.), and DMAP (31 mg, 0.2 equiv.) were added to be subjected to heating and refluxing for 18 hours. After checking that the reaction was terminated with TLC (SiO₂; hexane/ethyl acetate, 29:1), DCM (50 mL) was further added, and the organic layer was washed sequentially with distilled water (2 × 100 mL), saturated aqueous NaHCO₃ solution (2 × 100 mL), and saturated aqueous NaCl solution (1 × 40 mL). The filtrate filtered after removing moisture from the obtained organic layer with anhydrous MgSO₄ was distilled under reduced pressure, and then the obtained mixture was purified by column chromatography (SiO₂; hexane/ethyl acetate, 49:1 to 39:1, v/v) to obtain compound 109 (603 mg, 81 %) in a yellow liquid.

¹H NMR (CDCl₃, 400 MHz): δ 0.85-0.88 (t, *J* = 6.1 Hz, 3H), 1.04 (s, 9H), 1.25 (s, 8H), 1.44-1.66 (m, 8H), 1.68-1.89 (s, 1H), 2.26-2.31 (m, 2H), 2.42-2.43 (m, 1H), 3.09-3.16 (m, 2H), 3.51-3.55 (m, 1H), 3.66-3.68 (m, 2H), 5.00 (m, 1H), 7.25-7.42 (m, 6H), 7.65-7.66 (m, 4H); ¹³C NMR (CDCl₃, 100 MHz): δ 14.10, 19.27, 22.59, 24.79, 25.17, 26.78, 28.82, 29.18, 30.55, 31.71, 34.33, 34.33, 34.66, 38.49, 40.21, 56.12. 65.09, 74.38, 127.69, 129.69, 129.73, 133.45, 133.52, 135.57, 135.64, 173.10.

### 2-32-2. 1-Hydroxyoctan-2-yl 5-(1,2-dithiolan-3-yl)pentanoate (110)

The reaction vessel was subjected to flame-drying to remove moisture, cooled to 20°C, and filled with nitrogen gas. Thereafter, the compound 109 (600 mg, 1 equiv.) and dry THF (30 mL) were injected with a syringe and stirred for 1 minute. Then, n-Bu₄NF solution (1M, THF, 1.26 mL, 1.2 equiv.) was added dropwise with a syringe for 10 minutes, and then the reaction mixture solution was stirred at 20°C for 1 hour. After checking that the reaction was terminated with TLC (SiO₂; hexane/ethyl acetate, 8:2), dilution was performed by adding distilled water (2 × 50 mL), followed by extraction with DCM (3 × 40 mL). After collecting an organic layer and washing with saturated aqueous NaCl solution, a mixture obtained by distilling, under reduced pressure, the filtrate filtered after removing moisture with anhydrous Na₂SO₄ was purified by column chromatography (SiO₂; hexane/ethyl acetate, 9:1, v/v) to obtain compound 108 (297 mg, 85 %) in a yellow oil state.

¹H NMR (CDCl₃, 400 MHz): δ 0.82-0.81 (t, *J =* 6.7 Hz, 3H), 1.23-1.21 (m, 8H), 1.41-1.71 (m, 8H), 2.02 (m, 1H), 2.43-2.47 (m, 3H), 3.12-3.19 (m, 2H), 3.55-3.56 (qui, *J =* 6.7 Hz, 1H), 4.02-4.03 (m, 1H), 4.21-4.27 (dd, *J* = 2.7, 12.00 Hz, 1H), 5.05 (m, 1H); ¹³C NMR (CDCl₃, 100 MHz): δ 14.11, 19.72, 22.73, 31.81, 25.32, 28.04, 29.02, 30.73, 32.71, 33.41, 34.24, 34.63, 38.55, 40.33, 56.34, 65.56, 70.73, 173.14, 173.65, 178.45.

### 2-32-3. 5-((2-((5-(1,2-Dithiolan-3-yl)pentanoyl)oxy)octyl)oxy)-5-oxopentanoic acid (111)

In a reaction vessel, compound 81 (204 mg, 2 equiv.) and DMAP (274 mg, 2.5 equiv.) were put in a solution in which compound 110 (300 mg, 1 equiv.) and DCM (25 mL) were added, and then stirring was performed at room temperature. After checking that the reaction was terminated with TLC (SiO₂; hexane/ethyl acetate, 8:2) after vigorously stirring the reaction mixture at room temperature for 19 hours, acidification was performed with 1M aqueous HCl solution. After collecting an organic layer by extracting the obtained mixture solution with DCM (3 × 50 mL) and then washing with saturated aqueous NaCl solution (1 × 50 mL), a mixture obtained by distilling, under reduced pressure, the filtrate filtered after removing moisture with anhydrous Na₂SO₄ was purified by column chromatography (SiO₂; hexane/ethyl acetate, 9:1 to 8:2, v/v) to obtain compound 111 (302 mg, 75 %) in a yellow liquid state.

¹H NMR (CDCl₃, 400 MHz): δ 0.86-0.89 (t, *J =* 6.7 Hz, 3H), 1.27-1.29 (m, 8H), 1.45-1.70 (m, 8H), 1.88-2.02 (m, 5H), 2.30-2.39 (t, *J =* 8.0 Hz, 2H), 2.41-2.48 (m, 8H), 3.11-3.18 (m, 2H), 3.55-3.58 (qui, *J =* 6.8 Hz, 1H), 3.98-4.03 (m, 1H), 4.24-4.28 (dd, *J =* 2.7, 11.9 Hz, 1H), 5.08-5.09 (m, 1H); ¹³C NMR (CDCl₃, 100 MHz): δ 14.10, 19.72, 22.79, 31.79, 29.31, 25.32, 28.01, 29.03, 30.72, 32.75, 33.31, 34.23, 34.62, 38.51, 40.20, 56.33, 65.55, 70.71, 173.11, 173.60, 178.43.

### 2-32-4. 2-((5-(1,2-Dithiolan-3-yl)pentanoyl)oxy)octyl (17-hexyl-2,8,8-trimethyl-5,10,14 -trioxo-6,15-dioxa-2,11-diazapentacosan-9-yl) glutarate (Compound 38)

After putting compound 111 (215 mg, 1.3 equiv.) in a reaction vessel to dissolve in DCM (30 mL), EDCI•HCl (113 mg, 1.6 equiv.) and DMAP (9 mg, 0.2 equiv.) were added and stirred vigorously in the presence of argon gas at 5°C for 10 minutes. A solution obtained by dissolving compound 88 (200 mg, 1 equiv.) in DCM (10 mL) was added dropwise at 5°C and stirred at room temperature for 21 hours. After checking that the reaction was terminated with TLC (SiO₂; ethyl acetate), DCM (50 mL) was further added, followed by washing with saturated aqueous anhydrous NaHCO₃ solution (2 x 50 mL) and saturated aqueous NaCl solution (1 × 20 mL) sequentially. The filtrate filtered after removing moisture from the obtained organic layer with anhydrous MgSO₄ was distilled under reduced pressure and then purified by column chromatography (SiO₂; ethyl acetate/hexane, 5:5 to 7:3, v/v) to obtain compound 38 (251 mg, 70 %) in a yellow liquid state.

¹H NMR (CDCl₃, 400 MHz): δ 0.86-0.89 (t, *J* = 6.9 Hz, 9H), 1.05-1.06 (d, *J =* 6.5 Hz, 6H), 1.26 (m, 33H), 1.45-1.71 (m, 10H), 1.90-1.99 (m, 3H), 2.28 (s, 6H), 2.30-2.34 (t, *J* = 7.4 Hz, 2H), 2.38-2.41 (t, *J* = 7.2 Hz, 2H), 2.44-2.61 (m, 8H), 2.67-2.72 (m, 1H), 3.08-3.21 (m, 2H), 3.34-3.39 (m, 1H), 3.53-3.60 (m, 2H), 3.79-3.82 (d, *J =* 11.2 Hz, 1H), 3.96-4.04 (m, 4H), 4.23-4.26 (m, 1H), 4.85 (s, 1H), 5.06-5.09 (m, 1H), 7.43-7.46 (t, *J =* 5.7 Hz, 1H);¹³_{C} NMR (CDCl₃, 100 MHz): δ 14.01, 14.08, 14.09, 20.04, 20.26, 22.02, 22.51, 22.63, 22.65, 24.56, 25.06, 26.63, 26.67, 28.69, 29.00, 29.29, 29.54, 29.58, 29.92, 30.71, 31.15, 31.59, 31.79, 31.87, 33.02, 33.04, 33.29, 33.79, 33.83, 34.57, 35.05, 37.19, 37.23, 38.46, 40.20, 45.19, 55.10, 56.30, 64.94, 67.57, 67.57, 69.57, 71.69, 76.61, 167.81, 171.66, 171.92, 172.35, 172.39, 173.12.

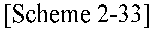

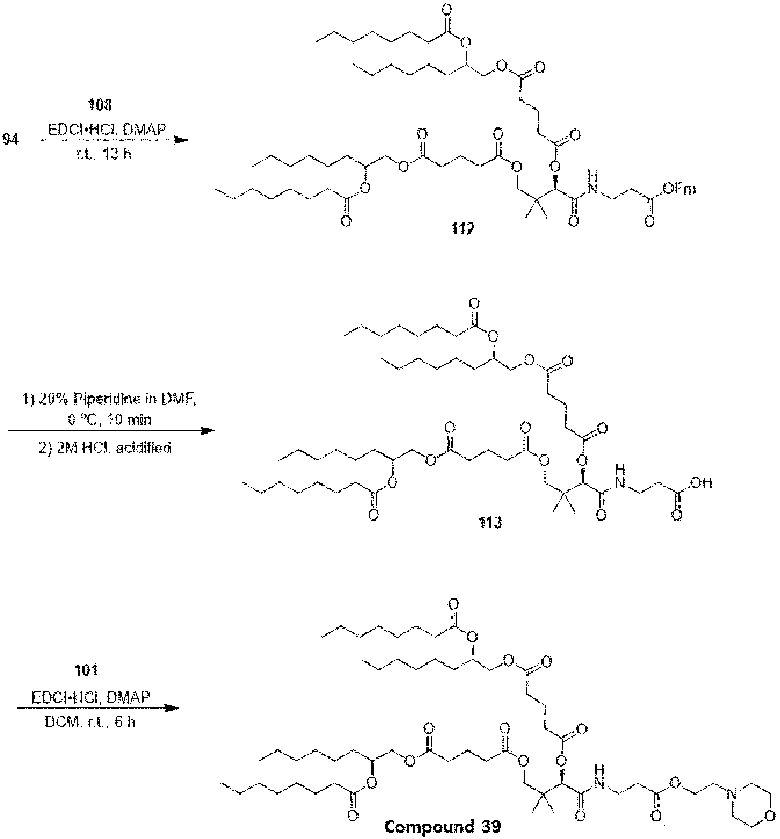

### 2-33-1. O,O'-(4-((3-((9H-Fluoren-9-yl)methoxy)-3-oxopropyl)amino)-2,2-dimethyl-4-oxobutane-1,3-diyl) bis(2-foctanoyloxy)octyl) diglutarate] (112)

Compound 94 (150 mg, 1 equiv.), compound 108 (335 mg, 2.3 equiv.), EDCI.HCl (181 mg, 2.5 equiv.), and DMAP (9 mg, 0.2 equiv.) were put in a reaction vessel along with DCM (50 mL), and then stirring was performed at room temperature for 13 hours. After checking that the reaction was terminated with TLC (SiO₂; hexane/ethyl acetate, 9:1), DCM (100 mL) was further added, followed by washing with saturated aqueous NaHCO₃ solution (2 x 200 mL) and saturated aqueous NaCl solution (1 × 200 mL) sequentially. The obtained organic layer was filtered by removing moisture with anhydrous MgSO₄, and the mixture obtained by distilling the filtrate under reduced pressure was purified by column chromatography (SiO₂; ethyl acetate/hexane, 1:9, v/v) to obtain compound 112 (300 mg, 70%) in a clear liquid state.

¹H NMR (CDCl₃, 400 MHz): δ 0.85-0.88 (t, *J =* 6.9 Hz, 12H), 1.00 (s, 3H), 1.04 (s, 3H), 1.27 (m, 36H), 1.55-1.59 (m, 9H), 1.73 (s, 1H), 1.90-1.97 (m, 4H), 2.26-2.31 (m, 4H), 2.34-2.41 (m, 6H), 2.44-2.48 (t, *J* = 7.2 Hz, 3H), 2.61-2.64 (t, *J* = 5.9 Hz, 2H), 3.45-3.59 (m, 2H), 3.83-3.86 (m, 1H), 3.96-4.04 (m, 3H), 4.18-4.23 (m, 3H), 4.38-4.40 (m, 2H), 4.95 (s, 1H), 5.04-5.09 (m, 2H), 6.67-6.70 (t, *J =* 5.9 Hz, 1H), 7.29-7.33 (t, *J =* 7.4 Hz, 2H), 7.39-7.42 (t, *J* = 7.4 Hz, 2H), 7.57-7.58 (d, *J =* 6.4 Hz, 2H), 7.76-7.77 (d, *J =* 7.5 Hz, 2H);¹³C NMR (CDCl₃, 100 MHz): δ 14.03, 14.06, 19.69, 20.10, 20.87, 21.26, 22.52, 22.59, 24.88, 25.07, 28.92, 29.03, 29.06, 30.71, 31.60, 31.65, 32.95, 33.12, 33.21, 33.40, 33.64, 34.12, 34.64, 37.36, 46.66, 64.73, 64.80, 66.70, 69.21, 71.71, 71.84, 120.08, 124.99, 127.16, 127.89, 141.29, 143.55, 143.62, 167.91, 171.57, 172.32, 172.39, 172.47, 172.65, 173.65.

### 2-33-2. 17-Hexyl-7,7-dimethyl-6-((5-((2-(octanoyloxy)octyl)oxy)-5-oxopentanoyl)oxy)-5,10,14,19-tetraoxo-9,15,18-trioxa-4-azahexacosanoic acid (113)

A solution of 20% piperidine:80% DMF (v/v) was prepared in a reaction vessel and then cooled to 0°C. In another reaction vessel, compound 112 (300 mg) was dissolved in DMF (20 mL) and cooled to 0°C with stirring for 5 minutes. Thereafter, the cooled 20% piperidine solution (30 mL) was added in the presence of argon gas and stirred for 10 minutes. After checking that the reaction was terminated with TLC (SiO₂; hexane/ethyl acetate, 5:5), 1 M aqueous HCl solution was added to adjust to pH 3, and ethyl acetate (200 mL) was further added to the reaction mixture solution for dilution. The organic layer was then washed with distilled water (2 × 200 mL) and saturated aqueous NaCl solution (2 × 200 mL) sequentially. The obtained organic layer was filtered after removing moisture with anhydrous MgSO₄ to distill the filtrate under reduced pressure and then was purified by column chromatography (SiO₂; ethyl acetate/hexane/acetic acid, 0:1:0.1 to 5:5:0.1, v/v/v) to obtain compound 113 (238 mg, 94 %) in a clear liquid state.

¹H NMR (CDCl₃, 400 MHz): δ 0.84-0.87 (t, *J =* 6.5 Hz, 12H), 1.01 (s, 3H), 1.05 (s, 3H), 1.28 (m, 35H), 1.56-1.60 (m, 9H), 1.74 (s, 1H), 1.91-1.96 (m, 4H), 2.25-2.33 (m, 4H), 2.35-2.42 (m, 6H), 2.45-2.49 (t, *J* = 7.7 Hz, 3H), 2.62-2.65 (t, *J* = 5.7 Hz, 2H), 3.46-3.58 (m, 2H), 3.84-3.87 (m, 1H), 3.96-4.01 (m, 3H), 4.17-4.26 (m, 2H), 4.96 (s, 1H), 5.05-5.10 (m, 2H), 6.68-6.72 (t, *J =* 5.7 Hz, 1H);¹³_{C} NMR (CDCl₃, 100 MHz): δ 14.00, 19.82, 19.87, 21.03, 20.87, 21.23, 22.48, 22.54, 24.84, 25.02, 28.86, 29.00, 30.65, 31.55, 31.60, 32.86, 33.06, 33.21, 33.34, 34.10, 34.76, 37.28, 64.78, 69.24, 71.76, 72.05, 168.02, 171.67, 172.53, 172.65, 172.79, 173.63, 173.82.

### 2-33-3. O,O'-(2,2-Dimethyl-4-((3-(2-morpholinoethoxy)-3-oxopropyl)amino)-4-oxobutane-1,3-diyl) bis(2-(octanoyloxy)octyl) diglutarate] (Compound 39)

After putting compound 113 (200 mg, 1 equiv.) in a reaction vessel and adding DCM (30 mL) to dissolve, EDCI•HCl (60 mg, 1.5 equiv.) and DMAP (5 mg, 0.2 equiv.) were added, and then a reaction mixture solution was stirred vigorously at 5°C in the presence of argon gas for 10 minutes. Thereafter, a solution obtained by dissolving compound 101 (30 mg, 1.1 equiv.) in DCM (10 mL) was added dropwise while maintaining at 5°C and then stirred at room temperature for 6 hours. After checking that the reaction was terminated with TLC (SiO₂; ethyl acetate), DCM (100 mL) was further added, an organic layer was washed with saturated aqueous NaHCO₃ solution (2 × 200 mL) and saturated aqueous NaCl solution (1 × 200 mL), and then the filtrate filtered after removing moisture from the obtained organic layer with anhydrous MgSO₄ was distilled under reduced pressure. The reaction mixture was then purified by column chromatography (SiO₂; ethyl acetate/hexane, 7:3 to 10:0, v/v) to obtain compound 39 (172 mg, 75%) in a clear liquid state.

¹HNMR (CDCl₃, 400 MHz): δ 0.84-0.88 (t, *J =* 6.9 Hz, 12H), 1.00 (s, 3H), 1.04 (s, 3H), 1.26-1.28 (m, 34H), 1.57-1.61 (m, 8H), 1.89-1.99 (m, 4H), 2.27-2.30 (t, *J* = 7.5 Hz, 4H), 2.34-2.40 (m, 6H), 2.45-2.49 (m, 6H), 2.52-2.55 (t, *J* = 6 Hz, 2H), 2.58-2.61 (t, *J =* 5.8 Hz, 2H), 3.44-3.56 (m, 2H), 3.67-3.69 (t, *J =* 4.6 Hz, 4H), 3.82-3.85 (d, *J =* 11 Hz, 1H), 3.98-4.04 (m, 3H), 4.16-4.26 (m, 4H), 4.94 (s, 1H), 5.05-5.06 (m, 2H), 6.70-6.72 (t, *J* = 6 Hz, 1H);¹³_{C} NMR (CDCl₃, 100 MHz): δ 13.99, 14.02, 19.93, 19.97, 20.09, 20.85, 21.25, 22.49, 22.56, 24.86, 25.05, 28.88, 28.99, 29.03, 30.69, 31.57, 31.62, 32.93, 33.11, 33.20, 33.38, 33.64, 34.10, 34.66, 37.33, 53.80, 56.98, 61.77, 64.67, 64.67, 64.70, 64.76, 66.84, 69.18, 71.70, 71.84, 76.63, 167.81, 171.53, 172.29, 172.34, 172.39, 172.50, 173.46, 173.49.

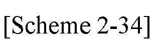

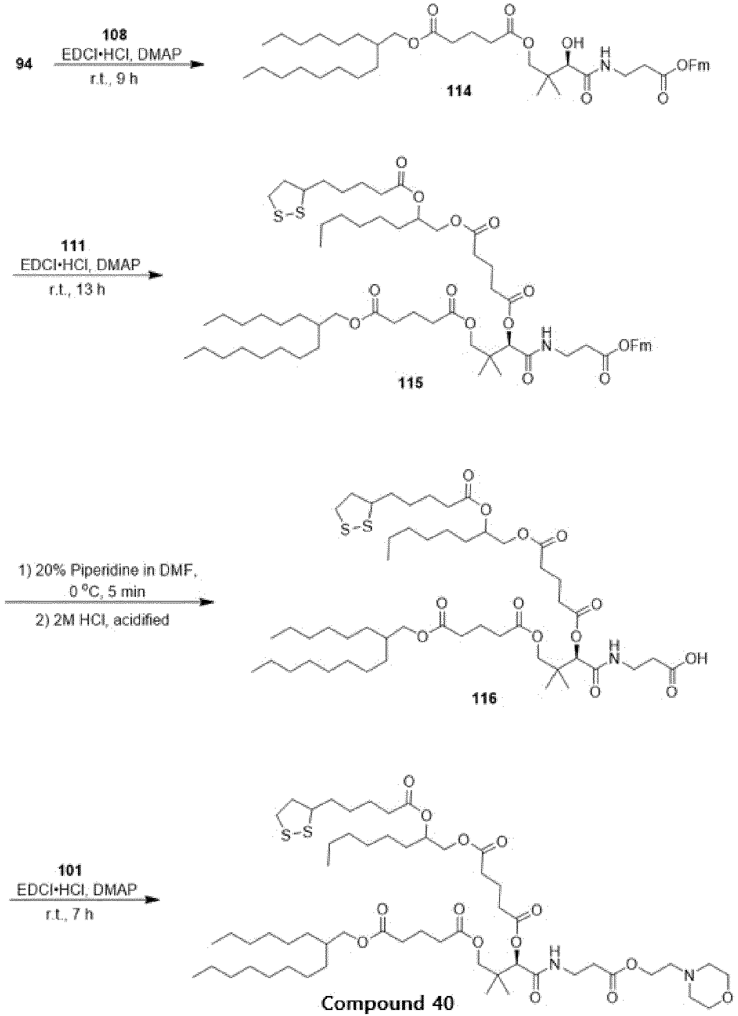

### 2-34-1. 4-((3-((9H-Fluoren-9-yl)methoxy)-3-oxopropyl)amino)-3-hydroxy-2,2-dimethyl-4-oxobutyl (2-hexyldecyl) glutaratel (114)

After putting compound 108 (1.07 g, 1.1 equiv.), EDCI•HCl (0.72 g, 1.5 equiv.), and DMAP (46 mg, 0.15 equiv.) in a reaction vessel and adding DCM (20 mL), stirring was performed vigorously in the presence of argon gas at room temperature for 20 minutes. Compound 94 (1 g, 1 equiv.) was dissolved in DCM (20 mL) in another reaction vessel, cooled to 5°C, and then added dropwise to a reaction mixture prepared in advance at 5°C in the presence of argon gas for 30 minutes. Thereafter, stirring was performed at room temperature for 9 hours, termination of the reaction was checked with TLC (SiO₂; hexane/ethyl acetate, 6:4), and a solvent was removed by distillation under reduced pressure. Thereafter, DCM (50 mL) was further added and washed with saturated aqueous NaHCO₃ solution (2 × 100 mL) and saturated aqueous NaCl solution (1 × 100 mL). The filtrate filtered after removing moisture from collected organic layers with anhydrous MgSO₄ was concentrated by distillation under reduced pressure and purified by column chromatography (SiO₂; EtOAc/hexane, 1:9 to 4:6, v/v) to obtain compound 114 (1.49 g, 72%) in a clear liquid state.

¹H NMR (CDCl₃, 400 MHz): δ 0.85-0.89 (t, *J* = 6.7 Hz, 9H), 1.04 (s, 3H), 1.25 (m, 25H), 1.60 (bs, 1H), 1.92-1.98 (m, 2H), 2.34-2.47 (m, 4H), 2.60-2.63 (t, *J* = 5.9 Hz, 2H), 3.50-3.55 (m, 2H), 3.60-3.61 (d, *J =* 5.8 Hz, 1H), 3.73-3.76 (d, *J =* 11, 1H), 3.84-3.86 (d, *J =* 5.7, 1H), 3.95-3.96 (d, *J* = 5.6, 2H), 4.16-4.21 (m, 2H), 4.39-4.42 (m, 2H), 7.00-7.03 (t, *J* = 11.7, 1H), 7.29-7.32 (t, *J* = 7.4, 2H), 7.38-4.41 (t, *J* = 7.4, 2H), 7.55-7.57 (d, *J* = 7.4, 2H), 7.74-7.76 (d, *J =* 7.52, 2H); ¹³C NMR (CDCl₃, 100 MHz): δ 14.09, 14.10, 19.57, 20.25, 21.37, 22.64, 22.66, 26.64, 26.69, 29.30, 29.55, 26.59, 29.94, 31.22, 31.80, 31.89, 33.37, 33.38, 34.08, 34.62, 37.26, 38.62, 46.68, 68.57, 67.46, 70.56, 74.65, 102.07, 124.94, 127.15, 127.85, 141.30, 143.54, 171.92, 172.15, 173.25, 173.40.

### 2-34-2. 2-((5-(1,2-Dithiolan-3-yl)pentanoyl)oxy)octyl (1-(9H-fluoren-9-yl)-19-hexyl-9,9-dimethyl-3,7,12,16-tetraoxo-2,11,17-trioxa-6-azaheptacosan-8-yl) glutarate (115)

After dissolving compound 114 (1.49 g, 1 equiv.) in DCM (50 mL) in a reaction vessel, compound 111 (1.08 g, 1.2 equiv.), EDCI•HCl (0.58 g, 1.5 equiv.), and DMAP (37 mg, 0.15 equiv.) were added and stirred at room temperature for 13 hours. After checking that the reaction was terminated with TLC (SiO₂; hexane/ethyl acetate, 7:3), DCM (100 mL) was further added, an organic layer was washed with saturated aqueous NaHCO₃ solution (2 × 200 mL) and saturated aqueous NaCl solution (1 × 200 mL), and then the filtrate filtered after removing moisture from the collected organic layer with anhydrous MgSO₄ was concentrated by distillation under reduced pressure. The obtained reaction mixture was purified by column chromatography (SiO₂; ethyl acetate/hexane, 1:9 to 3:7, v/v) to obtain compound 115 (1.41 g, 60%) in a light yellow liquid state.

¹H NMR (CDCl₃, 400 MHz): δ 0.85-0.89 (t, *J* = 3.2 Hz, 9H), 1.00 (s, 3H), 1.04 (s, 3H), 1.25 (m, 34H), 1.44-1.65 (m, 12H), 1.84-1.97 (m, 5H), 2.27-2.30 (t, *J* = 6.5 Hz, 2H), 2.32-2.48 (m, 9H), 2.60-2.63 (t, *J* = 6.0 Hz, 2H), 3.04-3.17 (m, 2H), 3.44-3.58 (m, 3H), 3.82-3.85 (t, *J =* 12.0 Hz, 1H), 3.94-4.01 (m, 3H), 4.02-4.05 (t, *J =* 11.0 Hz, 1H), 4.18-4.25 (m, 2H), 4.37-4.39 (d, *J =* 7.2 Hz, 2H), 4.94-4.95 (m, 1H), 5.05 (bs, 1H), 6.68-6.71 (t, *J =* 5.56 Hz, 1H), 7.28-7.32 (t, *J* = 7.7 Hz, 2H), 7.37-7.41 (t, *J* = 7.4 Hz, 2H), 7.56-7.58 (d, *J* = 7.4 Hz, 2H), 7.74-7.76 (d, *J* = 7.4 Hz, 2H); ¹³C NMR (CDCl₃, 100 MHz): δ 14.03, 14.09, 14.11, 19.95, 19.99, 20.11, 20.85, 21.32, 22.21, 22.62, 22.65, 24.56, 25.05, 26.62, 26.66, 28.67, 28.99, 29.27, 29.53, 29.57, 29.92, 30.63, 31.21, 31.58, 31.78, 31.87, 33.20, 33.33, 33.63, 33.82, 34.55, 34.66, 37.25, 37.34, 38.44, 40.18, 46.66, 56.29, 64.87, 66.67, 67.29, 69.17, 71.75, 76.69, 120.06, 124.99, 127.15, 127.85, 141.26, 143.56, 167.91, 171.55, 172.30, 172.40, 172.56, 173.03, 173.06.

### 2-34-3. 21-(1,2-Dithiolan-3-yl)-15-hexyl-6-(1-((5-((2-hexyldecyl)oxy)-5-oxopentanoyl)oxy)-2-methylpropan-2-yl)-5,8,12,17-tetraoxo-7,13,16-trioxa-4-azahenicosanoic acid (116)

A solution of 20% piperidine:80% DMF (v/v) was prepared in a reaction vessel and left to cool at 0°C. Compound 115 (1.41 g) was added to another reaction vessel to dissolve in DMF (20 mL), stirring was performed at 0°C for 5 minutes, and then the prepared 20% piperidine solution (60 mL) was added to the reaction mixture in the presence of argon gas, followed by stirring for 5 minutes. After checking that the reaction was terminated with TLC (SiO₂; ethyl acetate), 1 M aqueous HCl solution was added to acidify to pH 3, and then the reaction mixture was diluted with ethyl acetate (200 mL) and washed with distilled water (2 × 200 mL) and saturated aqueous NaCl solution (2 × 200 mL). The filtrate filtered after removing moisture from collected organic layers with anhydrous Na₂SO₄ was concentrated by distillation under reduced pressure and then purified by column chromatography (SiO₂; ethyl acetate/MeOH, 10:0 to 9:1, v/v) to obtain compound 116 (0.79 g, 66%) in a yellow liquid.

¹H NMR (CDCl₃, 400 MHz): δ 0.85-0.88 (t*, J* = 3.1 Hz, 9H), 1.01 (s, 3H), 1.10 (s, 3H), 1.24 (m, 35H), 1.45-1.67 (m, 12H), 1.85-1.92 (m, 5H), 2.28-2.32 (t, *J =* 6.4 Hz, 2H), 2.31-2.47 (m, 9H), 2.61-2.62 (t, *J* = 6.1 Hz, 2H), 3.05-3.18 (m, 2H), 3.45-3.59 (m, 3H), 3.85-3.88 (t, *J* = 9.0 Hz, 1H), 3.95-4.02 (m, 3H), 4.03-4.07 (t, *J =* 11.0 Hz, 1H), 4.21 (d, *J =* 7.1 Hz, 1H), 4.90-4.93 (m, 1H), 5.06 (bs, 1H), 6.69-6.72 (t, *J =* 5.62 Hz, 1H); ¹³C NMR (CDCl₃, 100 MHz): δ 14.01, 14.10, 19.94, 20.01, 20.13, 20.84, 21.35, 22.25, 22.63, 22.66, 24.57, 25.04, 26.63, 26.68, 28.68, 20.03, 29.28, 29.54, 29.55, 29.91, 30.64, 31.22, 31.57, 31.79, 31.87, 33.21, 33.34, 33.64, 33.81, 34.56, 34.67, 37.23, 37.33, 38.45, 40.19, 56.27, 66.66, 67.32, 69.19, 71.78, 76.71, 167.96, 171.57, 172.33, 172.42, 172.57, 173.02, 173.09.

### 2-34-4. 2-((5-(1,2-Dithiolan-3-yl)pentanoyl)oxy)octyl (20-hexyl-10,10-dimethyl-1-morpholino-4,8,13,17-tetraoxo-3,12,18-trioxa-7-azaoctacosan-9-yl) glutarate (Compound 40)

Compound 116 (434 mg, 1 equiv.) was dissolved in DCM (30 mL) in a reaction vessel, EDCI•HCl (126 mg, 1.5 equiv.) and DMAP (11 mg, 0.2 equiv.) were added and stirred vigorously in the presence of argon gas at 5°C. Then, a solution in which compound 101 (70 mg, 1.2 equiv.) was dissolved in DCM (10 mL) was added dropwise at 5°C and stirred at room temperature for 7 hours. After checking that the reaction was terminated with TLC (SiO₂; ethyl acetate), DCM (100 mL) was further added and washed with saturated aqueous NaHCO₃ solution (2 × 200 mL) and saturated aqueous NaCl solution (1 × 200 mL). The filtrate filtered after removing moisture from the collected organic layers with anhydrous MgSO₄ was concentrated by distillation under reduced pressure, and then the obtained reaction mixture was purified by column chromatography (SiO₂; ethyl acetate/hexane, 5:5 to 10:0, v/v) to obtain compound 40 (378 mg, 78 %) as a yellow liquid state.

¹H NMR (CDCl₃, 400 MHz): δ 0.80-0.83 (t, *J =* 5.8 Hz, 9H), 0.96 (s, 3H), 0.83 (s, 3H), 1.21 (m, 30H), 1.39-1.43 (m, 3H), 1.51-1.65 (m, 8H), 1.84-1.92 (m, 5H), 2.25-2.36 (m, 8H), 2.38-2.44 (m, 7H), 2.47-2.50 (t, *J =* 5.9 Hz, 2H), 2.54-2.57 (t, *J =* 5.8 Hz, 2H), 3.01-3.14 (m, 2H), 3.39-3.52 (m, 3H), 3.62-3.64 (t, *J =* 4.2 Hz, 4H), 3.77-3.80 (m, 1H), 3.91-4.00 (m, 4H), 4.01-4.20 (m, 3H), 4.88-4.90 (m, 1H), 5.01 (bs, 1H), 6.67-6.69 (t, *J =* 5.64 Hz, 1H); ¹³C NMR (CDCl₃, 100 MHz): δ 14.02, 14.09, 19.94, 19.97, 20.08, 20.87, 21.28, 22.50, 22.61, 22.64, 24.56, 25.06, 26.61, 26.65, 28.69, 28.99, 29.27, 29.52, 29.57, 29.91, 30.69, 31.19, 31.58, 31.77, 31.86, 32.95, 33.02, 33.19, 33.32, 33.64, 33.83, 34.56, 34.65, 37.24, 37.32, 38.45, 40.20, 53.80, 56.29, 56.97, 61.78, 64.87, 66.84, 67.30, 69.16, 71.63, 71.77, 76.67, 167.80, 171.56, 172.32, 172.42, 172.58, 172.99, 173.05, 173.12.

### Example 3: Synthesis of butyl lithocholic acid

Lithocholic acid and n-butanol were put into a reaction vessel, and then HCl solution (35~37% aqueous solution, 1 equiv.) was injected, followed by stirring at room temperature for 24 hours. After distilling alcohol using a rotary vacuum distiller, the mixture was dissolved again in DCM to extract impurities with saturated aqueous NaHCO₃ solution and saturated brine. Thereafter, the DCM layer was filtered after removing moisture using anhydrous Na₂SO₄, and the filtered solution was dried using the rotary vacuum distiller and then purified by column chromatography (SiO₂, DCM/MeOH, a volume ratio of 19:1~49:1). Thereby, a lithocholic acid derivative compound (92%) was obtained.

MS (ESI-MS) calcd. for C₂₈H₄₆NO₂ [M+H]⁺ 428.3523, found 428.3525.

### Example 4: Synthesis of trehalose-based lipid

After dissolving trehalose dihydrate (200 mg, 0.53 mmol) in pyridine (5 mL), TBTU (421 mg, 1.3 mmol), DIPEA (320 µℓ, 1.2 mmol), and oleic acid (370 µℓ, 1.2 mmol) were put in order, and argon gas was charged, followed by stirring at room temperature for 20 hours. After removing solvent using a rotary vacuum distiller, drying was performed after primary purification using column chromatography (SiO₂, MeOH/EtOAc 2:98 →10:90). After washing the dried solid several times with EtOAc, it was completely dried to obtain 287 mg (62%) of 6,6'-trehalose dioleate.

¹H NMR (400 MHz, MeO_{D}) δ 5.38 (t, *J =* 4.5 Hz, 4H), 5.08 (d, *J =* 3.8 Hz, 2H), 4.39 (dd, *J* = 2.2, 11.9 Hz, 2H), 4.23 (dd, *J* = 5.1, 11.9 Hz, 2H), 4.04 (ddd, *J* = 2.1, 5.3, 10.2 Hz, 2H), 3.81 (dd, *J* = 9.4, 9.6 Hz, 2H), 3.50 (dd, *J =* 3.6, 9.6 Hz, 2H), 3.35 (dd, *J =* 8.8, 10.0 Hz, 2H), 2.04-2.09 (m, 8H), 2.37 (t, *J* = 7.4 Hz, 4H), 1.61-1.67 (m, 4H), 1.29-1.41 (m, 40H), 0.93 (t, *J* = 6.8 Hz, 6H); ¹³C NMR (100 MHz, MeO_{D}) δ 174.0, 129.5, 129.4, 93.8, 73.2, 71.8, 70.5, 70.1, 63.0, 33.7, 31.7, 29.5, 29.4, 29.2, 29.1, 29.0, 28.9, 28.8, 28.8, 26.8, 24.7, 22.4, 13.1.

### Example 5: Preparation of nucleic acid molecules by RNA platform

Prepared was an RNA platform which has an IRES element derived from encephalomyocarditis virus (EMCV) and into which a nucleic acid sequence encoding Renilla Luciferase (R/L), known as one of the reporter genes, is inserted as a target sequence. A template DNA was designed and, using the IVT process, nucleic acid molecules for a single-stranded RNA platform were prepared.

Prepared was an RNA platform which includes a Cap1 structure and into which a nucleic acid sequence encoding influenza hemagglutinin (HA) is inserted as a target sequence. A template DNA was designed and, using the IVT process, nucleic acid molecules for a single-stranded RNA platform were prepared.

### Example 6: Preparation of lipid nanoparticles

After preparing lipid nanoparticles with RNA solution (50 mM sodium citrate buffer, 110 mM NaCl, pH=4.0) at a concentration of 0.625 mg/mL and a lipid mixture solution (Ethanol) by means of a laboratory mixer and emulsifier (NanoAssemblr Spark, Precision Nanosystems, Inc.), provision was followed by undergoing solvent conversion with physiological saline or PBS using a centrifugation filter tube (UFC5010, Amicon).

Specifically, preparation was carried out in the same content as in Tables 1 to 3 below.

**TABLE 1**

| | Ionized lipid (mol%) | Helper lipid (mol%) | Cholesterol (mol%) | PEG-containing lipids (mol%) | Additive (mol%) |
|---|---|---|---|---|---|
| LNP98 | DLin-MC3-DMA (25) | DOPE (10) | Butyl lithocholate (38.5) | DMG-PEG (1.5) | 6,6'-Trehalose dioleate (25) |
| LNP126 | SM-102 (25) | DOPE (10) | Butyl lithocholate (38.5) | DMG-PEG (1.5) | 6,6'-Trehalose dioleate (25) |
| LNP127 | DLin-MC3-DMA (25) | Compound 10 (25) DOPE (10) | Butyl lithocholate (38.5) | DMG-PEG (1.5) | - |
| LNP128 | SM-102 (50) | DSPC (10) | Cholesterol (38.5) | DMG-PEG (1.5) | - |
| LNP130 | DLin-MC3-DMA (25) | Compound 10 (10) | Butyl lithocholate (38.5) | DMG-PEG (1.5) | 6,6'-Trehalose dioleate (25) |
| LNP131 | SM-102 (25) | Compound 10 (25) DOPE (10) | Butyl lithocholate (38.5) | DMG-PEG (1.5) | - |
| LNP132 | SM-102 (25) | Compound 10 (35) | Butyl lithocholate (38.5) | DMG-PEG (1.5) | - |
| LNP133 | SM-102 (50) | Compound 10 (10) | Cholesterol (38.5) | DMG-PEG (1.5) | - |
| LNP136 | SM-102 (25) | Compound 10 (10) | Butyl lithocholate (38.5) | DMG-PEG (1.5) | 6,6'-Trehalose dioleate (25) |
| LNP138 | SM-102 (25) | Compound 11 (10) | Butyl lithocholate (38.5) | DMG-PEG (1.5) | 6,6'-Trehalose dioleate (25) |
| LNP139 | SM-102 (50) | Compound 11 (10) | Cholesterol (38.5) | DMG-PEG (1.5) | - |

**TABLE 2**

| | Ionized lipid (mol%) | Helper lipid (mol%) | Cholesterol (mol%) | PEG-containing lipids (mol%) | Additive (mol%) |
|---|---|---|---|---|---|
| LNP98 | DLin-MC3-DMA (25) | DOPE (10) | Butyl lithocholate (38.5) | DMG-PEG (1.5) | 6,6'-Trehalose dioleate (25) |
| LNP126 | SM-102 (25) | DOPE (10) | Butyl lithocholate (38.5) | DMG-PEG (1.5) | 6,6'-Trehalose dioleate (25) |
| LNP109 | DLin-MC3-DMA (25) | DOPE (10) | Vitamin A (Retinoic acid) (38.5) | DMG-PEG (1.5) | 6,6'-Trehalose dioleate (25) |
| LNP110 | DLin-MC3-DMA (25) | DOPE (10) | Butyl lithocholate/C oenzyme Q10 (19.25/ 19.25) | DMG-PEG (1.5) | 6,6'-Trehalose dioleate (25) |
| LNP111 | DLin-MC3-DMA (25) | DOPE (10) | α-Tocopherol (38.5) | DMG-PEG (1.5) | 6,6'-Trehalose dioleate (25) |
| LNP114 | DLin-MC3-DMA (25) | DOPE (10) | Vitamin K (38.5) | DMG-PEG (1.5) | 6,6'-Trehalose dioleate (25) |
| LNP115 | DLin-MC3-DMA (25) | DOPE (10) | Vitamin K2 (38.5) | DMG-PEG (1.5) | 6,6'-Trehalose dioleate (25) |
| LNP117 | DLin-MC3-DMA (25) | DOPE (10) | Vitamine C palimate (38.5) | DMG-PEG (1.5) | 6,6'-Trehalose dioleate (25) |
| LNP118 | DLin-MC3-DMA (25) | DOPE (10) | Beta-carotene (38.5) | DMG-PEG (1.5) | 6,6'-Trehalose dioleate (25) |
| LNP119 | DLin-MC3-DMA (34) | DOPE (10) | Coenzyme Q10 (20.5) | DMG-PEG (1.5) | 6,6'-Trehalose dioleate (34) |
| LNP123 | DLin-MC3-DMA (25) | DOPE (10) | Retinol (38.5) | DMG-PEG (1.5) | 6,6'-Trehalose dioleate (25) |
| LNP168 | DLin-MC3-DMA (25) | DOPE (10) | Luteolin (38.5) | DMG-PEG (1.5) | 6,6'-Trehalose dioleate (25) |
| LNP200 | SM-102 (25) | DOPE (10) | 3-Oxo-5β-cholanoic acid (38.5) | DMG-PEG (1.5) | 6,6'-Trehalose dioleate (25) |

**TABLE 3**

| | Ionized lipid (mol%) | Helper lipid (mol%) | Cholesterol (mol%) | PEG-containing lipids (mol%) | Additive (mol%) |
|---|---|---|---|---|---|
| LNP98 | DLin-MC3-DMA (25) | DOPE (10) | Butyl lithocholate (38.5) | DMG-PEG (1.5) | 6,6'-trehalose dioleate (25) |
| LNP126 | SM-102 (25) | DOPE (10) | Butyl lithocholate (38.5) | DMG-PEG (1.5) | 6,6'-trehalose dioleate (25) |
| LNP128 | SM-102 (50) | DSPC (10) | Cholesterol (38.5) | DMG-PEG (1.5) | - |
| LNP142 | SM-102 (25) | Compound 11 (10) | Vitamin A (38.5) | DMG-PEG (1.5) | 6,6'-trehalose dioleate (25) |
| LNP 146 | Compound 12 (25) | DOPE (10) | Butyl lithocholate (38.5) | DMG-PEG (1.5) | 6,6'-trehalose dioleate (25) |
| LNP 147 | Compound 13 (25) | DOPE (10) | Butyl lithocholate (38.5) | DMG-PEG (1.5) | 6,6'-trehalose dioleate (25) |
| LNP148 | Compound 12 (25) | Compound 11 (10) | Vitamin A (38.5) | DMG-PEG (1.5) | 6,6'-trehalose dioleate (25) |
| LNP149 | Compound 13 (25) | Compound 11 (10) | Vitamin A (38.5) | DMG-PEG (1.5) | 6,6'-trehalose dioleate (25) |
| LNP150 | Compound 12 (50) | Compound 11 (10) | Vitamin A (38.5) | DMG-PEG (1.5) | - |
| LNP151 | Compound 13 (50) | Compound 11 (10) | Vitamin A (38.5) | DMG-PEG (1.5) | - |
| LNP152 | Compound 24 (25) | DOPE (10) | Butyl lithocholate (38.5) | DMG-PEG (1.5) | 6,6'-trehalose dioleate (25) |
| LNP153 | Compound 24 (50) | DSPC (10) | Cholesterol (38.5) | DMG-PEG (1.5) | - |
| LNP159 | Compound 16 (25) | DOPE (10) | Butyl lithocholate (38.5) | DMG-PEG (1.5) | 6,6'-trehalose dioleate (25) |
| LNP 160 | Compound 16 (50) | DSPC (10) | Cholesterol (38.5) | DMG-PEG (1.5) | - |
| LNP 166 | Compound 14 (25) | DOPE (10) | Butyl lithocholate (38.5) | DMG-PEG (1.5) | 6,6'-trehalose dioleate (25) |
| LNP167 | Compound 14 (50) | DSPC (10) | Cholesterol (38.5) | DMG-PEG (1.5) | - |
| LNP178 | Compound 16 (50) | Compound 11 (10) | Cholesterol (38.5) | DMG-PEG (1.5) | |
| LNP179 | Compound 14 (50) | Compound 11 (10) | Cholesterol (38.5) | DMG-PEG (1.5) | - |
| LNP181 | Compound 16 (25) | Compound 11 (10) | Butyl lithocholate (38.5) | DMG-PEG (1.5) | 6,6'-trehalose dioleate (25) |
| LNP182 | Compound 14 (25) | Compound 11 (10) | Butyl lithocholate (38.5) | DMG-PEG (1.5) | 6,6'-trehalose dioleate (25) |
| LNP185 | Compound 16 (25) | Compound 11 (10) | Quercetin (38.5) | DMG-PEG (1.5) | 6,6'-trehalose dioleate (25) |
| LNP187 | Compound 16 (25) | Compound 11 (10) | 3-Oxo-5β-cholanoic acid (38.5) | DMG-PEG (1.5) | 6,6'-trehalose dioleate (25) |
| LNP 192 | Compound 15 (25) | DOPE (10) | Butyl lithocholate (38.5) | DMG-PEG (1.5) | 6,6'-trehalose dioleate (25) |
| LNP193 | Compound 15 (50) | DSPC (10) | Cholesterol (38.5) | DMG-PEG (1.5) | - |
| LNP205 | Compound 14 (50) | Compound 11 (10) | 3-Oxo-5β-cholanoic acid (38.5) | DMG-PEG (1.5) | |
| LNP208 | Compound 16 (30) | Compound 11 (10) | 3-Oxo-5β-cholanoic acid (28.2) | DMG-PEG (1.8) | 6,6'-trehalose dioleate (30) |
| LNP209 | Compound 16 (30) | Compound 11 (10) | CoenzymeQ10 (28.2) | DMG-PEG (1.8) | 6,6'-trehalose dioleate (30) |
| LNP210 | Compound 16 (30) | Compound 11 (30) | 3-Oxo-5β-cholanoic acid (38.5) | DMG-PEG (1.5) | |
| LNP211 | Compound 18 (25) | DOPE (10) | Butyl lithocholate (38.5) | DMG-PEG (1.5) | 6,6'-trehalose dioleate (25) |
| LNP212 | Compound 18 (50) | DSPC (10) | Cholesterol (38.5) | DMG-PEG (1.5) | - |
| LNP213 | Compound 18 (25) | Compound 11 (10) | 3-Oxo-5β-cholanoic acid (38.5) | DMG-PEG (1.5) | 6,6'-trehalose dioleate (25) |
| LNP214 | Compound 18 (30) | Compound 11 (10) | 3-Oxo-5β-cholanoic acid (28.2) | DMG-PEG (1.8) | 6,6'-trehalose dioleate (30) |
| LNP216 | Compound 14 (25) | Compound 11 (10) | 3-Oxo-5β-cholanoic acid (38.5) | DMG-PEG (1.5) | 6,6'-trehalose dioleate (25) |
| LNP217 | Compound 14 (30) | Compound 11 (10) | 3-Oxo-5β-cholanoic acid (28.2) | DMG-PEG (1.8) | 6,6'-trehalose dioleate (30) |
| LNP224 | Compound 17 (25) | DOPE (10) | Butyl lithocholate (38.5) | DMG-PEG (1.5) | 6,6'-trehalose dioleate (25) |
| LNP225 | Compound 17 (50) | DSPC (10) | Cholesterol (38.5) | DMG-PEG (1.5) | - |
| LNP226 | Compound 17 (25) | Compound 11 (10) | 3-Oxo-5β-cholanoic acid (38.5) | DMG-PEG (1.5) | 6,6'-trehalose dioleate (25) |
| LNP227 | Compound 19 (25) | DOPE (10) | Butyl lithocholate (38.5) | DMG-PEG (1.5) | 6,6'-trehalose dioleate (25) |
| LNP228 | Compound 19 (50) | DSPC (10) | Cholesterol (38.5) | DMG-PEG (1.5) | - |
| LNP229 | Compound 19 (25) | Compound 11 (10) | 3-Oxo-5β-cholanoic acid (38.5) | DMG-PEG (1.5) | 6,6'-trehalose dioleate (25) |
| LNP230 | Compound 20 (25) | DOPE (10) | Butyl lithocholate (38.5) | DMG-PEG (1.5) | 6,6'-trehalose dioleate (25) |
| LNP231 | Compound 20 (50) | DSPC (10) | Cholesterol (38.5) | DMG-PEG (1.5) | - |
| LNP232 | Compound 20 (25) | Compound 11 (10) | 3-Oxo-5β-cholanoic acid (38.5) | DMG-PEG (1.5) | 6,6'-trehalose dioleate (25) |
| LNP256 | Compound 23 (25) | DOPE (10) | Butyl lithocholate (38.5) | DMG-PEG (1.5) | 6,6'-trehalose dioleate (25) |
| LNP257 | Compound 23 (50) | DSPC (10) | Cholesterol (38.5) | DMG-PEG (1.5) | - |
| LNP278 | Compound 21 (25) | DOPE (10) | Butyl lithocholate (38.5) | DMG-PEG (1.5) | 6,6'-trehalose dioleate (25) |
| LNP279 | Compound 21 (50) | DSPC (10) | Cholesterol (38.5) | DMG-PEG (1.5) | - |
| LNP280 | Compound 22 (25) | DOPE (10) | Butyl lithocholate (38.5) | DMG-PEG (1.5) | 6,6'-trehalose dioleate (25) |
| LNP281 | Compound 22 (50) | DSPC (10) | Cholesterol (38.5) | DMG-PEG (1.5) | - |
| LNP291 | Compound 25 (25) | DOPE (10) | Butyl lithocholate (38.5) | DMG-PEG (1.5) | 6,6'-trehalose dioleate (25) |
| LNP292 | Compound 25 (50) | DSPC (10) | Cholesterol (38.5) | DMG-PEG (1.5) | - |
| LNP293 | Compound 25 (25) | Compound 11 (10) | 3-Oxo-5β-cholanoic acid (38.5) | DMG-PEG (1.5) | 6,6'-trehalose dioleate (25) |
| LNP294 | Compound 26 (25) | DOPE (10) | Butyl lithocholate (38.5) | DMG-PEG (1.5) | 6,6'-trehalose dioleate (25) |
| LNP295 | Compound 26 (50) | DSPC (10) | Cholesterol (38.5) | DMG-PEG (1.5) | - |
| LNP296 | Compound 26 (25) | Compound 11 (10) | 3-Oxo-5β-cholanoic acid (38.5) | DMG-PEG (1.5) | 6,6'-trehalose dioleate (25) |
| LNP3 10 | Compound 27 (25) | DOPE (10) | Butyl lithocholate (38.5) | DMG-PEG (1.5) | 6,6'-trehalose dioleate (25) |
| LNP311 | Compound 27 (50) | DSPC (10) | Cholesterol (38.5) | DMG-PEG (1.5) | - |
| LNP312 | Compound 27 (25) | Compound 11 (10) | 3-Oxo-5β-cholanoic acid (38.5) | DMG-PEG (1.5) | 6,6'-trehalose dioleate (25) |
| LNP313 | Compound 28 (25) | DOPE (10) | Butyl lithocholate (38.5) | DMG-PEG (1.5) | 6,6'-trehalose dioleate (25) |
| LNP314 | Compound 28 (50) | DSPC (10) | Cholesterol (38.5) | DMG-PEG (1.5) | - |
| LNP315 | Compound 28 (25) | Compound 11 (10) | 3-Oxo-5β-cholanoic acid (38.5) | DMG-PEG (1.5) | 6,6'-trehalose dioleate (25) |
| LNP323 | Compound 29 (25) | DOPE (10) | Butyl lithocholate (38.5) | DMG-PEG (1.5) | 6,6'-trehalose dioleate (25) |
| LNP324 | Compound 29 (50) | DSPC (10) | Cholesterol (38.5) | DMG-PEG (1.5) | - |
| LNP325 | Compound 30 (25) | DOPE (10) | Butyl lithocholate (38.5) | DMG-PEG (1.5) | 6,6'-trehalose dioleate (25) |
| LNP326 | Compound 30 (50) | DSPC (10) | Cholesterol (38.5) | DMG-PEG (1.5) | - |
| LNP327 | Compound 31 (25) | DOPE (10) | Butyl lithocholate (38.5) | DMG-PEG (1.5) | 6,6'-trehalose dioleate (25) |
| LNP328 | Compound 31 (50) | DSPC (10) | Cholesterol (38.5) | DMG-PEG (1.5) | - |
| LNP329 | Compound 32 (25) | DOPE (10) | Butyl lithocholate (38.5) | DMG-PEG (1.5) | 6,6'-trehalose dioleate (25) |
| LNP330 | Compound 32 (50) | DSPC (10) | Cholesterol (38.5) | DMG-PEG (1.5) | - |
| LNP331 | Compound 35 (25) | DOPE (10) | Butyl lithocholate (38.5) | DMG-PEG (1.5) | 6,6'-trehalose dioleate (25) |
| LNP332 | Compound 35 (50) | DSPC (10) | Cholesterol (38.5) | DMG-PEG (1.5) | - |
| LNP333 | Compound 36 (25) | DOPE (10) | Butyl lithocholate (38.5) | DMG-PEG (1.5) | 6,6'-trehalose dioleate (25) |
| LNP334 | Compound 36 (50) | DSPC (10) | Cholesterol (38.5) | DMG-PEG (1.5) | - |
| LNP335 | Compound 33 (25) | DOPE (10) | Butyl lithocholate (38.5) | DMG-PEG (1.5) | 6,6'-trehalose dioleate (25) |
| LNP336 | Compound 33 (50) | DSPC (10) | Cholesterol (38.5) | DMG-PEG (1.5) | - |
| LNP337 | Compound 34 (25) | DOPE (10) | Butyl lithocholate (38.5) | DMG-PEG (1.5) | 6,6'-trehalose dioleate (25) |
| LNP338 | Compound 34 (50) | DSPC (10) | Cholesterol (38.5) | DMG-PEG (1.5) | - |
| LNP412 | Compound 37 (50) | DSPC (10) | Cholesterol (38.5) | DMG-PEG (1.5) | - |
| LNP417 | Compound 38 (50) | DSPC (10) | Cholesterol (38.5) | DMG-PEG (1.5) | - |
| LNP422 | Compound 39 (50) | DSPC (10) | Cholesterol (38.5) | DMG-PEG (1.5) | - |
| LNP425 | Compound 40 (50) | DSPC (10) | Cholesterol (38.5) | DMG-PEG (1.5) | - |

### Experimental Example 1: Evaluation on delivery efficiency through identification of protein expression level

mRNA-LNP was injected into the ears of ICR mice by I.D. in an amount of 5 µg/20 µl based on RNA to identify expression.

The experimental process is as follows:
Each experimental group was prepared in a dose of 5 µg/20 µl based on RNA and then injected into each ear of mice by I.D. Thereafter, the expression was identified according to the maximum expression time of a target protein. In the case of R/L on which the experiment was actually carried out, expression and maintenance were identified according to two time periods which are 6 hours and 24 hours.

The specific experimental process is as follows:
After anesthesia using an individual respiratory anesthesia machine, a drug to be administered that is suitable for the experimental condition was injected via an insulin syringe. Thereafter, euthanasia was performed using CO₂ at each corresponding time, and then ears were cut off. While dipping the cut ears in 300 µl of 1X Renilla Lyswas buffer, ears were completely crushed using scissors and a homogenizer instrument. Thereafter, 20 µl was taken from the mixture solution, transferred to a white 96-well plate, and added with 100 µl of Promega's Renilla luciferase assay substrate, and then luminescence was measured to compare expression.

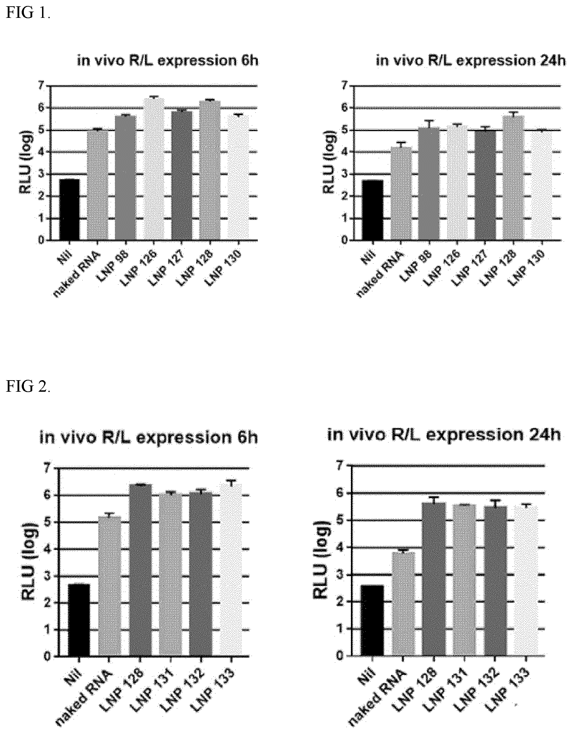

FIG. 1 shows results of analyzing an amount of R/L expressed 6 hours and 24 hours after injecting lipid nanoparticles (LNP 127, 130) of a novel helper lipid composition including Renilla Luciferase (R/L) mRNA into the ears of mice (i.d.). As shown in FIG. 1, comparing LNP 98 and LNP 127, the protein expression efficiency was maintained even when Chemical Formula 10 was replaced with helper lipid. In addition, comparing LNP 98 and LNP 130, the expression efficiency was maintained to a certain extent even when the proportion of helper lipid was increased by replacing the additive with Chemical Formula 10.

FIG. 2 shows results of analyzing an amount of R/L expressed 6 hours and 24 hours after injecting lipid nanoparticles (LNP 131, 132, 133) of the novel helper lipid composition including Renilla Luciferase (R/L) mRNA into the ears of mice (i.d.). As shown in FIG. 2, comparing LNP 128 and LNP 133, the protein expression efficiency was maintained even when Chemical Formula 10 was replaced with helper lipid. In addition, comparing LNP 131 and LNP 132, no significant difference was observed when Chemical Formula 10 was mixed with DOPE or used alone, determining that reduction in the content of ionized lipids by half does not dramatically affect the protein expression efficiency.

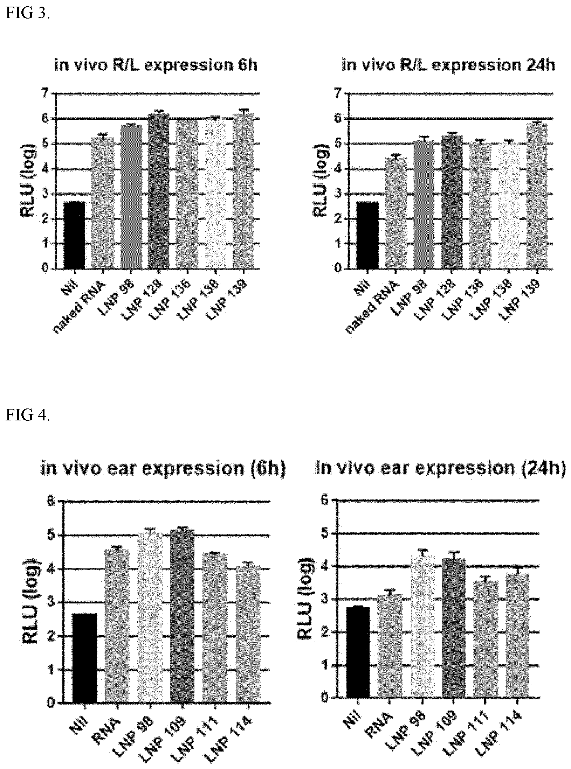

FIG. 3 shows results of analyzing an amount of R/L expressed 6 hours and 24 hours after injecting lipid nanoparticles (LNP 136, 138, 139) of the novel helper lipid composition including Renilla Luciferase (R/L) mRNA into the ears of mice (i.d.). Referring to FIG. 3, comparing LNP 128 and LNP 139, the protein expression efficiency was maintained at 6 hours even when Chemical Formula 11 was replaced with helper lipid, and the expression efficiency was rather increased at 24 hours. In addition, LNP 136 and LNP 138 with Chemical Formulas 10 and 11 added also played a valid role as helper lipid, proving the replaceability.

FIG. 4 shows results of analyzing an amount of R/L expressed 6 hours and 24 hours after injecting lipid nanoparticles (LNP 109, 111, 114) of a novel cholesterol replaced composition including Renilla Luciferase (R/L) mRNA into the ears of mice (i.d.). Comparing LNP 98 and LNP 109 in FIG. 4, no significant difference was observed in the protein expression efficiency when cholesterol was replaced with vitamin A, determining that cholesterol may be replaceable with vitamin A. In addition, for LNP 111 and LNP 114, the protein expression efficiency was decreased in the case of vitamin E or vitamin K.

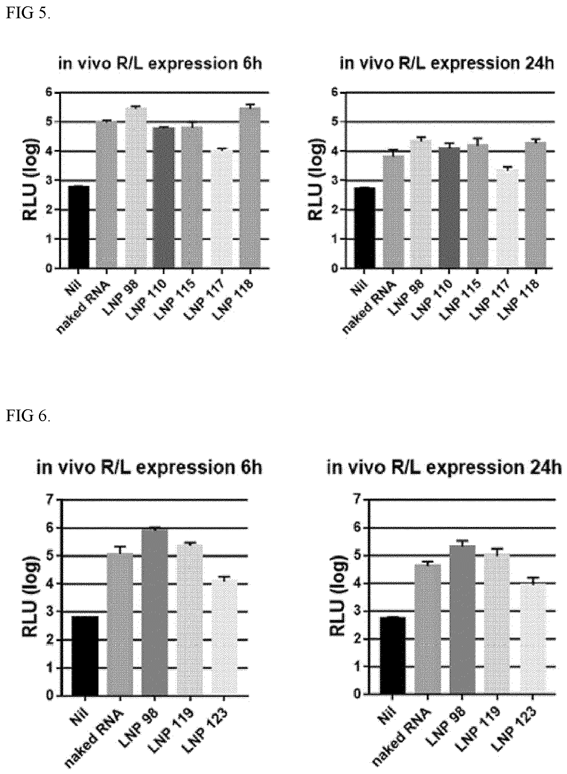

FIG. 5 shows results of analyzing an amount of R/L expressed 6 hours and 24 hours after injecting lipid nanoparticles (LNP 110, 115, 117, 118) of the novel cholesterol replaced composition including Renilla Luciferase (R/L) mRNA into the ears of mice (i.d.). Comparing LNP 98 and LNP 110 in FIG. 5, although cholesterol was partially mixed with butyl lithocholate due to solubility of coenzyme Q10 instead of whole replacement, the protein expression efficiency was rather decreased. In addition, for LNP 115 and 117, the protein expression efficiency was decreased after replacing cholesterol in the case of vitamin K2 and vitamin C palmitate. In addition, for LNP 118, when replaced with beta-carotene, no significant difference was observed in the protein expression efficiency of LNP 98, confirming the replaceability with cholesterol.

FIG. 6 shows results of analyzing an amount of R/L expressed 6 hours and 24 hours after injecting lipid nanoparticles (LNP 119, 123) of the novel cholesterol replaced composition including Renilla Luciferase (R/L) mRNA into the ears of mice (i.d.). Comparing LNP 98 and LNP 119 in FIG. 6, although the ratio of coenzyme Q10 was changed due to the solubility of coenzyme Q10, the expression efficiency was slightly lower than that of butyl lithocholate, such that a search for an appropriate ratio seems to be required. In addition, comparing LNP 109 (see FIG. 4) and LNP 123, in the case of retinol, although vitamin A (retinoic acid) has a form reduced to alcohol, it did not stably replace cholesterol unlike vitamin A.

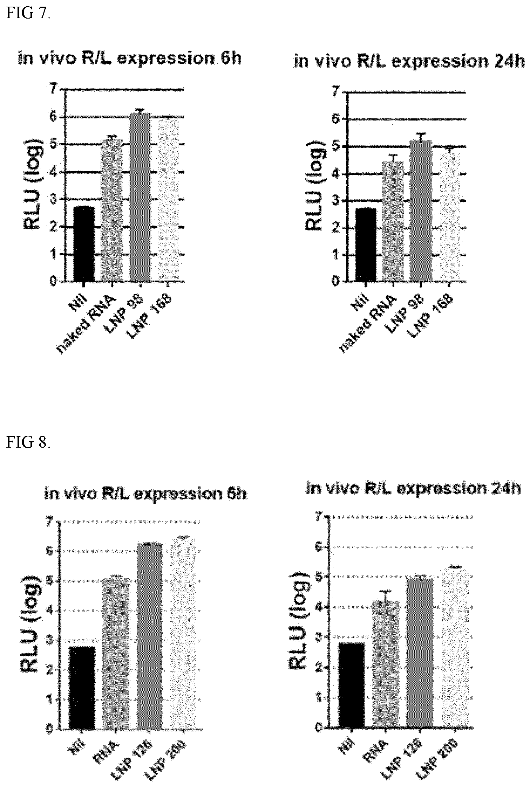

FIG. 7 shows results of analyzing an amount of R/L expressed 6 hours and 24 hours after injecting lipid nanoparticles (LNP 168) of the novel cholesterol replaced composition including Renilla Luciferase (R/L) mRNA into the ears of mice (i.d.). Comparing LNP 98 and LNP 168 in FIG. 7, it was found that the use of luteolin may retain the structure to a certain extent since there was no significant difference in the protein expression efficiency. The luteolin, a kind of flavonoids and phytochemical that is called vitamin P in a broad sense, is a substance known to have antioxidant action as well as functions of eliminating active oxygen and regulating metabolism and immune system.

FIG. 8 shows results of analyzing an amount of R/L expressed 6 hours and 24 hours after injecting lipid nanoparticles (LNP 200) of the novel cholesterol replaced composition including Renilla Luciferase (R/L) mRNA into the ears of mice (i.d.). Comparing LNP 126 and LNP 200 in FIG. 8, in the case of 3-oxo-5β-cholanoic acid, the protein expression efficiency was more increased when butyl lithocholate was replaced. It is considered that the 3-oxo-5β-cholanoic acid may act as an inhibitor of ROR-γt related to immune activity to suppress mRNA degradation by the in vivo immune system prior to protein expression.

In conclusion, vitamin A, beta-carotene, luteolin, and 3-oxo-5β-cholanoic acid showed excellent effects as substitutes for the structure-retaining compound.

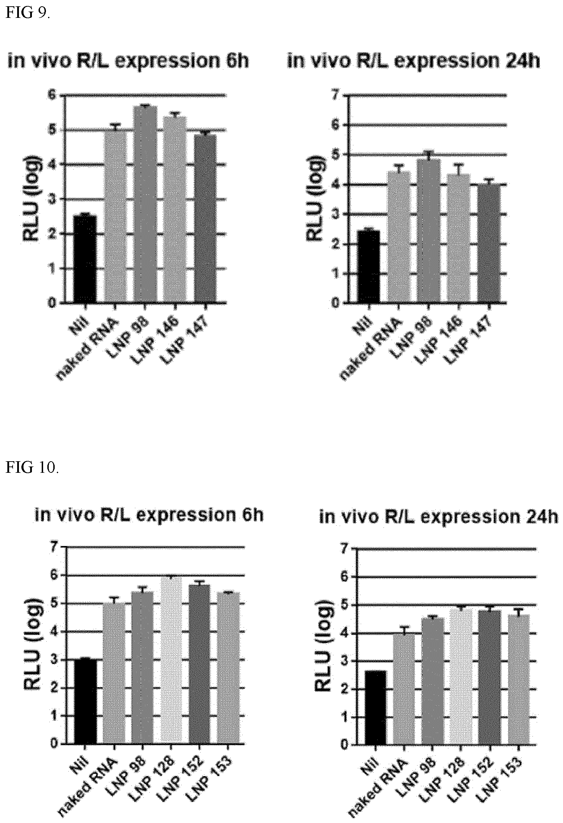

FIG. 9 shows results of analyzing an amount of R/L expressed 6 hours and 24 hours after injecting lipid nanoparticles (LNP 146, 147) of a novel ionized lipid composition including Renilla Luciferase (R/L) mRNA into the ears of mice (i.d.). Referring to FIG. 9, when compounds 12 and 13 were introduced in place of MC3, the ionized lipid, in LNP 98 (LNP 146 and LNP 147), respectively, the protein expression efficiency was slightly decreased in both cases, compared to LNP 98.

FIG. 10 shows results of analyzing an amount of R/L expressed 6 hours and 24 hours after injecting lipid nanoparticles (LNP 152, 153) of the novel ionized lipid composition including Renilla Luciferase (R/L) mRNA into the ears of mice (i.d.). Referring to FIG. 10, when compound 24 was introduced in place of MC3, the ionized lipid, in LNP 98 (LNP 152), the protein expression efficiency was increased compared to LNP 98, but when compound 24 was introduced in place of SM-102 in LNP 128 (LNP 153), the protein expression efficiency was decreased compared to LNP 128.

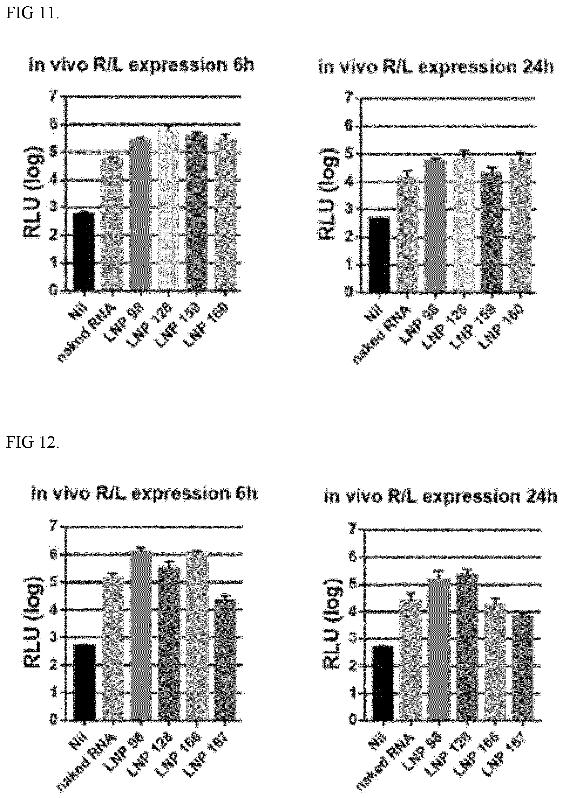

FIG. 11 shows results of analyzing an amount of R/L expressed 6 hours and 24 hours after injecting lipid nanoparticles (LNP 159, 160) of the novel ionized lipid composition including Renilla Luciferase (R/L) mRNA into the ears of mice (i.d.). Referring to FIG. 11, when compound 16 was introduced in place of DLin-MC3-DMA, the ionized lipid, in LNP 98 (LNP 159), the initial protein expression efficiency was slightly increased compared to LNP 98, but the persistence of expression was decreased. When compound 16 was introduced in place of SM-102 in LNP 128 (LNP 160), the protein expression efficiency was slightly decreased compared to LNP 128.

FIG. 12 shows results of analyzing an amount of R/L expressed 6 hours and 24 hours after injecting lipid nanoparticles (LNP 166, 167) of the novel ionized lipid composition including Renilla Luciferase (R/L) mRNA into the ears of mice (i.d.). Referring to FIG. 12, when compound 14 was introduced in place of MC3, the ionized lipid, in LNP 98 (LNP 166), the protein expression efficiency was maintained similarly at 6 hours compared to LNP 98, but the protein expression efficiency was significantly deceased at 24 hours. When compound 14 was introduced in place of SM-102 in LNP 128 (LNP 167), the protein expression efficiency was significantly deceased compared to LNP 128.

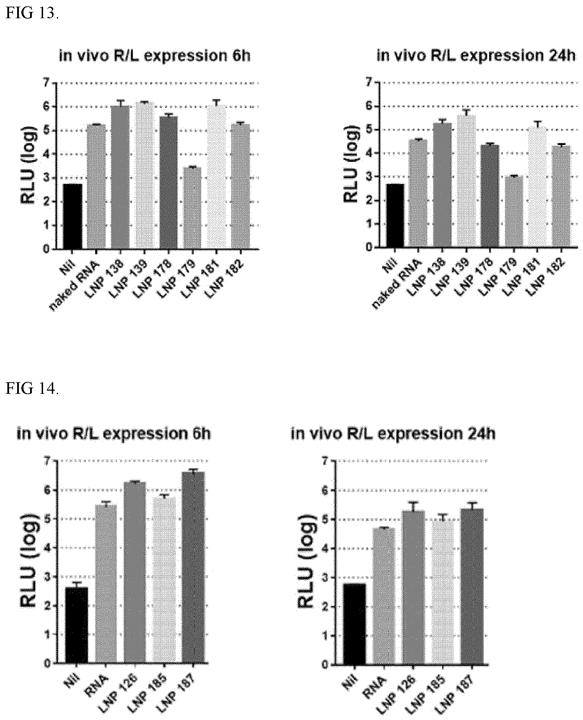

FIG. 13 shows results of analyzing an amount of R/L expressed 6 hours and 24 hours after injecting lipid nanoparticles (LNP 178, 179, 181, 182) of the novel ionized lipid composition including Renilla Luciferase (R/L) mRNA into the ears of mice (i.d.). In FIG. 13, when compound 16 (LNP 178) and compound 14 (LNP 179) were used in place of SM-102, the ionized lipid of LNP 139 using compound 10 as helper lipid, LNP 178 showed slightly decreased protein expression efficiency and LNP 179 showed significantly increased protein expression efficiency. When compound 16 (LNP 181) and compound 14 (LNP 182) were used in place of SM-102, the ionized lipid of LNP 138 using compound 11 as helper lipid, LNP 181 maintained the expression efficiency at 6 hours, and the expression efficiency was decreased slightly at 24 hours.

FIG. 14 shows results of analyzing an amount of R/L expressed 6 hours and 24 hours after injecting lipid nanoparticles (LNP 185, 187) of the novel ionized lipid composition including Renilla Luciferase (R/L) mRNA into the ears of mice (i.d.). In FIG. 14, as a result of replacing butyl lithocholate of LNP 181 with quercetin (LNP 185) and 3-oxo-5β-cholanoic acid (LNP 187) for optimization of LNP composition using compound 16 as the ionized lipid, the protein expression efficiency of LNP 187 was greatly increased. Quercetin is a plant flavonoid known to have excellent antioxidant power as well as neuroprotective, cardioprotective, and anti-inflammatory effects, but the protein expression efficiency was not increased significantly. Using 3-oxo-5β-cholanoic acid, which showed the most superior delivery efficiency among substitute compounds for cholesterol, it was found that the protein expression efficiency was further increased while persistence was enhanced, when replacing with 3-oxo-5β-cholanoic acid in place of butyl lithocholate which is cholesterol in LNP 181.

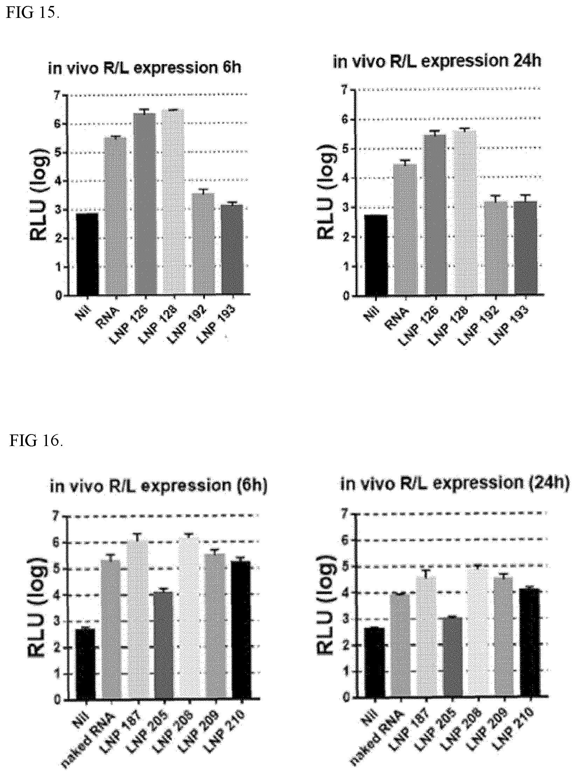

FIG. 15 shows results of analyzing an amount of R/L expressed 6 hours and 24 hours after injecting lipid nanoparticles (LNP 192, 193) of the novel ionized lipid composition including Renilla Luciferase (R/L) mRNA into the ears of mice (i.d.). Referring to FIG. 15, when ionized lipids of LNP 126 and LNP 128 were introduced into compound 15 in place of SM-102 (LNP 192 and LNP 193), the protein expression efficiency was significantly decreased compared to LNP 126 and LNP 128, respectively.

FIG. 16 shows results of analyzing an amount of R/L expressed 6 hours and 24 hours after injecting lipid nanoparticles (LNP 205, 208, 209, 210) of the novel ionized lipid composition including Renilla Luciferase (R/L) mRNA into the ears of mice (i.d.). In FIG. 16, the protein expression efficiency was significantly decreased in the case of LNP 205, in which the helper lipid of LNP 167 was replaced with compound 11 and cholesterol with 3-oxo-5β-cholanoic acid. The protein expression efficiency was slightly increased in the case of LNP 208 whose composition ratio of LNP 187 was changed. When 3-oxo-5β-cholanoic acid of LNP 208 was changed to coenzyme Q10, the protein expression efficiency was decreased, and, in the case of LNP 210 in which the helper lipid of LNP 160 was replaced with compound 11 and cholesterol with 3-oxo-5β-cholanoic acid, the protein expression efficiency was also decreased.

Referring to FIGS. 1 to 16, as a lipid nanoparticle composition including ionized lipid 16 among the lipid nanoparticle compositions of the present disclosure, the highest protein expression efficiency was shown when the helper lipid includes compound 11, cholesterol includes 3-oxo-5β-cholanoic acid, PEG-lipid includes DMG-PEG (LNP 187).

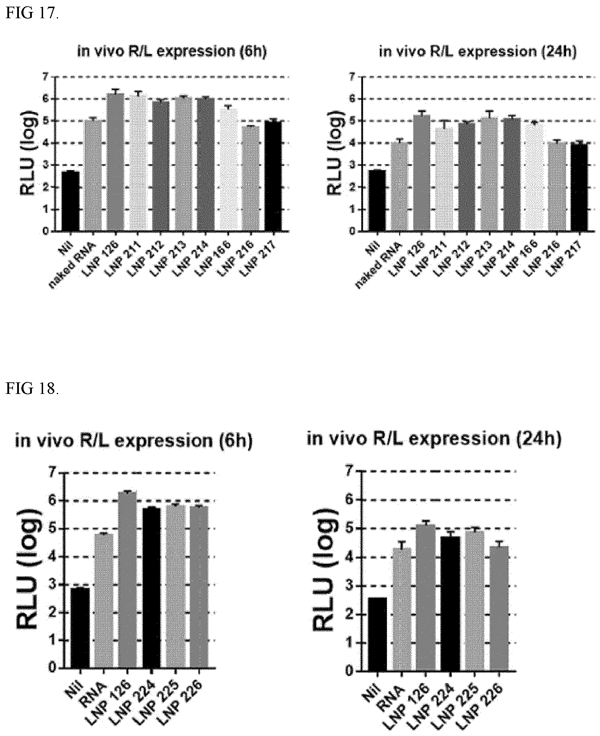

FIG. 17 shows results of analyzing an amount of R/L expressed 6 hours and 24 hours after injecting lipid nanoparticles (LNP 211, 212, 213, 214, 216, 217) of the novel ionized lipid composition including Renilla Luciferase (R/L) mRNA into the ears of mice (i.d.). Referring to FIG. 17, when ionized lipids of LNP 126 and LNP 128 were introduced into compound 18 in place of SM-102 (LNP 211 and LNP 212), the protein expression efficiency was slightly decreased compared to LNP 126. In particular, in the case of LNP 211, the expression efficiency was maintained to a certain extent at 6 hours, but that at 24 hours was greatly decreased. Comparing LNP 211 with LNP 213 and LNP 214, the protein expression efficiency was further increased at 24 hours when butyl lithocolate was changed to 3-oxo-5β-cholanoic acid. However, when compound 14 was used as the ionized lipid, comparing LNP 166 with LNP 216 and LNP 217, the protein expression efficiency was significantly decreased when butyl lithocolate was changed to 3-oxo-5β-cholanoic acid.

FIG. 18 shows results of analyzing a protein expression efficiency and an amount of R/L expressed 6 hours and 24 hours after injecting lipid nanoparticles (LNP 224, 225, 226) of the novel ionized lipid composition including Renilla Luciferase (R/L) mRNA into the ears of mice (i.d.). Referring to FIG. 18, when ionized lipids of LNP 126 and LNP 128 were introduced into compound 17 in place of SM-102 (LNP 224 and LNP 225), the protein expression efficiency was slightly decreased compared to LNP 126.

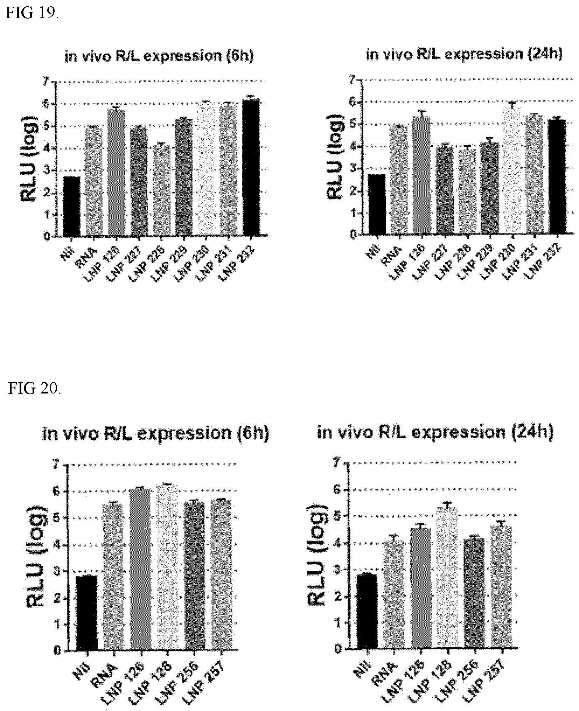

FIG. 19 shows results of analyzing a protein expression efficiency and an amount of R/L expressed 6 hours and 24 hours after injecting lipid nanoparticles (LNP 227, 228, 229, 230, 231, 232) of the novel ionized lipid composition including Renilla Luciferase (R/L) mRNA into the ears of mice (i.d.). Referring to FIG. 19, when ionized lipids of LNP 126 and LNP 128 were introduced into compound 19 in place of SM-102 (LNP 227 and LNP 228), the protein expression efficiency was significantly decreased compared to LNP 126. When ionized lipid of LNP 187 was introduced into compound 19 in place of compound 16, the protein expression efficiency was not increased significantly. On the other hand, when ionized lipids of LNP 126 and LNP 128 were introduced into compound 20 in place of SM-102 (LNP 230 and LNP 231), the protein expression efficiency was further increased compared to LNP 126. In particular, in the case of LNP 230, the expression efficiency was significantly increased at both 6 and 24 hours. In addition, when ionized lipid of LNP 187 was introduced into compound 20 in place of compound 16 (LNP 232), the protein expression efficiency was further increased at 6 hours, proving that optimization using 3-oxo-5β-cholanoic acid was well applied.

FIG. 20 shows results of analyzing an amount of R/L expressed 6 hours and 24 hours after injecting lipid nanoparticles (LNP 256, 257) of the novel ionized lipid composition including Renilla Luciferase (R/L) mRNA into the ears of mice (i.d.). Referring to FIG. 20, when compound 23 was introduced in place of SM-102, the ionized lipid, in LNP 126 and LNP 128 (LNP 256 and LNP 257), respectively, the protein expression efficiency was significantly decreased compared to LNP 126 and LNP 128.

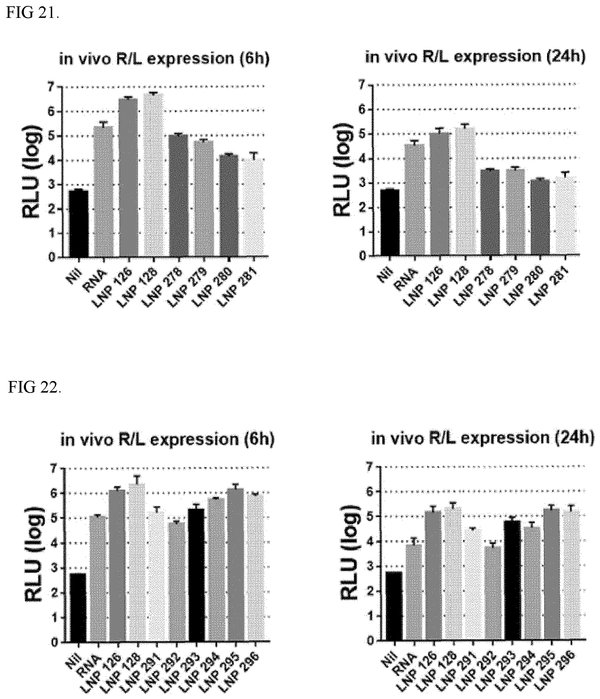

FIG. 21 shows results of analyzing an amount of R/L expressed 6 hours and 24 hours after injecting lipid nanoparticles (LNP 278, 279, 280, 281) of the novel ionized lipid composition including Renilla Luciferase (R/L) mRNA into the ears of mice (i.d.). In FIG. 21, the protein expression efficiency was significantly decreased in both cases when compound 21 (LNP 278) and compound 22 (LNP 280) were introduced in place of SM-102, the ionized lipid, in LNP 126 and when compound 21 (LNP 279) and compound 22 (LNP 281) were introduced in place of SM-102, the ionized lipid, in LNP 128.

Referring to FIGS. 10 to 21, the expression efficiency of LNP 230, LNP 231, and LNP 232 using compound 20 as the ionized lipid among compounds represented by Chemical Formula 4 was the highest.

FIG. 22 shows results of analyzing an amount of R/L expressed 6 hours and 24 hours after injecting lipid nanoparticles (LNP 291, 292, 293, 294, 295) of the novel ionized lipid included composition including Renilla Luciferase (R/L) mRNA into the ears of mice (i.d.). Referring to FIG. 22, when ionized lipids of LNP 126 and LNP 128 were introduced into compound 25 in place of SM-102 (LNP 291 and LNP 292), the protein expression efficiency was decreased compared to LNP 126. When ionized lipid of LNP187 was introduced into compound 25 in place of compound 16 (LNP 293), the protein expression efficiency was not increased significantly, but the protein expression efficiency was increased slightly compared to LNP 291. On the other hand, when ionized lipids of LNP 126 and LNP 128 were introduced into compound 26 in place of SM-102 (LNP 294 and LNP 295), the protein expression efficiency was maintained or slightly increased compared to LNP 126. In addition, when ionized lipids of LNP 187 were introduced into compound 26 in place of compound 16 (LNP 296), the protein expression efficiency was significantly increased compared to LNP 294, proving that optimization using 3-oxo-5β-cholanoic acid was well applied.

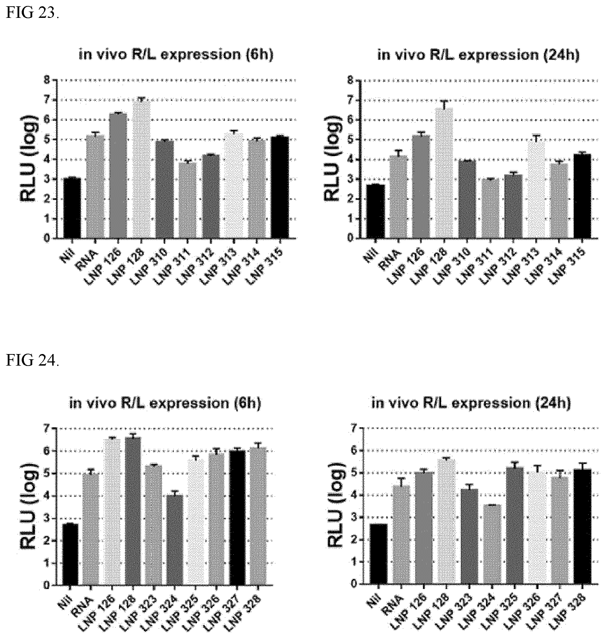

FIG. 23 shows results of analyzing an amount of R/L expressed 6 hours and 24 hours after injecting lipid nanoparticles (LNP 310, 311, 312, 313, 314, 315) of the novel cholesterol replaced composition including Renilla Luciferase (R/L) mRNA into the ears of mice (i.d.). Referring to FIG. 23, when ionized lipids of LNP 126 and LNP 128 were introduced into compound 27 in place of SM-102 (LNP 310 and LNP 311), the protein expression efficiency was decreased compared to LNP 126. When ionized lipid of LNP187 was introduced into compound 27 in place of compound 16 (LNP 312), the protein expression efficiency was slightly decreased compared to LNP 310. On the other hand, when ionized lipids of LNP 126 and LNP 128 were introduced into compound 28 in place of SM-102 (LNP 313 and LNP 314), the protein expression efficiency was decreased compared to LNP 126. In addition, when ionized lipid of LNP 187 was introduced into compound 28 in place of compound 16 (LNP 315), the protein expression efficiency was decreased compared to LNP 313. When compounds 27 and 28 were used as ionized lipids, it was discovered that optimization using compounds 11 and 3-oxo-5β-cholanoic acid was not suitable.

Referring to FIGS. 22 to 23, it may be found that the expression efficiency of LNP 295, the lipid nanoparticle including compound 26 as ionized lipid among compounds represented by Chemical Formula 8 was the highest.

FIG. 24 shows results of analyzing an amount of R/L expressed 6 hours and 24 hours after injecting lipid nanoparticles (LNP 323, 324, 325, 326, 327, 328) of a composition including novel ionized lipid including Renilla Luciferase (R/L) mRNA into the ears of mice (i.d.). Referring to FIG. 24, when compound 29 was introduced in place of SM-102, the ionized lipid, in LNP 126 and LNP 128 (LNP 323 and LNP 324), respectively, the protein expression efficiency was significantly decreased. On the other hand, when compound 30 was introduced in place of SM-102, the ionized lipid, in LNP 126 and LNP 128 (LNP 325 and LNP 326), respectively, and when compound 31 was introduced (LNP 327 and LNP 328), respectively, the protein expression efficiency was slightly decreased.

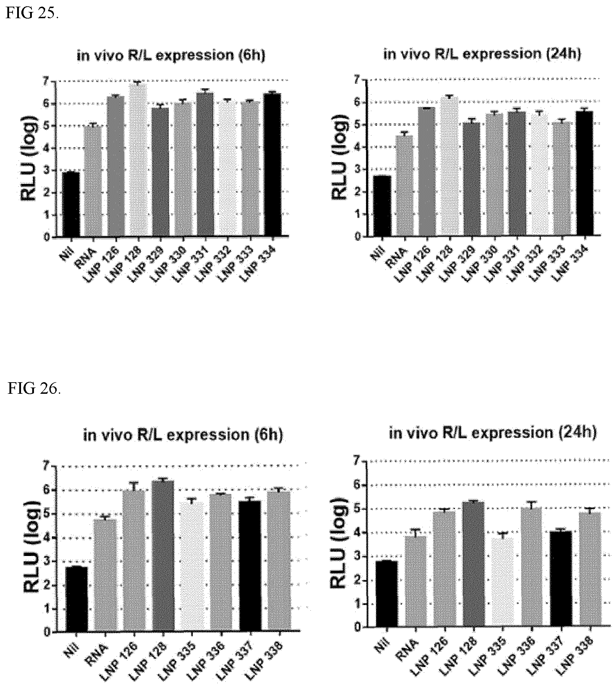

FIG. 25 shows results of analyzing an amount of R/L expressed 6 hours and 24 hours after injecting lipid nanoparticles (LNP 329, 330, 331, 332, 333, 334) of the composition including novel ionized lipid including Renilla Luciferase (R/L) mRNA into the ears of mice (i.d.). Referring to FIG. 25, when compound 32 was introduced in place of SM-102, the ionized lipid, in LNP 126 and LNP 128 (LNP 329 and LNP 330), respectively, the protein expression efficiency was slightly decreased. The protein expression efficiency at 6 hours compared to LNP 126 was increased by 40% and 20% respectively, particularly in the case of LNP 331 including compound 35 and LNP 334 including compound 36 among the cases when compound 35 was introduced in place of SM-102, the ionized lipid, in LNP 126 and LNP 128 (LNP 331 and LNP 332), respectively, and when compound 36 was introduced (LNP 333 and LNP 334), respectively.

FIG. 26 shows results of analyzing an amount of R/L expressed 6 hours and 24 hours after injecting lipid nanoparticles (LNP 335, 336, 337, 338) of the composition including novel ionized lipid including Renilla Luciferase (R/L) mRNA into the ears of mice (i.d.). Referring to FIG. 26, when compound 33 was introduced in place of SM-102, the ionized lipid, in LNP 126 and LNP 128 (LNP 335 and LNP 336), respectively, and compound 34 was introduced (LNP 337 and LNP 338), respectively, the protein expression efficiency at 6 hours was maintained or slightly decreased compared to LNP 126.

Referring to FIGS. 24 to 26, LNP 331 of a composition including compound 35 as the ionized lipid and LNP 334 of a composition including compound 36 as the ionized lipid among compounds represented by Chemical Formula 6 showed the highest protein expression efficiency.

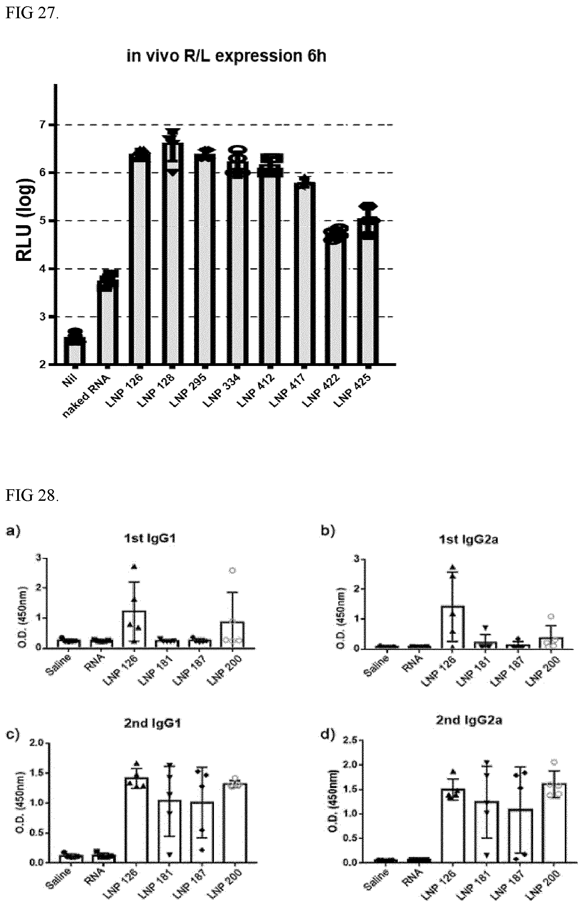

FIG. 27 shows a result of analyzing an amount of R/L expressed 6 hours after injecting lipid nanoparticles (LNP 412, 417, 422, 425) of a composition including novel ionized lipid including Renilla Luciferase (R/L) mRNA into the ears of mice (i.d.). In FIG. 27, when compounds 37, 38, 39, and 40 were introduced instead of SM-102, which is an ionized lipid, in LNP 128 (LNP 412, 417, 422, 425), respectively, the protein expression efficiency was slightly reduced. However, since the protein expression efficiency was similar when compound 37 was introduced (LNP 412) compared to compound 26 (LNP 295) instead of SM-102, which is an ionized lipid, in LNP 128, it was found that the additional ester bonds introduced in compound 37 did not significantly affect the inclusion of mRNA compared to compound 26.

### Experimental Example 2: HA-specific antibody immune response enhanced by mRNA-formulated LNP

In order to investigate the effect of LNP on antibody production, mRNA encoding HA, a surface antigen of influenza virus, was encapsulated in LNP, mice (BALB/c) aged 6 weeks were immunized with 10 µg thereof intramuscularly based on mRNA, and the antibody response was observed through the level of IgG1 and IgG2a against HA-specific antibodies in the blood using ELISA 2 weeks after primary immunity or secondary immunity.

The specific experimental process is as follows:

A drug to be administered that is suitable for the experimental condition was injected via an insulin syringe. Thereafter, blood collection was performed using a respiratory anesthesia machine on each corresponding date. The experiment was carried out after separating the serum from the obtained blood.

FIG. 28 shows results obtained by formulating (LNP 126, 181, 187, 200) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d). Referring to FIG. 28, it was found that, in the case of LNP 181, LNP 187, and LNP 200, the level of IgG1 and IgG2a was decreased compared to LNP 126, and the immune effect was not significant. In particular, although the protein expression efficiency of LNP 187 was increased compared to LNP 126 (FIG. 14), the immune effect was significantly reduced, ensuring the applicability as a nucleic acid treatment which is based on human immune activation and enables delivery without side effects.

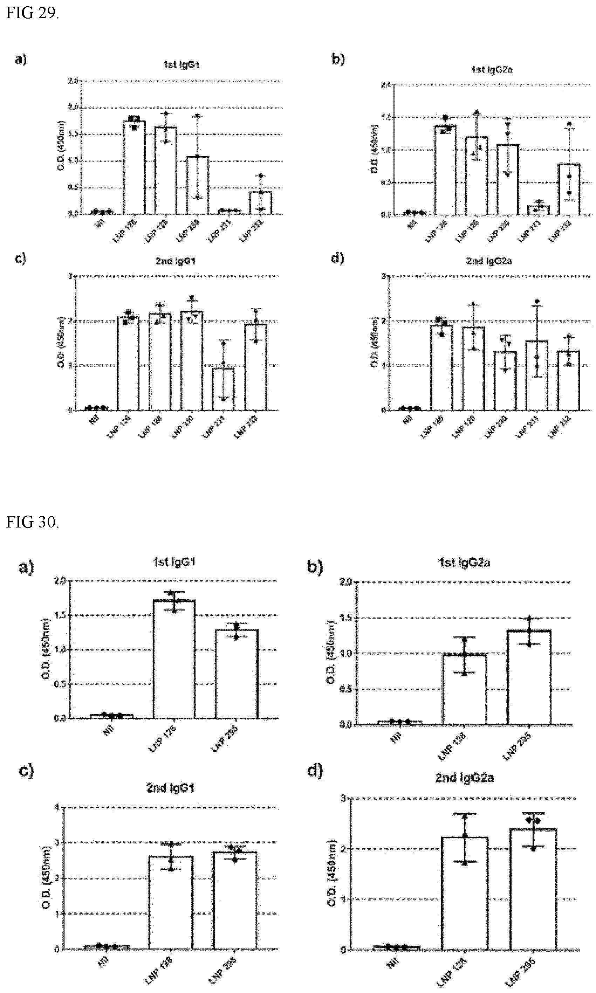

FIG. 29 shows results obtained by formulating (LNP 126, 128, 230, 231, 232) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d). Referring to FIG. 29, in the case of LNP 230, LNP 231, and LNP 128, comparing with LNP126 and LNP 128, the immune effect was not significant since the level of IgG1 and IgG2a was decreased in primary immunity, while the level of IgG2a was still lower than that of LNP 126 and LNP 128 with large difference among individuals in secondary immunity. In particular, in the case of LNP 231, the level of both IgG1 and IgG2a was decreased after primary immunity and secondary immunity, showing the applicability as a nucleic acid treatment which is based on human immune activation and enables delivery without side effects.

FIG. 30 shows results obtained by formulating (LNP 128, 295) mRNA encoding surface antigen of influenza virus, immunizing mice by intramuscular administration, and identifying HA-specific antibody responses in the blood by IgG1 (a, c) and IgG2a (b, d) levels 2 weeks after primary immunity (a~b) and secondary immunity (c~d). Referring to FIG. 30, in the case of LNP 295, the applicability as an mRNA vaccine was determined from an increase in the level of IgG1 and IgG2a to a degree similar to LNP 128 in both primary and secondary immunity, which is consistent with the result that the concentration of MCP-1 in the blood was increased significantly compared to the positive control LNP 128 in FIG. 29.

Experimental Example 3: Protein expression level of MCP-1 increased by HA-encoding mRNA-formulated LNP.

Monocyte chemoattracted protein-1 (MCP-1) is a type of chemokine that moves monocytes toward vascular endothelial cells to be attached thereto and known to play an important role in the immune response. To determine the effect of LNP on the immune response, mRNA encoding R/L was sealed in LNP, 5 µg/20 µl of mRNA-LNP based on RNA was injected into the ears of ICR mice by I.D., and blood was collected 6 hours later to measure the concentration via ELISA.

The specific experimental process is as follows:

A drug to be administered that is suitable for the experimental condition was injected via an insulin syringe. After 6 hours, blood collection was performed using a respiratory anesthetic machine. The experiment was carried out after separating the serum from the obtained blood. Expression level was compared using Invitrogen's MCP-1 mouse uncoated ELISA kit.

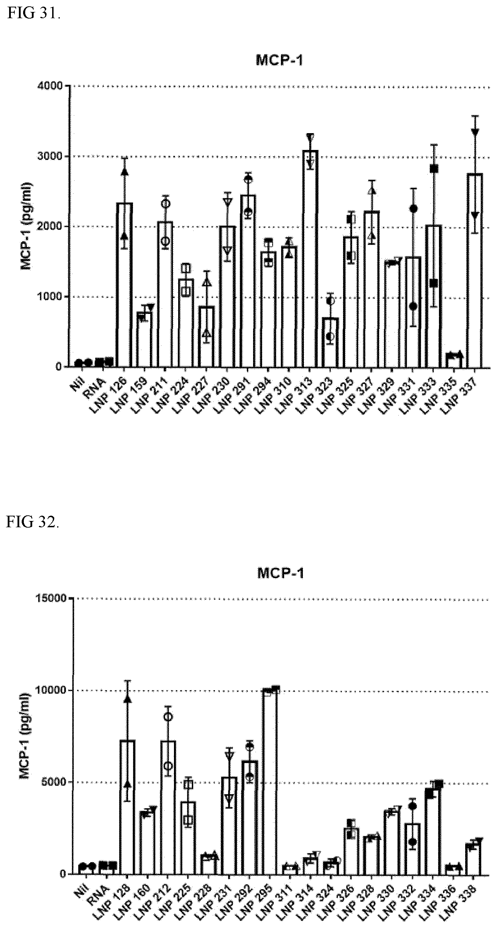

FIG. 31 shows results obtained by formulating (LNP 126, 159, 211, 224, 227, 230, 291, 294, 310, 313, 323, 325, 327, 329, 331, 333, 335, 337) mRNA encoding Renilla Luciferase (R/L) to administer the mRNA into the ear of ICR mice by I.D. and measuring the concentration of MCP-1 from the blood collected after 6 hours. Referring to FIG. 31, in the case of LNP 313 and LNP 337, the concentration of MCP-1 in the blood was increased compared to the positive control LNP 126, and LNP 291 and LNP 327 showed a similar concentration of MCP-1 in the blood compared to LNP 126. Thereamong, LNP 327 showed a high concentration of MCP-1 while maintaining the protein expression efficiency to a certain extent, such that the applicability may be expected as an mRNA vaccine.

FIG. 32 shows results obtained by formulating (LNP 128, 160, 212, 225, 228, 231, 292, 295, 311, 314, 324, 326, 328, 330, 332, 334, 336, 338) mRNA encoding Renilla Luciferase (R/L) to administer the mRNA into the ear of ICR mice by I.D. and measuring the concentration of MCP-1 from the blood collected after 6 hours. Referring to FIG. 32, it was observed that, in the case of LNP 295, the concentration of MCP-1 in the blood was increased significantly compared to positive control LNP 128. Since the MCP-1 concentration of LNP 212, LNP 231, LNP 292, and LNP 334 was also not low, the applicability of LNP 334, which had the high protein expression efficiency thereamong, may also be expected as an mRNA vaccine along with LNP 295.

In the case of LNP (LNP 181, 187, 230, 231, 232) of a composition including compound 16 or compound 17 in FIGS. 9 to 32, the protein expression efficiency was significantly high, but no obvious antibody response was observed. On the other hand, in the case of LNP 295 including compound 26, the antibody response was significant after immunization. For LNP (LNP 334), which includes compound 36, the concentration of MCP-1 in the blood after subcutaneous injection was significant. This means that ionized lipids of a new structure of the present disclosure may be configured for each use in relation to the applicability as therapeutic agents (LNP 181, 187, 230, 231, 232) to decrease antibody responses while increasing the protein expression efficiency as well as the applicability as prophylactic agents (LNP 295, 334) in which antibody responses should take place.

As the specific part of the present disclosure was described in detail above, it will be apparent to those skilled in the art that such specific techniques are only preferred embodiments, and the scope of the present disclosure is not limited thereby. Therefore, the substantial scope of the present disclosure will be defined by the appended claims and their equivalents.

## Claims

1. A compound selected from a compound represented by the following Chemical Formula 1, a stereoisomer thereof, a racemate thereof, or a pharmaceutically acceptable salt thereof: 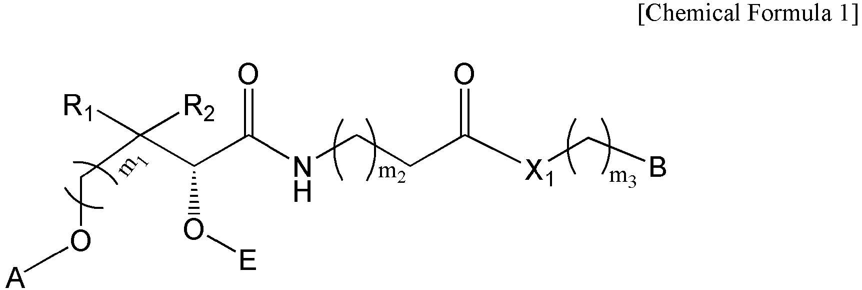 wherein, in the Chemical Formula 1,
R₁ and R₂ are the same or different and each is hydrogen or a saturated hydrocarbon with 1 to 2 carbon atoms,
X₁ is O, NH, or S,
m₁, m₂, and m₃ are the same or different and each is an integer of 1 to 3, and
A is hydrogen or a compound represented by the following Chemical Formula 1-1, 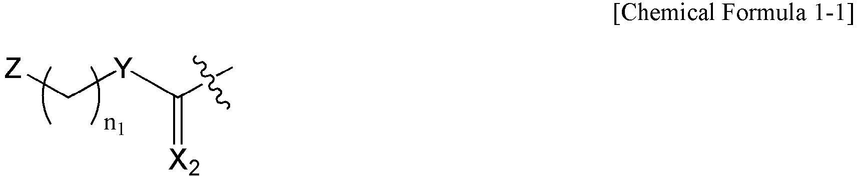
wherein, in the Chemical Formula 1-1,
Z is NR¹R²; a heterocyclic or aromatic compound with 3 to 8 carbon atoms; or linear or branched, saturated or unsaturated hydrocarbon with 6 to 22 carbon atoms,
the R¹ and R² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and the hydrocarbon includes or does not include ester, ether, amide, carbamate, carbonate, or disulfide bonds,
X₂ is O or S,
Y is CH₂, NH, or O,
n₁ is an integer of 0 to 3, and
B is a compound represented by the following Chemical Formula 1-2 or NR¹'R²', wherein the R¹' and R²' are the same or different and each is linear or branched, saturated or unsaturated hydrocarbon with 1 to 6 carbon atoms, and wherein the hydrocarbon includes or does not include ester, ether, amide, carbamate, carbonate, or disulfide bonds, and R¹' and R²' are connected to each other to form a heterocyclic or aromatic ring with 3 to 8 carbon atoms, 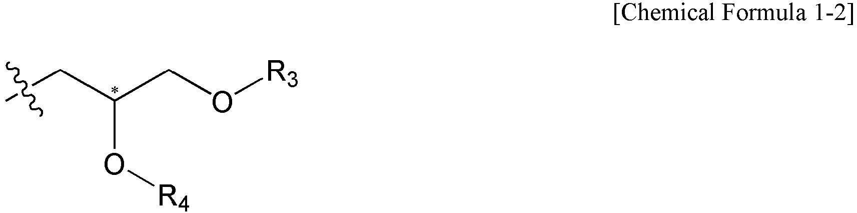
wherein, in the Chemical Formula 1-2,
R₃ and R₄ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
E is hydrogen or a compound represented by the following Chemical Formula 1-3, 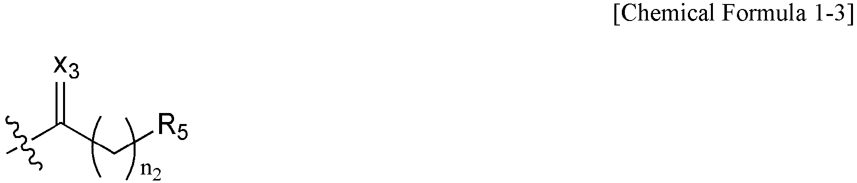
wherein, in the Chemical Formula 1-3,
X₃ is O or S,
n₂ is an integer of 0 to 3, and
R₅ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with thiolane or dithiolane.

2. The compound of claim 1, wherein the compound is a compound represented by the following Chemical Formula 2: 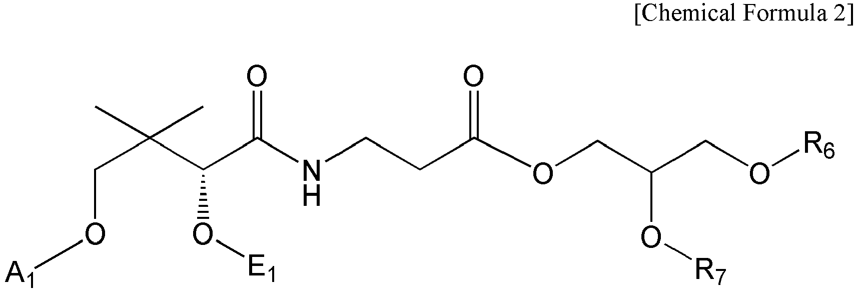 wherein, in the Chemical Formula 2,
A₁ is hydrogen or a compound represented by the following Chemical Formula 2-1, 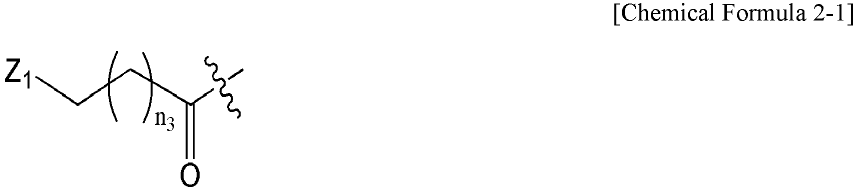
wherein, in the Chemical Formula 2-1,
Z₁ is NR³R⁴; or a heterocyclic or aromatic compound with 3 to 8 carbon atoms, wherein the R³ and R⁴ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4) alkylamino(C1~2) alkyl,
n₃ is an integer of 0 to 3, and
E₁ is hydrogen or a compound represented by the following Chemical Formula 2-2, 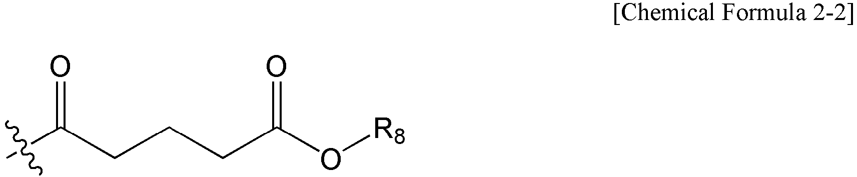
wherein, in the Chemical Formula 2-2,
R₈ is linear or branched, saturated or unsaturated hydrocarbon with 6 to 22 carbon atoms, wherein the hydrocarbon includes or does not include ester, ether, amide, carbamate, carbonate, or disulfide bonds, and is substituted or unsubstituted with dithiolane, and
R₆ and R₇ are the same or different, and each is linear or branched, saturated or unsaturated hydrocarbon with 6 to 22 carbon atoms.

3. The compound of claim 1, wherein the compound represented by the Chemical Formula 1 is represented by the following Chemical Formula 3: 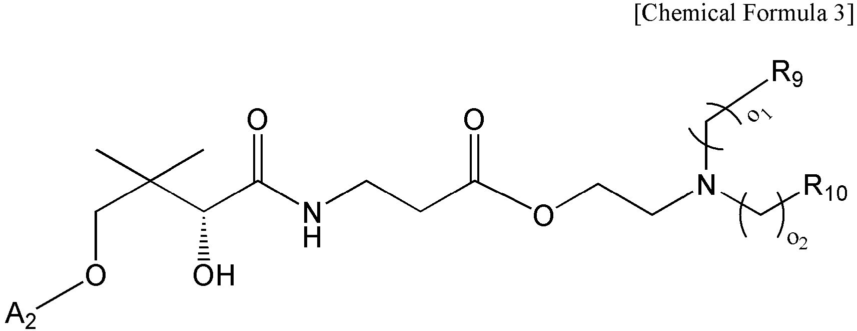
wherein, in the Chemical Formula 3,
A₂ is hydrogen or a compound represented by the following Chemical Formula 3-1, 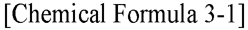 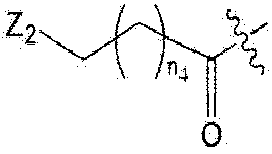
wherein, in the Chemical Formula 3-1,
Z₂ is NR⁵R⁶; or a heterocyclic or aromatic compound with 3 to 8 carbon atoms, wherein R⁵ and R⁶ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl,
n₄ is an integer of 0 to 3,
R₉ and R₁₀ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
o₁ and o₂ are the same or different, and each is an integer of 2 to 10.

4. The compound of claim 1, wherein the compound represented by the Chemical Formula 1 is represented by the following Chemical Formula 4: 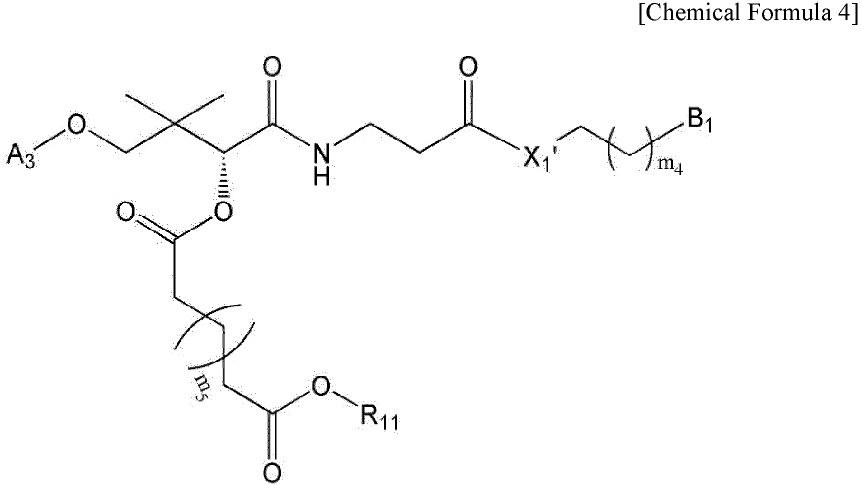
wherein, in the Chemical Formula 4,
X₁' is O, NH, or S,
m₄ and m₅ are the same or different and each is an integer of 0 to 3, and
A₃ is a compound represented by the Chemical Formula 4-1, 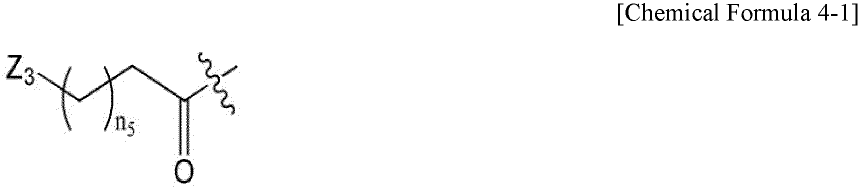
wherein, in the Chemical Formula 4-1,
Z₃ is NR⁷R⁸; heterocyclic or aromatic compound with 3 to 8 carbon atoms; or linear or branched, saturated or unsaturated hydrocarbon with 6 to 22 carbon atoms,
the R⁷ and R⁸ are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, wherein the hydrocarbon includes or does not include ester, ether, amide, carbamate, carbonate, or disulfide bonds,
n₅ is an integer of 0 to 3,
B₁ is a compound represented by the following Chemical Formula 4-2; NR⁹R¹⁰; or linear or branched, saturated or unsaturated hydrocarbon with 6 to 22 carbon atoms, wherein R⁹ and R¹⁰ are the same or different, and each is linear or branched, saturated or unsaturated hydrocarbon with 1 to 6 carbon atoms, wherein the hydrocarbon includes or does not include ester, ether, amide, carbamate, carbonate, or disulfide bonds, and R⁹ and R¹⁰ are connected to each other to form a heterocyclic or aromatic ring with 3 to 8 carbon atoms, 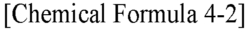 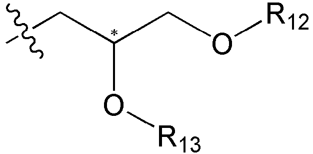
wherein, in the Chemical Formula 4-2,
R₁₂ and R₁₃ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
R₁₁ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with dithiolane.

5. The compound of claim 2, wherein the compound represented by the Chemical Formula 2 is represented by Chemical Formula 5 or Chemical Formula 6: 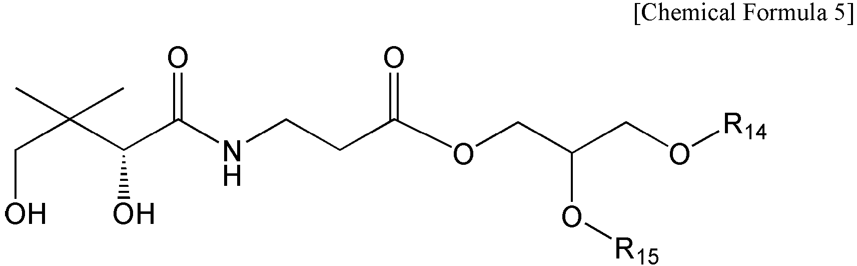 wherein, in the Chemical Formula 5,
the R₁₄ and R₁₅ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and 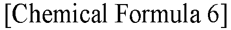 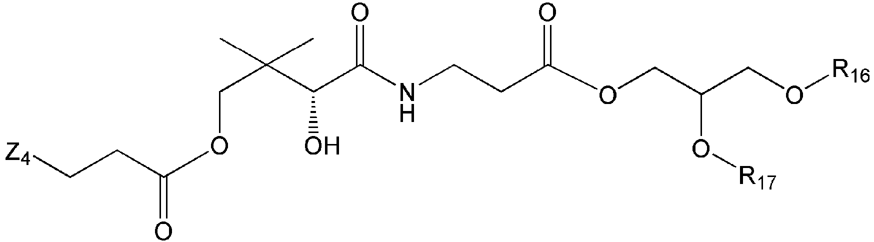
wherein, in the Chemical Formula 6,
the Z₄ is NR¹¹R¹²; or a heterocyclic or aromatic compound with 3 to 8 carbon atoms, wherein R¹¹ and R¹² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and
the R₁₆ and R₁₇ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds.

6. The compound of claim 3, wherein the compound represented by the Chemical Formula 3 is represented by Chemical Formula 7: 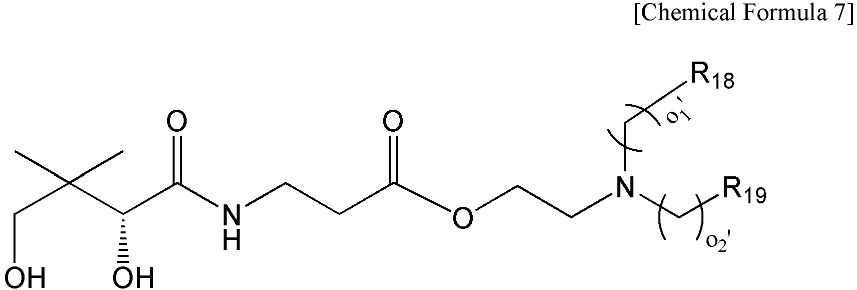 wherein, in the Chemical Formula 7,
the R₁₈ and R₁₉ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
o₁' and o₂' are the same or different, and each is an integer of 3 to 8.

7. The compound of claim 4, wherein the compound represented by the Chemical Formula 4 is represented by Chemical Formula 8 or Chemical Formula 9: 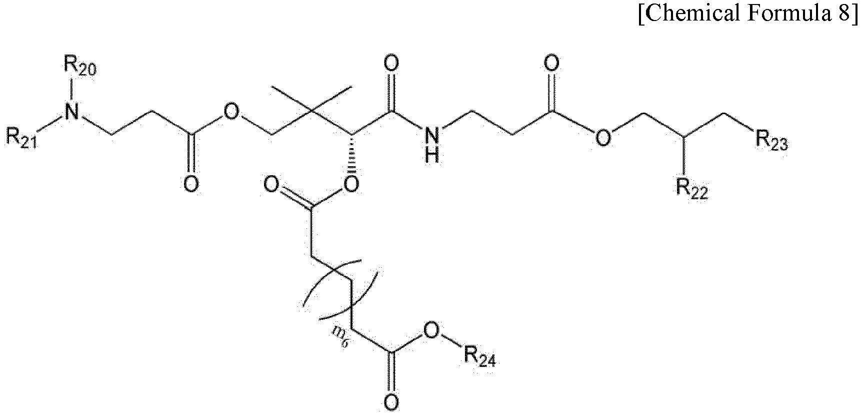 wherein in the Chemical Formula 8,
m₆ is an integer of 0 to 3,
R₂₀ and R₂₁ are the same or different, and each is linear or branched, saturated or unsaturated hydrocarbon with 1 to 6 carbon atoms; the R₂₀ and R₂₁ are connected to each other to form a heterocyclic ring with 3 to 8 carbon atoms,
R₂₂ and R₂₃ are the same or different, and each is hydrogen or linear or branched, saturated or unsaturated hydrocarbon with 6 to 22 carbon atoms, wherein the hydrocarbon includes or does not include ester, ether, amide, carbamate, carbonate, or disulfide bonds, and
R₂₄ is linear or branched, saturated or unsaturated hydrocarbon with 6 to 22 carbon atoms, wherein the hydrocarbon includes or does not include ester bonds or disulfide bonds and is substituted or unsubstituted with dithiolane, 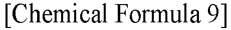 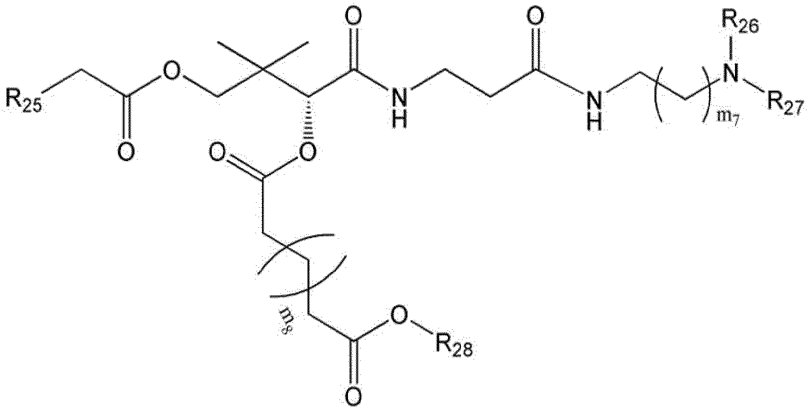
wherein, in the Chemical Formula 9,
m₇ and m₈ are the same or different and each is an integer of 0 to 3,
R₂₅ is linear or branched, saturated or unsaturated hydrocarbon with 1 to 6 carbon atoms, wherein the hydrocarbon includes or does not include ester bonds or disulfide bonds,
R₂₆ and R₂₇ are the same or different, and each is linear or branched, saturated or unsaturated hydrocarbon with 6 to 22 carbon atoms; the R₂₆ and R₂₇ are connected to each other to form a heterocyclic or aromatic ring with 3 to 8 carbon atoms, and
R₂₈ is linear or branched, saturated or unsaturated hydrocarbon with 6 to 22 carbon atoms, wherein the hydrocarbon includes or does not include ester bonds or disulfide bonds and is substituted or unsubstituted with dithiolane.

8. The compound of claim 1, wherein the heterocyclic ring is selected from the group consisting of thiophene, furan, pyrosol, pyridine, pyran, oxazine, thiazine, morpholine, pyrrolidine, piperidine, piperazine, pyrazole, pyridine, and dithiolane that are substituted or unsubstituted with 1 to 4 carbon atoms.

9. The compound of claim 1, wherein the compound is one or more types selected from the group consisting of Chemical Formula 10 to Chemical Formula 40.

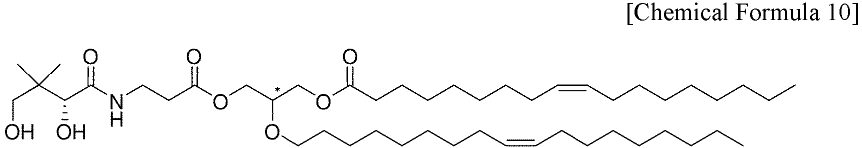

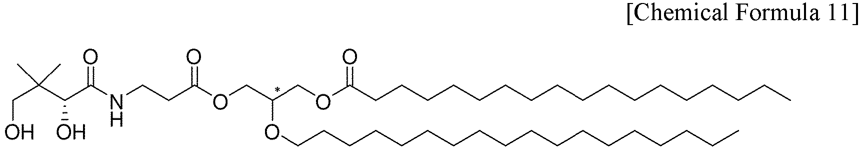

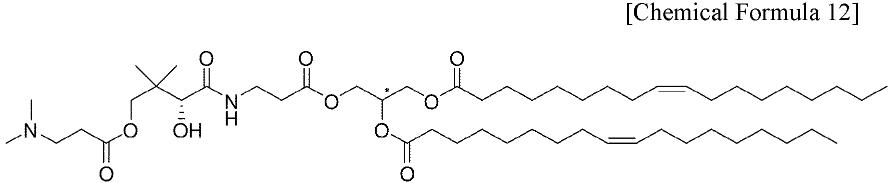

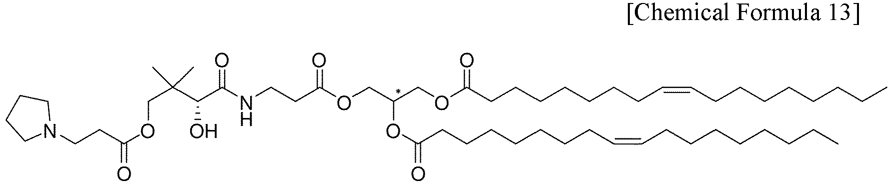

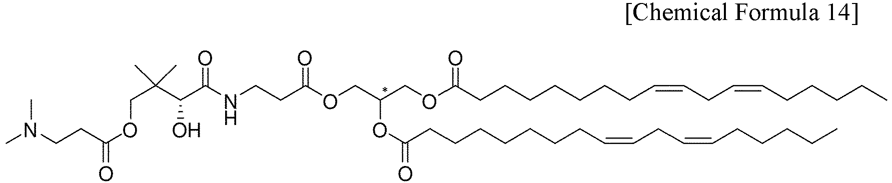

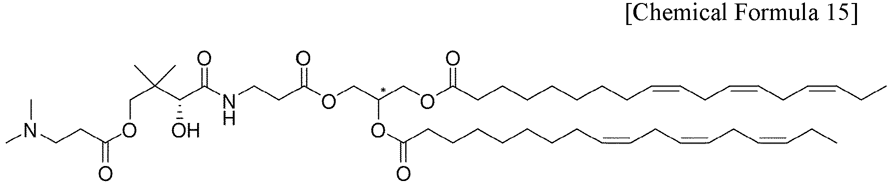

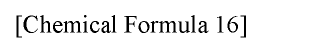

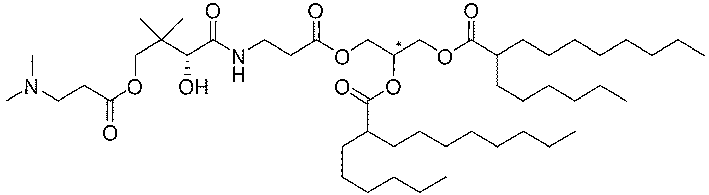

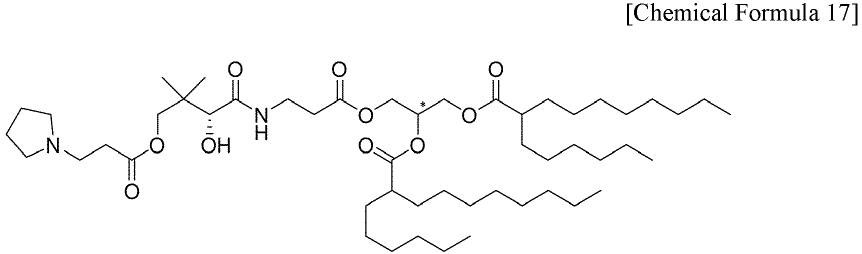

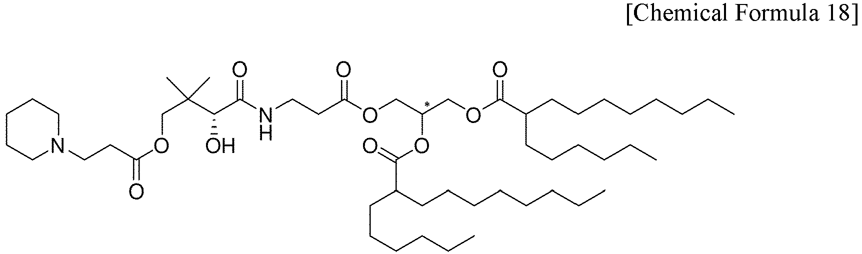

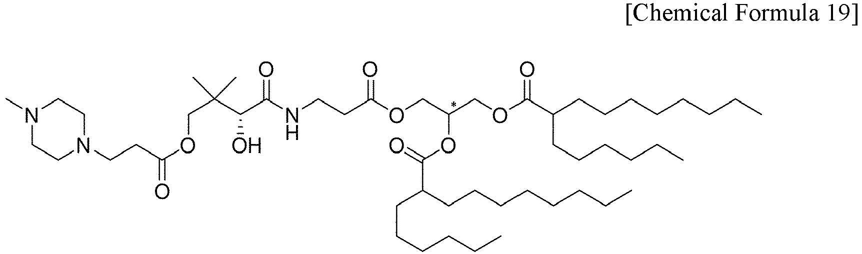

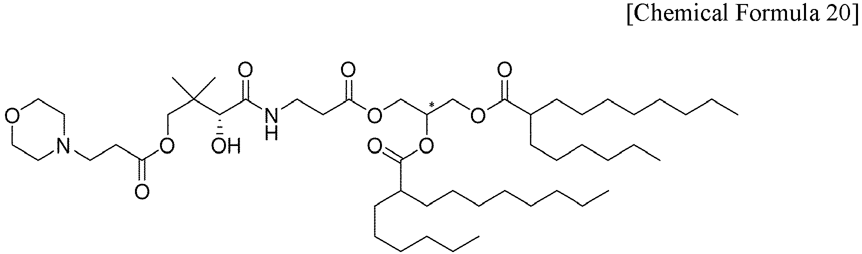

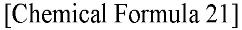

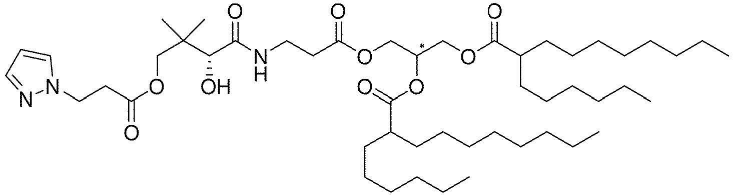

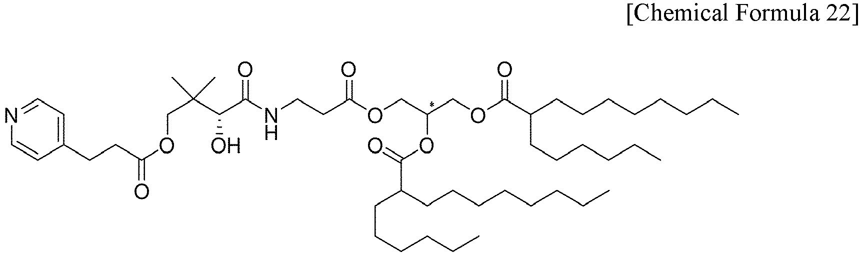

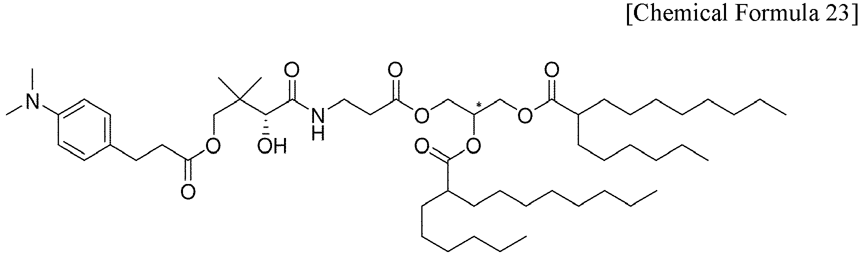

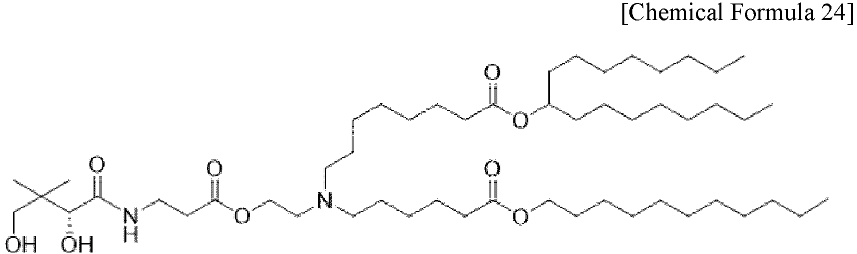

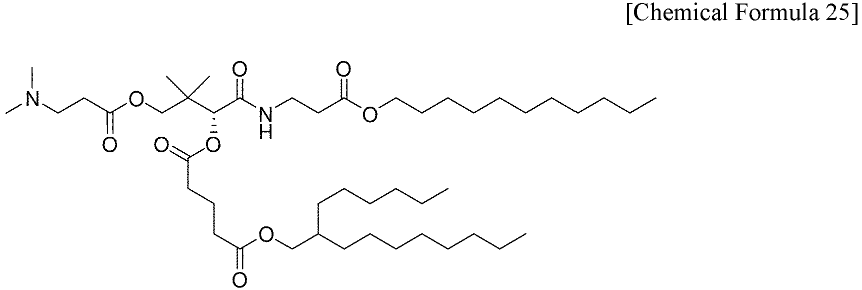

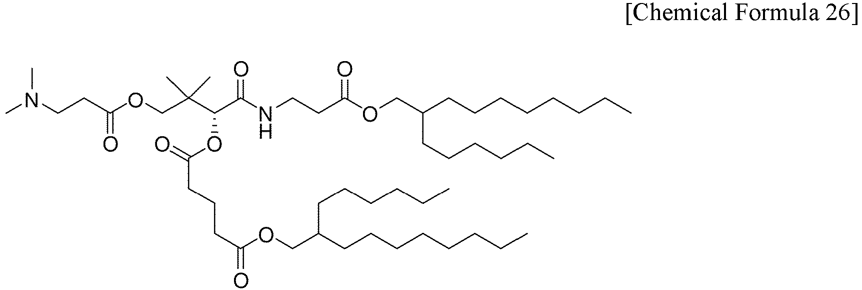

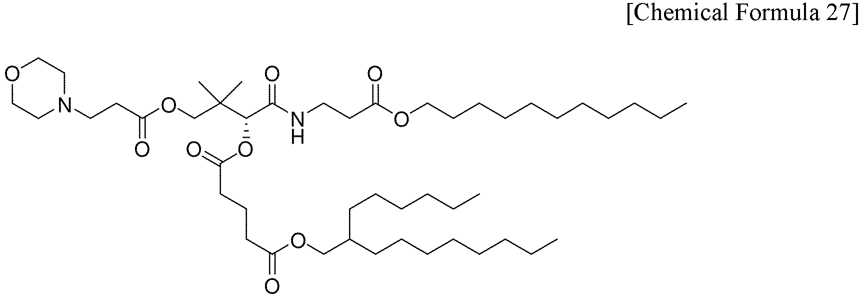

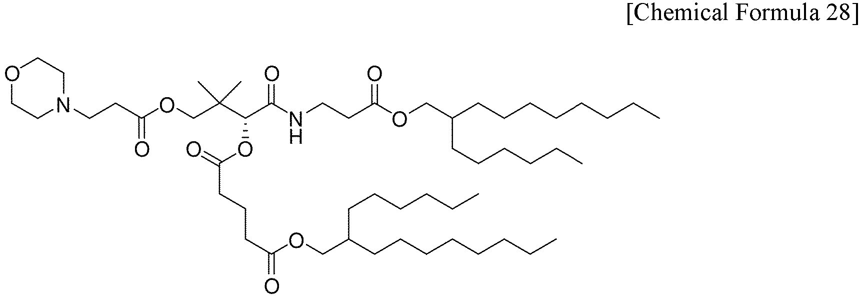

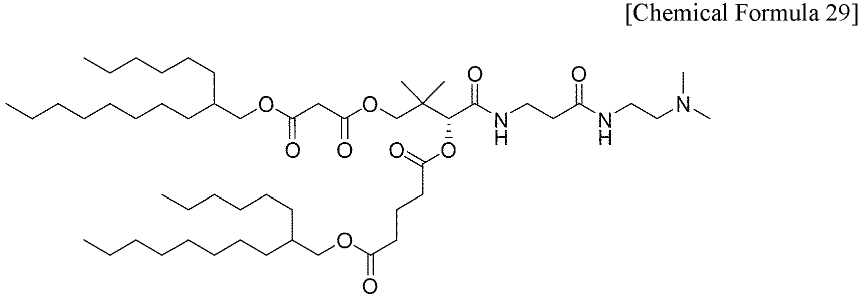

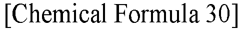

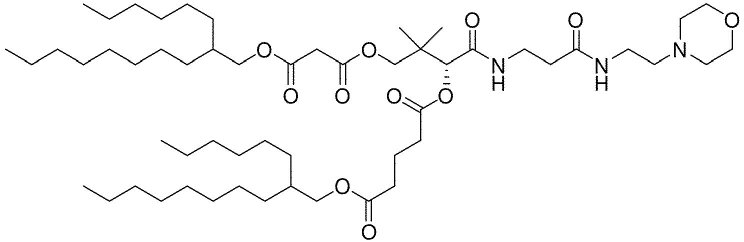

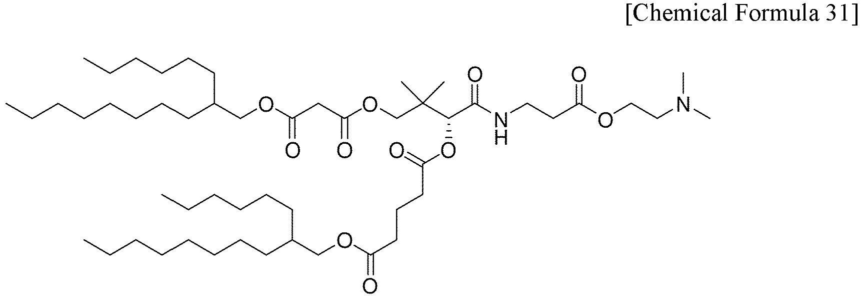

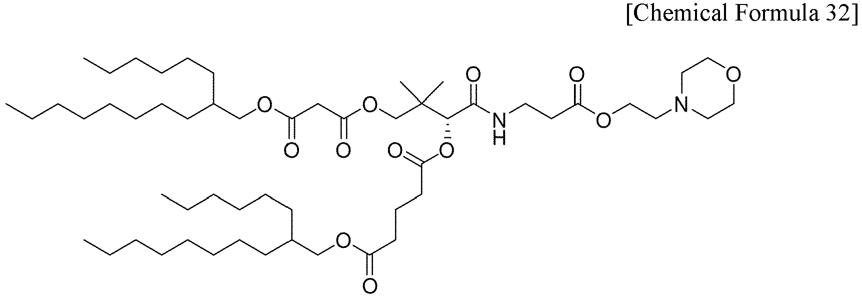

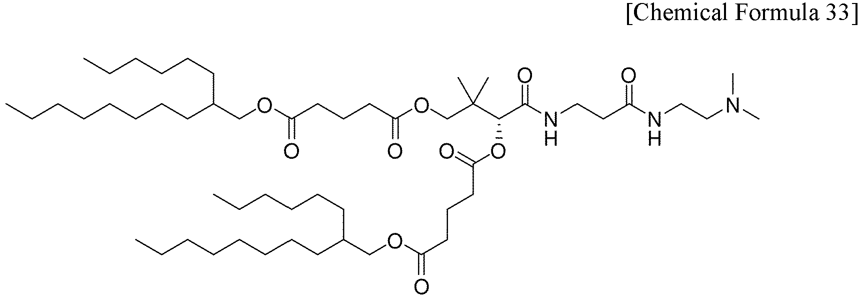

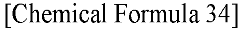

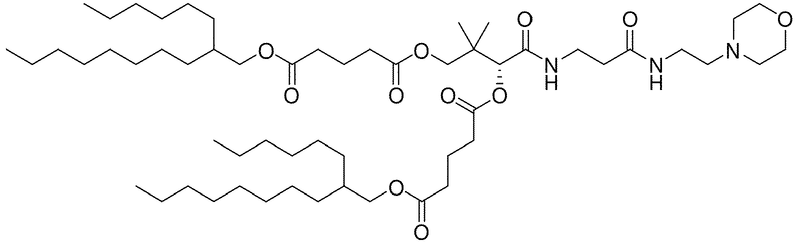

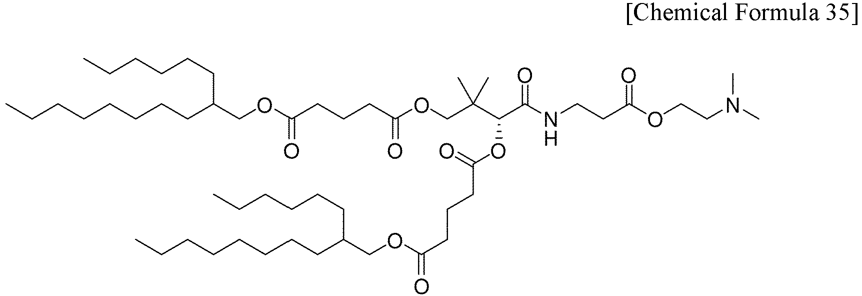

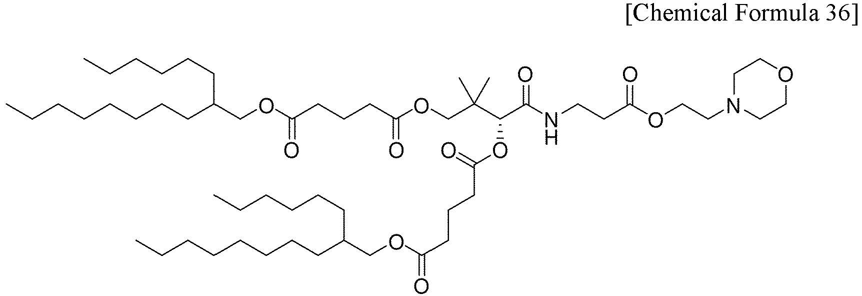

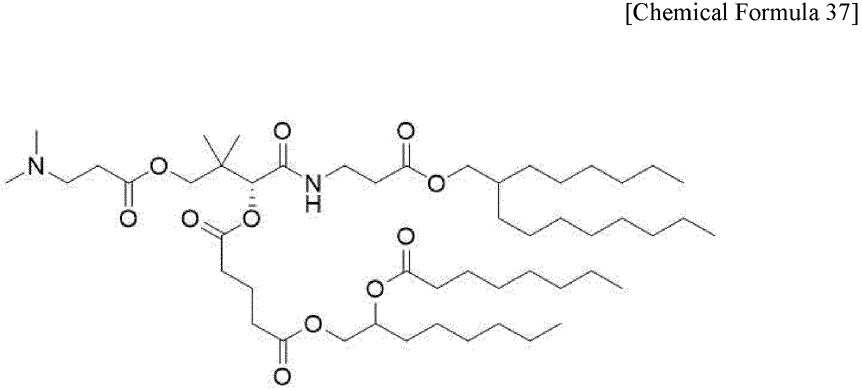

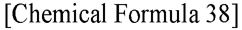

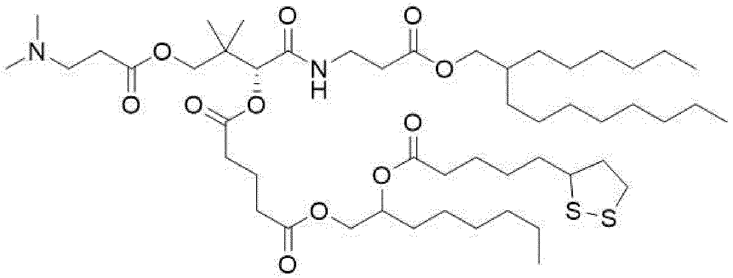

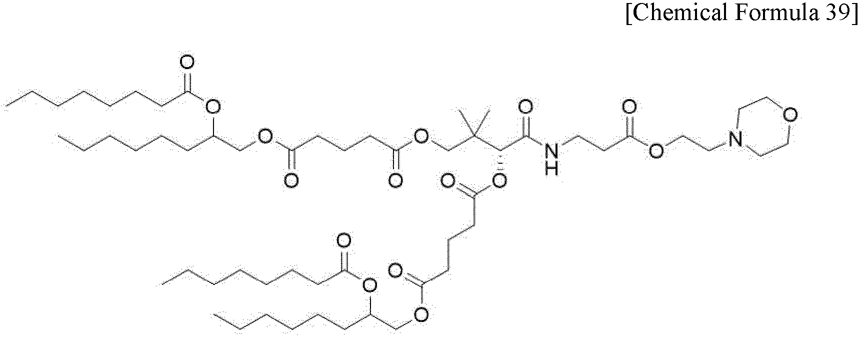

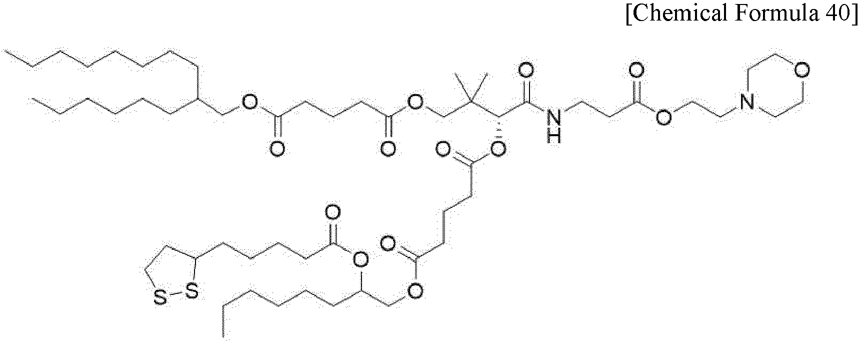

10. A lipid nanoparticle composition, comprising the compound of claim 1.

11. The lipid nanoparticle composition of claim 10, wherein the compound is ionized lipid or helper lipid.

12. A lipid nanoparticle composition, comprising a compound selected from a compound represented by the following Chemical Formula 1, a stereoisomer thereof, a racemate thereof, or a pharmaceutically acceptable salt thereof: 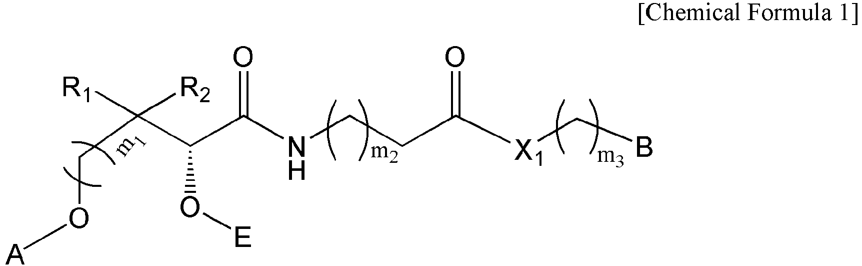 wherein, in the Chemical Formula 1,
R₁ and R₂ are the same or different and each is hydrogen or a saturated hydrocarbon with 1 to 2 carbon atoms,
X₁ is O, NH, or S,
m₁, m₂, and m₃ are the same or different and each is an integer of 1 to 3, and
A is hydrogen or a compound represented by the following Chemical Formula 1-1, 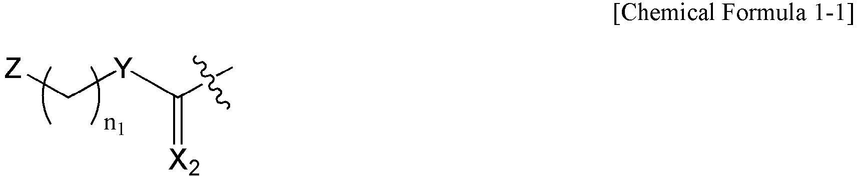
wherein, in the Chemical Formula 1-1,
Z is NR¹R²; a heterocyclic or aromatic compound with 3 to 8 carbon atoms; or linear or branched, saturated or unsaturated hydrocarbon with 6 to 22 carbon atoms,
the R¹ and R² are the same or different, and each is linear or branched, saturated or unsaturated hydrocarbon with 1 to 6 carbon atoms; the heterocyclic or aromatic ring is substituted or unsubstituted with (C1~C4)alkyl or di(C1~C4)alkylamino(C1~2)alkyl, and the hydrocarbon includes or does not include ester, ether, amide, carbamate, carbonate, or disulfide bonds,
X₂ is O or S,
Y is CH₂, NH, or O,
n₁ is an integer of 0 to 3, and
B is a compound represented by the following Chemical Formula 1-2 or NR^{1'}R^{2'}, wherein the R^{1'} and R^{2'} are the same or different, and each is linear or branched, saturated or unsaturated hydrocarbon with 1 to 6 carbon atoms, wherein the hydrocarbon includes or does not include ester, ether, amide, carbamate, carbonate, or disulfide bonds, and R^{1'} and R^{2'} are connected to each other to form a heterocyclic or aromatic ring with 3 to 8 carbon atoms, 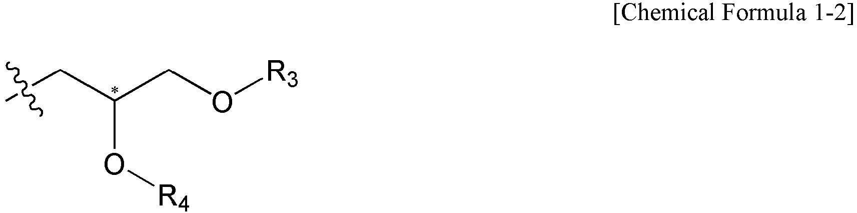
wherein, in the Chemical Formula 1-2,
R₃ and R₄ are the same or different, and each is linear or branched, saturated or unsaturated hydrocarbon with 6 to 22 carbon atoms, wherein the hydrocarbon includes or does not include ester, ether, amide, carbamate, carbonate, or disulfide bonds, and
E is hydrogen or a compound represented by the following Chemical Formula 1-3, and 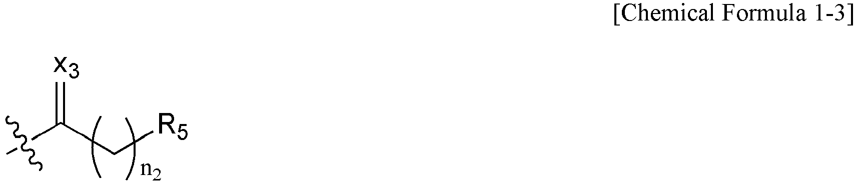
wherein, in the Chemical Formula 1-3,
X₃ is O or S,
n₂ is an integer of 0 to 3, and
R₅ is linear or branched, saturated or unsaturated hydrocarbon with 6 to 22 carbon atoms, wherein the hydrocarbon includes or does not include ester, ether, amide, carbamate, carbonate, or disulfide bonds and is substituted or unsubstituted with thiolane or dithiolane.

13. The lipid nanoparticle composition of claim 12, wherein the composition comprises one or more selected from the group consisting of helper lipid, structure-retaining lipid, PEG-lipid, and additives.

14. The lipid nanoparticle composition of claim 13, wherein the helper lipid is one or more selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinoleoyl-sn-glycero-3-phosphocholine, 1,2-diaracydonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diaracydonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidylethanolamine (DSPE), dipalmitoyl-phosphatidylethanolamine (DPPE), 1,2-dimyristoyl-sn-glycero-3-phosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, and lysophosphatidylethanolamine (LPE).

15. The lipid nanoparticle composition of claim 13, wherein the structure-retaining lipid is one or more selected from the group consisting of cholesterol, bile acid derivatives including butyl lithocholate, cholanic acid derivatives, lithocholic acid derivatives, flavonoid, vitamin A and derivatives thereof, vitamin E, vitamin K, coenzyme Q10, and beta-carotene.

16. The lipid nanoparticle composition of claim 13, wherein the PEG-lipid is one or more selected from the group consisting of PEG-modified phosphatidylethanolamine, PEG-modified phosphatidic acid, PEG-modified ceramide, PEG-modified dialkylamine, PEG-modified diacylglycerol, PEG-modified dialkyl glycerol, and a mixture thereof.

17. The lipid nanoparticle composition of claim 13, wherein the additive is a trehalose derivative represented by the following Chemical Formula 43: 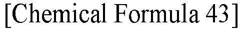 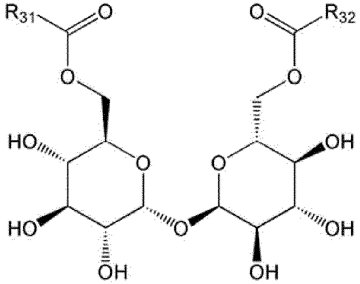 wherein, in the Chemical Formula 43,
R₃₁ and R₃₂ are the same or different, and each is linear or branched, saturated or unsaturated hydrocarbon with 6 to 22 carbon atoms.

18. The lipid nanoparticle composition of claim 13, wherein the composition comprises ionized lipid, helper lipid, structure-retaining lipid, PEG-lipid, and additives, and
the composition comprises 20 to 60 mol% of ionized lipid, 5 to 40 mol% of helper lipid, 25 to 45 mol% of structure-retaining lipid, 1 to 3 mol% of PEG-lipid, and 0 to 25 mol% of additives.

19. The lipid nanoparticle composition of claim 13, wherein the composition comprises 20 to 50 mol% of ionized lipid, 5 to 15 mol% of helper lipid, 25 to 45 mol% of structure-retaining lipid, 1 to 3 mol% of PEG-lipid, and 0 to 25 mol% of additives.

20. The lipid nanoparticle composition of claim 19, wherein the composition comprises any one compound of the Chemical Formula 12 to Chemical Formula 40 as the ionized lipid, DSPC, DOPE, and a compound of the Chemical Formula 10 or Chemical Formula 11 as the helper lipid, cholesterol, lithocholic acid derivative or cholanic acid derivative as the structure-retaining lipid, myristoyl diglyceride (DMG)-PEG as the PEG-lipid, and 6,6'-trehalose dioleate as the additive:

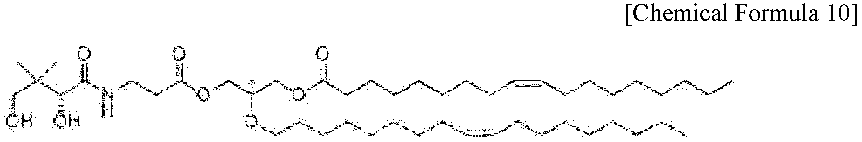

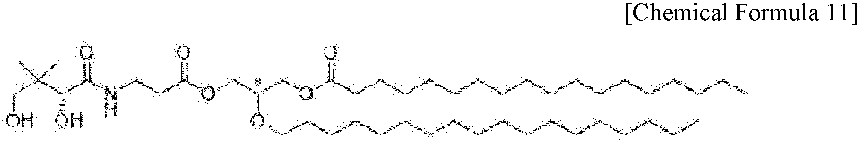

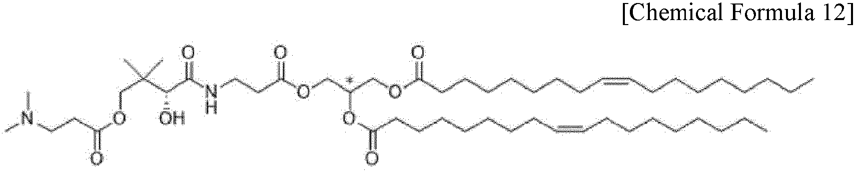

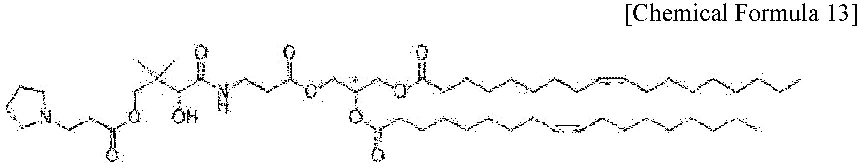

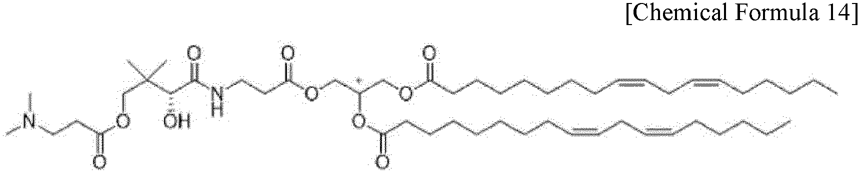

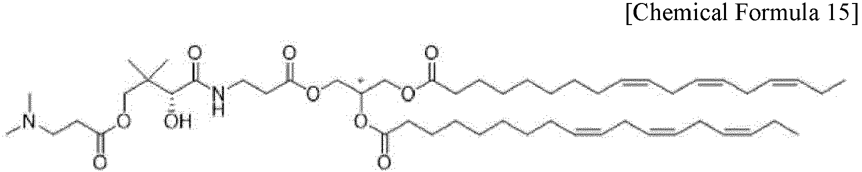

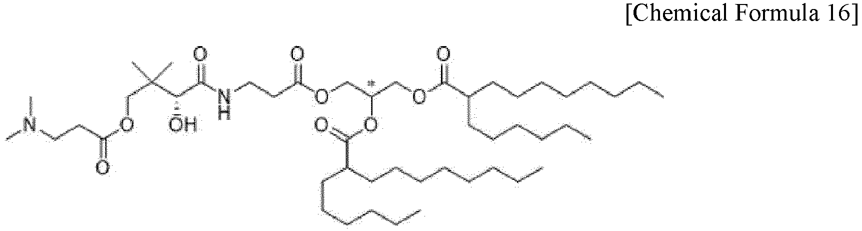

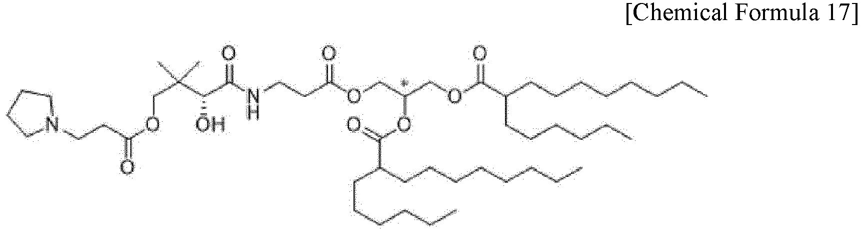

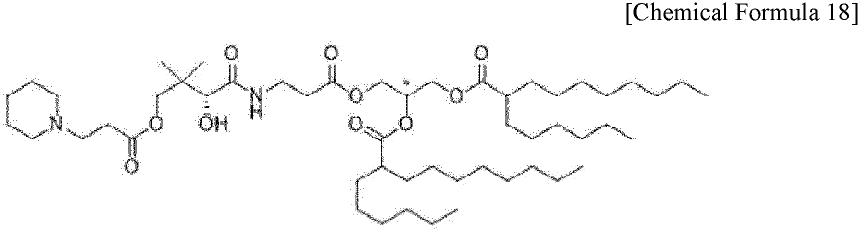

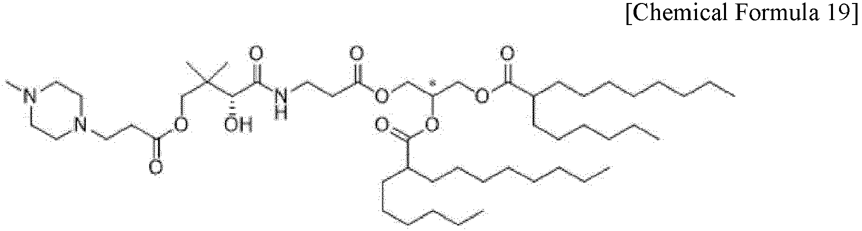

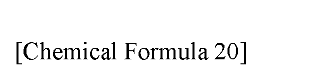

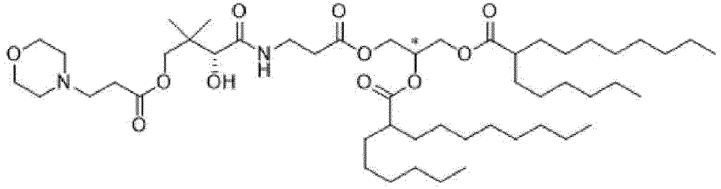

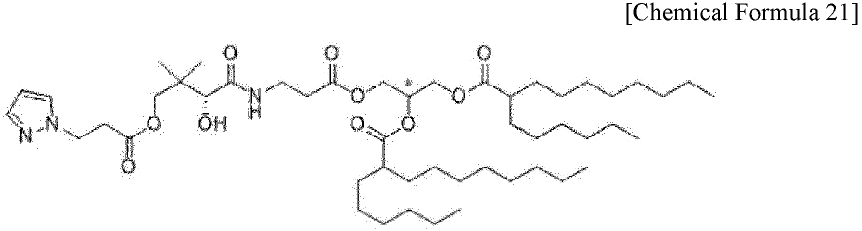

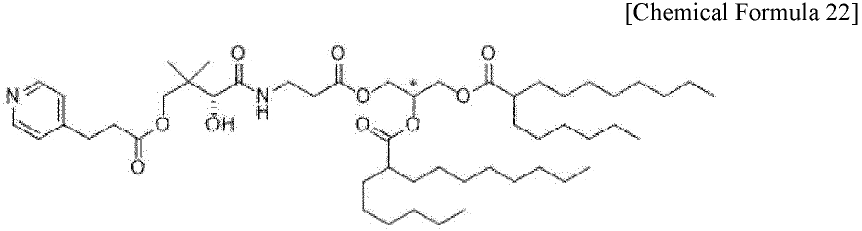

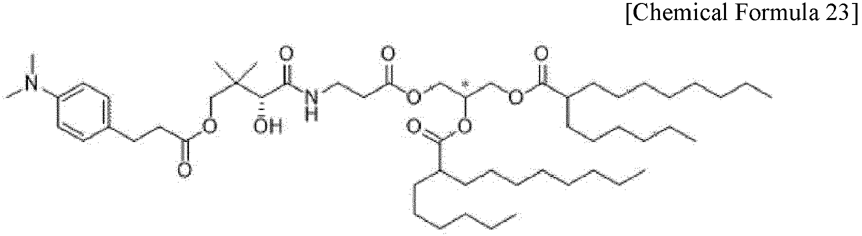

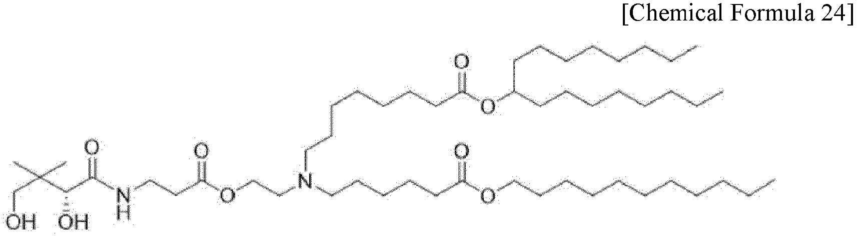

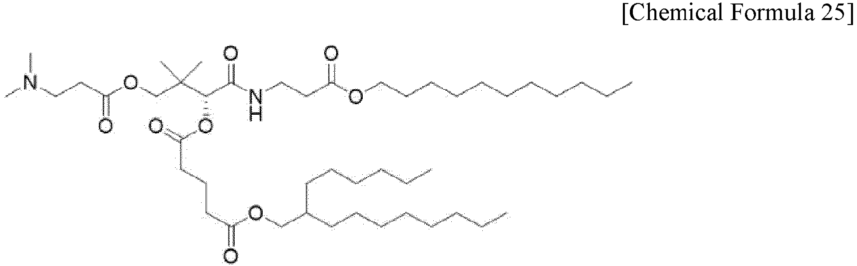

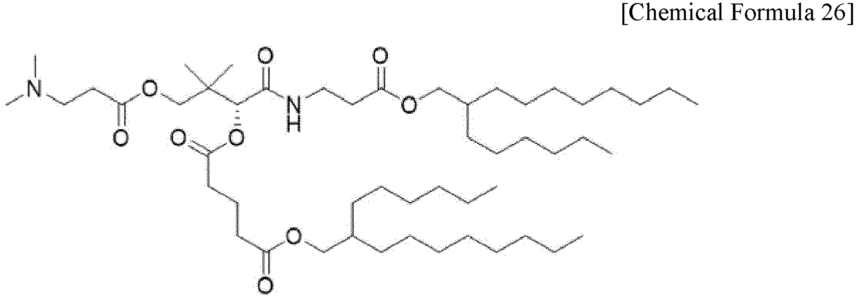

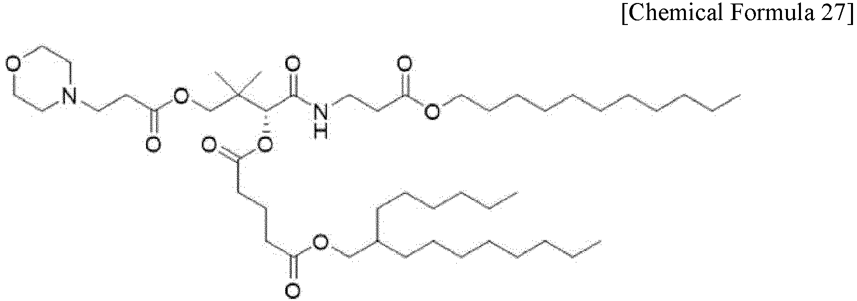

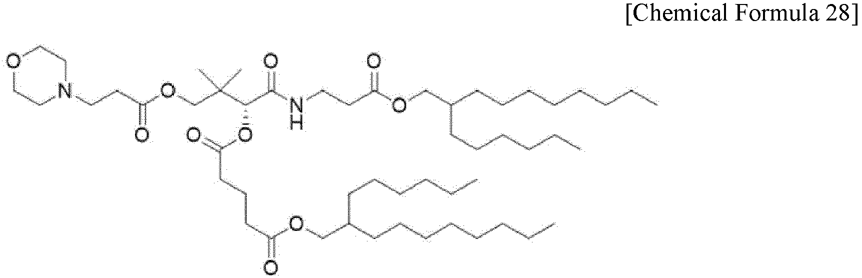

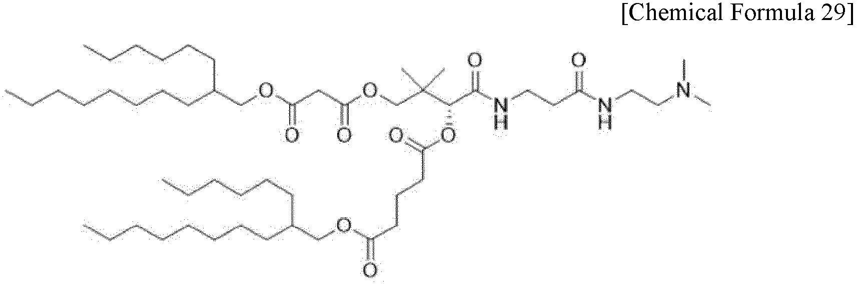

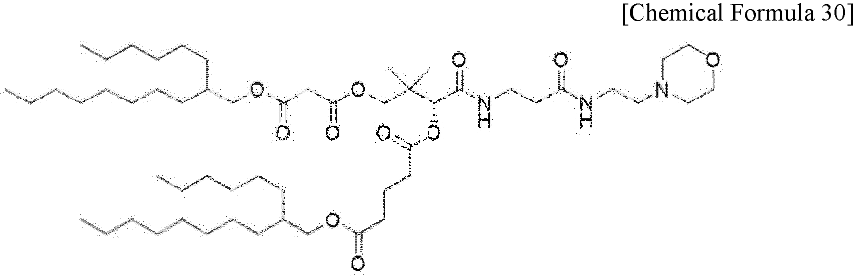

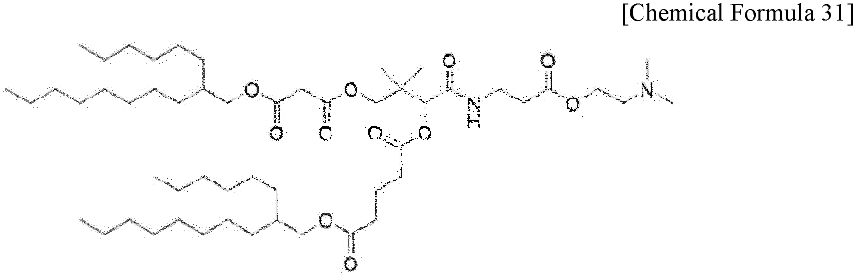

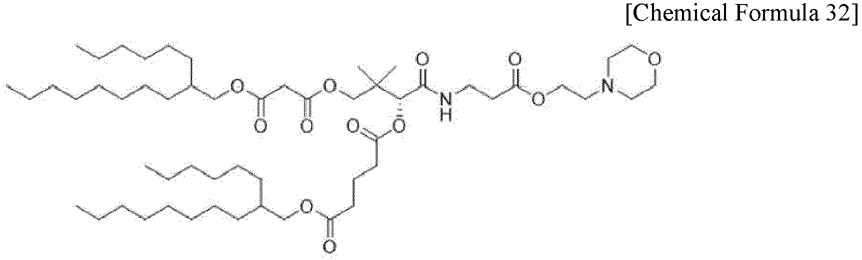

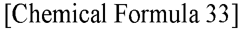

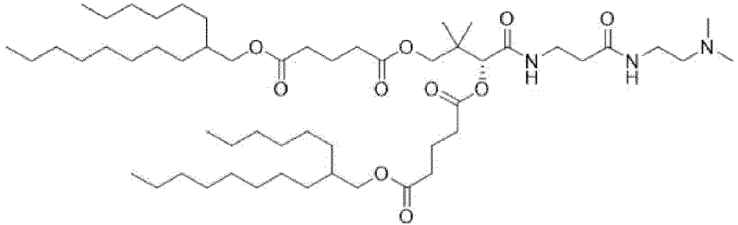

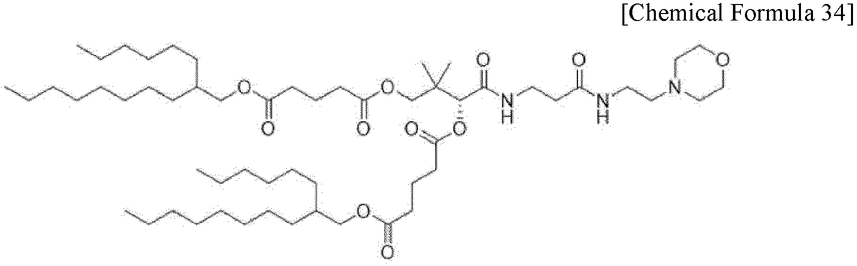

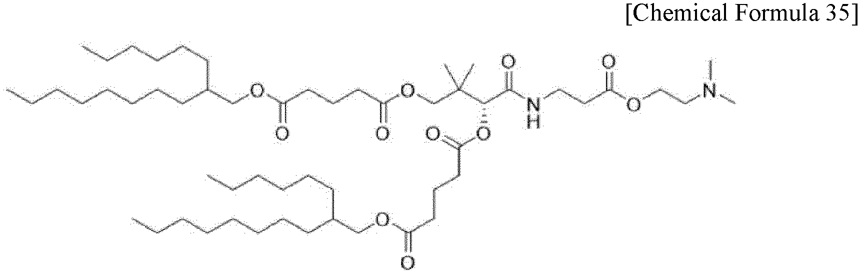

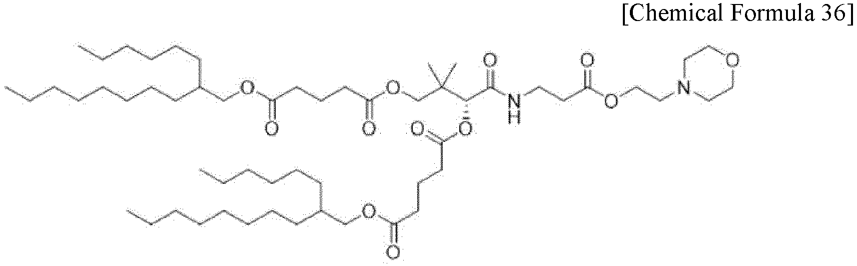

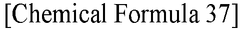

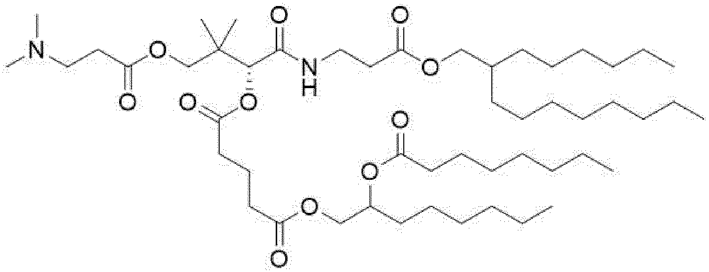

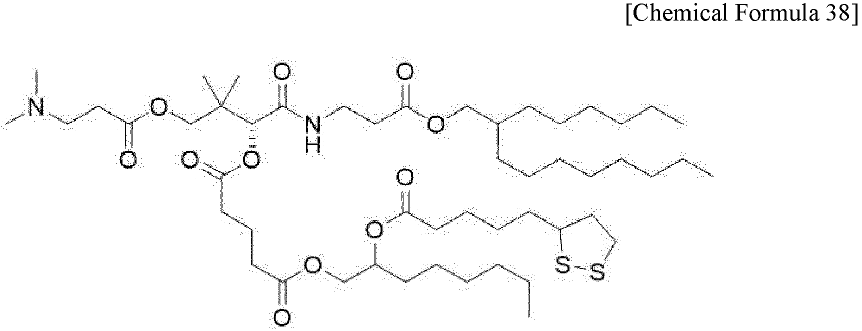

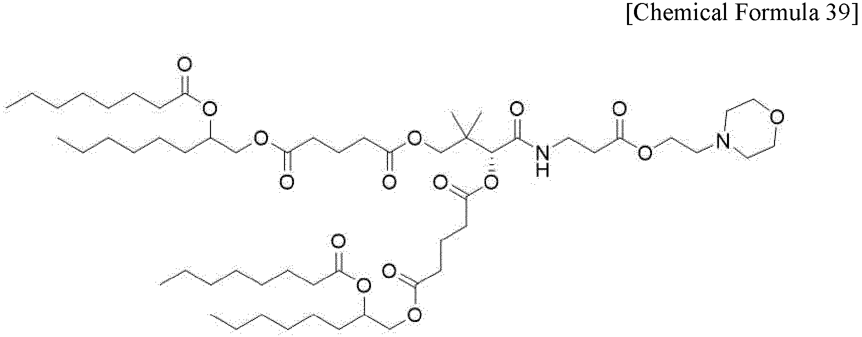

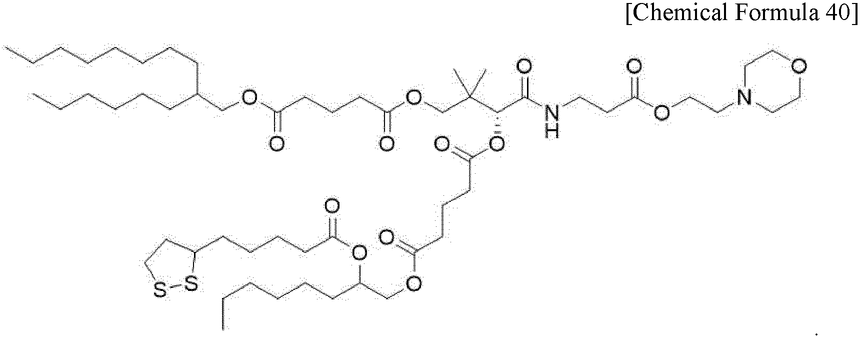

21. The lipid nanoparticle composition of claim 10 or claim 12, further comprising a therapeutic or prophylactic agent.

22. The lipid nanoparticle composition of claim 21, wherein the therapeutic or prophylactic agent is a vaccine or compound capable of inducing an immune response.

23. The lipid nanoparticle composition of claim 21, wherein the therapeutic or prophylactic agent is selected from the group consisting of small interfering RNA (siRNA), asymmetric interfering RNA (aiRNA), microRNA (miRNA), dicer-substrate RNA (dsRNA), small hairpin RNA (shRNA), messenger RNA (mRNA), and a mixture thereof.

24. A composition for drug delivery, comprising:
the lipid nanoparticle composition according to claim 10 or claim 12; and
a therapeutic or prophylactic agent.

25. An immune boosting composition, comprising the lipid nanoparticle composition according to claim 10 or claim 12.
